(19)

**Europäisches Patentamt**
**European Patent Office**
**Office européen des brevets**

(11) **EP 4 130 259 A1**

(12) **EUROPEAN PATENT APPLICATION**
published in accordance with Art. 153(4) EPC

(43) Date of publication:
**08.02.2023 Bulletin 2023/06**

(21) Application number: **21780961.5**

(22) Date of filing: **31.03.2021**

(51) International Patent Classification (IPC):
*C12N 15/09* (1990.01)      *C12N 15/113* (2010.01)
*C12Q 1/6837* (2018.01)      *C12Q 1/6883* (2018.01)

(52) Cooperative Patent Classification (CPC):
**C12N 15/09; C12N 15/113; C12Q 1/6837;**
**C12Q 1/6883**

(86) International application number:
**PCT/JP2021/013807**

(87) International publication number:
**WO 2021/201092 (07.10.2021 Gazette 2021/40)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **31.03.2020 JP 2020064383**

(71) Applicants:
• **Toray Industries, Inc.**
  **Tokyo 103-8666 (JP)**
• **National Center for Geriatrics and Gerontology**
  **Obu-shi, Aichi 474-8511 (JP)**

(72) Inventors:
• **KASHIMURA Chiori**
  **Kamakura-shi, Kanagawa 248-8555 (JP)**
• **SUDO Hiroko**
  **Kamakura-shi, Kanagawa 248-8555 (JP)**

• **YOSHIMOTO Makiko**
  **Kamakura-shi, Kanagawa 248-8555 (JP)**
• **NIIDA Shumpei**
  **Obu-shi, Aichi 474-8511 (JP)**
• **ITO Kengo**
  **Obu-shi, Aichi 474-8511 (JP)**
• **SHIGEMIZU Daichi**
  **Obu-shi, Aichi 474-8511 (JP)**
• **KATO Takashi**
  **Obu-shi, Aichi 474-8511 (JP)**
• **NAKAMURA Akinori**
  **Obu-shi, Aichi 474-8511 (JP)**

(74) Representative: **Mewburn Ellis LLP**
  **Aurora Building**
  **Counterslip**
  **Bristol BS1 6BX (GB)**

Remarks:
The complete document including Reference
Table(s) and the Sequence Listing(s) can be
downloaded from the EPO website

(54) **KIT OR DEVICE AND METHOD FOR DETECTING HIPPOCAMPAL ATROPHY**

(57)      This invention relates to a kit or device for detection of hippocampal atrophy comprising a nucleic acid(s) capable of specifically binding to particular hippocampal atrophy marker miRNA(s) or to complementary strand(s) thereof, and a method for detecting hippocampal atrophy comprising measuring such miRNA(s) *in vitro*.

EP 4 130 259 A1

**Description**

TECHNICAL FIELD

The present invention relates to a kit or device for detecting hippocampal atrophy, comprising a nucleic acid capable of specifically binding to a specific miRNA or a complementary strand thereof, which can be used for examining hippocampal atrophy in the brain of a subject, and a method for detecting hippocampal atrophy, comprising measuring the expression level of the miRNA.

BACKGROUND ART

The hippocampus is a part of a region referred to as "hippocampal formation" in the brain limbic system, which is a brain organ that lies deep within the temporal lobe of the brain cortex. Physiological functions of the hippocampus are associated with intellectual functions, memory, and spatial awareness ability. The hippocampus stores a memory until a new memory (i.e., a recent memory) is immobilized as a long-term memory. Accordingly, atrophy or damage occurred in the hippocampus would adversely affect formation of new memory and cause impaired perception and memory.

Hippocampal atrophy is caused by drop-out of nerve cells constituting the hippocampus. The whole brain, including the hippocampus, is known to become atrophic with aging. Examples of diseases involving particularly significant hippocampal atrophy due to causes other than aging include Alzheimer's disease (Alzheimer's dementia), frontotemporal lobar degeneration, depression, post-traumatic stress disorder, and schizophrenia.

The results of examination of an extent of atrophy in the hippocampal region are considered as one aspect of information for diagnosis of Alzheimer's disease (N: Neurodegeneration). An example of a common method for examining the hippocampus is diagnostic imaging by brain MRI or the like. FDG-PET, which is a method of diagnostic imaging comprising analyzing sugar metabolism in the brain, is expected as a method for determining the neuronal cell death leading to hippocampal atrophy (Non-Patent Literature 1).

Development of biomarker tests aimed at diagnosis of Alzheimer's disease characterized by hippocampal atrophy is in progress. For example, a relatively less invasive method for assaying amyloid $\beta$, which is considered to cause Alzheimer's disease, in a small amount of plasma by immunoprecipitation and mass spectrometry has been reported (Non-Patent Literature 2). It is also reported that a patient with Alzheimer's disease can be detected by another blood testing with a sensitivity of 81% and a specificity of 79%, by assaying the expression levels of 451 types of miRNAs in the serum samples (including hsa-miR-1228-5p, hsa-miR-6088, hsa-miR-4525, and hsa-miR-4741) (Patent Literature 1).

Similarly, regarding a method of biochemical diagnosis of neurodegenerative diseases, such as Alzheimer's disease, using nucleic acids, there is a report indicating that a patient with Alzheimer's disease can be detected by measuring the expression level of miRNA such as hsa-miR-486-3p in the blood components (Patent Literature 2).

Also, there is a report indicating that a patient with Alzheimer's disease can be detected by assaying, for example, hsa-miR-6805-5p in the blood sample (Patent Literature 3).

There is a report indicating that a patient with Alzheimer's disease and a patient with mild dementia can be detected by assaying, for example, hsa-miR-320b in the blood sample (Patent Literature 4).

There is also a report indicating that the expression level of an miRNA such as hsa-miR-663a in the serum of a patient with Alzheimer's disease is significantly lower than that in a healthy individual (Non-Patent Literature 3).

There is a report indicating that a patient with Alzheimer's disease can be detected by assaying, for example, hsa-miR-760 or hsa-miR-887-3p in the cerebrospinal fluid (Patent Literature 5).

There is a report indicating that a patient with dementia can be detected by assaying, for example, hsa-miR-4463 in the sample (Patent Literature 6).

There is also a report indicating that a patient with Alzheimer's disease can be detected by assaying the expression levels of miRNAs in the microvesicles of the body fluid, such as hsa-miR-128-1-5p, hsa-miR-128-2-5p, hsa-miR-187-5p, hsa-miR-711, and hsa-miR-328-5p, for the purpose of diagnosis of neurodegenerative disease such as Alzheimer's disease (Patent Literature 7).

There is also a report indicating that the expression levels of miRNA in the brain tissue, such as hsa-miR-4674 or hsa-miR-6722-3p, would be significantly altered in a patient with Alzheimer's disease (Non-Patent Literature 4).

Concerning the influence of nucleic acids on nerve cells, there is a report indicating that hsa-miR-204-3p is associated with expression of a protein that is critical for formation of nerve cells (Non-Patent Literature 5).

There is a report concerning the tau protein that is shown to be associated with Alzheimer's disease as with amyloid $\beta$, indicating that the total amount of tau proteins in the cerebrospinal fluid is highly correlated with the volume of the hippocampus/amygdala regions determined by quantification of MRI images with the use of software (FreeSurfer) (p < 0.001) (Non-Patent Literature 6).

Dementia, in general, is a progressive disease and is accompanied by irreversible degeneration of brain neurons. In diagnosis of dementia, accordingly, it is critical to measure an extent of hippocampal atrophy. In order to implement medical interventions in accordance with the symptoms of patients for treatment of dementia, it is critical to stratify patients in accordance with types of dementia based on pathological findings. The diagnostic markers that enable objective and quantitative measurements of an extent of hippocampal atrophy are needed. However, the methods described in Patent Literatures 1 to 7 and Non-Patent Literatures 1 to 6 do not have sufficient performances as the methods for diagnosis of an extent of hippocampal atrophy for the reasons described below.

In a MRI test, which is a common brain imaging test, a margin of error occurs in quantified values depending on imaging apparatuses used, how to perform imaging, or physicians who make a diagnosis; thus, there is no generalized threshold value for hippocampal atrophy concerning the quantified hippocampal volume. While a MRI test has been extensively used, some institutions are not equipped with facilities for brain MRI. In addition, it is difficult for persons who have magnetic metals in their bodies or persons with claustrophobia to undergo MRI. Accordingly, MRI is not versatile for any person and thus is disadvantageous in terms of convenience. While FDG-PET is shown as the brain imaging test in Non-Patent Literature 1, institutions where FDG-PET can be performed are limited. While safety is secured, in addition, FDG-PET has the risk of the use of radioactive substances. As described above, brain imaging tests for detecting hippocampal atrophy or synaptic degeneration are problematic mainly in terms of convenience for patients, safety, and quantitative performance.

The test method described in Non-Patent Literature 2 is limited to assay involving the use of mass spectrometry analysis equipment and it thus requires sophisticated measurement technique. In addition, by what mechanism APP669-771 (amyloid β protein) to be assayed would vary remains unknown, and it does not lead the direct correlation thereof with hippocampal atrophy.

In Patent Literature 1, patients are classified positive for Alzheimer's disease by physicians based on clinical findings. Accordingly, this test is not able to perform stratified tests, nor to find the direct correlation thereof with hippocampal atrophy. The same applies to Patent Literatures 2 to 4, Patent Literatures 6 and 7, and Non-Patent Literature 3, and markers capable of directly detecting hippocampal atrophy are not disclosed therein.

The method described in Non-Patent Literature 4 requires the use of brain tissue samples and such method cannot be used as a convenient test.

Non-Patent Literature 5 does not directly demonstrate the correlation between the indicated nucleic acids and hippocampal atrophy. Thus, it cannot be said that miRNA disclosed therein is capable of being used as a hippocampal atrophy marker.

Concerning the methods described in Patent Literature 5 and Non-Patent Literature 6, sampling of cerebrospinal fluid is highly invasive and imposes a significant burden on a patient.

A brain imaging test directly shows hippocampal atrophy. At present, however, use thereof is limited due to the facilities or the burden on patients, and thus the convenience and versatility thereof are low. While it is preferable to perform blood testing as a low-invasive and convenient biochemical diagnosis in screening prior to such imaging diagnosis, none of currently available marker testing techniques are capable of detection of hippocampal atrophy in a low-invasive manner. Accordingly, development of diagnostic markers that enable accurate differentiation between atrophy and non-atrophy of the hippocampus with the use of blood samples is demanded.

CITATION LIST

Patent Literature

Patent Literature 1: International Publication WO 2019/159884
Patent Literature 2: JP Patent Publication No. 2017-184642 A
Patent Literature 3: International Publication WO 2019/014457
Patent Literature 4: U.S. Patent Application Publication No. 2019/136320
Patent Literature 5: U.S. Patent Application Publication No. 2014/272993
Patent Literature 6: International Publication WO 2019/048500
Patent Literature 7: U.S. Patent Application Publication No. 2019/249250

Non-Patent Literature

Non-Patent Literature 1: Daniel H.S. et al., The Journal of Nuclear Medicine, Apr. 2004, vol. 45, No. 4

Non-Patent Literature 2: Nakamura A. et al., Nature, Jan. 31, 2018, vol. 554, pp. 249-254
Non-Patent Literature 3: Grasso M. et al., Neurobiol. Aging., Dec. 2019; 84: 240.e1-240.e12.
Non-Patent Literature 4: Kumar S. et al., Hum. Mol. Genet., Oct. 2017, 1; 26 (19): 3808-3822
Non-Patent Literature 5: Bhagat R. et al., Cell Death Differ., Nov. 2018, (10): 1837-1854
Non-Patent Literature 6. Emrah Duzel et al., Alzhemer's & Dementia Monitoring, 2018, 10, 782-790

## SUMMARY OF INVENTION

### Problem to be Solved by Invention

The object of the present invention is to provide a hippocampal atrophy marker that enables detection of hippocampal atrophy without MRI and a means for and a method enabling objective and effective detection of hippocampal atrophy with the use of such marker.

### Means for Solution to Problem

The present inventors have conducted diligent studies to attain the object and found hippocampal atrophy markers that enable detection of an extent of atrophy of hippocampal region, which is also closely associated with dementia. This has led to the completion of the present invention.

Specifically, the present invention encompasses the following aspects.

(1) A kit for detection of hippocampal atrophy, comprising a nucleic acid(s) capable of specifically binding to one or more polynucleotide(s) selected from the group consisting of hippocampal atrophy markers: miR-3131, miR-6757-5p, miR-4706, miR-5001-5p, miR-3180-3p, miR-642b-3p, miR-4655-5p, miR-6819-5p, miR-937-5p, miR-4688, miR-6741-5p, miR-7107-5p, miR-4271, miR-1229-5p, miR-4707-5p, miR-6808-5p, miR-4656, miR-6076, miR-6762-5p, miR-7109-5p, miR-6732-5p, miR-3195, miR-7150, miR-642a-3p, miR-1249-5p, miR-3185, miR-4689, miR-3141, miR-6840-3p, miR-3135b, miR-1914-3p, miR-4446-3p, miR-4433b-3p, miR-6877-5p, miR-6848-5p, miR-3620-5p, miR-6825-5p, miR-5739, miR-3663-3p, miR-4695-5p, miR-3162-5p, miR-3679-5p, miR-8059, miR-7110-5p, miR-1275, miR-6779-5p, miR-197-5p, miR-6845-5p, miR-4327, miR-4723-5p, miR-4530, miR-6771-5p, miR-614, miR-92a-2-5p, miR-6891-5p, miR-6124, miR-4687-3p, miR-4442, miR-7977, miR-6785-5p, miR-4497, miR-8071, miR-663b, miR-3180, miR-4251, miR-1285-3p, miR-6870-5p, miR-4484, miR-4476, miR-6749-5p, miR-4454, miR-6893-5p, miR-6085, miR-4787-5p, miR-149-3p, miR-7704, miR-6125, miR-6090, miR-3197, miR-6850-5p, miR-4467, miR-6885-5p, miR-6803-5p, miR-6798-5p, miR-6780b-5p, miR-6768-5p, miR-5100, miR-6724-5p, miR-6879-5p, miR-7108-5p, miR-4649-5p, miR-4739, miR-6089, miR-1908-5p, miR-4516, miR-2861, miR-4492, miR-4294, miR-6791-5p, miR-1469, miR-6752-5p, miR-4730, miR-6126, miR-6869-5p, miR-1268a, miR-6799-5p, miR-8069, miR-3621, and miR-4763-3p, or to a complementary strand(s) of the polynucleotide(s).

(2) The kit according to (1), wherein the nucleic acid(s) are polynucleotide(s) selected from the group consisting of the following polynucleotides (a) to (e):

(a) a polynucleotide consisting of a nucleotide sequence shown by any of SEQ ID NOs: 1 to 109 or a nucleotide sequence derived from the nucleotide sequence by the replacement of u with t, a variant thereof, a derivative thereof, or a fragment thereof comprising 15 or more consecutive nucleotides;

(b) a polynucleotide comprising a nucleotide sequence shown by any of SEQ ID NOs: 1 to 109, a variant thereof, a derivative thereof, or a fragment thereof comprising 15 or more consecutive nucleotides;

(c) a polynucleotide consisting of a nucleotide sequence complementary to a nucleotide sequence shown by any of SEQ ID NOs: 1 to 109 or a nucleotide sequence derived from the nucleotide sequence by the replacement of u with t, a variant thereof, a derivative thereof, or a fragment thereof comprising 15 or more consecutive nucleotides;

(d) a polynucleotide comprising a nucleotide sequence complementary to a nucleotide sequence shown by any of SEQ ID NOs: 1 to 109 or a nucleotide sequence derived from the nucleotide sequence by the replacement of u with t, a variant thereof, a derivative thereof, or a fragment thereof comprising 15 or more consecutive nucleotides; and

(e) a polynucleotide hybridizing under stringent conditions to any of the polynucleotides (a) to (d).

(3) The kit according to (1) or (2), wherein the kit further comprises a nucleic acid(s) capable of specifically binding to one or more polynucleotide(s) selected from the group consisting of other hippocampal atrophy markers: miR-1228-5p, miR-760, miR-187-5p, miR-7111-Sp, miR-6088, miR-6805-3p, miR-4640-5p, miR-6721-5p, miR-6880-5p, miR-711, miR-128-1-5p, miR-4525, miR-486-3p, miR-6756-5p, miR-1260b, miR-3184-5p, miR-6075, miR-204-3p, miR-4728-5p, miR-4534, miR-4758-5p, miR-8063, miR-6836-3p, miR-6789-5p, miR-744-5p, miR-1909-3p, miR-887-3p, miR-4745-5p, miR-4433a-3p, miR-5090, miR-296-5p, miR-939-5p, miR-3648, miR-3196, miR-6722-3p, miR-6805-5p, miR-1202, miR-6775-5p, miR-6087, miR-6765-5p, miR-6875-5p, miR-4674, miR-1233-5p, miR-7114-5p, miR-5787, miR-8072, miR-3619-3p, miR-4632-5p, miR-6800-5p, miR-4634, miR-4486, miR-6727-5p, miR-4505, miR-4725-3p, miR-1538, miR-320b, miR-1915-5p, miR-328-5p, miR-6820-5p, miR-6726-5p, miR-3665, miR-638, miR-762, miR-4466, miR-3940-5p, miR-1237-5p, miR-575, miR-3656, miR-4488, miR-4281, miR-6781-5p, miR-4532, miR-4665-5p, miR-6816-5p, miR-4508, miR-6784-5p, miR-6786-5p, miR-4741, miR-1343-5p, miR-1227-5p, miR-4734, miR-3960, miR-128-2-5p, miR-6743-5p, miR-663a, miR-6729-5p, miR-1915-3p, miR-1268b, miR-4651, miR-3178, and miR-4463, or to a complementary strand(s) of the polynucleotide(s).

(4) The kit according to (3), wherein the nucleic acid(s) are polynucleotide(s) selected from the group consisting of the following polynucleotides (f) to (j):

(f) a polynucleotide consisting of a nucleotide sequence shown by any of SEQ ID NOs: 110 to 200 or a nucleotide sequence derived from the nucleotide sequence by the replacement of u with t, a variant thereof, a derivative thereof, or a fragment thereof comprising 15 or more consecutive nucleotides;

(g) a polynucleotide comprising a nucleotide sequence shown by any of SEQ ID NOs: 110 to 200, a variant thereof, a derivative thereof, or a fragment thereof comprising 15 or more consecutive nucleotides;

(h) a polynucleotide consisting of a nucleotide sequence complementary to a nucleotide sequence shown by any of SEQ ID NOs: 110 to 200 or a nucleotide sequence derived from the nucleotide sequence by the replacement of u with t, a variant thereof, a derivative thereof, or a fragment thereof comprising 15 or more consecutive nucleotides;

(i) a polynucleotide comprising a nucleotide sequence complementary to a nucleotide sequence shown by any of SEQ ID NOs: 110 to 200 or a nucleotide sequence derived from the nucleotide sequence by the replacement of u with t, a variant thereof, a derivative thereof, or a fragment thereof comprising 15 or more consecutive nucleotides; and

(j) a polynucleotide hybridizing under stringent conditions to any of the polynucleotides (f) to (i).

(5) A device for detection of hippocampal atrophy comprising a nucleic acid(s) capable of specifically binding to one or more polynucleotide(s) selected from the group consisting of hippocampal atrophy markers: miR-3131, miR-6757-5p, miR-4706, miR-5001-5p, miR-3180-3p, miR-642b-3p, miR-4655-5p, miR-6819-5p, miR-937-5p, miR-4688, miR-6741-5p, miR-7107-5p, miR-4271, miR-1229-5p, miR-4707-5p, miR-6808-5p, miR-4656, miR-6076, miR-6762-5p, miR-7109-5p, miR-6732-5p, miR-3195, miR-7150, miR-642a-3p, miR-1249-5p, miR-3185, miR-4689, miR-3141, miR-6840-3p, miR-3135b, miR-1914-3p, miR-4446-3p, miR-4433b-3p, miR-6877-5p, miR-6848-5p, miR-3620-5p, miR-6825-5p, miR-5739, miR-3663-3p, miR-4695-5p, miR-3162-5p, miR-3679-5p, miR-8059, miR-7110-5p, miR-1275, miR-6779-5p, miR-197-5p, miR-6845-5p, miR-4327, miR-4723-5p, miR-4530, miR-6771-5p, miR-614, miR-92a-2-5p, miR-6891-5p, miR-6124, miR-4687-3p, miR-4442, miR-7977, miR-6785-5p, miR-4497, miR-8071, miR-663b, miR-3180, miR-4251, miR-1285-3p, miR-6870-5p, miR-4484, miR-4476, miR-6749-5p, miR-4454, miR-6893-5p, miR-6085, miR-4787-5p, miR-149-3p, miR-7704, miR-6125, miR-6090, miR-3197, miR-6850-5p, miR-4467, miR-6885-5p, miR-6803-5p, miR-6798-5p, miR-6780b-5p, miR-6768-5p, miR-5100, miR-6724-5p, miR-6879-5p, miR-7108-5p, miR-4649-5p, miR-4739, miR-6089, miR-1908-5p, miR-4516, miR-2861, miR-4492, miR-4294, miR-6791-5p, miR-1469, miR-6752-5p, miR-4730, miR-6126, miR-6869-5p, miR-1268a, miR-6799-5p, miR-8069, miR-3621, and miR-4763-3p, or to a complementary strand(s) of the polynucleotide(s).

(6) The device according to (5), wherein the nucleic acid(s) are polynucleotide(s) selected from the group consisting of the following polynucleotides (a) to (e):

(a) a polynucleotide consisting of a nucleotide sequence shown by any of SEQ ID NOs: 1 to 109 or a nucleotide sequence derived from the nucleotide sequence by the replacement of u with t, a variant thereof, a derivative thereof, or a fragment thereof comprising 15 or more consecutive nucleotides;

(b) a polynucleotide comprising a nucleotide sequence shown by any of SEQ ID NOs: 1 to 109, a variant thereof, a derivative thereof, or a fragment thereof comprising 15 or more consecutive nucleotides;

(c) a polynucleotide consisting of a nucleotide sequence complementary to a nucleotide sequence shown by any of SEQ ID NOs: 1 to 109 or a nucleotide sequence derived from the nucleotide sequence by the replacement of u with t, a variant thereof, a derivative thereof, or a fragment thereof comprising 15 or more consecutive nucleotides;

(d) a polynucleotide comprising a nucleotide sequence complementary to a nucleotide sequence shown by any of SEQ ID NOs: 1 to 109 or a nucleotide sequence derived from the nucleotide sequence by the replacement of u with t, a variant thereof, a derivative thereof, or a fragment thereof comprising 15 or more consecutive nucleotides; and

(e) a polynucleotide hybridizing under stringent conditions to any of the polynucleotides (a) to (d).

(7) The device according to (5) or (6), wherein the device further comprises a nucleic acid(s) capable of specifically binding to one or more polynucleotide(s) selected from the group consisting of other hippocampal atrophy markers: miR-1228-5p, miR-760, miR-187-5p, miR-7111-Sp, miR-6088, miR-6805-3p, miR-4640-5p, miR-6721-5p, miR-6880-5p, miR-711, miR-128-1-5p, miR-4525, miR-486-3p, miR-6756-5p, miR-1260b, miR-3184-5p, miR-6075, miR-204-3p, miR-4728-5p, miR-4534, miR-4758-5p, miR-8063, miR-6836-3p, miR-6789-5p, miR-744-5p, miR-1909-3p, miR-887-3p, miR-4745-5p, miR-4433a-3p, miR-5090, miR-296-5p, miR-939-5p, miR-3648, miR-3196, miR-6722-3p, miR-6805-5p, miR-1202, miR-6775-5p, miR-6087, miR-6765-5p, miR-6875-5p, miR-4674, miR-1233-5p, miR-7114-5p, miR-5787, miR-8072, miR-3619-3p, miR-4632-5p, miR-6800-5p, miR-4634, miR-4486, miR-6727-5p, miR-4505, miR-4725-3p, miR-1538, miR-320b, miR-1915-5p, miR-328-5p, miR-6820-5p, miR-6726-5p, miR-3665, miR-638, miR-762, miR-4466, miR-3940-5p, miR-1237-5p, miR-575, miR-3656, miR-4488, miR-4281, miR-6781-5p, miR-4532, miR-4665-5p, miR-6816-5p, miR-4508, miR-6784-5p, miR-6786-5p, miR-4741, miR-1343-5p, miR-1227-5p, miR-4734, miR-3960, miR-128-2-5p, miR-6743-5p, miR-663a, miR-6729-5p, miR-1915-3p, miR-1268b, miR-4651, miR-3178, and miR-4463, or to a complementary strand(s) of the polynucleotide(s).

(8) The device according to (7), wherein the nucleic acid(s) are polynucleotide(s) selected from the group consisting of the following polynucleotides (f) to (j):

(f) a polynucleotide consisting of a nucleotide sequence shown by any of SEQ ID NOs: 110 to 200 or a nucleotide sequence derived from the nucleotide sequence by the replacement of u with t, a variant thereof, a derivative thereof, or a fragment thereof comprising 15 or more consecutive nucleotides;

(g) a polynucleotide comprising a nucleotide sequence shown by any of SEQ ID NOs: 110 to 200, a variant thereof, a derivative thereof, or a fragment thereof comprising 15 or more consecutive nucleotides;

(h) a polynucleotide consisting of a nucleotide sequence complementary to a nucleotide sequence shown by any of SEQ ID NOs: 110 to 200 or a nucleotide sequence derived from the nucleotide sequence by the replacement of u with t, a variant thereof, a derivative thereof, or a fragment thereof comprising 15 or more consecutive nucleotides;

(i) a polynucleotide comprising a nucleotide sequence complementary to a nucleotide sequence shown by any of SEQ ID NOs: 110 to 200 or a nucleotide sequence derived from the nucleotide sequence by the replacement of u with t, a variant thereof, a derivative thereof, or a fragment thereof comprising 15 or more consecutive nucleotides; and

(j) a polynucleotide hybridizing under stringent conditions to any of the polynucleotides (f) to (i).

(9) The device according to any one of (5) to (8), wherein the device is a device for measurement by a hybridization technique.

(10) The device according to (9), wherein the hybridization technique is a nucleic acid array technique.

(11) A method for detecting hippocampal atrophy, comprising: measuring an expression level(s) of one or more polynucleotide(s) selected from the group consisting of hippocampal atrophy markers: miR-3131, miR-6757-5p, miR-4706, miR-5001-5p, miR-3180-3p, miR-642b-3p, miR-4655-5p, miR-6819-5p, miR-937-5p, miR-4688, miR-6741-5p, miR-7107-5p, miR-4271, miR-1229-5p, miR-4707-5p, miR-6808-5p, miR-4656, miR-6076, miR-6762-5p, miR-7109-5p, miR-6732-5p, miR-3195, miR-7150, miR-642a-3p, miR-1249-5p, miR-3185, miR-4689, miR-3141, miR-6840-3p, miR-3135b, miR-1914-3p, miR-4446-3p, miR-4433b-3p, miR-6877-5p, miR-6848-5p, miR-3620-5p, miR-6825-5p, miR-5739, miR-3663-3p, miR-4695-5p, miR-3162-5p, miR-3679-5p, miR-8059, miR-7110-5p, miR-1275, miR-6779-5p, miR-197-5p, miR-6845-5p, miR-4327, miR-4723-5p, miR-4530, miR-6771-5p, miR-614, miR-92a-2-5p, miR-6891-5p, miR-6124, miR-4687-3p, miR-4442, miR-7977, miR-6785-5p, miR-4497, miR-8071, miR-663b, miR-3180, miR-4251, miR-1285-3p, miR-6870-5p, miR-4484, miR-4476, miR-6749-5p, miR-4454, miR-6893-5p, miR-6085, miR-4787-5p, miR-149-3p, miR-7704, miR-6125, miR-6090, miR-3197, miR-6850-5p, miR-4467, miR-6885-5p, miR-6803-5p, miR-6798-5p, miR-6780b-5p, miR-6768-5p, miR-5100, miR-6724-5p, miR-6879-5p, miR-7108-5p, miR-4649-5p, miR-4739, miR-6089, miR-1908-5p, miR-4516, miR-2861, miR-4492, miR-4294, miR-6791-5p, miR-1469, miR-6752-5p, miR-4730, miR-6126, miR-6869-5p, miR-1268a, miR-6799-5p, miR-8069, miR-3621, and miR-4763-3p in a sample from a subject; and evaluating whether or not the subject's hippocampus has become atrophic by using the measured expression level(s).

(12) The method according to (11), wherein the expression level(s) of the polynucleotide(s) are measured using the kit according to any one of (1) to (4) or the device according to any one of (5) to (10).

(13) The method according to (12), comprising: assigning the measured expression level(s) of the polynucleotide(s) to a discriminant capable of distinctively discriminating between atrophy and non-atrophy of hippocampus, wherein the discriminant is prepared using expression level(s) of the polynucleotide(s) in samples from subjects known to have hippocampal atrophy and expression level(s) of the polynucleotide(s) in samples from subjects known to have no hippocampal atrophy as training samples; and thereby evaluating whether there is atrophy or non-atrophy of hippocampus.

(14) The method according to (12) or (13), wherein the nucleic acid(s) are polynucleotide(s) selected from the group consisting of the following polynucleotides (a) to (e):

(a) a polynucleotide consisting of a nucleotide sequence shown by any of SEQ ID NOs: 1 to 109 or a nucleotide sequence derived from the nucleotide sequence by the replacement of u with t, a variant thereof, a derivative thereof, or a fragment thereof comprising 15 or more consecutive nucleotides;

(b) a polynucleotide comprising a nucleotide sequence shown by any of SEQ ID NOs: 1 to 109, a variant thereof, a derivative thereof, or a fragment thereof comprising 15 or more consecutive nucleotides;

(c) a polynucleotide consisting of a nucleotide sequence complementary to a nucleotide sequence shown by any of SEQ ID NOs: 1 to 109 or a nucleotide sequence derived from the nucleotide sequence by the replacement of u with t, a variant thereof, a derivative thereof, or a fragment thereof comprising 15 or more consecutive nucleotides;

(d) a polynucleotide comprising a nucleotide sequence complementary to a nucleotide sequence shown by any of SEQ ID NOs: 1 to 109 or a nucleotide sequence derived from the nucleotide sequence by the replacement of u with t, a variant thereof, a derivative thereof, or a fragment thereof comprising 15 or more consecutive nucleotides; and

(e) a polynucleotide hybridizing under stringent conditions to any of the polynucleotides (a) to (d).

(15) The method according to any one of (12) to (14), wherein the kit or device further comprises nucleic acid(s) capable of specifically binding to one or more polynucleotide(s) selected from the group consisting of other hippocampal atrophy markers: miR-1228-5p, miR-760, miR-187-5p, miR-7111-5p, miR-6088, miR-6805-3p, miR-4640-5p, miR-6721-5p, miR-6880-5p, miR-711, miR-128-1-5p, miR-4525, miR-486-3p, miR-6756-5p, miR-1260b, miR-3184-5p, miR-6075, miR-204-3p, miR-4728-5p, miR-4534, miR-4758-5p, miR-8063, miR-6836-3p, miR-6789-5p, miR-744-5p, miR-1909-3p, miR-887-3p, miR-4745-5p, miR-4433a-3p, miR-5090, miR-296-5p, miR-939-5p, miR-3648, miR-3196, miR-6722-3p, miR-6805-5p, miR-1202, miR-6775-5p, miR-6087, miR-6765-5p, miR-6875-5p, miR-4674, miR-1233-5p, miR-7114-5p, miR-5787, miR-8072, miR-3619-3p, miR-4632-5p, miR-6800-5p, miR-4634, miR-4486, miR-6727-5p, miR-4505, miR-4725-3p, miR-1538, miR-320b, miR-1915-5p, miR-328-5p, miR-6820-5p, miR-6726-5p, miR-3665, miR-638, miR-762, miR-4466, miR-3940-5p, miR-1237-5p, miR-575, miR-3656, miR-4488, miR-4281, miR-6781-5p, miR-4532, miR-4665-5p, miR-6816-5p, miR-4508, miR-6784-5p, miR-6786-5p, miR-4741, miR-1343-5p, miR-1227-5p, miR-4734, miR-3960, miR-128-2-5p, miR-6743-5p, miR-663a, miR-6729-5p, miR-1915-3p, miR-1268b, miR-4651, miR-3178, and miR-4463, or to a complementary strand(s) of the polynucleotide(s).

(16) The method according to (15), wherein the nucleic acid(s) are polynucleotide(s) selected from the group consisting of the following polynucleotides (f) to (j):

(f) a polynucleotide consisting of a nucleotide sequence shown by any of SEQ ID NOs: 110 to 200 or a nucleotide sequence derived from the nucleotide sequence by the replacement of u with t, a variant thereof, a derivative thereof, or a fragment thereof comprising 15 or more consecutive nucleotides;

(g) a polynucleotide comprising a nucleotide sequence shown by any of SEQ ID NOs: 110 to 200, a variant thereof, a derivative thereof, or a fragment thereof comprising 15 or more consecutive nucleotides;

(h) a polynucleotide consisting of a nucleotide sequence complementary to a nucleotide sequence shown by any of SEQ ID NOs: 110 to 200 or a nucleotide sequence derived from the nucleotide sequence by the replacement of u with t, a variant thereof, a derivative thereof, or a fragment thereof comprising 15 or more consecutive nucleotides;

(i) a polynucleotide comprising a nucleotide sequence complementary to a nucleotide sequence shown by any of SEQ ID NOs: 110 to 200 or a nucleotide sequence derived from the nucleotide sequence by the replacement of u with t, a variant thereof, a derivative thereof, or a fragment thereof comprising 15 or more consecutive nucleotides; and

(j) a polynucleotide hybridizing under stringent conditions to any of the polynucleotides (f) to (i).

(17) The method according to any one of (11) to (16), wherein the subject is a human.

(18) The method according to any one of (11) to (17), wherein the sample is blood, serum, or plasma.

<Definition of Terms>

The terms used herein are defined as described below.

Abbreviations or terms such as nucleotide, polynucleotide, DNA, and RNA abide by "Guidelines for the preparation of specification which contain nucleotide and/or amino acid sequences" (edited by Japan Patent Office) and common use in the art.

The term "polynucleotide" used herein refers to a nucleic acid including any of RNA, DNA, and RNA/DNA (chimera). The DNA includes any of cDNA, genomic DNA, and synthetic DNA. The RNA includes any of total RNA, mRNA, rRNA, miRNA, siRNA, snoRNA, snRNA, non-coding RNA and synthetic RNA. Here the "synthetic DNA" and the "synthetic RNA" refer to a DNA and an RNA artificially prepared using, for example, an automatic nucleic acid synthesizer, on the basis of predetermined nucleotide sequences (which may be any of natural and non-natural sequences). The "non-natural sequence" is intended to be used in a broad sense and includes, for example, a sequence comprising substitution, deletion, insertion, and/or addition of one or more nucleotides (i.e., a variant sequence) and a sequence comprising one or more modified nucleotides (i.e., a modified sequence), which are different from the natural sequence. Herein, the term "polynucleotide" is used interchangeably with the term "nucleic acid." The polynucleotide comprises two or more nucleotides.

The term "fragment" used herein is a polynucleotide having a nucleotide sequence that consists of a consecutive portion of a polynucleotide and desirably has a length of 15 or more nucleotides, preferably 17 or more nucleotides, more preferably 19 or more nucleotides.

The term "gene" used herein is intended to include not only RNA and double-stranded DNA but also each single-stranded DNA such as a plus(+) strand (or a sense strand) or a complementary strand (or an antisense strand) constituting the duplex. The gene is not particularly limited by its length.

Thus, the "gene" used herein includes any of double-stranded DNA including human genomic DNA, single-stranded DNA (plus strand), single-stranded DNA having a sequence complementary to the plus strand (complementary strand), cDNA, microRNA (miRNA), their fragments, human genome, and their transcripts, unless otherwise specified. The "gene" includes not only a "gene" shown by a particular nucleotide sequence (or SEQ ID NO) but also "nucleic acids" encoding RNAs having biological functions equivalent to RNA encoded by the gene, for example, a congener (i.e., a homolog or an ortholog), a variant (e.g., a genetic polymorph), and a derivative. Specific examples of such a "nucleic acid" encoding a congener, a variant, or a derivative can include a "nucleic acid" having a nucleotide sequence hybridizing under stringent conditions described later to a complementary sequence of a nucleotide sequence shown by any of SEQ ID NOs: 1 to 740 or a nucleotide sequence derived from the nucleotide sequence by the replacement of u with t. Regardless whether or not there is a difference in functional region, the "gene" can comprise, for example, expression control regions, coding region, exons, or introns. The "gene" may be contained in a cell or may exist alone after being released from a cell. Alternatively, the "gene" may be in a state enclosed in a vesicle called exosome.

The term "exosome" used herein is a vesicle that is encapsulated by lipid bilayer and secreted from a cell. The exosome is derived from a multivesicular endosome and may incorporate biomaterials such as "genes" (e.g., RNA or DNA) or proteins when released into an extracellular environment. The exosome is known to be contained in a body fluid such as blood, serum, plasma, cerebrospinal fluid, or lymph.

The term "transcript" used herein refers to an RNA produced by using a DNA sequence of a gene as a template. RNA polymerase binds to a site called promoter located upstream of the gene and adds ribonucleotides complementary to the nucleotide sequence of the DNA to the 3' end to produce an RNA. This RNA contains not only the gene sequence itself but also the whole sequence from a transcription initiation site to the end of a polyA sequence, including expression control regions, coding region, exons, and introns.

Unless otherwise specified, the term "microRNA (miRNA)" used herein is intended to mean a non-coding RNA having typically 15 to 25 nucleotides that is transcribed as an RNA precursor having a hairpin-like structure, cleaved by a dsRNA-cleaving enzyme having RNase III cleavage activity, and/or integrated into a protein complex called RISC, and that is involved in the suppression of translation of mRNA. Also, the term "miRNA" used herein includes not only a so-called "miRNA" shown by a particular nucleotide sequence (or SEQ ID NO) but also a precursor of the "miRNA" (pre-miRNA or pri-miRNA) and miRNA having biological functions equivalent to the miRNA shown by the particular nucleotide sequence (or SEQ ID NO), and another "miRNA" which is a congener thereof (i.e., a homolog or an ortholog), a variant such as a genetic polymorph, and a derivative. Such specific "miRNA," which is a precursor, a congener, a variant, or a derivative, can be identified, for example, by using "miRBase" (version 21). Examples of the "miRNA" which is a precursor, a congener, a variant, or a derivative include a "miRNA" having a nucleotide sequence hybridizing under stringent conditions described later to a complementary sequence of any particular nucleotide sequence shown by any of SEQ ID NOs: 1 to 740. Note that the "miRBase" (version 21) (http://www.mirbase.org/) is a database on the Web that provides miRNA nucleotide sequences, annotations, and prediction of their target genes. All the miRNAs registered in the "miRBase" are limited to miRNAs that have been cloned or miRNAs that have been reported to be expressed and processed *in vivo.* The term "miRNA" used herein may be a gene product of a miR gene. Such a gene product includes a mature miRNA (e.g., a 15- to 25-nucleotide or 19-to 25-nucleotide non-coding RNA involved in the suppression of translation of mRNA, as described above) or a miRNA precursor (e.g., pre-miRNA or pri-miRNA as described above).

The term "probe" used herein includes a polynucleotide, for example, a polynucleotide that is used for specifically detecting an RNA resulting from the expression of a gene or a polynucleotide derived from the RNA, and/or a polynucleotide complementary thereto.

The term "primer" used herein includes a polynucleotide, for example, consecutive polynucleotides capable of specifically recognizing (i.e., specifically binding to) and amplifying an RNA resulting from the expression of a gene or a polynucleotide derived from the RNA, and/or a polynucleotide complementary thereto.

In this context, the "complementary" polynucleotide (reverse strand) means a polynucleotide in a base-complementary relationship based on A:T (U) and G:C base pairs to the full-length or partial sequence of a polynucleotide consisting of a nucleotide sequence defined by any of SEQ ID NOs: 1 to 740 or a nucleotide sequence derived from the nucleotide sequence by the replacement of u with t (here, this full-length or partial sequence is referred to as a plus strand for the sake of convenience). However, such "complementary" polynucleotide is not limited to a sequence completely complementary to the nucleotide sequence of the plus strand of interest and may have a complementary relationship to an extent that permits hybridization under stringent conditions to the plus strand of interest.

The term "complementary strand" used herein refers to a single-stranded polynucleotide consisting of a nucleotide sequence that is completely complementary (i.e., at a level of 100%) to a single-stranded polynucleotide of interest.

The term "stringent conditions" used herein refers to conditions under which a polynucleotide, such as a nucleic acid probe or primer, hybridizes to its target polynucleotide at a detectable higher extent, e.g., at a measurement value equal to or larger than "(a mean of background measurement values) + (a standard error of the background measurement values) x 2", than that to other polynucleotides. The stringent conditions are nucleotide sequence-dependent and vary depending on an environment where hybridization is performed. A target polynucleotide complementary (e.g., 100% complementary) to a polynucleotide can be specified by controlling the stringency of hybridization and/or washing conditions. Specific examples of the "stringent conditions" will be mentioned later.

The term "Tm value" used herein means a temperature at which the double-stranded moiety of a polynucleotide is denatured into single strands so that the double strands and the single strands exist at a ratio of 1:1.

The term "variant" used herein means, in the context of a nucleic acid, a natural variant attributed to polymorphism, mutation, or the like; a variant containing a deletion, substitution, addition, and/or insertion of 1, 2 or 3 or more (e.g., 1 to several) nucleotides in a nucleotide sequence shown by any SEQ ID NO (e.g., any of SEQ ID NOs: 1 to 740 herein) or a nucleotide sequence derived from the nucleotide sequence by the replacement of u with t, or a partial sequence thereof; a variant containing a deletion, substitution, addition, and/or insertion of one or more nucleotides in a nucleotide sequence of a precursor RNA (a premature miRNA) of the nucleotide sequence shown by any of the above-mentioned SEQ ID NOs, a nucleotide sequence derived from the nucleotide sequence by the replacement of u with t, or a partial sequence thereof; a variant having percent (%) identity of approximately 90% or higher, approximately 95% or higher, approximately 97% or higher, approximately 98% or higher, or approximately 99% or higher to each of these nucleotide sequences or the partial sequences thereof; or a nucleic acid hybridizing under the stringent conditions defined above to a polynucleotide or an oligonucleotide comprising each of these nucleotide sequences or the partial sequences thereof. The term "oligonucleotide" used herein refers to a polynucleotide of 2 to 100 nucleotides in length.

The term "several" used herein means an integer of approximately 10, 9, 8, 7, 6, 5, 4, 3, or 2.

The variant as used herein can be produced with the use of a well-known technique such as site-directed mutagenesis, or mutagenesis using PCR.

The term "percent (%) identity" used herein can be determined with or without an introduced gap, using a protein or gene search system based on BLAST (https://blast.ncbi.nlm.nih.gov/Blast.cgi) or FASTA (http://www.genome.jp/tools/fasta/) (Zheng Zhang et al., 2000, J. Comput. Biol., Vol. 7, pp. 203-214; Altschul, S.F. et al., 1990, Journal of Molecular Biology, Vol. 215, pp. 403-410; and Pearson, W.R. et al., 1988, Proc. Natl. Acad. Sci. U.S.A., Vol. 85, pp. 2444-2448).

The term "derivative" used herein is meant to include a modified nucleic acid, for example, but not limited to, a derivative labeled with a fluorophore or the like, a derivative containing a modified nucleotide (e.g., a nucleotide containing a group such as halogen, alkyl such as methyl, alkoxy such as methoxy, thio, or carboxymethyl, and a nucleotide that has undergone base rearrangement, double bond saturation, deamination, replacement of an oxygen molecule with a sulfur molecule, etc.), PNA (peptide nucleic acid; Nielsen, P.E. et al., 1991, Science, Vol. 254, pp. 1497-500), and LNA (locked nucleic acid; Obika, S. et al., 1998, Tetrahedron Lett., Vol. 39, pp. 5401-5404).

As used herein, the "nucleic acid" capable of specifically binding to a polynucleotide selected from the groups of the hippocampal atrophy marker miRNAs or to a complementary strand of the polynucleotide is a synthesized or prepared nucleic acid and, for example, includes a "nucleic acid probe" or a "primer," as specific examples. Such "nucleic acid" may be used directly or indirectly for detecting the presence or absence of hippocampal atrophy in a subject, for diagnosing the presence or absence or the degree of amelioration of hippocampal atrophy or the therapeutic sensitivity of hippocampal atrophy, or for screening for a candidate substance useful in the prevention, amelioration, or treatment of hippocampal atrophy. The "nucleic acid" includes an oligonucleotide and a polynucleotide capable of specifically recognizing and binding to a transcript consisting of a nucleotide sequence shown by any of SEQ ID NOs: 1 to 740 associated with hippocampal atrophy or a synthetic cDNA nucleic acid thereof, or a complementary strand thereto, *in vivo,* or particularly, in a sample such as a body fluid (e.g., blood or urine). These polynucleotides can be effectively used on the basis of the properties described above, as probes for detecting the aforementioned gene/miRNA expressed *in vivo,* in tissues, in cells, or the like, or as primers for amplifying the aforementioned gene/miRNA expressed *in vivo.*

The term "detection" used herein is interchangeable with the term "examination," "measurement," "detection," or "determination support." As used herein, the term "evaluation" is meant to include diagnosing- or evaluation-supporting on the basis of examination results or measurement results.

The term "subject" used herein means a mammal such as a primate including a human and a monkey, such as a chimpanzee or a gorilla, a pet animal including a dog and a cat, a livestock animal including cattle, a horse, sheep, and a goat, a rodent including a mouse and a rat, and animals raised in a zoo. The subject is preferably a human. Meanwhile, a "subject with normal hippocampus," "subject with hippocampal atrophy," or "subject with no hippocampal atrophy" also means such a mammal which is an animal with no hippocampal atrophy to be detected, unless otherwise specified. The "subject with normal hippocampus," the "subject with hippocampal atrophy," and the "subject with no hippocampal atrophy" are preferably a human. While the term "subject" is used interchangeably with the term "patient," and a more typical "patient" is a human.

The term "hippocampus" used herein refers to a cortical region that is located at the base of the inferior horn of the lateral ventricle in the interior part of the temporal lobe of the cerebrum and is essential for formation of explicit memory, such as episodic memory.

The term "atrophy" used herein refers to a state in which the volume of the organ or tissue that has once developed to a normal volume is decreased due to various causes. The term "hippocampal atrophy" used herein indicates that the hippocampus size is decreased to an extent such that functions of the hippocampus would be adversely affected. The term "hippocampal atrophy" typically indicates that the ratio of the hippocampal volume (%) is decreased to 0.6% or less of the whole brain volume. As described above, in general, hippocampal atrophy gradually advances with aging. In addition to aging, diseases associated with hippocampal atrophy are, for example, but not limited to, Alzheimer's disease, frontotemporal lobar degeneration, depression, post-traumatic stress disorder, and schizophrenia.

The term "an extent of hippocampal atrophy" used herein refers to the degree of hippocampal atrophy of a patient, and the extent of hippocampal atrophy can be determined using the volume ratio of the hippocampus to the whole brain (%) as the indicator.

The "patient with no hippocampal atrophy" used herein includes an individual in which the volume ratio of the hippocampus to the whole brain (i.e., the proportion of the hippocampal volume in the whole brain volume) is 0.63% or more, 0.64% or more, 0.68% or more, or 0.86% or more.

The "patient with hippocampal atrophy" used herein inlcudes an individual in which the volume ratio of the hippocampus to the whole brain (i.e., the proportion of the hippocampal volume in the whole brain volume) is 0.60% or less, 0.59% or less, 0.57% or less, or 0.38% or less.

The term "Alzheimer's dementia" or "Alzheimer's disease" used herein refers to dementia in which amyloid β accumulation and phosphorylated tau accumulation are observed and neural degeneration characterized by hippocampal atrophy is caused in the brain.

The term "patient with mild dementia" used herein means a patient who does not develop dementia but exhibits signs of dementia. More specifically, the "patient with mild dementia" refers to a patient who scores 25 or lower on the dementia examination MMSE.

The term "P" or "P value" used herein refers to a probability at which a more extreme statistic than that actually calculated from data under null hypothesis is observed in a statistical test. Thus, smaller "P" or "P value" is regarded as being a more significant difference between subjects to be compared.

The term "sensitivity" used herein means a value of (the number of true positives) / (the number of true positives + the number of false negatives). High sensitivity allows hippocampal atrophy to be detected early and enables an early medical intervention.

The term "specificity" used herein means a value of (the number of true negatives) / (the number of true negatives + the number of false positives). High specificity prevents needless extra examination for hippocampal non-atrophy subjects, such as subjects with normal cognitive functions, misjudged as being hippocampal atrophy patients, leading to reduction in burden on patients and reduction in medical expense.

The term "accuracy" used herein means a value of (the number of true positives + the number of true negatives) / (the total number of cases). The accuracy indicates the ratio of samples that are identified correctly to all samples, and serves as a primary index for evaluating discrimination performance.

"$R^2$" indicates a value of the square of a correlation coefficient R herein. The correlation coefficient R is an indicator for the correlation between two variables.

"RMSE" used herein is an abbreviation for a root mean squared error, which is an indicator used to evaluate an error of a regression equation model. RMSE becomes smaller as the difference between the observed value and the estimated value becomes smaller.

"MAE" used herein is an abbreviation for a mean absolute error, which is also an indicator used to evaluate an error of a regression equation model as with RMSE. MAE becomes smaller as the difference between the observed value and the estimated value becomes smaller.

As used herein, the "sample" that is subjected to determination, detection, or diagnosis refers to a tissue and a biological material in which the expression level(s) of the hippocampal atrophy marker gene(s)/miRNA(s) of the present invention may vary, e.g., depending on the presence or absence of hippocampal atrophy, or as hippocampal atrophy progresses, or as therapeutic effects on hippocampal atrophy are exerted. Specifically, the sample may be a brain tissue or neurons, a nerve tissue, a cerebrospinal fluid, a bone marrow aspirate, an organ, skin; and a body fluid such as blood, urine, saliva, sweat, and tissue exudate; serum or plasma prepared from blood; and feces, hair, or the like. The "sample" further extracted therefrom may be, specifically, RNA or miRNA.

The term "hsa-miR-3131 gene" or "hsa-miR-3131" used herein includes the hsa-miR-3131 gene (miRBase Accession No. MIMAT0014996) shown by SEQ ID NO: 1, a homolog or an ortholog of a different organism species, and the like. The hsa-miR-3131 gene can be obtained by a method described in Stark MS et al., 2010, PLoS One, Vol. 5, e9685. Also, "hsa-mir-3131" (miRBase Accession No. MI0014151; SEQ ID NO: 201) having a hairpin-like structure is known as a precursor of "hsa-miR-3131."

The term "hsa-miR-6757-5p gene" or "hsa-miR-6757-5p" used herein includes the hsa-miR-6757-5p gene (miRBase Accession No. MIMAT0027414) shown by SEQ ID NO: 2, a homolog or an ortholog of a different organism species, and the like. The hsa-miR-6757-5p gene can be obtained by a method described in Ladewig E et al., 2012, Genome Res., Vol. 22, pp. 1634-1645. Also, "hsa-mir-6757" (miRBase Accession No. MI0022602; SEQ ID NO: 202) having a hairpin-like structure is known as a precursor of "hsa-miR-6757-5p."

The term "hsa-miR-4706 gene" or "hsa-miR-4706" used herein includes the hsa-miR-4706 gene (miRBase Accession No. MIMAT0019806) shown by SEQ ID NO: 3, a homolog or an ortholog of a different organism species, and the like. The hsa-miR-4706 gene can be obtained by a method described in Persson H et al., 2011, Cancer Res., Vol. 71, pp. 78-86. Also, "hsa-mir-4706" (miRBase Accession No. MI0017339; SEQ ID NO: 203) having a hairpin-like structure is known as a precursor of "hsa-miR-4706."

The term "hsa-miR-5001-5p gene" or "hsa-miR-5001-5p" used herein includes the hsa-miR-5001-5p gene (miRBase Accession No. MIMAT0021021) shown by SEQ ID NO: 4, a homolog or an ortholog of a different organism species, and the like. The hsa-miR-5001-5p gene can be obtained by a method described in Hansen TB et al., 2011, RNA Biol., Vol. 8, pp. 378-383. Also, "hsa-mir-5001" (miRBase Accession No. MI0017867; SEQ ID NO: 204) having a hairpin-like structure is known as a precursor of "hsa-miR-5001-5p."

The term "hsa-miR-3180-3p gene" or "hsa-miR-3180-3p" used herein includes the hsa-miR-3180-3p gene (miRBase Accession No. MIMAT0015058) shown by SEQ ID NO: 5, a homolog or an ortholog of a different organism species, and the like. The hsa-miR-3180-3p gene can be obtained by a method described in Creighton CJ et al., 2010, PLoS One, Vol. 5, e9637. Also, "hsa-mir-3180-1, hsa-mir-3180-2, and hsa-mir-3180-3" (miRBase Accession Nos. MI0014214, MI0014215, and MI0014217; SEQ ID NOs: 205, 206, and 207) each having a hairpin-like structure are known as precursors of "hsa-miR-3180-3p."

The term "hsa-miR-642b-3p gene" or "hsa-miR-642b-3p" used herein includes the hsa-miR-642b-3p gene (miRBase Accession No. MIMAT0018444) shown by SEQ ID NO: 6, a homolog or an ortholog of a different organism species, and the like. The hsa-miR-642b-3p gene can be obtained by a method described in Witten D et al., 2010, BMC Biol., Vol. 8, p. 58. Also, "hsa-mir-642b" (miRBase Accession No. MI0016685; SEQ ID NO: 208) having a hairpin-like structure is known as a precursor of "hsa-miR-642b-3p."

The term "hsa-miR-4655-5p gene" or "hsa-miR-4655-5p" used herein includes the hsa-miR-4655-5p gene (miRBase Accession No. MIMAT0019721) shown by SEQ ID NO: 7, a homolog or an ortholog of a different organism species, and the like. The hsa-miR-4655-5p gene can be obtained by a method described in Persson H et al., 2011, Cancer Res., Vol. 71, pp. 78-86. Also, "hsa-mir-4655" (miRBase Accession No. MI0017283; SEQ ID NO: 209) having a hairpin-like structure is known as a precursor of "hsa-miR-4655-5p."

The term "hsa-miR-6819-5p gene" or "hsa-miR-6819-5p" used herein includes the hsa-miR-6819-5p gene (miRBase Accession No. MIMAT0027538) shown by SEQ ID NO: 8, a homolog or an ortholog of a different organism species, and the like. The hsa-miR-6819-5p gene can be obtained by a method described in Ladewig E et al., 2012, Genome Res., Vol. 22, pp. 1634-1645. Also, "hsa-mir-6819" (miRBase Accession No. MI0022664; SEQ ID NO: 210) having a hairpin-like structure is known as a precursor of "hsa-miR-6819-5p."

The term "hsa-miR-937-5p gene" or "hsa-miR-937-5p" used herein includes the hsa-miR-937-5p gene (miRBase Accession No. MIMAT0022938) shown by SEQ ID NO: 9, a homolog or an ortholog of a different organism species, and the like. The hsa-miR-937-5p gene can be obtained by a method described in Lui WO et al., 2007, Cancer Res., Vol. 67, pp. 6031-6043. Also, "hsa-mir-937" (miRBase Accession No. MI0005759; SEQ ID NO: 211) having a hairpin-like structure is known as a precursor of "hsa-miR-937-5p."

The term "hsa-miR-4688 gene" or "hsa-miR-4688" used herein includes the hsa-miR-4688 gene (miRBase Accession No. MIMAT0019777) shown by SEQ ID NO: 10, a homolog or an ortholog of a different organism species, and the like. The hsa-miR-4688 gene can be obtained by a method described in Persson H et al., 2011, Cancer Res., Vol. 71, pp. 78-86. Also, "hsa-mir-4688" (miRBase Accession No. MI0017321; SEQ ID NO: 212) having a hairpin-like structure is known as a precursor of "hsa-miR-4688."

The term "hsa-miR-6741-5p gene" or "hsa-miR-6741-5p" used herein includes the hsa-miR-6741-5p gene (miRBase Accession No. MIMAT0027383) shown by SEQ ID NO: 11, a homolog or an ortholog of a different organism species, and the like. The hsa-miR-6741-5p gene can be obtained by a method described in Ladewig E et al., 2012, Genome Res., Vol. 22, pp. 1634-1645. Also, "hsa-mir-6741" (miRBase Accession No. MI0022586; SEQ ID NO: 213) having a hairpin-like structure is known as a precursor of "hsa-miR-6741-5p."

The term "hsa-miR-7107-5p gene" or "hsa-miR-7107-5p" used herein includes the hsa-miR-7107-5p gene (miRBase Accession No. MIMAT0028111) shown by SEQ ID NO: 12, a homolog or an ortholog of a different organism species, and the like. The hsa-miR-7107-5p gene can be obtained by a method described in Ladewig E et al., 2012, Genome Res., Vol. 22, pp. 1634-1645. Also, "hsa-mir-7107" (miRBase Accession No. MI0022958; SEQ ID NO: 214) having a hairpin-like structure is known as a precursor of "hsa-miR-7107-5p."

The term "hsa-miR-4271 gene" or "hsa-miR-4271" used herein includes the hsa-miR-4271 gene (miRBase Accession No. MIMAT0016901) shown by SEQ ID NO: 13, a homolog or an ortholog of a different organism species, and the like. The hsa-miR-4271 gene can be obtained by a method described in Goff LA et al., 2009, PLoS One, Vol. 4, e7192. Also, "hsa-mir-4271" (miRBase Accession No. MI0015879; SEQ ID NO: 215) having a hairpin-like structure is known as a precursor of "hsa-miR-4271."

The term "hsa-miR-1229-5p gene" or "hsa-miR-1229-5p" used herein includes the hsa-miR-1229-Sp gene (miRBase Accession No. MIMAT0022942) shown by SEQ ID NO: 14, a homolog or an ortholog of a different organism species, and the like. The hsa-miR-1229-5p gene can be obtained by a method described in Berezikov E et al., 2007, Mol. Cell, Vol. 28, pp. 328-336. Also, "hsa-mir-1229" (miRBase Accession No. MI0006319; SEQ ID NO: 216) having a hairpin-like structure is known as a precursor of "hsa-miR-1229-5p."

The term "hsa-miR-4707-5p gene" or "hsa-miR-4707-5p" used herein includes the hsa-miR-4707-5p gene (miRBase Accession No. MIMAT0019807) shown by SEQ ID NO: 15, a homolog or an ortholog of a different organism species, and the like. The hsa-miR-4707-5p gene can be obtained by a method described in Persson H et al., 2011, Cancer Res., Vol. 71, pp. 78-86. Also, "hsa-mir-4707" (miRBase Accession No. MI0017340; SEQ ID NO: 217) having a hairpin-like structure is known as a precursor of "hsa-miR-4707-5p."

The term "hsa-miR-6808-5p gene" or "hsa-miR-6808-5p" used herein includes the hsa-miR-6808-5p gene (miRBase Accession No. MIMAT0027516) shown by SEQ ID NO: 16, a homolog or an ortholog of a different organism species, and the like. The hsa-miR-6808-5p gene can be obtained by a method described in Ladewig E et al., 2012, Genome Res., Vol. 22, pp. 1634-1645. Also, "hsa-mir-6808" (miRBase Accession No. MI0022653; SEQ ID NO: 218) having a hairpin-like structure is known as a precursor of "hsa-miR-6808-5p."

The term "hsa-miR-4656 gene" or "hsa-miR-4656" used herein includes the hsa-miR-4656 gene (miRBase Accession No. MIMAT0019723) shown by SEQ ID NO: 17, a homolog or an ortholog of a different organism species, and the like. The hsa-miR-4656 gene can be obtained by a method described in Persson H et al., 2011, Cancer Res., Vol. 71, pp. 78-86. Also, "hsa-mir-4656" (miRBase Accession No. MI0017284; SEQ ID NO: 219) having a hairpin-like structure is known as a precursor of "hsa-miR-4656."

The term "hsa-miR-6076 gene" or "hsa-miR-6076" used herein includes the hsa-miR-6076 gene (miRBase Accession No. MIMAT0023701) shown by SEQ ID NO: 18, a homolog or an ortholog of a different organism species, and the like. The hsa-miR-6076 gene can be obtained by a method described in Voellenkle C et al., 2012, RNA, Vol. 18, pp. 472-484. Also, "hsa-mir-6076" (miRBase Accession No. MI0020353; SEQ ID NO: 220) having a hairpin-like structure is known as a precursor of "hsa-miR-6076."

The term "hsa-miR-6762-5p gene" or "hsa-miR-6762-5p" used herein includes the hsa-miR-6762-5p gene (miRBase Accession No. MIMAT0027424) shown by SEQ ID NO: 19, a homolog or an ortholog of a different organism species, and the like. The hsa-miR-6762-5p gene can be obtained by a method described in Ladewig E et al., 2012, Genome Res., Vol. 22, pp. 1634-1645. Also, "hsa-mir-6762" (miRBase Accession No. MI0022607; SEQ ID NO: 221) having a hairpin-like structure is known as a precursor of "hsa-miR-6762-5p."

The term "hsa-miR-7109-5p gene" or "hsa-miR-7109-5p" used herein includes the hsa-miR-7109-5p gene (miRBase Accession No. MIMAT0028115) shown by SEQ ID NO: 20, a homolog or an ortholog of a different organism species, and the like. The hsa-miR-7109-5p gene can be obtained by a method described in Ladewig E et al., 2012, Genome Res., Vol. 22, pp. 1634-1645. Also, "hsa-mir-7109" (miRBase Accession No. MI0022960; SEQ ID NO: 222) having a hairpin-like structure is known as a precursor of "hsa-miR-7109-5p."

The term "hsa-miR-6732-5p gene" or "hsa-miR-6732-5p" used herein includes the hsa-miR-6732-5p gene (miRBase Accession No. MIMAT0027365) shown by SEQ ID NO: 21, a homolog or an ortholog of a different organism species, and the like. The hsa-miR-6732-5p gene can be obtained by a method described in Ladewig E et al., 2012, Genome Res., Vol. 22, pp. 1634-1645. Also, "hsa-mir-6732" (miRBase Accession No. MI0022577; SEQ ID NO: 223) having a hairpin-like structure is known as a precursor of "hsa-miR-6732-5p."

The term "hsa-miR-3195 gene" or "hsa-miR-3195" used herein includes the hsa-miR-3195 gene (miRBase Accession No. MIMAT0015079) shown by SEQ ID NO: 22, a homolog or an ortholog of a different organism species, and the like. The hsa-miR-3195 gene can be obtained by a method described in Stark MS et al., 2010, PLoS One, Vol. 5, e9685. Also, "hsa-mir-3195" (miRBase Accession No. MI0014240; SEQ ID NO: 224) having a hairpin-like structure is known as a precursor of "hsa-miR-3195."

The term "hsa-miR-7150 gene" or "hsa-miR-7150" used herein includes the hsa-miR-7150 gene (miRBase Accession No. MIMAT0028211) shown by SEQ ID NO: 23, a homolog or an ortholog of a different organism species, and the like. The hsa-miR-7150 gene can be obtained by a method described in Oulas A. et al., 2009, Nucleic Acids Res., Vol. 37, pp. 3276-3287. Also, "hsa-mir-7150" (miRBase Accession No. MI0023610; SEQ ID NO: 225) having a hairpin-like structure is known as a precursor of "hsa-miR-7150."

The term "hsa-miR-642a-3p gene" or "hsa-miR-642a-3p" used herein includes the hsa-miR-642a-3p gene (miRBase Accession No. MIMAT0020924) shown by SEQ ID NO: 24, a homolog or an ortholog of a different organism species, and the like. The hsa-miR-642a-3p gene can be obtained by a method described in Cummins JM et al., 2006, Proc. Natl. Acad. Sci., U.S.A., Vol. 103, pp. 3687-3692. Also, "hsa-mir-642a" (miRBase Accession No. MI0003657; SEQ ID NO: 226) having a hairpin-like structure is known as a precursor of "hsa-miR-642a-3p."

The term "hsa-miR-1249-5p gene" or "hsa-miR-1249-5p" used herein includes the hsa-miR-1249-5p gene (miRBase Accession No. MIMAT0032029) shown by SEQ ID NO: 25, a homolog or an ortholog of a different organism species, and the like. The hsa-miR-1249-5p gene can be obtained by a method described in Morin RD et al., 2008, Genome Res., Vol. 18, pp. 610-621. Also, "hsa-mir-1249" (miRBase Accession No. MI0006384; SEQ ID NO: 227) having a hairpin-like structure is known as a precursor of "hsa-miR-1249-5p."

The term "hsa-miR-3185 gene" or "hsa-miR-3185" used herein includes the hsa-miR-3185 gene (miRBase Accession No. MIMAT0015065) shown by SEQ ID NO: 26, a homolog or an ortholog of a different organism species, and the like. The hsa-miR-3185 gene can be obtained by a method described in Stark MS et al., 2010, PLoS One, Vol. 5, e9685. Also, "hsa-mir-3185" (miRBase Accession No. MI0014227; SEQ ID NO: 228) having a hairpin-like structure is known as a precursor of "hsa-miR-3185."

The term "hsa-miR-4689 gene" or "hsa-miR-4689" used herein includes the hsa-miR-4689 gene (miRBase Accession No. MIMAT0019778) shown by SEQ ID NO: 27, a homolog or an ortholog of a different organism species, and the like. The hsa-miR-4689 gene can be obtained by a method described in Persson H et al., 2011, Cancer Res., Vol. 71, pp. 78-86. Also, "hsa-mir-4689" (miRBase Accession No. MI0017322; SEQ ID NO: 229) having a hairpin-like structure is known as a precursor of "hsa-miR-4689."

The term "hsa-miR-3141 gene" or "hsa-miR-3141" used herein includes the hsa-miR-3141 gene (miRBase Accession No. MIMAT0015010) shown by SEQ ID NO: 28, a homolog or an ortholog of a different organism species, and the like. The hsa-miR-3141 gene can be obtained by a method described in Stark MS et al., 2010, PLoS One, Vol. 5, e9685. Also, "hsa-mir-3141" (miRBase Accession No. MI0014165; SEQ ID NO: 230) having a hairpin-like structure is known as a precursor of "hsa-miR-3141."

The term "hsa-miR-6840-3p gene" or "hsa-miR-6840-3p" used herein includes the hsa-miR-6840-3p gene (miRBase Accession No. MIMAT0027583) shown by SEQ ID NO: 29, a homolog or an ortholog of a different organism species, and the like. The hsa-miR-6840-3p gene can be obtained by a method described in Ladewig E et al., 2012, Genome Res., Vol. 22, pp. 1634-1645. Also, "hsa-mir-6840" (miRBase Accession No. MI0022686; SEQ ID NO: 231) having a hairpin-like structure is known as a precursor of "hsa-miR-6840-3p."

The term "hsa-miR-3135b gene" or "hsa-miR-3135b" used herein includes the hsa-miR-3135b gene (miRBase Accession No. MIMAT0018985) shown by SEQ ID NO: 30, a homolog or an ortholog of a different organism species, and the like. The hsa-miR-3135b gene can be obtained by a method described in Jima DD et al., 2010, Blood, Vol. 116, e118-e127. Also, "hsa-mir-3135b" (miRBase Accession No. MI0016809; SEQ ID NO: 232) having a hairpin-like structure is known as a precursor of "hsa-miR-3135b."

The term "hsa-miR-1914-3p gene" or "hsa-miR-1914-3p" used herein includes the hsa-miR-1914-3p gene (miRBase Accession No. MIMAT0007890) shown by SEQ ID NO: 31, a homolog or an ortholog of a different organism species, and the like. The hsa-miR-1914-3p gene can be obtained by a method described in Bar M et al., 2008, Stem Cells, Vol. 26, pp. 2496-2505. Also, "hsa-mir-1914" (miRBase Accession No. MI0008335; SEQ ID NO: 233) having a hairpin-like structure is known as a precursor of "hsa-miR-1914-3p."

The term "hsa-miR-4446-3p gene" or "hsa-miR-4446-3p" used herein includes the hsa-miR-4446-3p gene (miRBase Accession No. MIMAT0018965) shown by SEQ ID NO: 32, a homolog or an ortholog of a different organism species, and the like. The hsa-miR-4446-3p gene can be obtained by a method described in Jima DD et al., 2010, Blood, Vol. 116, e118-e127. Also, "hsa-mir-4446" (miRBase Accession No. MI0016789; SEQ ID NO: 234) having a hairpin-like structure is known as a precursor of "hsa-miR-4446-3p."

The term "hsa-miR-4433b-3p gene" or "hsa-miR-4433b-3p" used herein includes the hsa-miR-4433b-3p gene (miRBase Accession No. MIMAT0030414) shown by SEQ ID NO: 33, a homolog or an ortholog of a different organism species, and the like. The hsa-miR-4433b-3p gene can be obtained by a method described in Ple H et al., 2012, PLoS One, Vol. 7, e50746. Also, "hsa-mir-4433b" (miRBase Accession No. MI0025511; SEQ ID NO: 235) having a hairpin-like structure is known as a precursor of "hsa-miR-4433b-3p."

The term "hsa-miR-6877-5p gene" or "hsa-miR-6877-5p" used herein includes the hsa-miR-6877-5p gene (miRBase Accession No. MIMAT0027654) shown by SEQ ID NO: 34, a homolog or an ortholog of a different organism species, and the like. The hsa-miR-6877-5p gene can be obtained by a method described in Ladewig E et al., 2012, Genome Res., Vol. 22, pp. 1634-1645. Also, "hsa-mir-6877" (miRBase Accession No. MI0022724; SEQ ID NO: 236) having a hairpin-like structure is known as a precursor of "hsa-miR-6877-5p."

The term "hsa-miR-6848-5p gene" or "hsa-miR-6848-5p" used herein includes the hsa-miR-6848-5p gene (miRBase Accession No. MIMAT0027596) shown by SEQ ID NO: 35, a homolog or an ortholog of a different organism species, and the like. The hsa-miR-6848-5p gene can be obtained by a method described in Ladewig E et al., 2012, Genome Res., Vol. 22, pp. 1634-1645. Also, "hsa-mir-6848" (miRBase Accession No. MI0022694; SEQ ID NO: 237) having a hairpin-like structure is known as a precursor of "hsa-miR-6848-5p."

The term "hsa-miR-3620-5p gene" or "hsa-miR-3620-5p" used herein includes the hsa-miR-3620-5p gene (miRBase Accession No. MIMAT0022967) shown by SEQ ID NO: 36, a homolog or an ortholog of a different organism species, and the like. The hsa-miR-3620-5p gene can be obtained by a method described in Witten D et al., 2010, BMC Biol., Vol. 8, p. 58. Also, "hsa-mir-3620" (miRBase Accession No. MI0016011; SEQ ID NO: 238) having a hairpin-like structure is known as a precursor of "hsa-miR-3620-5p."

The term "hsa-miR-6825-5p gene" or "hsa-miR-6825-5p" used herein includes the hsa-miR-6825-5p gene (miRBase Accession No. MIMAT0027550) shown by SEQ ID NO: 37, a homolog or an ortholog of a different organism species, and the like. The hsa-miR-6825-5p gene can be obtained by a method described in Ladewig E et al., 2012, Genome Res., Vol. 22, pp. 1634-1645. Also, "hsa-mir-6825" (miRBase Accession No. MI0022670; SEQ ID NO: 239) having a hairpin-like structure is known as a precursor of "hsa-miR-6825-5p."

The term "hsa-miR-5739 gene" or "hsa-miR-5739" used herein includes the hsa-miR-5739 gene (miRBase Accession No. MIMAT0023116) shown by SEQ ID NO: 38, a homolog or an ortholog of a different organism species, and the like. The hsa-miR-5739 gene can be obtained by a method described in Yoo JK et al., 2011, Biochem. Biophys. Res. Commun., Vol. 415, pp. 258-262. Also, "hsa-mir-5739" (miRBase Accession No. MI0019412; SEQ ID NO: 240) having a hairpin-like structure is known as a precursor of "hsa-miR-5739."

The term "hsa-miR-3663-3p gene" or "hsa-miR-3663-3p" used herein includes the hsa-miR-3663-3p gene (miRBase Accession No. MIMAT0018085) shown by SEQ ID NO: 39, a homolog or an ortholog of a different organism species, and the like. The hsa-miR-3663-3p gene can be obtained by a method described in Liao JY et al., 2010, PLoS One, Vol. 5, e10563. Also, "hsa-mir-3663" (miRBase Accession No. MI0016064; SEQ ID NO: 241) having a hairpin-like structure is known as a precursor of "hsa-miR-3663-3p."

The term "hsa-miR-4695-5p gene" or "hsa-miR-4695-5p" used herein includes the hsa-miR-4695-5p gene (miRBase Accession No. MIMAT0019788) shown by SEQ ID NO: 40, a homolog or an ortholog of a different organism species, and the like. The hsa-miR-4695-5p gene can be obtained by a method described in Persson H et al., 2011, Cancer Res., Vol. 71, pp. 78-86. Also, "hsa-mir-4695" (miRBase Accession No. MI0017328; SEQ ID NO: 242) having a hairpin-like structure is known as a precursor of "hsa-miR-4695-5p."

The term "hsa-miR-3162-5p gene" or "hsa-miR-3162-5p" used herein includes the hsa-miR-3162-5p gene (miRBase Accession No. MIMAT0015036) shown by SEQ ID NO: 41, a homolog or an ortholog of a different organism species, and the like. The hsa-miR-3162-5p gene can be obtained by a method described in Stark MS et al., 2010, PLoS One, Vol. 5, e9685. Also, "hsa-mir-3162" (miRBase Accession No. MI0014192; SEQ ID NO: 243) having a hairpin-like structure is known as a precursor of "hsa-miR-3162-5p."

The term "hsa-miR-3679-5p gene" or "hsa-miR-3679-5p" used herein includes the hsa-miR-3679-5p gene (miRBase Accession No. MIMAT0018104) shown by SEQ ID NO: 42, a homolog or an ortholog of a different organism species, and the like. The hsa-miR-3679-5p gene can be obtained by a method described in Creighton CJ et al., 2010, PLoS One, Vol. 5, e9637. Also, "hsa-mir-3679" (miRBase Accession No. MI0016080; SEQ ID NO: 244) having a hairpin-like structure is known as a precursor of "hsa-miR-3679-5p."

The term "hsa-miR-8059 gene" or "hsa-miR-8059" used herein includes the hsa-miR-8059 gene (miRBase Accession No. MIMAT0030986) shown by SEQ ID NO: 43, a homolog or an ortholog of a different organism species, and the like. The hsa-miR-8059 gene can be obtained by a method described in Wang HJ et al., 2013, Shock, Vol. 39, pp. 480-487. Also, "hsa-mir-8059" (miRBase Accession No. MI0025895; SEQ ID NO: 245) having a hairpin-like structure is known as a precursor of "hsa-miR-8059."

The term "hsa-miR-7110-5p gene" or "hsa-miR-7110-5p" used herein includes the hsa-miR-7110-5p gene (miRBase Accession No. MIMAT0028117) shown by SEQ ID NO: 44, a homolog or an ortholog of a different organism species, and the like. The hsa-miR-7110-5p gene can be obtained by a method described in Ladewig E et al., 2012, Genome Res., Vol. 22, pp. 1634-1645. Also, "hsa-mir-7110" (miRBase Accession No. MI0022961; SEQ ID NO: 246) having a hairpin-like structure is known as a precursor of "hsa-miR-7110-5p."

The term "hsa-miR-1275 gene" or "hsa-miR-1275" used herein includes the hsa-miR-1275 gene (miRBase Accession No. MIMAT0005929) shown by SEQ ID NO: 45, a homolog or an ortholog of a different organism species, and the like. The hsa-miR-1275 gene can be obtained by a method described in Morin RD et al., 2008, Genome Res.,

Vol. 18, pp. 610-621. Also, "hsa-mir-1275" (miRBase Accession No. MI0006415; SEQ ID NO: 247) having a hairpin-like structure is known as a precursor of "hsa-miR-1275."

The term "hsa-miR-6779-5p gene" or "hsa-miR-6779-5p" used herein includes the hsa-miR-6779-5p gene (miRBase Accession No. MIMAT0027458) shown by SEQ ID NO: 46, a homolog or an ortholog of a different organism species, and the like. The hsa-miR-6779-5p gene can be obtained by a method described in Ladewig E et al., 2012, Genome Res., Vol. 22, pp. 1634-1645. Also, "hsa-mir-6779" (miRBase Accession No. MI0022624; SEQ ID NO: 248) having a hairpin-like structure is known as a precursor of "hsa-miR-6779-5p."

The term "hsa-miR-197-5p gene" or "hsa-miR-197-5p" used herein includes the hsa-miR-197-5p gene (miRBase Accession No. MIMAT0022691) shown by SEQ ID NO: 47, a homolog or an ortholog of a different organism species, and the like. The hsa-miR-197-5p gene can be obtained by a method described in Lagos-Quintana M et al., 2003, RNA, Vol. 9, pp. 175-179. Also, "hsa-mir-197" (miRBase Accession No. MI0000239; SEQ ID NO: 249) having a hairpin-like structure is known as a precursor of "hsa-miR-197-5p."

The term "hsa-miR-6845-5p gene" or "hsa-miR-6845-5p" used herein includes the hsa-miR-6845-5p gene (miRBase Accession No. MIMAT0027590) shown by SEQ ID NO: 48, a homolog or an ortholog of a different organism species, and the like. The hsa-miR-6845-5p gene can be obtained by a method described in Ladewig E et al., 2012, Genome Res., Vol. 22, pp. 1634-1645. Also, "hsa-mir-6845" (miRBase Accession No. MI0022691; SEQ ID NO: 250) having a hairpin-like structure is known as a precursor of "hsa-miR-6845-5p."

The term "hsa-miR-4327 gene" or "hsa-miR-4327" used herein includes the hsa-miR-4327 gene (miRBase Accession No. MIMAT0016889) shown by SEQ ID NO: 49, a homolog or an ortholog of a different organism species, and the like. The hsa-miR-4327 gene can be obtained by a method described in Goff LA et al., 2009, PLoS One, Vol. 4, e7192. Also, "hsa-mir-4327" (miRBase Accession No. MI0015867; SEQ ID NO: 251) having a hairpin-like structure is known as a precursor of "hsa-miR-4327."

The term "hsa-miR-4723-5p gene" or "hsa-miR-4723-5p" used herein includes the hsa-miR-4723-5p gene (miRBase Accession No. MIMAT0019838) shown by SEQ ID NO: 50, a homolog or an ortholog of a different organism species, and the like. The hsa-miR-4723-5p gene can be obtained by a method described in Persson H et al., 2011, Cancer Res., Vol. 71, pp. 78-86. Also, "hsa-mir-4723" (miRBase Accession No. MI0017359; SEQ ID NO: 252) having a hairpin-like structure is known as a precursor of "hsa-miR-4723-5p."

The term "hsa-miR-4530 gene" or "hsa-miR-4530" used herein includes the hsa-miR-4530 gene (miRBase Accession No. MIMAT0019069) shown by SEQ ID NO: 51, a homolog or an ortholog of a different organism species, and the like. The hsa-miR-4530 gene can be obtained by a method described in Jima DD et al., 2010, Blood, Vol. 116, e118-e127. Also, "hsa-mir-4530" (miRBase Accession No. MI0016897; SEQ ID NO: 253) having a hairpin-like structure is known as a precursor of "hsa-miR-4530."

The term "hsa-miR-6771-5p gene" or "hsa-miR-6771-5p" used herein includes the hsa-miR-6771-5p gene (miRBase Accession No. MIMAT0027442) shown by SEQ ID NO: 52, a homolog or an ortholog of a different organism species, and the like. The hsa-miR-6771-5p gene can be obtained by a method described in Ladewig E et al., 2012, Genome Res., Vol. 22, pp. 1634-1645. Also, "hsa-mir-6771" (miRBase Accession No. MI0022616; SEQ ID NO: 254) having a hairpin-like structure is known as a precursor of "hsa-miR-6771-5p."

The term "hsa-miR-614 gene" or "hsa-miR-614" used herein includes the hsa-miR-614 gene (miRBase Accession No. MIMAT0003282) shown by SEQ ID NO: 53, a homolog or an ortholog of a different organism species, and the like. The hsa-miR-614 gene can be obtained by a method described in Cummins JM et al., 2006, Proc. Natl. Acad. Sci., U.S.A., Vol. 103, pp. 3687-3692. Also, "hsa-mir-614" (miRBase Accession No. MI0003627; SEQ ID NO: 255) having a hairpin-like structure is known as a precursor of "hsa-miR-614."

The term "hsa-miR-92a-2-5p gene" or "hsa-miR-92a-2-5p" used herein includes the hsa-miR-92a-2-5p gene (miRBase Accession No. MIMAT0004508) shown by SEQ ID NO: 54, a homolog or an ortholog of a different organism species, and the like. The hsa-miR-92a-2-5p gene can be obtained by a method described in Mourelatos Z et al., 2002, Genes Dev., Vol. 16, pp. 720-728. Also, "hsa-mir-92a-2" (miRBase Accession No. MI0000094; SEQ ID NO: 256) having a hairpin-like structure is known as a precursor of "hsa-miR-92a-2-5p."

The term "hsa-miR-6891-5p gene" or "hsa-miR-6891-5p" used herein includes the hsa-miR-6891-5p gene (miRBase Accession No. MIMAT0027682) shown by SEQ ID NO: 55, a homolog or an ortholog of a different organism species, and the like. The hsa-miR-6891-5p gene can be obtained by a method described in Ladewig E et al., 2012, Genome Res., Vol. 22, pp. 1634-1645. Also, "hsa-mir-6891" (miRBase Accession No. MI0022738; SEQ ID NO: 257) having a hairpin-like structure is known as a precursor of "hsa-miR-6891-5p."

The term "hsa-miR-6124 gene" or "hsa-miR-6124" used herein includes the hsa-miR-6124 gene (miRBase Accession No. MIMAT0024597) shown by SEQ ID NO: 56, a homolog or an ortholog of a different organism species, and the like. The hsa-miR-6124 gene can be obtained by a method described in Smith JL et al., 2012, J. Virol., Vol. 86, pp. 5278-5287. Also, "hsa-mir-6124" (miRBase Accession No. MI0021258; SEQ ID NO: 258) having a hairpin-like structure is known as a precursor of "hsa-miR-6124."

The term "hsa-miR-4687-3p gene" or "hsa-miR-4687-3p" used herein includes the hsa-miR-4687-3p gene (miRBase Accession No. MIMAT0019775) shown by SEQ ID NO: 57, a homolog or an ortholog of a different organism species, and the like. The hsa-miR-4687-3p gene can be obtained by a method described in Persson H et al., 2011, Cancer Res., Vol. 71, pp. 78-86. Also, "hsa-mir-4687" (miRBase Accession No. MI0017319; SEQ ID NO: 259) having a hairpin-like structure is known as a precursor of "hsa-miR-4687-3p."

The term "hsa-miR-4442 gene" or "hsa-miR-4442" used herein includes the hsa-miR-4442 gene (miRBase Accession No. MIMAT0018960) shown by SEQ ID NO: 58, a homolog or an ortholog of a different organism species, and the like. The hsa-miR-4442 gene can be obtained by a method described in Jima DD et al., 2010, Blood, Vol. 116, e118-e127. Also, "hsa-mir-4442" (miRBase Accession No. MI0016785; SEQ ID NO: 260) having a hairpin-like structure is known as a precursor of "hsa-miR-4442."

The term "hsa-miR-7977 gene" or "hsa-miR-7977" used herein includes the hsa-miR-7977 gene (miRBase Accession No. MIMAT0031180) shown by SEQ ID NO: 59, a homolog or an ortholog of a different organism species, and the like. The hsa-miR-7977 gene can be obtained by a method described in Velthut-Meikas A et al., 2013, Mol. Endocrinol., online version. Also, "hsa-mir-7977" (miRBase Accession No. MI0025753; SEQ ID NO: 261) having a hairpin-like structure is known as a precursor of "hsa-miR-7977."

The term "hsa-miR-6785-5p gene" or "hsa-miR-6785-5p" used herein includes the hsa-miR-6785-5p gene (miRBase Accession No. MIMAT0027470) shown by SEQ ID NO: 60, a homolog or an ortholog of a different organism species, and the like. The hsa-miR-6785-5p gene can be obtained by a method described in Ladewig E et al., 2012, Genome Res., Vol. 22, pp. 1634-1645. Also, "hsa-mir-6785" (miRBase Accession No. MI0022630; SEQ ID NO: 262) having a hairpin-like structure is known as a precursor of "hsa-miR-6785-5p."

The term "hsa-miR-4497 gene" or "hsa-miR-4497" used herein includes the hsa-miR-4497 gene (miRBase Accession No. MIMAT0019032) shown by SEQ ID NO: 61, a homolog or an ortholog of a different organism species, and the like. The hsa-miR-4497 gene can be obtained by a method described in Jima DD et al., 2010, Blood, Vol. 116, e118-e127. Also, "hsa-mir-4497" (miRBase Accession No. MI0016859; SEQ ID NO: 263) having a hairpin-like structure is known as a precursor of "hsa-miR-4497."

The term "hsa-miR-8071 gene" or "hsa-miR-8071" used herein includes the hsa-miR-8071 gene (miRBase Accession No. MIMAT0030998) shown by SEQ ID NO: 62, a homolog or an ortholog of a different organism species, and the like. The hsa-miR-8071 gene can be obtained by a method described in Wang HJ et al., 2013, Shock, Vol. 39, pp. 480-487. Also, "hsa-mir-8071-1 and hsa-mir-8071-2" (miRBase Accession Nos. MI0025907 and MI0026417; SEQ ID NOs: 264 and 265) each having a hairpin-like structure are known as precursors of "hsa-miR-8071."

The term "hsa-miR-663b gene" or "hsa-miR-663b" used herein includes the hsa-miR-663b gene (miRBase Accession No. MIMAT0005867) shown by SEQ ID NO: 63, a homolog or an ortholog of a different organism species, and the like. The hsa-miR-663b gene can be obtained by a method described in Takada S et al., 2008, Leukemia, Vol. 22, pp. 1274-1278. Also, "hsa-mir-663b" (miRBase Accession No. MI0006336; SEQ ID NO: 266) having a hairpin-like structure is known as a precursor of "hsa-miR-663b."

The term "hsa-miR-3180 gene" or "hsa-miR-3180" used herein includes the hsa-miR-3180 gene (miRBase Accession No. MIMAT0018178) shown by SEQ ID NO: 64, a homolog or an ortholog of a different organism species, and the like. The hsa-miR-3180 gene can be obtained by a method described in Creighton CJ et al., 2010, PLoS One, Vol. 5, e9637. Also, "hsa-mir-3180-4 and hsa-mir-3180-5" (miRBase Accession Nos. MI0016408 and MI0016409; SEQ ID NOs: 266 and 268) each having a hairpin-like structure are known as precursors of "hsa-miR-3180."

The term "hsa-miR-4251 gene" or "hsa-miR-4251" used herein includes the hsa-miR-4251 gene (miRBase Accession No. MIMAT0016883) shown by SEQ ID NO: 65, a homolog or an ortholog of a different organism species, and the like. The hsa-miR-4251 gene can be obtained by a method described in Goff LA et al., 2009, PLoS One, Vol. 4, e7192. Also, "hsa-mir-4251" (miRBase Accession No. MI0015861; SEQ ID NO: 269) having a hairpin-like structure is known as a precursor of "hsa-miR-4251."

The term "hsa-miR-1285-3p gene" or "hsa-miR-1285-3p" used herein includes the hsa-miR-1285-3p gene (miRBase Accession No. MIMAT0005876) shown by SEQ ID NO: 66, a homolog or an ortholog of a different organism species, and the like. The hsa-miR-1285-3p gene can be obtained by a method described in Morin RD et al., 2008, Genome Res., Vol. 18, pp. 10-621. Also, "hsa-mir-1285-1 and hsa-mir-1285-2" (miRBase Accession Nos. MI0006346 and MI0006347; SEQ ID NOs: 270 and 271) each having a hairpin-like structure are known as precursors of "hsa-miR-1285-3p."

The term "hsa-miR-6870-5p gene" or "hsa-miR-6870-5p" used herein includes the hsa-miR-6870-5p gene (miRBase Accession No. MIMAT0027640) shown by SEQ ID NO: 67, a homolog or an ortholog of a different organism species, and the like. The hsa-miR-6870-5p gene can be obtained by a method described in Ladewig E et al., 2012, Genome Res., Vol. 22, pp. 1634-1645. Also, "hsa-mir-6870" (miRBase Accession No. MI0022717; SEQ ID NO: 272) having a hairpin-like structure is known as a precursor of "hsa-miR-6870-5p."

The term "hsa-miR-4484 gene" or "hsa-miR-4484" used herein includes the hsa-miR-4484 gene (miRBase Accession No. MIMAT0019018) shown by SEQ ID NO: 68, a homolog or an ortholog of a different organism species, and the like. The hsa-miR-4484 gene can be obtained by a method described in Jima DD et al., 2010, Blood, Vol. 116, e118-e127. Also, "hsa-mir-4484" (miRBase Accession No. MI0016845; SEQ ID NO: 273) having a hairpin-like structure is known as a precursor of "hsa-miR-4484."

The term "hsa-miR-4476 gene" or "hsa-miR-4476" used herein includes the hsa-miR-4476 gene (miRBase Accession No. MIMAT0019003) shown by SEQ ID NO: 69, a homolog or an ortholog of a different organism species, and the like. The hsa-miR-4476 gene can be obtained by a method described in Jima DD et al., 2010, Blood, Vol. 116, e118-e127. Also, "hsa-miR-4476" (miRBase Accession No. MI0016828; SEQ ID NO: 274) having a hairpin-like structure is known as a precursor of "hsa-miR-4476."

The term "hsa-miR-6749-5p gene" or "hsa-miR-6749-5p" used herein includes the hsa-miR-6749-5p gene (miRBase Accession No. MIMAT0027398) shown by SEQ ID NO: 70, a homolog or an ortholog of a different organism species, and the like. The hsa-miR-6749-5p gene can be obtained by a method described in Ladewig E et al., 2012, Genome Res., Vol. 22, pp. 1634-1645. Also, "hsa-mir-6749" (miRBase Accession No. MI0022594; SEQ ID NO: 275) having a hairpin-like structure is known as a precursor of "hsa-miR-6749-5p."

The term "hsa-miR-4454 gene" or "hsa-miR-4454" used herein includes the hsa-miR-4454 gene (miRBase Accession No. MIMAT0018976) shown by SEQ ID NO: 71, a homolog or an ortholog of a different organism species, and the like. The hsa-miR-4454 gene can be obtained by a method described in Jima DD et al., 2010, Blood, Vol. 116, e118-e127. Also, "hsa-miR-4454" (miRBase Accession No. MI0016800; SEQ ID NO: 276) having a hairpin-like structure is known as a precursor of "hsa-miR-4454."

The term "hsa-miR-6893-5p gene" or "hsa-miR-6893-5p" used herein includes the hsa-miR-6893-5p gene (miRBase Accession No. MIMAT0027686) shown by SEQ ID NO: 72, a homolog or an ortholog of a different organism species, and the like. The hsa-miR-6893-5p gene can be obtained by a method described in Ladewig E et al., 2012, Genome Res., Vol. 22, pp. 1634-1645. Also, "hsa-mir-6893" (miRBase Accession No. MI0022740; SEQ ID NO: 277) having a hairpin-like structure is known as a precursor of "hsa-miR-6893-5p."

The term "hsa-miR-6085 gene" or "hsa-miR-6085" used herein includes the hsa-miR-6085 gene (miRBase Accession No. MIMAT0023710) shown by SEQ ID NO: 73, a homolog or an ortholog of a different organism species, and the like. The hsa-miR-6085 gene can be obtained by a method described in Voellenkle C et al., 2012, RNA, Vol. 18, pp. 472-484. Also, "hsa-mir-6085" (miRBase Accession No. MI0020362; SEQ ID NO: 278) having a hairpin-like structure is known as a precursor of "hsa-miR-6085."

The term "hsa-miR-4787-5p gene" or "hsa-miR-4787-5p" used herein includes the hsa-miR-4787-5p gene (miRBase Accession No. MIMAT0019956) shown by SEQ ID NO: 74, a homolog or an ortholog of a different organism species, and the like. The hsa-miR-4787-5p gene can be obtained by a method described in Persson H et al., 2011, Cancer Res., Vol. 71, pp. 78-86. Also, "hsa-mir-4787" (miRBase Accession No. MI0017434; SEQ ID NO: 279) having a hairpin-like structure is known as a precursor of "hsa-miR-4787-5p."

The term "hsa-miR-149-3p gene" or "hsa-miR-149-3p" used herein includes the hsa-miR-149-3p gene (miRBase Accession No. MIMAT0004609) shown by SEQ ID NO: 75, a homolog or an ortholog of a different organism species, and the like. The hsa-miR-149-3p gene can be obtained by a method described in Lagos-Quintana M et al., 2002, Curr. Biol., Vol. 12, pp. 735-739. Also, "hsa-mir-149" (miRBase Accession No. MI0000478; SEQ ID NO: 280) having a hairpin-like structure is known as a precursor of "hsa-miR-149-3p."

The term "hsa-miR-7704 gene" or "hsa-miR-7704" used herein includes the hsa-miR-7704 gene (miRBase Accession No. MIMAT0030019) shown by SEQ ID NO: 76, a homolog or an ortholog of a different organism species, and the like. The hsa-miR-7704 gene can be obtained by a method described in Swaminathan S et al., 2013, Biochem. Biophys. Res. Commun., Vol. 434, pp. 228-234. Also, "hsa-mir-7704" (miRBase Accession No. MI0025240; SEQ ID NO: 281) having a hairpin-like structure is known as a precursor of "hsa-miR-7704."

The term "hsa-miR-6125 gene" or "hsa-miR-6125" used herein includes the hsa-miR-6125 gene (miRBase Accession No. MIMAT0024598) shown by SEQ ID NO: 77, a homolog or an ortholog of a different organism species, and the like. The hsa-miR-6125 gene can be obtained by a method described in Smith JL et al., 2012, J. Virol., Vol. 86, pp. 5278-5287. Also, "hsa-mir-6125" (miRBase Accession No. MI0021259; SEQ ID NO: 282) having a hairpin-like structure is known as a precursor of "hsa-miR-6125."

The term "hsa-miR-6090 gene" or "hsa-miR-6090" used herein includes the hsa-miR-6090 gene (miRBase Accession No. MIMAT0023715) shown by SEQ ID NO: 78, a homolog or an ortholog of a different organism species, and the like. The hsa-miR-6090 gene can be obtained by a method described in Yoo JK et al., 2012, Stem Cells Dev., Vol. 21, pp. 2049-2057. Also, "hsa-mir-6090" (miRBase Accession No. MI0020367; SEQ ID NO: 283) having a hairpin-like structure is known as a precursor of "hsa-miR-6090."

The term "hsa-miR-3197 gene" or "hsa-miR-3197" used herein includes the hsa-miR-3197 gene (miRBase Accession No. MIMAT0015082) shown by SEQ ID NO: 79, a homolog or an ortholog of a different organism species, and the like. The hsa-miR-3197 gene can be obtained by a method described in Stark MS et al., 2010, PLoS One, Vol. 5, e9685. Also, "hsa-mir-3197" (miRBase Accession No. MI0014245; SEQ ID NO: 284) having a hairpin-like structure is known as a precursor of "hsa-miR-3197."

The term "hsa-miR-6850-5p gene" or "hsa-miR-6850-5p" used herein includes the hsa-miR-6850-5p gene (miRBase Accession No. MIMAT0027600) shown by SEQ ID NO: 80, a homolog or an ortholog of a different organism species, and the like. The hsa-miR-6850-5p gene can be obtained by a method described in Ladewig E et al., 2012, Genome Res., Vol. 22, pp. 1634-1645. Also, "hsa-mir-6850" (miRBase Accession No. MI0022696; SEQ ID NO: 285) having a hairpin-like structure is known as a precursor of "hsa-miR-6850-5p."

The term "hsa-miR-4467 gene" or "hsa-miR-4467" used herein includes the hsa-miR-4467 gene (miRBase Accession No. MIMAT0018994) shown by SEQ ID NO: 81, a homolog or an ortholog of a different organism species, and the like. The hsa-miR-4467 gene can be obtained by a method described in Jima DD et al., 2010, Blood, Vol. 116, e118-e127. Also, "hsa-mir-4467" (miRBase Accession No. MI0016818; SEQ ID NO: 286) having a hairpin-like structure is known as a precursor of "hsa-miR-4467."

The term "hsa-miR-6885-5p gene" or "hsa-miR-6885-5p" used herein includes the hsa-miR-6885-5p gene (miRBase Accession No. MIMAT0027670) shown by SEQ ID NO: 82, a homolog or an ortholog of a different organism species, and the like. The hsa-miR-6885-5p gene can be obtained by a method described in Ladewig E et al., 2012, Genome Res., Vol. 22, pp. 1634-1645. Also, "hsa-mir-6885" (miRBase Accession No. MI0022732; SEQ ID NO: 287) having a hairpin-like structure is known as a precursor of "hsa-miR-6885-5p."

The term "hsa-miR-6803-5p gene" or "hsa-miR-6803-5p" used herein includes the hsa-miR-6803-5p gene (miRBase Accession No. MIMAT0027506) shown by SEQ ID NO: 83, a homolog or an ortholog of a different organism species, and the like. The hsa-miR-6803-5p gene can be obtained by a method described in Ladewig E et al., 2012, Genome Res., Vol. 22, pp. 1634-1645. Also, "hsa-mir-6803" (miRBase Accession No. MI0022648; SEQ ID NO: 288) having a hairpin-like structure is known as a precursor of "hsa-miR-6803-5p."

The term "hsa-miR-6798-5p gene" or "hsa-miR-6798-5p" used herein includes the hsa-miR-6798-5p gene (miRBase Accession No. MIMAT0027496) shown by SEQ ID NO: 84, a homolog or an ortholog of a different organism species, and the like. The hsa-miR-6798-5p gene can be obtained by a method described in Ladewig E et al., 2012, Genome Res., Vol. 22, pp. 1634-1645. Also, "hsa-mir-6798" (miRBase Accession No. MI0022643; SEQ ID NO: 289) having a hairpin-like structure is known as a precursor of "hsa-miR-6798-5p."

The term "hsa-miR-6780b-5p gene" or "hsa-miR-6780b-5p" used herein includes the hsa-miR-6780b-5p gene (miRBase Accession No. MIMAT0027572) shown by SEQ ID NO: 85, a homolog or an ortholog of a different organism species, and the like. The hsa-miR-6780b-5p gene can be obtained by a method described in Ladewig E et al., 2012, Genome Res., Vol. 22, pp. 1634-1645. Also, "hsa-mir-6780b" (miRBase Accession No. MI0022681; SEQ ID NO: 290) having a hairpin-like structure is known as a precursor of "hsa-miR-6780b-5p."

The term "hsa-miR-6768-5p gene" or "hsa-miR-6768-5p" used herein includes the hsa-miR-6768-5p gene (miRBase Accession No. MIMAT0027436) shown by SEQ ID NO: 86, a homolog or an ortholog of a different organism species, and the like. The hsa-miR-6768-5p gene can be obtained by a method described in Ladewig E et al., 2012, Genome Res., Vol. 22, pp. 1634-1645. Also, "hsa-mir-6768" (miRBase Accession No. MI0022613; SEQ ID NO: 291) having a hairpin-like structure is known as a precursor of "hsa-miR-6768-5p."

The term "hsa-miR-5100 gene" or "hsa-miR-5100" used herein includes the hsa-miR-5100 gene (miRBase Accession No. MIMAT0022259) shown by SEQ ID NO: 87, a homolog or an ortholog of a different organism species, and the like. The hsa-miR-5100 gene can be obtained by a method described in Tandon M et al., 2012, Oral Dis., Vol. 18, pp. 127-131. Also, "hsa-mir-5100" (miRBase Accession No. MI0019116; SEQ ID NO: 292) having a hairpin-like structure is known as a precursor of "hsa-miR-5100."

The term "hsa-miR-6724-5p gene" or "hsa-miR-6724-5p" used herein includes the hsa-miR-6724-5p gene (miRBase Accession No. MIMAT0025856) shown by SEQ ID NO: 88, a homolog or an ortholog of a different organism species, and the like. The hsa-miR-6724-5p gene can be obtained by a method described in Li Y et al., 2012, Gene, Vol. 497, pp. 330-335. Also, "hsa-mir-6724-1, hsa-mir-6724-2, hsa-mir-6724-3, and hsa-mir-6724-4" (miRBase Accession Nos. MI0022559, MI0031516, MI0031517, and MI0031518; SEQ ID NOs: 293, 294, 295, and 296) each having a hairpin-like structure are known as precursors of "hsa-miR-6724-5p."

The term "hsa-miR-6879-5p gene" or "hsa-miR-6879-5p" used herein includes the hsa-miR-6879-5p gene (miRBase Accession No. MIMAT0027658) shown by SEQ ID NO: 89, a homolog or an ortholog of a different organism species, and the like. The hsa-miR-6879-5p gene can be obtained by a method described in Ladewig E et al., 2012, Genome Res., Vol. 22, pp. 1634-1645. Also, "hsa-mir-6879" (miRBase Accession No. MI0022726; SEQ ID NO: 297) having a hairpin-like structure is known as a precursor of "hsa-miR-6879-5p."

The term "hsa-miR-7108-5p gene" or "hsa-miR-7108-5p" used herein includes the hsa-miR-7108-5p gene (miRBase Accession No. MIMAT0028113) shown by SEQ ID NO: 90, a homolog or an ortholog of a different organism species, and the like. The hsa-miR-7108-5p gene can be obtained by a method described in Ladewig E et al., 2012, Genome Res., Vol. 22, pp. 1634-1645. Also, "hsa-mir-7108" (miRBase Accession No. MI0022959; SEQ ID NO: 298) having a hairpin-like structure is known as a precursor of "hsa-miR-7108-5p."

The term "hsa-miR-4649-5p gene" or "hsa-miR-4649-5p" used herein includes the hsa-miR-4649-5p gene (miRBase Accession No. MIMAT0019711) shown by SEQ ID NO: 91, a homolog or an ortholog of a different organism species, and the like. The hsa-miR-4649-5p gene can be obtained by a method described in Persson H et al., 2011, Cancer Res., Vol. 71, pp. 78-86. Also, "hsa-mir-4649" (miRBase Accession No. MI0017276; SEQ ID NO: 299) having a hairpin-like structure is known as a precursor of "hsa-miR-4649-5p."

The term "hsa-miR-4739 gene" or "hsa-miR-4739" used herein includes the hsa-miR-4739 gene (miRBase Accession No. MIMAT0019868) shown by SEQ ID NO: 92, a homolog or an ortholog of a different organism species, and the like. The hsa-miR-4739 gene can be obtained by a method described in Persson H et al., 2011, Cancer Res., Vol. 71, pp. 78-86. Also, "hsa-mir-4739" (miRBase Accession No. MI0017377; SEQ ID NO: 300) having a hairpin-like structure is known as a precursor of "hsa-miR-4739."

The term "hsa-miR-6089 gene" or "hsa-miR-6089" used herein includes the hsa-miR-6089 gene (miRBase Accession No. MIMAT0023714) shown by SEQ ID NO: 93, a homolog or an ortholog of a different organism species, and the like. The hsa-miR-6089 gene can be obtained by a method described in Yoo JK et al., 2012, Stem Cells Dev., Vol. 21, pp. 2049-2057. Also, "hsa-mir-6089-1 and hsa-mir-6089-2" (miRBase Accession Nos. MI0020366 and MI0023563; SEQ ID NOs: 301 and 302) each having a hairpin-like structure are known as precursors of "hsa-miR-6089."

The term "hsa-miR-1908-5p gene" or "hsa-miR-1908-5p" used herein includes the hsa-miR-1908-5p gene (miRBase Accession No. MIMAT0007881) shown by SEQ ID NO: 94, a homolog or an ortholog of a different organism species, and the like. The hsa-miR-1908-5p gene can be obtained by a method described in Bar M et al., 2008, Stem Cells, Vol. 26, pp. 2496-2505. Also, "hsa-mir-1908" (miRBase Accession No. MI0008329; SEQ ID NO: 303) having a hairpin-like structure is known as a precursor of "hsa-miR-1908-5p."

The term "hsa-miR-4516 gene" or "hsa-miR-4516" used herein includes the hsa-miR-4516 gene (miRBase Accession No. MIMAT0019053) shown by SEQ ID NO: 95, a homolog or an ortholog of a different organism species, and the like. The hsa-miR-4516 gene can be obtained by a method described in Jima DD et al., 2010, Blood, Vol. 116, e118-e127. Also, "hsa-mir-4516" (miRBase Accession No. MI0016882; SEQ ID NO: 304) having a hairpin-like structure is known as a precursor of "hsa-miR-4516."

The term "hsa-miR-2861 gene" or "hsa-miR-2861" used herein includes the hsa-miR-2861 gene (miRBase Accession No. MIMAT0013802) shown by SEQ ID NO: 96, a homolog or an ortholog of a different organism species, and the like. The hsa-miR-2861 gene can be obtained by a method described in Li H et al., 2009, J. Clin. Invest., Vol. 119, pp. 3666-3677. Also, "hsa-mir-2861" (miRBase Accession No. MI0013006; SEQ ID NO: 305) having a hairpin-like structure is known as a precursor of "hsa-miR-2861."

The term "hsa-miR-4492 gene" or "hsa-miR-4492" used herein includes the hsa-miR-4492 gene (miRBase Accession No. MIMAT0019027) shown by SEQ ID NO: 97, a homolog or an ortholog of a different organism species, and the like. The hsa-miR-4492 gene can be obtained by a method described in Jima DD et al., 2010, Blood, Vol. 116, e118-e127. Also, "hsa-mir-4492" (miRBase Accession No. MI0016854; SEQ ID NO: 306) having a hairpin-like structure is known as a precursor of "hsa-miR-4492."

The term "hsa-miR-4294 gene" or "hsa-miR-4294" used herein includes the hsa-miR-4294 gene (miRBase Accession No. MIMAT0016849) shown by SEQ ID NO: 98, a homolog or an ortholog of a different organism species, and the like. The hsa-miR-4294 gene can be obtained by a method described in Goff LA et al., 2009, PLoS One, Vol. 4, e7192. Also, "hsa-mir-4294" (miRBase Accession No. MI0015827; SEQ ID NO: 307) having a hairpin-like structure is known as a precursor of "hsa-miR-4294."

The term "hsa-miR-6791-5p gene" or "hsa-miR-6791-5p" used herein includes the hsa-miR-6791-5p gene (miRBase Accession No. MIMAT0027482) shown by SEQ ID NO: 99, a homolog or an ortholog of a different organism species, and the like. The hsa-miR-6791-5p gene can be obtained by a method described in Ladewig E et al., 2012, Genome Res., Vol. 22, pp. 1634-1645. Also, "hsa-mir-6791" (miRBase Accession No. MI0022636; SEQ ID NO: 308) having a hairpin-like structure is known as a precursor of "hsa-miR-6791-5p."

The term "hsa-miR-1469 gene" or "hsa-miR-1469" used herein includes the hsa-miR-1469 gene (miRBase Accession No. MIMAT0007347) shown by SEQ ID NO: 100, a homolog or an ortholog of a different organism species, and the like. The hsa-miR-1469 gene can be obtained by a method described in Kawaji H et al., 2008, BMC Genomics, Vol. 9, p. 157. Also, "hsa-mir-1469" (miRBase Accession No. MI0007074; SEQ ID NO: 309) having a hairpin-like structure is known as a precursor of "hsa-miR-1469."

The term "hsa-miR-6752-5p gene" or "hsa-miR-6752-5p" used herein includes the hsa-miR-6752-5p gene (miRBase Accession No. MIMAT0027404) shown by SEQ ID NO: 101, a homolog or an ortholog of a different organism species, and the like. The hsa-miR-6752-5p gene can be obtained by a method described in Ladewig E et al., 2012, Genome Res., Vol. 22, pp. 1634-1645. Also, "hsa-mir-6752" (miRBase Accession No. MI0022597; SEQ ID NO: 310) having a hairpin-like structure is known as a precursor of "hsa-miR-6752-5p."

The term "hsa-miR-4730 gene" or "hsa-miR-4730" used herein includes the hsa-miR-4730 gene (miRBase Accession No. MIMAT0019852) shown by SEQ ID NO: 102, a homolog or an ortholog of a different organism species, and the like. The hsa-miR-4730 gene can be obtained by a method described in Persson H et al., 2011, Cancer Res., Vol. 71, pp. 78-86. Also, "hsa-mir-4730" (miRBase Accession No. MI0017367; SEQ ID NO: 311) having a hairpin-like structure is known as a precursor of "hsa-miR-4730."

The term "hsa-miR-6126 gene" or "hsa-miR-6126" used herein includes the hsa-miR-6126 gene (miRBase Accession No. MIMAT0024599) shown by SEQ ID NO: 103, a homolog or an ortholog of a different organism species, and the like. The hsa-miR-6126 gene can be obtained by a method described in Smith JL et al., 2012, J. Virol., Vol. 86, pp. 5278-5287. Also, "hsa-mir-6126" (miRBase Accession No. MI0021260; SEQ ID NO: 312) having a hairpin-like structure is known as a precursor of "hsa-miR-6126."

The term "hsa-miR-6869-5p gene" or "hsa-miR-6869-5p" used herein includes the hsa-miR-6869-5p gene (miRBase Accession No. MIMAT0027638) shown by SEQ ID NO: 104, a homolog or an ortholog of a different organism species, and the like. The hsa-miR-6869-5p gene can be obtained by a method described in Ladewig E et al., 2012, Genome Res., Vol. 22, pp. 1634-1645. Also, "hsa-mir-6869" (miRBase Accession No. MI0022716; SEQ ID NO: 313) having a hairpin-like structure is known as a precursor of "hsa-miR-6869-5p."

The term "hsa-miR-1268a gene" or "hsa-miR-1268a" used herein includes the hsa-miR-1268a gene (miRBase Accession No. MIMAT0005922) shown by SEQ ID NO: 105, a homolog or an ortholog of a different organism species, and the like. The hsa-miR-1268a gene can be obtained by a method described in Morin RD et al., 2008, Genome Res., Vol. 18, pp. 610-621. Also, "hsa-mir-1268a" (miRBase Accession No. MI0006405; SEQ ID NO: 314) having a hairpin-like structure is known as a precursor of "hsa-miR-1268a."

The term "hsa-miR-6799-5p gene" or "hsa-miR-6799-5p" used herein includes the hsa-miR-6799-5p gene (miRBase Accession No. MIMAT0027498) shown by SEQ ID NO: 106, a homolog or an ortholog of a different organism species, and the like. The hsa-miR-6799-5p gene can be obtained by a method described in Ladewig E et al., 2012, Genome Res., Vol. 22, pp. 1634-1645. Also, "hsa-mir-6799" (miRBase Accession No. MI0022644; SEQ ID NO: 315) having a hairpin-like structure is known as a precursor of "hsa-miR-6799-5p."

The term "hsa-miR-8069 gene" or "hsa-miR-8069" used herein includes the hsa-miR-8069 gene (miRBase Accession No. MIMAT0030996) shown by SEQ ID NO: 107, a homolog or an ortholog of a different organism species, and the like. The hsa-miR-8069 gene can be obtained by a method described in Wang HJ et al., 2013, Shock, Vol. 39, pp. 480-487. Also, "hsa-mir-8069-1 and hsa-mir-8069-2" (miRBase Accession Nos. MI0025905 and MI0031519; SEQ ID NOs: 316 and 317) each having a hairpin-like structure are known as precursors of "hsa-miR-8069. "

The term "hsa-miR-3621 gene" or "hsa-miR-3621" used herein includes the hsa-miR-3621 gene (miRBase Accession No. MIMAT0018002) shown by SEQ ID NO: 108, a homolog or an ortholog of a different organism species, and the like. The hsa-miR-3621 gene can be obtained by a method described in Witten D et al., 2010, BMC Biol., Vol. 8, p. 58. Also, "hsa-mir-3621" (miRBase Accession No. MI0016012; SEQ ID NO: 318) having a hairpin-like structure is known as a precursor of "hsa-miR-3621."

The term "hsa-miR-4763-3p gene" or "hsa-miR-4763-3p" used herein includes the hsa-miR-4763-3p gene (miRBase Accession No. MIMAT0019913) shown by SEQ ID NO: 109, a homolog or an ortholog of a different organism species, and the like. The hsa-miR-4763-3p gene can be obtained by a method described in Persson H et al., 2011, Cancer Res., Vol. 71, pp. 78-86. Also, "hsa-mir-4763" (miRBase Accession No. MI0017404; SEQ ID NO: 319) having a hairpin-like structure is known as a precursor of "hsa-miR-4763-3p."

The term "hsa-miR-1228-5p gene" or "hsa-miR-1228-5p" used herein includes the hsa-miR-1228-Sp gene (miRBase Accession No. MIMAT0005582) shown by SEQ ID NO: 110, a homolog or an ortholog of a different organism species, and the like. The hsa-miR-1228-5p gene can be obtained by a method described in Berezikov E et al., 2007, Mol. Cell, Vol. 28, pp. 328-336. Also, "hsa-mir-1228" (miRBase Accession No. MI0006318; SEQ ID NO: 320) having a hairpin-like structure is known as a precursor of "hsa-miR-1228-5p."

The term "hsa-miR-760 gene" or "hsa-miR-760" used herein includes the hsa-miR-760 gene (miRBase Accession No. MIMAT0004957) shown by SEQ ID NO: 111, a homolog or an ortholog of a different organism species, and the like. The hsa-miR-760 gene can be obtained by a method described in Berezikov E et al., 2006, Genome Res., Vol. 16, pp. 1289-1298. Also, "hsa-mir-760" (miRBase Accession No. MI0005567; SEQ ID NO: 321) having a hairpin-like structure is known as a precursor of "hsa-miR-760."

The term "hsa-miR-187-5p gene" or "hsa-miR-187-5p" used herein includes the hsa-miR-187-5p gene (miRBase Accession No. MIMAT0004561) shown by SEQ ID NO: 112, a homolog or an ortholog of a different organism species, and the like. The hsa-miR-187-5p gene can be obtained by a method described in Lim LP et al., 2003, Science, Vol. 299, p. 1540. Also, "hsa-mir-187" (miRBase Accession No. MI0000274; SEQ ID NO: 322) having a hairpin-like structure is known as a precursor of "hsa-miR-187-5p."

The term "hsa-miR-7111-5p gene" or "hsa-miR-7111-5p" used herein includes the hsa-miR-7111-Sp gene (miRBase Accession No. MIMAT0028119) shown by SEQ ID NO: 113, a homolog or an ortholog of a different organism species, and the like. The hsa-miR-7111-Sp gene can be obtained by a method described in Ladewig E et al., 2012, Genome Res., Vol. 22, pp. 1634-1645. Also, "hsa-mir-7111" (miRBase Accession No. MI0022962; SEQ ID NO: 323) having a hairpin-like structure is known as a precursor of "hsa-miR-7111-Sp."

The term "hsa-miR-6088 gene" or "hsa-miR-6088" used herein includes the hsa-miR-6088 gene (miRBase Accession No. MIMAT0023713) shown by SEQ ID NO: 114, a homolog or an ortholog of a different organism species, and the like. The hsa-miR-6088 gene can be obtained by a method described in Yoo JK et al., 2012, Stem Cells Dev., Vol. 21, pp. 2049-2057. Also, "hsa-mir-6088" (miRBase Accession No. MI0020365; SEQ ID NO: 324) having a hairpin-like structure is known as a precursor of "hsa-miR-6088."

The term "hsa-miR-6805-3p gene" or "hsa-miR-6805-3p" used herein includes the hsa-miR-6805-3p gene (miRBase Accession No. MIMAT0027511) shown by SEQ ID NO: 115, a homolog or an ortholog of a different organism species, and the like. The hsa-miR-6805-3p gene can be obtained by a method described in Ladewig E et al., 2012, Genome Res., Vol. 22, pp. 1634-1645. Also, "hsa-mir-6805" (miRBase Accession No. MI0022650; SEQ ID NO: 325) having a hairpin-like structure is known as a precursor of "hsa-miR-6805-3p."

The term "hsa-miR-4640-5p gene" or "hsa-miR-4640-5p" used herein includes the hsa-miR-4640-5p gene (miRBase Accession No. MIMAT0019699) shown by SEQ ID NO: 116, a homolog or an ortholog of a different organism species, and the like. The hsa-miR-4640-5p gene can be obtained by a method described in Persson H et al., 2011, Cancer Res., Vol. 71, pp. 78-86. Also, "hsa-mir-4640" (miRBase Accession No. MI0017267; SEQ ID NO: 326) having a hairpin-like structure is known as a precursor of "hsa-miR-4640-5p."

The term "hsa-miR-6721-5p gene" or "hsa-miR-6721-5p" used herein includes the hsa-miR-6721-5p gene (miRBase Accession No. MIMAT0025852) shown by SEQ ID NO: 117, a homolog or an ortholog of a different organism species, and the like. The hsa-miR-6721-5p gene can be obtained by a method described in Li Y et al., 2012, Gene, Vol. 497, pp. 330-335. Also, "hsa-mir-6721" (miRBase Accession No. MI0022556; SEQ ID NO: 327) having a hairpin-like structure is known as a precursor of "hsa-miR-6721-5p."

The term "hsa-miR-6880-5p gene" or "hsa-miR-6880-5p" used herein includes the hsa-miR-6880-5p gene (miRBase Accession No. MIMAT0027660) shown by SEQ ID NO: 118, a homolog or an ortholog of a different organism species, and the like. The hsa-miR-6880-5p gene can be obtained by a method described in Ladewig E et al., 2012, Genome Res., Vol. 22, pp. 1634-1645. Also, "hsa-mir-6880" (miRBase Accession No. MI0022727; SEQ ID NO: 328) having a hairpin-like structure is known as a precursor of "hsa-miR-6880-5p."

The term "hsa-miR-711 gene" or "hsa-miR-711" used herein includes the hsa-miR-711 gene (miRBase Accession No. MIMAT0012734) shown by SEQ ID NO: 119, a homolog or an ortholog of a different organism species, and the like. The hsa-miR-711 gene can be obtained by a method described in Artzi S et al., 2008, BMC Bioinformatics, Vol. 9, p. 39. Also, "hsa-mir-711" (miRBase Accession No. MI0012488; SEQ ID NO: 329) having a hairpin-like structure is known as a precursor of "hsa-miR-711."

The term "hsa-miR-128-1-5p gene" or "hsa-miR-128-1-5p" used herein includes the hsa-miR-128-1-5p gene (miRBase Accession No. MIMAT0026477) shown by SEQ ID NO: 120, a homolog or an ortholog of a different organism species, and the like. The hsa-miR-128-1-5p gene can be obtained by a method described in Lagos-Quintana M et al., 2002, Curr. Biol., Vol. 12, pp. 735-739. Also, "hsa-mir-128-1" (miRBase Accession No. MI0000447; SEQ ID NO: 330) having a hairpin-like structure is known as a precursor of "hsa-miR-128-1-5p."

The term "hsa-miR-4525 gene" or "hsa-miR-4525" used herein includes the hsa-miR-4525 gene (miRBase Accession No. MIMAT0019064) shown by SEQ ID NO: 121, a homolog or an ortholog of a different organism species, and the like. The hsa-miR-4525 gene can be obtained by a method described in Jima DD et al., 2010, Blood, Vol. 116, e118-e127. Also, "hsa-mir-4525" (miRBase Accession No. MI0016892; SEQ ID NO: 331) having a hairpin-like structure is known as a precursor of "hsa-miR-4525."

The term "hsa-miR-486-3p gene" or "hsa-miR-486-3p" used herein includes the hsa-miR-486-3p gene (miRBase Accession No. MIMAT0004762) shown by SEQ ID NO: 122, a homolog or an ortholog of a different organism species, and the like. The hsa-miR-486-3p gene can be obtained by a method described in Fu H et al., 2005, FEBS Lett., Vol. 579, pp. 3849-3854. Also, "hsa-mir-486-1 and hsa-mir-486-2" (miRBase Accession Nos. MI0002470 and MI0023622; SEQ ID NOs: 332 and 333) each having a hairpin-like structure are known as precursors of "hsa-miR-486-3p."

The term "hsa-miR-6756-5p gene" or "hsa-miR-6756-5p" used herein includes the hsa-miR-6756-5p gene (miRBase Accession No. MIMAT0027412) shown by SEQ ID NO: 123, a homolog or an ortholog of a different organism species, and the like. The hsa-miR-6756-5p gene can be obtained by a method described in Ladewig E et al., 2012, Genome Res., Vol. 22, pp. 1634-1645. Also, "hsa-mir-6756" (miRBase Accession No. MI0022601; SEQ ID NO: 334) having a hairpin-like structure is known as a precursor of "hsa-miR-6756-5p."

The term "hsa-miR-1260b gene" or "hsa-miR-1260b" used herein includes the hsa-miR-1260b gene (miRBase Accession No. MIMAT0015041) shown by SEQ ID NO: 124, a homolog or an ortholog of a different organism species, and the like. The hsa-miR-1260b gene can be obtained by a method described in Stark MS et al., 2010, PLoS One, Vol. 5, e9685. Also, "hsa-mir-1260b" (miRBase Accession No. MI0014197; SEQ ID NO: 335) having a hairpin-like structure is known as a precursor of "hsa-miR-1260b."

The term "hsa-miR-3184-5p gene" or "hsa-miR-3184-5p" used herein includes the hsa-miR-3184-5p gene (miRBase Accession No. MIMAT0015064) shown by SEQ ID NO: 125, a homolog or an ortholog of a different organism species, and the like. The hsa-miR-3184-5p gene can be obtained by a method described in Stark MS et al., 2010, PLoS One, Vol. 5, e9685. Also, "hsa-mir-3184" (miRBase Accession No. MI0014226; SEQ ID NO: 336) having a hairpin-like structure is known as a precursor of "hsa-miR-3184-5p."

The term "hsa-miR-6075 gene" or "hsa-miR-6075" used herein includes the hsa-miR-6075 gene (miRBase Accession No. MIMAT0023700) shown by SEQ ID NO: 126, a homolog or an ortholog of a different organism species, and the like. The hsa-miR-6075 gene can be obtained by a method described in Voellenkle C et al., 2012, RNA, Vol. 18, pp. 472-484. Also, "hsa-mir-6075" (miRBase Accession No. MI0020352; SEQ ID NO: 337) having a hairpin-like structure is known as a precursor of "hsa-miR-6075."

The term "hsa-miR-204-3p gene" or "hsa-miR-204-3p" used herein includes the hsa-miR-204-3p gene (miRBase Accession No. MIMAT0022693) shown by SEQ ID NO: 127, a homolog or an ortholog of a different organism species, and the like. The hsa-miR-204-3p gene can be obtained by a method described in Lim LP et al., 2003, Science, Vol. 299, p. 1540. Also, "hsa-mir-204" (miRBase Accession No. MI0000284; SEQ ID NO: 338) having a hairpin-like structure is known as a precursor of "hsa-miR-204-3p."

The term "hsa-miR-4728-5p gene" or "hsa-miR-4728-5p" used herein includes the hsa-miR-4728-5p gene (miRBase Accession No. MIMAT0019849) shown by SEQ ID NO: 128, a homolog or an ortholog of a different organism species, and the like. The hsa-miR-4728-5p gene can be obtained by a method described in Persson H et al., 2011, Cancer Res., Vol. 71, pp. 78-86. Also, "hsa-mir-4728" (miRBase Accession No. MI0017365; SEQ ID NO: 339) having a hairpin-like structure is known as a precursor of "hsa-miR-4728-5p."

The term "hsa-miR-4534 gene" or "hsa-miR-4534" used herein includes the hsa-miR-4534 gene (miRBase Accession No. MIMAT0019073) shown by SEQ ID NO: 129, a homolog or an ortholog of a different organism species, and the like. The hsa-miR-4534 gene can be obtained by a method described in Jima DD et al., 2010, Blood, Vol. 116, e118-e127. Also, "hsa-mir-4534" (miRBase Accession No. MI0016901; SEQ ID NO: 340) having a hairpin-like structure is known as a precursor of "hsa-miR-4534."

The term "hsa-miR-4758-5p gene" or "hsa-miR-4758-5p" used herein includes the hsa-miR-4758-5p gene (miRBase Accession No. MIMAT0019903) shown by SEQ ID NO: 130, a homolog or an ortholog of a different organism species, and the like. The hsa-miR-4758-5p gene can be obtained by a method described in Persson H et al., 2011, Cancer Res., Vol. 71, pp. 78-86. Also, "hsa-mir-4758" (miRBase Accession No. MI0017399; SEQ ID NO: 341) having a hairpin-like structure is known as a precursor of "hsa-miR-4758-5p."

The term "hsa-miR-8063 gene" or "hsa-miR-8063" used herein includes the hsa-miR-8063 gene (miRBase Accession No. MIMAT0030990) shown by SEQ ID NO: 131, a homolog or an ortholog of a different organism species, and the like. The hsa-miR-8063 gene can be obtained by a method described in Wang HJ et al., 2013, Shock, Vol. 39, pp. 480-487. Also, "hsa-mir-8063" (miRBase Accession No. MI0025899; SEQ ID NO: 342) having a hairpin-like structure is known as a precursor of "hsa-miR-8063."

The term "hsa-miR-6836-3p gene" or "hsa-miR-6836-3p" used herein includes the hsa-miR-6836-3p gene (miRBase Accession No. MIMAT0027575) shown by SEQ ID NO: 132, a homolog or an ortholog of a different organism species, and the like. The hsa-miR-6836-3p gene can be obtained by a method described in Ladewig E et al., 2012, Genome Res., Vol. 22, pp. 1634-1645. Also, "hsa-mir-6836" (miRBase Accession No. MI0022682; SEQ ID NO: 343) having a hairpin-like structure is known as a precursor of "hsa-miR-6836-3p."

The term "hsa-miR-6789-5p gene" or "hsa-miR-6789-5p" used herein includes the hsa-miR-6789-5p gene (miRBase Accession No. MIMAT0027478) shown by SEQ ID NO: 133, a homolog or an ortholog of a different organism species, and the like. The hsa-miR-6789-5p gene can be obtained by a method described in Ladewig E et al., 2012, Genome Res., Vol. 22, pp. 1634-1645. Also, "hsa-mir-6789" (miRBase Accession No. MI0022634; SEQ ID NO: 344) having a hairpin-like structure is known as a precursor of "hsa-miR-6789-5p."

The term "hsa-miR-744-5p gene" or "hsa-miR-744-5p" used herein includes the hsa-miR-744-5p gene (miRBase Accession No. MIMAT0004945) shown by SEQ ID NO: 134, a homolog or an ortholog of a different organism species, and the like. The hsa-miR-744-5p gene can be obtained by a method described in Berezikov E et al., 2006, Genome Res., Vol. 16, pp. 1289-1298. Also, "hsa-mir-744" (miRBase Accession No. MI0005559; SEQ ID NO: 345) having a hairpin-like structure is known as a precursor of "hsa-miR-744-5p."

The term "hsa-miR-1909-3p gene" or "hsa-miR-1909-3p" used herein includes the hsa-miR-1909-3p gene (miRBase Accession No. MIMAT0007883) shown by SEQ ID NO: 135, a homolog or an ortholog of a different organism species, and the like. The hsa-miR-1909-3p gene can be obtained by a method described in Bar M et al., 2008, Stem Cells, Vol. 26, pp. 2496-2505. Also, "hsa-mir-1909" (miRBase Accession No. MI0008330; SEQ ID NO: 346) having a hairpin-like structure is known as a precursor of "hsa-miR-1909-3p."

The term "hsa-miR-887-3p gene" or "hsa-miR-887-3p" used herein includes the hsa-miR-887-3p gene (miRBase Accession No. MIMAT0004951) shown by SEQ ID NO: 136, a homolog or an ortholog of a different organism species, and the like. The hsa-miR-887-3p gene can be obtained by a method described in Berezikov E et al., 2006, Genome Res., Vol. 16, pp. 1289-1298. Also, "hsa-mir-887" (miRBase Accession No. MI0005562; SEQ ID NO: 347) having a hairpin-like structure is known as a precursor of "hsa-miR-887-3p."

The term "hsa-miR-4745-5p gene" or "hsa-miR-4745-5p" used herein includes the hsa-miR-4745-5p gene (miRBase Accession No. MIMAT0019878) shown by SEQ ID NO: 137, a homolog or an ortholog of a different organism species, and the like. The hsa-miR-4745-5p gene can be obtained by a method described in Persson H et al., 2011, Cancer Res., Vol. 71, pp. 78-86. Also, "hsa-mir-4745" (miRBase Accession No. MI0017384; SEQ ID NO: 348) having a hairpin-like structure is known as a precursor of "hsa-miR-4745-5p."

The term "hsa-miR-4433a-3p gene" or "hsa-miR-4433a-3p" used herein includes the hsa-miR-4433a-3p gene (miRBase Accession No. MIMAT0018949) shown by SEQ ID NO: 138, a homolog or an ortholog of a different organism species, and the like. The hsa-miR-4433a-3p gene can be obtained by a method described in JimaDD et al., 2010, Blood, Vol. 116, e118-e127. Also, "hsa-mir-4433a" (miRBase Accession No. MI0016773; SEQ ID NO: 349) having a hairpin-like structure is known as a precursor of "hsa-miR-4433a-3p."

The term "hsa-miR-5090 gene" or "hsa-miR-5090" used herein includes the hsa-miR-5090 gene (miRBase Accession No. MIMAT0021082) shown by SEQ ID NO: 139, a homolog or an ortholog of a different organism species, and the like. The hsa-miR-5090 gene can be obtained by a method described in Ding N et al., 2011, J. Radiat. Res., Vol. 52, pp. 425-432. Also, "hsa-mir-5090" (miRBase Accession No. MI0017979; SEQ ID NO: 350) having a hairpin-like structure is known as a precursor of "hsa-miR-5090."

The term "hsa-miR-296-5p gene" or "hsa-miR-296-5p" used herein includes the hsa-miR-296-5p gene (miRBase Accession No. MIMAT0000690) shown by SEQ ID NO: 140, a homolog or an ortholog of a different organism species, and the like. The hsa-miR-296-5p gene can be obtained by a method described in Houbaviy HB et al., 2003, Dev. Cell., Vol. 5, pp. 351-358. Also, "hsa-mir-296" (miRBase Accession No. MI0000747; SEQ ID NO: 351) having a hairpin-like structure is known as a precursor of "hsa-miR-296-5p."

The term "hsa-miR-939-5p gene" or "hsa-miR-939-5p" used herein includes the hsa-miR-939-5p gene (miRBase Accession No. MIMAT0004982) shown by SEQ ID NO: 141, a homolog or an ortholog of a different organism species, and the like. The hsa-miR-939-5p gene can be obtained by a method described in Lui WO et al., 2007, Cancer Res., Vol. 67, pp. 6031-6043. Also, "hsa-mir-939" (miRBase Accession No. MI0005761; SEQ ID NO: 352) having a hairpin-like structure is known as a precursor of "hsa-miR-939-5p."

The term "hsa-miR-3648 gene" or "hsa-miR-3648" used herein includes the hsa-miR-3648 gene (miRBase Accession No. MIMAT0018068) shown by SEQ ID NO: 142, a homolog or an ortholog of a different organism species, and the like. The hsa-miR-3648 gene can be obtained by a method described in Meiri E et al., 2010, Nucleic Acids Res., Vol. 38, pp. 6234-6246. Also, "hsa-mir-3648-1 and hsa-mir-3648-2" (miRBase Accession Nos. MI0016048 and MI0031512; SEQ ID NOs: 353 and 354) each having a hairpin-like structure are known as precursors of "hsa-miR-3648."

The term "hsa-miR-3196 gene" or "hsa-miR-3196" used herein includes the hsa-miR-3196 gene (miRBase Accession No. MIMAT0015080) shown by SEQ ID NO: 143, a homolog or an ortholog of a different organism species, and the like. The hsa-miR-3196 gene can be obtained by a method described in Stark MS et al., 2010, PLoS One, Vol. 5, e9685. Also, "hsa-mir-3196" (miRBase Accession No. MI0014241; SEQ ID NO: 355) having a hairpin-like structure is known as a precursor of "hsa-miR-3196."

The term "hsa-miR-6722-3p gene" or "hsa-miR-6722-3p" used herein includes the hsa-miR-6722-3p gene (miRBase Accession No. MIMAT0025854) shown by SEQ ID NO: 144, a homolog or an ortholog of a different organism species, and the like. The hsa-miR-6722-3p gene can be obtained by a method described in Li Y et al., 2012, Gene, Vol. 497, pp. 330-335. Also, "hsa-mir-6722" (miRBase Accession No. MI0022557; SEQ ID NO: 356) having a hairpin-like structure is known as a precursor of "hsa-miR-6722-3p."

The term "hsa-miR-6805-5p gene" or "hsa-miR-6805-5p" used herein includes the hsa-miR-6805-5p gene (miRBase Accession No. MIMAT0027510) shown by SEQ ID NO: 145, a homolog or an ortholog of a different organism species, and the like. The hsa-miR-6805-5p gene can be obtained by a method described in Ladewig E et al., 2012, Genome Res., Vol. 22, pp. 1634-1645. Also, "hsa-mir-6805" (miRBase Accession No. MI0022650; SEQ ID NO: 325) having a hairpin-like structure is known as a precursor of "hsa-miR-6805-5p."

The term "hsa-miR-1202 gene" or "hsa-miR-1202" used herein includes the hsa-miR-1202 gene (miRBase Accession No. MIMAT0005865) shown by SEQ ID NO: 146, a homolog or an ortholog of a different organism species, and the like. The hsa-miR-1202 gene can be obtained by a method described in Marton S et al., 2008, Leukemia, Vol. 22, pp. 330-338. Also, "hsa-mir-1202" (miRBase Accession No. MI0006334; SEQ ID NO: 357) having a hairpin-like structure is known as a precursor of "hsa-miR-1202."

The term "hsa-miR-6775-5p gene" or "hsa-miR-6775-5p" used herein includes the hsa-miR-6775-5p gene (miRBase Accession No. MIMAT0027450) shown by SEQ ID NO: 147, a homolog or an ortholog of a different organism species, and the like. The hsa-miR-6775-5p gene can be obtained by a method described in Ladewig E et al., 2012, Genome Res., Vol. 22, pp. 1634-1645. Also, "hsa-mir-6775" (miRBase Accession No. MI0022620; SEQ ID NO: 358) having a hairpin-like structure is known as a precursor of "hsa-miR-6775-5p."

The term "hsa-miR-6087 gene" or "hsa-miR-6087" used herein includes the hsa-miR-6087 gene (miRBase Accession No. MIMAT0023712) shown by SEQ ID NO: 148, a homolog or an ortholog of a different organism species, and the like. The hsa-miR-6087 gene can be obtained by a method described in Yoo JK et al., 2012, Stem Cells Dev., Vol. 21, pp. 2049-2057. Also, "hsa-mir-6087" (miRBase Accession No. MI0020364; SEQ ID NO: 359) having a hairpin-like structure is known as a precursor of "hsa-miR-6087."

The term "hsa-miR-6765-5p gene" or "hsa-miR-6765-5p" used herein includes the hsa-miR-6765-5p gene (miRBase Accession No. MIMAT0027430) shown by SEQ ID NO: 149, a homolog or an ortholog of a different organism species, and the like. The hsa-miR-6765-5p gene can be obtained by a method described in Ladewig E et al., 2012, Genome Res., Vol. 22, pp. 1634-1645. Also, "hsa-mir-6765" (miRBase Accession No. MI0022610; SEQ ID NO: 360) having a hairpin-like structure is known as a precursor of "hsa-miR-6765-5p."

The term "hsa-miR-6875-5p gene" or "hsa-miR-6875-5p" used herein includes the hsa-miR-6875-5p gene (miRBase Accession No. MIMAT0027650) shown by SEQ ID NO: 150, a homolog or an ortholog of a different organism species, and the like. The hsa-miR-6875-5p gene can be obtained by a method described in Ladewig E et al., 2012, Genome Res., Vol. 22, pp. 1634-1645. Also, "hsa-mir-6875" (miRBase Accession No. MI0022722; SEQ ID NO: 361) having a hairpin-like structure is known as a precursor of "hsa-miR-6875-5p."

The term "hsa-miR-4674 gene" or "hsa-miR-4674" used herein includes the hsa-miR-4674 gene (miRBase Accession No. MIMAT0019756) shown by SEQ ID NO: 151, a homolog or an ortholog of a different organism species, and the like. The hsa-miR-4674 gene can be obtained by a method described in Persson H et al., 2011, Cancer Res., Vol. 71, pp. 78-86. Also, "hsa-mir-4674" (miRBase Accession No. MI0017305; SEQ ID NO: 362) having a hairpin-like structure is known as a precursor of "hsa-miR-4674."

The term "hsa-miR-1233-5p gene" or "hsa-miR-1233-5p" used herein includes the hsa-miR-1233-Sp gene (miRBase Accession No. MIMAT0022943) shown by SEQ ID NO: 152, a homolog or an ortholog of a different organism species, and the like. The hsa-miR-1233-5p gene can be obtained by a method described in Berezikov E et al., 2007, Mol. Cell, Vol. 28, pp. 328-336. Also, "hsa-mir-1233-1 and hsa-mir-1233-2" (miRBase Accession Nos. MI0006323 and MI0015973; SEQ ID NOs: 363 and 364) each having a hairpin-like structure are known as precursors of "hsa-miR-1233-5p."

The term "hsa-miR-7114-5p gene" or "hsa-miR-7114-5p" used herein includes the hsa-miR-7114-5p gene (miRBase Accession No. MIMAT0028125) shown by SEQ ID NO: 153, a homolog or an ortholog of a different organism species, and the like. The hsa-miR-7114-5p gene can be obtained by a method described in Ladewig E et al., 2012, Genome Res., Vol. 22, pp. 1634-1645. Also, "hsa-mir-7114" (miRBase Accession No. MI0022965; SEQ ID NO: 365) having a hairpin-like structure is known as a precursor of "hsa-miR-7114-5p."

The term "hsa-miR-5787 gene" or "hsa-miR-5787" used herein includes the hsa-miR-5787 gene (miRBase Accession No. MIMAT0023252) shown by SEQ ID NO: 154, a homolog or an ortholog of a different organism species, and the like. The hsa-miR-5787 gene can be obtained by a method described in Yoo H et al., 2011, Biochem. Biophys. Res. Commun., Vol. 415, pp. 567-572. Also, "hsa-mir-5787" (miRBase Accession No. MI0019797; SEQ ID NO: 366) having a hairpin-like structure is known as a precursor of "hsa-miR-5787."

The term "hsa-miR-8072 gene" or "hsa-miR-8072" used herein includes the hsa-miR-8072 gene (miRBase Accession No. MIMAT0030999) shown by SEQ ID NO: 155, a homolog or an ortholog of a different organism species, and the like. The hsa-miR-8072 gene can be obtained by a method described in Wang HJ et al., 2013, Shock, Vol. 39, pp. 480-487. Also, "hsa-mir-8072" (miRBase Accession No. MI0025908; SEQ ID NO: 367) having a hairpin-like structure is known as a precursor of "hsa-miR-8072."

The term "hsa-miR-3619-3p gene" or "hsa-miR-3619-3p" used herein includes the hsa-miR-3619-3p gene (miRBase Accession No. MIMAT0019219) shown by SEQ ID NO: 156, a homolog or an ortholog of a different organism species, and the like. The hsa-miR-3619-3p gene can be obtained by a method described in Witten D et al., 2010, BMC Biol., Vol. 8, p. 58. Also, "hsa-mir-3619" (miRBase Accession No. MI0016009; SEQ ID NO: 368) having a hairpin-like structure is known as a precursor of "hsa-miR-3619-3p."

The term "hsa-miR-4632-5p gene" or "hsa-miR-4632-5p" used herein includes the hsa-miR-4632-5p gene (miRBase Accession No. MIMAT0022977) shown by SEQ ID NO: 157, a homolog or an ortholog of a different organism species, and the like. The hsa-miR-4632-5p gene can be obtained by a method described in Persson H et al., 2011, Cancer Res., Vol. 71, pp. 78-86. Also, "hsa-mir-4632" (miRBase Accession No. MI0017259; SEQ ID NO: 369) having a hairpin-like structure is known as a precursor of "hsa-miR-4632-5p."

The term "hsa-miR-6800-5p gene" or "hsa-miR-6800-5p" used herein includes the hsa-miR-6800-5p gene (miRBase Accession No. MIMAT0027500) shown by SEQ ID NO: 158, a homolog or an ortholog of a different organism species, and the like. The hsa-miR-6800-5p gene can be obtained by a method described in Ladewig E et al., 2012, Genome Res., Vol. 22, pp. 1634-1645. Also, "hsa-mir-6800" (miRBase Accession No. MI0022645; SEQ ID NO: 370) having a hairpin-like structure is known as a precursor of "hsa-miR-6800-5p."

The term "hsa-miR-4634 gene" or "hsa-miR-4634" used herein includes the hsa-miR-4634 gene (miRBase Accession No. MIMAT0019691) shown by SEQ ID NO: 159, a homolog or an ortholog of a different organism species, and the like. The hsa-miR-4634 gene can be obtained by a method described in Persson H et al., 2011, Cancer Res., Vol. 71, pp. 78-86. Also, "hsa-mir-4634" (miRBase Accession No. MI0017261; SEQ ID NO: 371) having a hairpin-like structure is known as a precursor of "hsa-miR-4634."

The term "hsa-miR-4486 gene" or "hsa-miR-4486" used herein includes the hsa-miR-4486 gene (miRBase Accession No. MIMAT0019020) shown by SEQ ID NO: 160, a homolog or an ortholog of a different organism species, and the like. The hsa-miR-4486 gene can be obtained by a method described in Jima DD et al., 2010, Blood, Vol. 116, e118-e127. Also, "hsa-mir-4486" (miRBase Accession No. MI0016847; SEQ ID NO: 372) having a hairpin-like structure is known as a precursor of "hsa-miR-4486."

The term "hsa-miR-6727-5p gene" or "hsa-miR-6727-5p" used herein includes the hsa-miR-6727-5p gene (miRBase Accession No. MIMAT0027355) shown by SEQ ID NO: 161, a homolog or an ortholog of a different organism species, and the like. The hsa-miR-6727-5p gene can be obtained by a method described in Ladewig E et al., 2012, Genome Res., Vol. 22, pp. 1634-1645. Also, "hsa-mir-6727" (miRBase Accession No. MI0022572; SEQ ID NO: 373) having a hairpin-like structure is known as a precursor of "hsa-miR-6727-5p."

The term "hsa-miR-4505 gene" or "hsa-miR-4505" used herein includes the hsa-miR-4505 gene (miRBase Accession No. MIMAT0019041) shown by SEQ ID NO: 162, a homolog or an ortholog of a different organism species, and the like. The hsa-miR-4505 gene can be obtained by a method described in Jima DD et al., 2010, Blood, Vol. 116, e118-e127. Also, "hsa-mir-4505" (miRBase Accession No. MI0016868; SEQ ID NO: 374) having a hairpin-like structure is known as a precursor of "hsa-miR-4505."

The term "hsa-miR-4725-3p gene" or "hsa-miR-4725-3p" used herein includes the hsa-miR-4725-3p gene (miRBase Accession No. MIMAT0019844) shown by SEQ ID NO: 163, a homolog or an ortholog of a different organism species, and the like. The hsa-miR-4725-3p gene can be obtained by a method described in Persson H et al., 2011, Cancer Res., Vol. 71, pp. 78-86. Also, "hsa-mir-4725" (miRBase Accession No. MI0017362; SEQ ID NO: 375) having a hairpin-like structure is known as a precursor of "hsa-miR-4725-3p."

The term "hsa-miR-1538 gene" or "hsa-miR-1538" used herein includes the hsa-miR-1538 gene (miRBase Accession No. MIMAT0007400) shown by SEQ ID NO: 164, a homolog or an ortholog of a different organism species, and the like. The hsa-miR-1538 gene can be obtained by a method described in Azuma-Mukai, A et al., 2008, Proc. Natl. Acad. Sci. U.S.A., Vol. 105, pp. 7964-7969. Also, "hsa-mir-1538" (miRBase Accession No. MI0007259; SEQ ID NO: 376) having a hairpin-like structure is known as a precursor of "hsa-miR-1538."

The term "hsa-miR-320b gene" or "hsa-miR-320b" used herein includes the hsa-miR-320b gene (miRBase Accession No. MIMAT0005792) shown by SEQ ID NO: 165, a homolog or an ortholog of a different organism species, and the like. The hsa-miR-320b gene can be obtained by a method described in Berezikov E et al., 2006, Genome Res., Vol. 16, pp. 1289-1298. Also, "hsa-mir-320b-1 and hsa-mir-320b-2" (miRBase Accession Nos. MI0003776 and MI0003839; SEQ ID NOs: 377 and 378) each having a hairpin-like structure are known as precursors of "hsa-miR-320b."

The term "hsa-miR-1915-5p gene" or "hsa-miR-1915-5p" used herein includes the hsa-miR-1915-5p gene (miRBase Accession No. MIMAT0007891) shown by SEQ ID NO: 166, a homolog or an ortholog of a different organism species, and the like. The hsa-miR-1915-5p gene can be obtained by a method described in Bar M et al., 2008, Stem Cells, Vol. 26, pp. 2496-2505. Also, "hsa-mir-1915" (miRBase Accession No. MI0008336; SEQ ID NO: 379) having a hairpin-like structure is known as a precursor of "hsa-miR-1915-5p."

The term "hsa-miR-328-5p gene" or "hsa-miR-328-5p" used herein includes the hsa-miR-328-5p gene (miRBase Accession No. MIMAT0026486) shown by SEQ ID NO: 167, a homolog or an ortholog of a different organism species, and the like. The hsa-miR-328-5p gene can be obtained by a method described in Kim J et al., 2004, Proc. Natl. Acad. Sci. U.S.A., Vol. 101, pp. 360-365. Also, "hsa-mir-328" (miRBase Accession No. MI0000804; SEQ ID NO: 380) having a hairpin-like structure is known as a precursor of "hsa-miR-328-5p."

The term "hsa-miR-6820-5p gene" or "hsa-miR-6820-5p" used herein includes the hsa-miR-6820-5p gene (miRBase Accession No. MIMAT0027540) shown by SEQ ID NO: 168, a homolog or an ortholog of a different organism species, and the like. The hsa-miR-6820-5p gene can be obtained by a method described in Ladewig E et al., 2012, Genome Res., Vol. 22, pp. 1634-1645. Also, "hsa-mir-6820" (miRBase Accession No. MI0022665; SEQ ID NO: 381) having a hairpin-like structure is known as a precursor of "hsa-miR-6820-5p."

The term "hsa-miR-6726-5p gene" or "hsa-miR-6726-5p" used herein includes the hsa-miR-6726-5p gene (miRBase Accession No. MIMAT0027353) shown by SEQ ID NO: 169, a homolog or an ortholog of a different organism species, and the like. The hsa-miR-6726-5p gene can be obtained by a method described in Ladewig E et al., 2012, Genome Res., Vol. 22, pp. 1634-1645. Also, "hsa-mir-6726" (miRBase Accession No. MI0022571; SEQ ID NO: 382) having a hairpin-like structure is known as a precursor of "hsa-miR-6726-5p."

The term "hsa-miR-3665 gene" or "hsa-miR-3665" used herein includes the hsa-miR-3665 gene (miRBase Accession No. MIMAT0018087) shown by SEQ ID NO: 170, a homolog or an ortholog of a different organism species, and the like. The hsa-miR-3665 gene can be obtained by a method described in Xie X et al., 2005, Nature, Vol. 434, pp. 338-345. Also, "hsa-mir-3665" (miRBase Accession No. MI0016066; SEQ ID NO: 383) having a hairpin-like structure is known as a precursor of "hsa-miR-3665."

The term "hsa-miR-638 gene" or "hsa-miR-638" used herein includes the hsa-miR-638 gene (miRBase Accession No. MIMAT0003308) shown by SEQ ID NO: 171, a homolog or an ortholog of a different organism species, and the like. The hsa-miR-638 gene can be obtained by a method described in Cummins JM et al., 2006, Proc. Natl. Acad. Sci., U.S.A., Vol. 103, pp. 3687-3692. Also, "hsa-mir-638" (miRBase Accession No. MI0003653; SEQ ID NO: 384) having a hairpin-like structure is known as a precursor of "hsa-miR-638."

The term "hsa-miR-762 gene" or "hsa-miR-762" used herein includes the hsa-miR-762 gene (miRBase Accession No. MIMAT0010313) shown by SEQ ID NO: 172, a homolog or an ortholog of a different organism species, and the like. The hsa-miR-762 gene can be obtained by a method described in Berezikov E et al., 2006, Genome Res., Vol.

16, pp. 1289-1298. Also, "hsa-mir-762" (miRBase Accession No. MI0003892; SEQ ID NO: 385) having a hairpin-like structure is known as a precursor of "hsa-miR-762."

The term "hsa-miR-4466 gene" or "hsa-miR-4466" used herein includes the hsa-miR-4466 gene (miRBase Accession No. MIMAT0018993) shown by SEQ ID NO: 173, a homolog or an ortholog of a different organism species, and the like. The hsa-miR-4466 gene can be obtained by a method described in Jima DD et al., 2010, Blood, Vol. 116, e118-e127. Also, "hsa-mir-4466" (miRBase Accession No. MI0016817; SEQ ID NO: 386) having a hairpin-like structure is known as a precursor of "hsa-miR-4466."

The term "hsa-miR-3940-5p gene" or "hsa-miR-3940-5p" used herein includes the hsa-miR-3940-5p gene (miRBase Accession No. MIMAT0019229) shown by SEQ ID NO: 174, a homolog or an ortholog of a different organism species, and the like. The hsa-miR-3940-5p gene can be obtained by a method described in Liao JY et al., 2010, PLoS One, Vol. 5, e10563. Also, "hsa-mir-3940" (miRBase Accession No. MI0016597; SEQ ID NO: 387) having a hairpin-like structure is known as a precursor of "hsa-miR-3940-5p."

The term "hsa-miR-1237-5p gene" or "hsa-miR-1237-5p" used herein includes the hsa-miR-1237-5p gene (miRBase Accession No. MIMAT0022946) shown by SEQ ID NO: 175, a homolog or an ortholog of a different organism species, and the like. The hsa-miR-1237-5p gene can be obtained by a method described in Berezikov E et al., 2007, Mol. Cell, Vol. 28, pp. 328-336. Also, "hsa-mir-1237" (miRBase Accession No. MI0006327; SEQ ID NO: 388) having a hairpin-like structure is known as a precursor of "hsa-miR-1237-5p."

The term "hsa-miR-575 gene" or "hsa-miR-575" used herein includes the hsa-miR-575 gene (miRBase Accession No. MIMAT0003240) shown by SEQ ID NO: 176, a homolog or an ortholog of a different organism species, and the like. The hsa-miR-575 gene can be obtained by a method described in Cummins JM et al., 2006, Proc. Natl. Acad. Sci., U.S.A., Vol. 103, pp. 3687-3692. Also, "hsa-mir-575" (miRBase Accession No. MI0003582; SEQ ID NO: 389) having a hairpin-like structure is known as a precursor of "hsa-miR-575."

The term "hsa-miR-3656 gene" or "hsa-miR-3656" used herein includes the hsa-miR-3656 gene (miRBase Accession No. MIMAT0018076) shown by SEQ ID NO: 177, a homolog or an ortholog of a different organism species, and the like. The hsa-miR-3656 gene can be obtained by a method described in Meiri E et al., 2010, Nucleic Acids Res., Vol. 38, pp. 6234-6246. Also, "hsa-mir-3656" (miRBase Accession No. MI0016056; SEQ ID NO: 390) having a hairpin-like structure is known as a precursor of "hsa-miR-3656."

The term "hsa-miR-4488 gene" or "hsa-miR-4488" used herein includes the hsa-miR-4488 gene (miRBase Accession No. MIMAT0019022) shown by SEQ ID NO: 178, a homolog or an ortholog of a different organism species, and the like. The hsa-miR-4488 gene can be obtained by a method described in Jima DD et al., 2010, Blood, Vol. 116, e118-e127. Also, "hsa-mir-4488" (miRBase Accession No. MI0016849; SEQ ID NO: 391) having a hairpin-like structure is known as a precursor of "hsa-miR-4488."

The term "hsa-miR-4281 gene" or "hsa-miR-4281" used herein includes the hsa-miR-4281 gene (miRBase Accession No. MIMAT0016907) shown by SEQ ID NO: 179, a homolog or an ortholog of a different organism species, and the like. The hsa-miR-4281 gene can be obtained by a method described in Goff LA et al., 2009, PLoS One, Vol. 4, e7192. Also, "hsa-mir-4281" (miRBase Accession No. MI0015885; SEQ ID NO: 392) having a hairpin-like structure is known as a precursor of "hsa-miR-4281."

The term "hsa-miR-6781-5p gene" or "hsa-miR-6781-5p" used herein includes the hsa-miR-6781-5p gene (miRBase Accession No. MIMAT0027462) shown by SEQ ID NO: 180, a homolog or an ortholog of a different organism species, and the like. The hsa-miR-6781-5p gene can be obtained by a method described in Ladewig E et al., 2012, Genome Res., Vol. 22, pp. 1634-1645. Also, "hsa-mir-6781" (miRBase Accession No. MI0022626; SEQ ID NO: 393) having a hairpin-like structure is known as a precursor of "hsa-miR-6781-5p."

The term "hsa-miR-4532 gene" or "hsa-miR-4532" used herein includes the hsa-miR-4532 gene (miRBase Accession No. MIMAT0019071) shown by SEQ ID NO: 181, a homolog or an ortholog of a different organism species, and the like. The hsa-miR-4532 gene can be obtained by a method described in Jima DD et al., 2010, Blood, Vol. 116, e118-e127. Also, "hsa-mir-4532" (miRBase Accession No. MI0016899; SEQ ID NO: 394) having a hairpin-like structure is known as a precursor of "hsa-miR-4532."

The term "hsa-miR-4665-5p gene" or "hsa-miR-4665-5p" used herein includes the hsa-miR-4665-5p gene (miRBase Accession No. MIMAT0019739) shown by SEQ ID NO: 182, a homolog or an ortholog of a different organism species, and the like. The hsa-miR-4665-5p gene can be obtained by a method described in Persson H et al., 2011, Cancer Res., Vol. 71, pp. 78-86. Also, "hsa-mir-4665" (miRBase Accession No. MI0017295; SEQ ID NO: 395) having a hairpin-like structure is known as a precursor of "hsa-miR-4665-5p."

The term "hsa-miR-6816-5p gene" or "hsa-miR-6816-5p" used herein includes the hsa-miR-6816-5p gene (miRBase Accession No. MIMAT0027532) shown by SEQ ID NO: 183, a homolog or an ortholog of a different organism species, and the like. The hsa-miR-6816-5p gene can be obtained by a method described in Ladewig E et al., 2012, Genome Res., Vol. 22, pp. 1634-1645. Also, "hsa-mir-6816" (miRBase Accession No. MI0022661; SEQ ID NO: 396) having a hairpin-like structure is known as a precursor of "hsa-miR-6816-5p."

The term "hsa-miR-4508 gene" or "hsa-miR-4508" used herein includes the hsa-miR-4508 gene (miRBase Accession No. MIMAT0019045) shown by SEQ ID NO: 184, a homolog or an ortholog of a different organism species, and the like. The hsa-miR-4508 gene can be obtained by a method described in Jima DD et al., 2010, Blood, Vol. 116, e118-e127. Also, "hsa-mir-4508" (miRBase Accession No. MI0016872; SEQ ID NO: 397) having a hairpin-like structure is known as a precursor of "hsa-miR-4508."

The term "hsa-miR-6784-5p gene" or "hsa-miR-6784-5p" used herein includes the hsa-miR-6784-5p gene (miRBase Accession No. MIMAT0027468) shown by SEQ ID NO: 185, a homolog or an ortholog of a different organism species, and the like. The hsa-miR-6784-5p gene can be obtained by a method described in Ladewig E et al., 2012, Genome Res., Vol. 22, pp. 1634-1645. Also, "hsa-mir-6784" (miRBase Accession No. MI0022629; SEQ ID NO: 398) having a hairpin-like structure is known as a precursor of "hsa-miR-6784-5p."

The term "hsa-miR-6786-5p gene" or "hsa-miR-6786-5p" used herein includes the hsa-miR-6786-5p gene (miRBase Accession No. MIMAT0027472) shown by SEQ ID NO: 186, a homolog or an ortholog of a different organism species, and the like. The hsa-miR-6786-5p gene can be obtained by a method described in Ladewig E et al., 2012, Genome Res., Vol. 22, pp. 1634-1645. Also, "hsa-mir-6786" (miRBase Accession No. MI0022631; SEQ ID NO: 399) having a hairpin-like structure is known as a precursor of "hsa-miR-6786-5p."

The term "hsa-miR-4741 gene" or "hsa-miR-4741" used herein includes the hsa-miR-4741 gene (miRBase Accession No. MIMAT0019871) shown by SEQ ID NO: 187, a homolog or an ortholog of a different organism species, and the like. The hsa-miR-4741 gene can be obtained by a method described in Persson H et al., 2011, Cancer Res., Vol. 71, pp. 78-86. Also, "hsa-mir-4741" (miRBase Accession No. MI0017379; SEQ ID NO: 400) having a hairpin-like structure is known as a precursor of "hsa-miR-4741."

The term "hsa-miR-1343-5p gene" or "hsa-miR-1343-5p" used herein includes the hsa-miR-1343-5p gene (miRBase Accession No. MIMAT0027038) shown by SEQ ID NO: 188, a homolog or an ortholog of a different organism species, and the like. The hsa-miR-1343-5p gene can be obtained by a method described in Persson H et al., 2011, Cancer Res., Vol. 71, pp. 78-86. Also, "hsa-mir-1343" (miRBase Accession No. MI0017320; SEQ ID NO: 401) having a hairpin-like structure is known as a precursor of "hsa-miR-1343-5p."

The term "hsa-miR-1227-5p gene" or "hsa-miR-1227-5p" used herein includes the hsa-miR-1227-Sp gene (miRBase Accession No. MIMAT0022941) shown by SEQ ID NO: 189, a homolog or an ortholog of a different organism species, and the like. The hsa-miR-1227-5p gene can be obtained by a method described in Berezikov E et al., 2007, Mol. Cell, Vol. 28, pp. 328-336. Also, "hsa-mir-1227" (miRBase Accession No. MI0006316; SEQ ID NO: 402) having a hairpin-like structure is known as a precursor of "hsa-miR-1227-5p."

The term "hsa-miR-4734 gene" or "hsa-miR-4734" used herein includes the hsa-miR-4734 gene (miRBase Accession No. MIMAT0019859) shown by SEQ ID NO: 190, a homolog or an ortholog of a different organism species, and the like. The hsa-miR-4734 gene can be obtained by a method described in Persson H et al., 2011, Cancer Res., Vol. 71, pp. 78-86. Also, "hsa-mir-4734" (miRBase Accession No. MI0017371; SEQ ID NO: 403) having a hairpin-like structure is known as a precursor of "hsa-miR-4734."

The term "hsa-miR-3960 gene" or "hsa-miR-3960" used herein includes the hsa-miR-3960 gene (miRBase Accession No. MIMAT0019337) shown by SEQ ID NO: 191, a homolog or an ortholog of a different organism species, and the like. The hsa-miR-3960 gene can be obtained by a method described in Hu R et al., 2011, J. Biol. Chem., Vol. 286, pp. 12328-12339. Also, "hsa-mir-3960" (miRBase Accession No. MI0016964; SEQ ID NO: 404) having a hairpin-like structure is known as a precursor of "hsa-miR-3960."

The term "hsa-miR-128-2-5p gene" or "hsa-miR-128-2-5p" used herein includes the hsa-miR-128-2-5p gene (miRBase Accession No. MIMAT0031095) shown by SEQ ID NO: 192, a homolog or an ortholog of a different organism species, and the like. The hsa-miR-128-2-5p gene can be obtained by a method described in Lagos-Quintana M et al., 2002, Curr. Biol., Vol. 12, pp. 735-739. Also, "hsa-mir-128-2" (miRBase Accession No. MI0000727; SEQ ID NO: 405) having a hairpin-like structure is known as a precursor of "hsa-miR-128-2-5p."

The term "hsa-miR-6743-5p gene" or "hsa-miR-6743-5p" used herein includes the hsa-miR-6743-Sp gene (miRBase Accession No. MIMAT0027387) shown by SEQ ID NO: 193, a homolog or an ortholog of a different organism species, and the like. The hsa-miR-6743-5p gene can be obtained by a method described in Ladewig E et al., 2012, Genome Res., Vol. 22, pp. 1634-1645. Also, "hsa-mir-6743" (miRBase Accession No. MI0022588; SEQ ID NO: 406) having a hairpin-like structure is known as a precursor of "hsa-miR-6743-5p."

The term "hsa-miR-663a gene" or "hsa-miR-663a" used herein includes the hsa-miR-663a gene (miRBase Accession No. MIMAT0003326) shown by SEQ ID NO: 194, a homolog or an ortholog of a different organism species, and the like. The hsa-miR-663a gene can be obtained by a method described in Cummins JM et al., 2006, Proc. Natl. Acad. Sci., U.S.A., Vol. 103, pp. 3687-3692. Also, "hsa-mir-663a" (miRBase Accession No. MI0003672; SEQ ID NO: 407) having a hairpin-like structure is known as a precursor of "hsa-miR-663a."

The term "hsa-miR-6729-5p gene" or "hsa-miR-6729-5p" used herein includes the hsa-miR-6729-5p gene (miRBase Accession No. MIMAT0027359) shown by SEQ ID NO: 195, a homolog or an ortholog of a different organism species, and the like. The hsa-miR-6729-5p gene can be obtained by a method described in Ladewig E et al., 2012, Genome Res., Vol. 22, pp. 1634-1645. Also, "hsa-mir-6729" (miRBase Accession No. MI0022574; SEQ ID NO: 408) having a hairpin-like structure is known as a precursor of "hsa-miR-6729-5p."

The term "hsa-miR-1915-3p gene" or "hsa-miR-1915-3p" used herein includes the hsa-miR-1915-3p gene (miRBase Accession No. MIMAT0007892) shown by SEQ ID NO: 196, a homolog or an ortholog of a different organism species, and the like. The hsa-miR-1915-3p gene can be obtained by a method described in Bar M et al., 2008, Stem Cells, Vol. 26, pp. 2496-2505. Also, "hsa-mir-1915" (miRBase Accession No. MI0008336; SEQ ID NO: 379) having a hairpin-like structure is known as a precursor of "hsa-miR-1915-3p."

The term "hsa-miR-1268b gene" or "hsa-miR-1268b" used herein includes the hsa-miR-1268b gene (miRBase Accession No. MIMAT0018925) shown by SEQ ID NO: 197, a homolog or an ortholog of a different organism species, and the like. The hsa-miR-1268b gene can be obtained by a method described in Jima DD et al., 2010, Blood, Vol. 116, e118-e127. Also, "hsa-mir-1268b" (miRBase Accession No. MI0016748; SEQ ID NO: 409) having a hairpin-like structure is known as a precursor of "hsa-miR-1268b."

The term "hsa-miR-4651 gene" or "hsa-miR-4651" used herein includes the hsa-miR-4651 gene (miRBase Accession No. MIMAT0019715) shown by SEQ ID NO: 198, a homolog or an ortholog of a different organism species, and the like. The hsa-miR-4651 gene can be obtained by a method described in Persson H et al., 2011, Cancer Res., Vol. 71, pp. 78-86. Also, "hsa-mir-4651" (miRBase Accession No. MI0017279; SEQ ID NO: 410) having a hairpin-like structure is known as a precursor of "hsa-miR-4651."

The term "hsa-miR-3178 gene" or "hsa-miR-3178" used herein includes the hsa-miR-3178 gene (miRBase Accession No. MIMAT0015055) shown by SEQ ID NO: 199, a homolog or an ortholog of a different organism species, and the like. The hsa-miR-3178 gene can be obtained by a method described in Stark MS et al., 2010, PLoS One, Vol. 5, e9685. Also, "hsa-mir-3178" (miRBase Accession No. MI0014212; SEQ ID NO: 411) having a hairpin-like structure is known as a precursor of "hsa-miR-3178."

The term "hsa-miR-4463 gene" or "hsa-miR-4463" used herein includes the hsa-miR-4463 gene (miRBase Accession No. MIMAT0018987) shown by SEQ ID NO: 200, a homolog or an ortholog of a different organism species, and the like. The hsa-miR-4463 gene can be obtained by a method described in Jima DD et al., 2010, Blood, Vol. 116, e118-e127. Also, "hsa-mir-4463" (miRBase Accession No. MI0016811; SEQ ID NO: 412) having a hairpin-like structure is known as a precursor of "hsa-miR-4463."

A mature miRNA may become a variant due to the sequence cleaved shorter or longer by one to several flanking nucleotides, or due to substitution of nucleotides, when cut out as the mature miRNA from its RNA precursor having a hairpin-like structure. Such variant is called isomiR (Morin RD. et al., 2008, Genome Res., Vol. 18, pp. 610-621). The miRBase (version 21) shows the nucleotide sequences shown by SEQ ID NOs: 1 to 200 as well as a large number of variants and fragments thereof, called isomiRs, which have the nucleotide sequences shown by SEQ ID NOs: 413 to 740. These variants can also be obtained as variants derived from miRNAshaving the nucleotide sequences shown by SEQ ID NOs: 1 to 200.

Specifically, among the variants of polynucleotides each consisting of the nucleotide sequence shown by any of SEQ ID NOs: 1 to 17, 19 to 22, 24 to 27, 39 to 42, 44 to 48, 50 to 52, 54 to 64, 66, 68, 69, 71, 72, 74 to 77, 79 to 92, 94 to 97, 99, 102, 104 to 106, 109 to 125, 127, 128, 130, 132 to 143, 145, 150 to 153, 155, 156, 158 to 170, 173 to 176, 178, 180, 182 to 184, 187, 188, and 190 to 200 or nucleotide sequences derived from the nucleotide sequences by the replacement of u with t according to the present invention, the polynucleotides shown by SEQ ID NOs: 413, 415, 417, 419, 421, 423, 425, 427, 429, 431, 433, 435, 437, 439, 441, 443, 445, 447, 449, 451, 453, 455, 457, 459, 461, 463, 465, 467, 469, 471, 473, 475, 477, 479, 481, 483, 485, 487, 489, 491, 493, 495, 497, 499, 501, 503, 505, 507, 509, 511, 513, 515, 517, 519, 521, 523, 525, 527, 529, 531, 533, 535, 537, 539, 541, 543, 545, 547, 549, 551, 553, 555, 557, 559, 561, 563, 565, 567, 569, 571, 573, 575, 577, 579, 581, 583, 585, 587, 589, 591, 593, 595, 597, 599, 601, 603, 605, 607, 609, 611, 613, 615, 617, 619, 621, 623, 625, 627, 629, 631, 633, 635, 637, 639, 641, 643, 645, 647, 649, 651, 653, 655, 657, 659, 661, 663, 665, 667, 669, 671, 673, 675, 677, 679, 681, 683, 685, 687, 689, 691, 693, 695, 697, 699, 701, 703, 705, 707, 709, 711, 713, 715, 717, 719, 721, 723, 725, 727, 729, 731, 733, 735, 737, and 739 are cited, for example, as the longest variants registered in miRBase (version 21). Also among them, polynucleotides having sequences shown by SEQ ID NOs: 414, 416, 418, 420, 422, 424, 426, 428, 430, 432, 434, 436, 438, 440, 442, 444, 446, 448, 450, 452, 454, 456, 458, 460, 462, 464, 466, 468, 470, 472, 474, 476, 478, 480, 482, 484, 486, 488, 490, 492, 494, 496, 498, 500, 502, 504, 506, 508, 510, 512, 514, 516, 518, 520, 522, 524, 526, 528, 530, 532, 534, 536, 538, 540, 542, 544, 546, 548, 550, 552, 554, 556, 558, 560, 562, 564, 566, 568, 570, 572, 574, 576, 578, 580, 582, 584, 586, 588, 590, 592, 594, 596, 598, 600, 602, 604, 606, 608, 610, 612, 614, 616, 618, 620, 622, 624, 626, 628, 630, 632, 634, 636, 638, 640, 642, 644, 646, 648, 650, 652, 654, 656, 658, 660, 662, 664, 666, 668, 670, 672, 674, 676, 678, 680, 682, 684, 686, 688, 690, 692, 694, 696, 698, 700, 702, 704, 706, 708, 710, 712, 714, 716, 718, 720, 722, 724, 726, 728, 730, 732, 734, 736, 738, and 740 are cited as the shortest variants. In addition to these variants and fragments, a large number of isomiR polynucleotides corresponding to miRNAs having the nucleotide sequences shown by SEQ ID NOs: 1 to 200 are registered in the miRBase. Also, examples of polynucleotides comprising the nucleotide sequences shown by SEQ ID NOs: 1 to 200 also include polynucleotides shown by SEQ ID NOs: 201 to 412, which are precursors of the former nucleotide sequences.

The names and miRBase Accession Nos. (registration numbers) of genes/miRNAs comprising the nucleotide sequences shown by SEQ ID NOs: 1 to 740 are shown in Table 1.

As used herein, the phrase "capable of specifically binding" means that the nucleic acid probe or the primer used in the present invention is able to bind to a specific target nucleic acid and is substantially unable to bind to other nucleic acids.

Table 1

| SEQ ID NO: | Name of gene | miRBase Accession No. |
|---|---|---|
| 1 | hsa-miR-3131 | MIMAT0014996 |
| 2 | hsa-miR-6757-5p | MIMAT0027414 |
| 3 | hsa-miR-4706 | MIMAT0019806 |
| 4 | hsa-miR-5001-5p | MIMAT0021021 |
| 5 | hsa-miR-3180-3p | MIMAT0015058 |
| 6 | hsa-miR-642b-3p | MIMAT0018444 |
| 7 | hsa-miR-4655-5p | MIMAT0019721 |
| 8 | hsa-miR-6819-5p | MIMAT0027538 |
| 9 | hsa-miR-937-5p | MIMAT0022938 |
| 10 | hsa-miR-4688 | MIMAT0019777 |
| 11 | hsa-miR-6741-5p | MIMAT0027383 |
| 12 | hsa-miR-7107-5p | MIMAT0028111 |
| 13 | hsa-miR-4271 | MIMAT0016901 |
| 14 | hsa-miR-1229-5p | MIMAT0022942 |
| 15 | hsa-miR-4707-5p | MIMAT0019807 |
| 16 | hsa-miR-6808-5p | MIMAT0027516 |
| 17 | hsa-miR-4656 | MIMAT0019723 |
| 18 | hsa-miR-6076 | MIMAT0023701 |
| 19 | hsa-miR-6762-5p | MIMAT0027424 |
| 20 | hsa-miR-7109-5p | MIMAT0028115 |
| 21 | hsa-miR-6732-5p | MIMAT0027365 |
| 22 | hsa-miR-3195 | MIMAT0015079 |
| 23 | hsa-miR-7150 | MIMAT0028211 |
| 24 | hsa-miR-642a-3p | MIMAT0020924 |
| 25 | hsa-miR-1249-5p | MIMAT0032029 |
| 26 | hsa-miR-3185 | MIMAT0015065 |
| 27 | hsa-miR-4689 | MIMAT0019778 |
| 28 | hsa-miR-3141 | MIMAT0015010 |
| 29 | hsa-miR-6840-3p | MIMAT0027583 |
| 30 | hsa-miR-3135b | MIMAT0018985 |
| 31 | hsa-miR-1914-3p | MIMAT0007890 |
| 32 | hsa-miR-4446-3p | MIMAT0018965 |
| 33 | hsa-miR-4433b-3p | MIMAT0030414 |
| 34 | hsa-miR-6877-5p | MIMAT0027654 |
| 35 | hsa-miR-6848-5p | MIMAT0027596 |

(continued)

| SEQ ID NO: | Name of gene | miRBase Accession No. |
|---|---|---|
| 36 | hsa-miR-3620-5p | MIMAT0022967 |
| 37 | hsa-miR-6825-5p | MIMAT0027550 |
| 38 | hsa-miR-5739 | MIMAT0023116 |
| 39 | hsa-miR-3663-3p | MIMAT0018085 |
| 40 | hsa-miR-4695-5p | MIMAT0019788 |
| 41 | hsa-miR-3162-5p | MIMAT0015036 |
| 42 | hsa-miR-3679-5p | MIMAT0018104 |
| 43 | hsa-miR-8059 | MIMAT0030986 |
| 44 | hsa-miR-7110-5p | MIMAT0028117 |
| 45 | hsa-miR-1275 | MIMAT0005929 |
| 46 | hsa-miR-6779-5p | MIMAT0027458 |
| 47 | hsa-miR-197-5p | MIMAT0022691 |
| 48 | hsa-miR-6845-5p | MIMAT0027590 |
| 49 | hsa-miR-4327 | MIMAT0016889 |
| 50 | hsa-miR-4723-5p | MIMAT0019838 |
| 51 | hsa-miR-4530 | MIMAT0019069 |
| 52 | hsa-miR-6771-5p | MIMAT0027442 |
| 53 | hsa-miR-614 | MIMAT0003282 |
| 54 | hsa-miR-92a-2-5p | MIMAT0004508 |
| 55 | hsa-miR-6891-5p | MIMAT0027682 |
| 56 | hsa-miR-6124 | MIMAT0024597 |
| 57 | hsa-miR-4687-3p | MIMAT0019775 |
| 58 | hsa-miR-4442 | MIMAT0018960 |
| 59 | hsa-miR-7977 | MIMAT0031180 |
| 60 | hsa-miR-6785-5p | MIMAT0027470 |
| 61 | hsa-miR-4497 | MIMAT0019032 |
| 62 | hsa-miR-8071 | MIMAT0030998 |
| 63 | hsa-miR-663b | MIMAT0005867 |
| 64 | hsa-miR-3180 | MIMAT0018178 |
| 65 | hsa-miR-4251 | MIMAT0016883 |
| 66 | hsa-miR-1285-3p | MIMAT0005876 |
| 67 | hsa-miR-6870-5p | MIMAT0027640 |
| 68 | hsa-miR-4484 | MIMAT0019018 |
| 69 | hsa-miR-4476 | MIMAT0019003 |
| 70 | hsa-miR-6749-5p | MIMAT0027398 |
| 71 | hsa-miR-4454 | MIMAT0018976 |
| 72 | hsa-miR-6893-5p | MIMAT0027686 |
| 73 | hsa-miR-6085 | MIMAT0023710 |

(continued)

| SEQ ID NO: | Name of gene | miRBase Accession No. |
|---|---|---|
| 74 | hsa-miR-4787-5p | MIMAT0019956 |
| 75 | hsa-miR-149-3p | MIMAT0004609 |
| 76 | hsa-miR-7704 | MIMAT0030019 |
| 77 | hsa-miR-6125 | MIMAT0024598 |
| 78 | hsa-miR-6090 | MIMAT0023715 |
| 79 | hsa-miR-3197 | MIMAT0015082 |
| 80 | hsa-miR-6850-5p | MIMAT0027600 |
| 81 | hsa-miR-4467 | MIMAT0018994 |
| 82 | hsa-miR-6885-5p | MIMAT0027670 |
| 83 | hsa-miR-6803-5p | MIMAT0027506 |
| 84 | hsa-miR-6798-5p | MIMAT0027496 |
| 85 | hsa-miR-6780b-5p | MIMAT0027572 |
| 86 | hsa-miR-6768-5p | MIMAT0027436 |
| 87 | hsa-miR-5100 | MIMAT0022259 |
| 88 | hsa-miR-6724-5p | MIMAT0025856 |
| 89 | hsa-miR-6879-5p | MIMAT0027658 |
| 90 | hsa-miR-7108-5p | MIMAT0028113 |
| 91 | hsa-miR-4649-5p | MIMAT0019711 |
| 92 | hsa-miR-4739 | MIMAT0019868 |
| 93 | hsa-miR-6089 | MIMAT0023714 |
| 94 | hsa-miR-1908-5p | MIMAT0007881 |
| 95 | hsa-miR-4516 | MIMAT0019053 |
| 96 | hsa-miR-2861 | MIMAT0013802 |
| 97 | hsa-miR-4492 | MIMAT0019027 |
| 98 | hsa-miR-4294 | MIMAT0016849 |
| 99 | hsa-miR-6791-5p | MIMAT0027482 |
| 100 | hsa-miR-1469 | MIMAT0007347 |
| 101 | hsa-miR-6752-5p | MIMAT0027404 |
| 102 | hsa-miR-4730 | MIMAT0019852 |
| 103 | hsa-miR-6126 | MIMAT0024599 |
| 104 | hsa-miR-6869-5p | MIMAT0027638 |
| 105 | hsa-miR-1268a | MIMAT0005922 |
| 106 | hsa-miR-6799-5p | MIMAT0027498 |
| 107 | hsa-miR-8069 | MIMAT0030996 |
| 108 | hsa-miR-3621 | MIMAT0018002 |
| 109 | hsa-miR-4763-3p | MIMAT0019913 |
| 110 | hsa-miR-1228-5p | MIMAT0005582 |
| 111 | hsa-miR-760 | MIMAT0004957 |

(continued)

| SEQ ID NO: | Name of gene | miRBase Accession No. |
|---|---|---|
| 112 | hsa-miR-187-5p | MIMAT0004561 |
| 113 | hsa-miR-7111-5p | MIMAT0028119 |
| 114 | hsa-miR-6088 | MIMAT0023713 |
| 115 | hsa-miR-6805-3p | MIMAT0027511 |
| 116 | hsa-miR-4640-5p | MIMAT0019699 |
| 117 | hsa-miR-6721-5p | MIMAT0025852 |
| 118 | hsa-miR-6880-5p | MIMAT0027660 |
| 119 | hsa-miR-711 | MIMAT0012734 |
| 120 | hsa-miR-128-1-5p | MIMAT0026477 |
| 121 | hsa-miR-4525 | MIMAT0019064 |
| 122 | hsa-miR-486-3p | MIMAT0004762 |
| 123 | hsa-miR-6756-5p | MIMAT0027412 |
| 124 | hsa-miR-1260b | MIMAT0015041 |
| 125 | hsa-miR-3184-5p | MIMAT0015064 |
| 126 | hsa-miR-6075 | MIMAT0023700 |
| 127 | hsa-miR-204-3p | MIMAT0022693 |
| 128 | hsa-miR-4728-5p | MIMAT0019849 |
| 129 | hsa-miR-4534 | MIMAT0019073 |
| 130 | hsa-miR-4758-5p | MIMAT0019903 |
| 131 | hsa-miR-8063 | MIMAT0030990 |
| 132 | hsa-miR-6836-3p | MIMAT0027575 |
| 133 | hsa-miR-6789-5p | MIMAT0027478 |
| 134 | hsa-miR-744-5p | MIMAT0004945 |
| 135 | hsa-miR-1909-3p | MIMAT0007883 |
| 136 | hsa-miR-887-3p | MIMAT0004951 |
| 137 | hsa-miR-4745-5p | MIMAT0019878 |
| 138 | hsa-miR-4433a-3p | MIMAT0018949 |
| 139 | hsa-miR-5090 | MIMAT0021082 |
| 140 | hsa-miR-296-5p | MIMAT0000690 |
| 141 | hsa-miR-939-5p | MIMAT0004982 |
| 142 | hsa-miR-3648 | MIMAT0018068 |
| 143 | hsa-miR-3196 | MIMAT0015080 |
| 144 | hsa-miR-6722-3p | MIMAT0025854 |
| 145 | hsa-miR-6805-5p | MIMAT0027510 |
| 146 | hsa-miR-1202 | MIMAT0005865 |
| 147 | hsa-miR-6775-5p | MIMAT0027450 |
| 148 | hsa-miR-6087 | MIMAT0023712 |
| 149 | hsa-miR-6765-5p | MIMAT0027430 |

(continued)

| SEQ ID NO: | Name of gene | miRBase Accession No. |
|---|---|---|
| 150 | hsa-miR-6875-5p | MIMAT0027650 |
| 151 | hsa-miR-4674 | MIMAT0019756 |
| 152 | hsa-miR-1233-5p | MIMAT0022943 |
| 153 | hsa-miR-7114-5p | MIMAT0028125 |
| 154 | hsa-miR-5787 | MIMAT0023252 |
| 155 | hsa-miR-8072 | MIMAT0030999 |
| 156 | hsa-miR-3619-3p | MIMAT0019219 |
| 157 | hsa-miR-4632-5p | MIMAT0022977 |
| 158 | hsa-miR-6800-5p | MIMAT0027500 |
| 159 | hsa-miR-4634 | MIMAT0019691 |
| 160 | hsa-miR-4486 | MIMAT0019020 |
| 161 | hsa-miR-6727-5p | MIMAT0027355 |
| 162 | hsa-miR-4505 | MIMAT0019041 |
| 163 | hsa-miR-4725-3p | MIMAT0019844 |
| 164 | hsa-miR-1538 | MIMAT0007400 |
| 165 | hsa-miR-320b | MIMAT0005792 |
| 166 | hsa-miR-1915-5p | MIMAT0007891 |
| 167 | hsa-miR-328-5p | MIMAT0026486 |
| 168 | hsa-miR-6820-5p | MIMAT0027540 |
| 169 | hsa-miR-6726-5p | MIMAT0027353 |
| 170 | hsa-miR-3665 | MIMAT0018087 |
| 171 | hsa-miR-63 8 | MIMAT0003308 |
| 172 | hsa-miR-762 | MIMAT0010313 |
| 173 | hsa-miR-4466 | MIMAT0018993 |
| 174 | hsa-miR-3940-5p | MIMAT0019229 |
| 175 | hsa-miR-1237-5p | MIMAT0022946 |
| 176 | hsa-miR-575 | MIMAT0003240 |
| 177 | hsa-miR-3656 | MIMAT0018076 |
| 178 | hsa-miR-4488 | MIMAT0019022 |
| 179 | hsa-miR-4281 | MIMAT0016907 |
| 180 | hsa-miR-6781-5p | MIMAT0027462 |
| 181 | hsa-miR-4532 | MIMAT0019071 |
| 182 | hsa-miR-4665-5p | MIMAT0019739 |
| 183 | hsa-miR-6816-5p | MIMAT0027532 |
| 184 | hsa-miR-4508 | MIMAT0019045 |
| 185 | hsa-miR-6784-5p | MIMAT0027468 |
| 186 | hsa-miR-6786-5p | MIMAT0027472 |
| 187 | hsa-miR-4741 | MIMAT0019871 |

(continued)

| SEQ ID NO: | Name of gene | miRBase Accession No. |
|---|---|---|
| 188 | hsa-miR-1343-5p | MIMAT0027038 |
| 189 | hsa-miR-1227-5p | MIMAT0022941 |
| 190 | hsa-miR-4734 | MIMAT0019859 |
| 191 | hsa-miR-3960 | MIMAT0019337 |
| 192 | hsa-miR-128-2-5p | MIMAT0031095 |
| 193 | hsa-miR-6743-5p | MIMAT00273 87 |
| 194 | hsa-miR-663a | MIMAT0003326 |
| 195 | hsa-miR-6729-5p | MIMAT0027359 |
| 196 | hsa-miR-1915-3p | MIMAT0007892 |
| 197 | hsa-miR-1268b | MIMAT0018925 |
| 198 | hsa-miR-4651 | MIMAT0019715 |
| 199 | hsa-miR-3178 | MIMAT0015055 |
| 200 | hsa-miR-4463 | MIMAT0018987 |
| 201 | hsa-mir-3131 | MI0014151 |
| 202 | hsa-mir-6757 | MI0022602 |
| 203 | hsa-mir-4706 | MI0017339 |
| 204 | hsa-mir-5001 | MI0017867 |
| 205 | hsa-mir-3180-1 | MI0014214 |
| 206 | hsa-mir-3180-2 | MI0014215 |
| 207 | hsa-mir-3180-3 | MI0014217 |
| 208 | hsa-mir-642b | MI0016685 |
| 209 | hsa-mir-4655 | MI0017283 |
| 210 | hsa-mir-6819 | MI0022664 |
| 211 | hsa-mir-937 | MI0005759 |
| 212 | hsa-mir-4688 | MI0017321 |
| 213 | hsa-mir-6741 | MI0022586 |
| 214 | hsa-mir-7107 | MI0022958 |
| 215 | hsa-mir-4271 | MI0015879 |
| 216 | hsa-mir-1229 | MI0006319 |
| 217 | hsa-mir-4707 | MI0017340 |
| 218 | hsa-mir-6808 | MI0022653 |
| 219 | hsa-mir-4656 | MI0017284 |
| 220 | hsa-mir-6076 | MI0020353 |
| 221 | hsa-mir-6762 | MI0022607 |
| 222 | hsa-mir-7109 | MI0022960 |
| 223 | hsa-mir-6732 | MI0022577 |
| 224 | hsa-mir-3195 | MI0014240 |
| 225 | hsa-mir-7150 | MI0023610 |

(continued)

| SEQ ID NO: | Name of gene | miRBase Accession No. |
|---|---|---|
| 226 | hsa-mir-642a | MI0003657 |
| 227 | hsa-mir-1249 | MI0006384 |
| 228 | hsa-mir-3185 | MI0014227 |
| 229 | hsa-mir-4689 | MI0017322 |
| 230 | hsa-mir-3141 | MI0014165 |
| 231 | hsa-mir-6840 | MI0022686 |
| 232 | hsa-mir-3135b | MI0016809 |
| 233 | hsa-mir-1914 | MI0008335 |
| 234 | hsa-mir-4446 | MI0016789 |
| 235 | hsa-mir-4433b | MI0025511 |
| 236 | hsa-mir-6877 | MI0022724 |
| 237 | hsa-mir-6848 | MI0022694 |
| 238 | hsa-mir-3620 | MI0016011 |
| 239 | hsa-mir-6825 | MI0022670 |
| 240 | hsa-mir-5739 | MI0019412 |
| 241 | hsa-mir-3663 | MI0016064 |
| 242 | hsa-mir-4695 | MI0017328 |
| 243 | hsa-mir-3162 | MI0014192 |
| 244 | hsa-mir-3679 | MI0016080 |
| 245 | hsa-mir-8059 | MI0025895 |
| 246 | hsa-mir-7110 | MI0022961 |
| 247 | hsa-mir-1275 | MI0006415 |
| 248 | hsa-mir-6779 | MI0022624 |
| 249 | hsa-mir-197 | MI0000239 |
| 250 | hsa-mir-6845 | MI0022691 |
| 251 | hsa-mir-4327 | MI0015867 |
| 252 | hsa-mir-4723 | MI00173 59 |
| 253 | hsa-mir-4530 | MI0016897 |
| 254 | hsa-mir-6771 | MI0022616 |
| 255 | hsa-mir-614 | MI0003627 |
| 256 | hsa-mir-92a-2 | MI0000094 |
| 257 | hsa-mir-6891 | MI0022738 |
| 258 | hsa-mir-6124 | MI0021258 |
| 259 | hsa-mir-4687 | MI0017319 |
| 260 | hsa-mir-4442 | MI0016785 |
| 261 | hsa-mir-7977 | MI0025753 |
| 262 | hsa-mir-6785 | MI0022630 |
| 263 | hsa-mir-4497 | MI0016859 |

(continued)

| SEQ ID NO: | Name of gene | miRBase Accession No. |
|---|---|---|
| 264 | hsa-mir-8071-1 | MI0025907 |
| 265 | hsa-mir-8071-2 | MI0026417 |
| 266 | hsa-mir-663b | MI0006336 |
| 267 | hsa-mir-3180-4 | MI0016408 |
| 268 | hsa-mir-3180-5 | MI0016409 |
| 269 | hsa-mir-4251 | MI0015861 |
| 270 | hsa-mir-1285-1 | MI0006346 |
| 271 | hsa-mir-1285-2 | MI0006347 |
| 272 | hsa-mir-6870 | MI0022717 |
| 273 | hsa-mir-4484 | MI0016845 |
| 274 | hsa-mir-4476 | MI0016828 |
| 275 | hsa-mir-6749 | MI0022594 |
| 276 | hsa-mir-4454 | MI0016800 |
| 277 | hsa-mir-6893 | MI0022740 |
| 278 | hsa-mir-6085 | MI0020362 |
| 279 | hsa-mir-4787 | MI0017434 |
| 280 | hsa-mir-149 | MI0000478 |
| 281 | hsa-mir-7704 | MI0025240 |
| 282 | hsa-mir-6125 | MI0021259 |
| 283 | hsa-mir-6090 | MI0020367 |
| 284 | hsa-mir-3197 | MI0014245 |
| 285 | hsa-mir-6850 | MI0022696 |
| 286 | hsa-mir-4467 | MI0016818 |
| 287 | hsa-mir-6885 | MI0022732 |
| 288 | hsa-mir-6803 | MI0022648 |
| 289 | hsa-mir-6798 | MI0022643 |
| 290 | hsa-mir-6780b | MI0022681 |
| 291 | hsa-mir-6768 | MI0022613 |
| 292 | hsa-mir-5100 | MI0019116 |
| 293 | hsa-mir-6724-1 | MI0022559 |
| 294 | hsa-mir-6724-2 | MI0031516 |
| 295 | hsa-mir-6724-3 | MI0031517 |
| 296 | hsa-mir-6724-4 | MI0031518 |
| 297 | hsa-mir-6879 | MI0022726 |
| 298 | hsa-mir-7108 | MI0022959 |
| 299 | hsa-mir-4649 | MI0017276 |
| 300 | hsa-mir-4739 | MI0017377 |
| 301 | hsa-mir-6089-1 | MI0020366 |

(continued)

| SEQ ID NO: | Name of gene | miRBase Accession No. |
|---|---|---|
| 302 | hsa-mir-6089-2 | MI0023563 |
| 303 | hsa-mir-1908 | MI0008329 |
| 304 | hsa-mir-4516 | MI0016882 |
| 305 | hsa-mir-2861 | MI0013006 |
| 306 | hsa-mir-4492 | MI0016854 |
| 307 | hsa-mir-4294 | MI0015827 |
| 308 | hsa-mir-6791 | MI0022636 |
| 309 | hsa-mir-1469 | MI0007074 |
| 310 | hsa-mir-6752 | MI0022597 |
| 311 | hsa-mir-4730 | MI0017367 |
| 312 | hsa-mir-6126 | MI0021260 |
| 313 | hsa-mir-6869 | MI0022716 |
| 314 | hsa-mir-1268a | MI0006405 |
| 315 | hsa-mir-6799 | MI0022644 |
| 316 | hsa-mir-8069-1 | MI0025905 |
| 317 | hsa-mir-8069-2 | MI0031519 |
| 318 | hsa-mir-3621 | MI0016012 |
| 319 | hsa-mir-4763 | MI0017404 |
| 320 | hsa-mir-1228 | MI0006318 |
| 321 | hsa-mir-760 | MI0005567 |
| 322 | hsa-mir-187 | MI0000274 |
| 323 | hsa-mir-7111 | MI0022962 |
| 324 | hsa-mir-6088 | MI0020365 |
| 325 | hsa-mir-6805 | MI0022650 |
| 326 | hsa-mir-4640 | MI0017267 |
| 327 | hsa-mir-6721 | MI0022556 |
| 328 | hsa-mir-6880 | MI0022727 |
| 329 | hsa-mir-711 | MI0012488 |
| 330 | hsa-mir-128-1 | MI0000447 |
| 331 | hsa-mir-4525 | MI0016892 |
| 332 | hsa-mir-486-1 | MI0002470 |
| 333 | hsa-mir-486-2 | MI0023622 |
| 334 | hsa-mir-6756 | MI0022601 |
| 335 | hsa-mir-1260b | MI0014197 |
| 336 | hsa-mir-3184 | MI0014226 |
| 337 | hsa-mir-6075 | MI0020352 |
| 338 | hsa-mir-204 | MI0000284 |
| 339 | hsa-mir-4728 | MI0017365 |

(continued)

| SEQ ID NO: | Name of gene | miRBase Accession No. |
|---|---|---|
| 340 | hsa-mir-4534 | MI0016901 |
| 341 | hsa-mir-4758 | MI0017399 |
| 342 | hsa-mir-8063 | MI0025899 |
| 343 | hsa-mir-6836 | MI0022682 |
| 344 | hsa-mir-6789 | MI0022634 |
| 345 | hsa-mir-744 | MI0005559 |
| 346 | hsa-mir-1909 | MI0008330 |
| 347 | hsa-mir-887 | MI0005562 |
| 348 | hsa-mir-4745 | MI0017384 |
| 349 | hsa-mir-4433a | MI0016773 |
| 350 | hsa-mir-5090 | MI0017979 |
| 351 | hsa-mir-296 | MI0000747 |
| 352 | hsa-mir-939 | MI0005761 |
| 353 | hsa-mir-3648-1 | MI0016048 |
| 354 | hsa-mir-3 648-2 | MI0031512 |
| 355 | hsa-mir-3196 | MI0014241 |
| 356 | hsa-mir-6722 | MI0022557 |
| 357 | hsa-mir-1202 | MI0006334 |
| 358 | hsa-mir-6775 | MI0022620 |
| 359 | hsa-mir-6087 | MI0020364 |
| 360 | hsa-mir-6765 | MI0022610 |
| 361 | hsa-mir-6875 | MI0022722 |
| 362 | hsa-mir-4674 | MI0017305 |
| 363 | hsa-mir-1233-1 | MI0006323 |
| 364 | hsa-mir-1233-2 | MI0015973 |
| 365 | hsa-mir-7114 | MI0022965 |
| 366 | hsa-mir-5787 | MI0019797 |
| 367 | hsa-mir-8072 | MI0025908 |
| 368 | hsa-mir-3619 | MI0016009 |
| 369 | hsa-mir-4632 | MI0017259 |
| 370 | hsa-mir-6800 | MI0022645 |
| 371 | hsa-mir-4634 | MI0017261 |
| 372 | hsa-mir-4486 | MI0016847 |
| 373 | hsa-mir-6727 | MI0022572 |
| 374 | hsa-mir-4505 | MI0016868 |
| 375 | hsa-mir-4725 | MI0017362 |
| 376 | hsa-mir-1538 | MI0007259 |
| 377 | hsa-mir-320b-1 | MI0003776 |

(continued)

| SEQ ID NO: | Name of gene | miRBase Accession No. |
|---|---|---|
| 378 | hsa-mir-320b-2 | MI0003839 |
| 379 | hsa-mir-1915 | MI0008336 |
| 380 | hsa-mir-328 | MI0000804 |
| 381 | hsa-mir-6820 | MI0022665 |
| 382 | hsa-mir-6726 | MI0022571 |
| 383 | hsa-mir-3665 | MI0016066 |
| 384 | hsa-mir-63 8 | MI0003653 |
| 385 | hsa-mir-762 | MI0003892 |
| 386 | hsa-mir-4466 | MI0016817 |
| 387 | hsa-mir-3940 | MI0016597 |
| 388 | hsa-mir-1237 | MI0006327 |
| 389 | hsa-mir-575 | MI0003582 |
| 390 | hsa-mir-3656 | MI0016056 |
| 391 | hsa-mir-4488 | MI0016849 |
| 392 | hsa-mir-4281 | MI0015885 |
| 393 | hsa-mir-6781 | MI0022626 |
| 394 | hsa-mir-4532 | MI0016899 |
| 395 | hsa-mir-4665 | MI0017295 |
| 396 | hsa-mir-6816 | MI0022661 |
| 397 | hsa-mir-4508 | MI0016872 |
| 398 | hsa-mir-6784 | MI0022629 |
| 399 | hsa-mir-6786 | MI0022631 |
| 400 | hsa-mir-4741 | MI0017379 |
| 401 | hsa-mir-1343 | MI0017320 |
| 402 | hsa-mir-1227 | MI0006316 |
| 403 | hsa-mir-4734 | MI0017371 |
| 404 | hsa-mir-3960 | MI0016964 |
| 405 | hsa-mir-128-2 | MI0000727 |
| 406 | hsa-mir-6743 | MI0022588 |
| 407 | hsa-mir-663a | MI0003672 |
| 408 | hsa-mir-6729 | MI0022574 |
| 409 | hsa-mir-1268b | MI0016748 |
| 410 | hsa-mir-4651 | MI0017279 |
| 411 | hsa-mir-3178 | MI0014212 |
| 412 | hsa-mir-4463 | MI0016811 |
| 413 | isomiR sequence 1 of SEQ ID NO: 1 | - |
| 414 | isomiR sequence 2 of SEQ ID NO: 1 | - |
| 415 | isomiR sequence 1 of SEQ ID NO: 2 | - |

(continued)

| SEQ ID NO: | Name of gene | miRBase Accession No. |
|---|---|---|
| 416 | isomiR sequence 2 of SEQ ID NO: 2 | - |
| 417 | isomiR sequence 1 of SEQ ID NO: 3 | - |
| 418 | isomiR sequence 2 of SEQ ID NO: 3 | - |
| 419 | isomiR sequence 1 of SEQ ID NO: 4 | - |
| 420 | isomiR sequence 2 of SEQ ID NO: 4 | - |
| 421 | isomiR sequence 1 of SEQ ID NO: 5 | - |
| 422 | isomiR sequence 2 of SEQ ID NO: 5 | - |
| 423 | isomiR sequence 1 of SEQ ID NO: 6 | - |
| 424 | isomiR sequence 2 of SEQ ID NO: 6 | - |
| 425 | isomiR sequence 1 of SEQ ID NO: 7 | - |
| 426 | isomiR sequence 2 of SEQ ID NO: 7 | - |
| 427 | isomiR sequence 1 of SEQ ID NO: 8 | - |
| 428 | isomiR sequence 2 of SEQ ID NO: 8 | - |
| 429 | isomiR sequence 1 of SEQ ID NO: 9 | - |
| 430 | isomiR sequence 2 of SEQ ID NO: 9 | - |
| 431 | isomiR sequence 1 of SEQ ID NO: 10 | - |
| 432 | isomiR sequence 2 of SEQ ID NO: 10 | - |
| 433 | isomiR sequence 1 of SEQ ID NO: 11 | - |
| 434 | isomiR sequence 2 of SEQ ID NO: 11 | - |
| 435 | isomiR sequence 1 of SEQ ID NO: 12 | - |
| 436 | isomiR sequence 2 of SEQ ID NO: 12 | - |
| 437 | isomiR sequence 1 of SEQ ID NO: 13 | - |
| 438 | isomiR sequence 2 of SEQ ID NO: 13 | - |
| 439 | isomiR sequence 1 of SEQ ID NO: 14 | - |
| 440 | isomiR sequence 2 of SEQ ID NO: 14 | - |
| 441 | isomiR sequence 1 of SEQ ID NO: 15 | - |
| 442 | isomiR sequence 2 of SEQ ID NO: 15 | - |
| 443 | isomiR sequence 1 of SEQ ID NO: 16 | - |
| 444 | isomiR sequence 2 of SEQ ID NO: 16 | - |
| 445 | isomiR sequence 1 of SEQ ID NO: 17 | - |
| 446 | isomiR sequence 2 of SEQ ID NO: 17 | - |
| 447 | isomiR sequence 1 of SEQ ID NO: 19 | - |
| 448 | isomiR sequence 2 of SEQ ID NO: 19 | - |
| 449 | isomiR sequence 1 of SEQ ID NO: 20 | - |
| 450 | isomiR sequence 2 of SEQ ID NO: 20 | - |
| 451 | isomiR sequence 1 of SEQ ID NO: 21 | - |
| 452 | isomiR sequence 2 of SEQ ID NO: 21 | - |
| 453 | isomiR sequence 1 of SEQ ID NO: 22 | - |

(continued)

| SEQ ID NO: | Name of gene | miRBase Accession No. |
|---|---|---|
| 454 | isomiR sequence 2 of SEQ ID NO: 22 | - |
| 455 | isomiR sequence 1 of SEQ ID NO: 24 | - |
| 456 | isomiR sequence 2 of SEQ ID NO: 24 | - |
| 457 | isomiR sequence 1 of SEQ ID NO: 25 | - |
| 458 | isomiR sequence 2 of SEQ ID NO: 25 | - |
| 459 | isomiR sequence 1 of SEQ ID NO: 26 | - |
| 460 | isomiR sequence 2 of SEQ ID NO: 26 | - |
| 461 | isomiR sequence 1 of SEQ ID NO: 27 | - |
| 462 | isomiR sequence 2 of SEQ ID NO: 27 | - |
| 463 | isomiR sequence 1 of SEQ ID NO: 28 | - |
| 464 | isomiR sequence 2 of SEQ ID NO: 28 | - |
| 465 | isomiR sequence 1 of SEQ ID NO: 29 | - |
| 466 | isomiR sequence 2 of SEQ ID NO: 29 | - |
| 467 | isomiR sequence 1 of SEQ ID NO: 30 | - |
| 468 | isomiR sequence 2 of SEQ ID NO: 30 | - |
| 469 | isomiR sequence 1 of SEQ ID NO: 31 | - |
| 470 | isomiR sequence 2 of SEQ ID NO: 31 | - |
| 471 | isomiR sequence 1 of SEQ ID NO: 32 | - |
| 472 | isomiR sequence 2 of SEQ ID NO: 32 | - |
| 473 | isomiR sequence 1 of SEQ ID NO: 33 | - |
| 474 | isomiR sequence 2 of SEQ ID NO: 33 | - |
| 475 | isomiR sequence 1 of SEQ ID NO: 34 | - |
| 476 | isomiR sequence 2 of SEQ ID NO: 34 | - |
| 477 | isomiR sequence 1 of SEQ ID NO: 35 | - |
| 478 | isomiR sequence 2 of SEQ ID NO: 35 | - |
| 479 | isomiR sequence 1 of SEQ ID NO: 36 | - |
| 480 | isomiR sequence 2 of SEQ ID NO: 36 | - |
| 481 | isomiR sequence 1 of SEQ ID NO: 37 | - |
| 482 | isomiR sequence 2 of SEQ ID NO: 37 | - |
| 483 | isomiR sequence 1 of SEQ ID NO: 39 | - |
| 484 | isomiR sequence 2 of SEQ ID NO: 39 | - |
| 485 | isomiR sequence 1 of SEQ ID NO: 40 | - |
| 486 | isomiR sequence 2 of SEQ ID NO: 40 | - |
| 487 | isomiR sequence 1 of SEQ ID NO: 41 | - |
| 488 | isomiR sequence 2 of SEQ ID NO: 41 | - |
| 489 | isomiR sequence 1 of SEQ ID NO: 42 | - |
| 490 | isomiR sequence 2 of SEQ ID NO: 42 | - |
| 491 | isomiR sequence 1 of SEQ ID NO: 44 | - |

(continued)

| SEQ ID NO: | Name of gene | miRBase Accession No. |
|---|---|---|
| 492 | isomiR sequence 2 of SEQ ID NO: 44 | - |
| 493 | isomiR sequence 1 of SEQ ID NO: 45 | - |
| 494 | isomiR sequence 2 of SEQ ID NO: 45 | - |
| 495 | isomiR sequence 1 of SEQ ID NO: 46 | - |
| 496 | isomiR sequence 2 of SEQ ID NO: 46 | - |
| 497 | isomiR sequence 1 of SEQ ID NO: 47 | - |
| 498 | isomiR sequence 2 of SEQ ID NO: 47 | - |
| 499 | isomiR sequence 1 of SEQ ID NO: 48 | - |
| 500 | isomiR sequence 2 of SEQ ID NO: 48 | - |
| 501 | isomiR sequence 1 of SEQ ID NO: 50 | - |
| 502 | isomiR sequence 2 of SEQ ID NO: 50 | - |
| 503 | isomiR sequence 1 of SEQ ID NO: 51 | - |
| 504 | isomiR sequence 2 of SEQ ID NO: 51 | - |
| 505 | isomiR sequence 1 of SEQ ID NO: 52 | - |
| 506 | isomiR sequence 2 of SEQ ID NO: 52 | - |
| 507 | isomiR sequence 1 of SEQ ID NO: 54 | - |
| 508 | isomiR sequence 2 of SEQ ID NO: 54 | - |
| 509 | isomiR sequence 1 of SEQ ID NO: 55 | - |
| 510 | isomiR sequence 2 of SEQ ID NO: 55 | - |
| 511 | isomiR sequence 1 of SEQ ID NO: 56 | - |
| 512 | isomiR sequence 2 of SEQ ID NO: 56 | - |
| 513 | isomiR sequence 1 of SEQ ID NO: 57 | - |
| 514 | isomiR sequence 2 of SEQ ID NO: 57 | - |
| 515 | isomiR sequence 1 of SEQ ID NO: 58 | - |
| 516 | isomiR sequence 2 of SEQ ID NO: 58 | - |
| 517 | isomiR sequence 1 of SEQ ID NO: 59 | - |
| 518 | isomiR sequence 2 of SEQ ID NO: 59 | - |
| 519 | isomiR sequence 1 of SEQ ID NO: 60 | - |
| 520 | isomiR sequence 2 of SEQ ID NO: 60 | - |
| 521 | isomiR sequence 1 of SEQ ID NO: 61 | - |
| 522 | isomiR sequence 2 of SEQ ID NO: 61 | - |
| 523 | isomiR sequence 1 of SEQ ID NO: 62 | - |
| 524 | isomiR sequence 2 of SEQ ID NO: 62 | - |
| 525 | isomiR sequence 1 of SEQ ID NO: 63 | - |
| 526 | isomiR sequence 2 of SEQ ID NO: 63 | - |
| 527 | isomiR sequence 1 of SEQ ID NO: 64 | - |
| 528 | isomiR sequence 2 of SEQ ID NO: 64 | - |
| 529 | isomiR sequence 1 of SEQ ID NO: 66 | - |

(continued)

| SEQ ID NO: | Name of gene | miRBase Accession No. |
|---|---|---|
| 530 | isomiR sequence 2 of SEQ ID NO: 66 | - |
| 531 | isomiR sequence 1 of SEQ ID NO: 68 | - |
| 532 | isomiR sequence 2 of SEQ ID NO: 68 | - |
| 533 | isomiR sequence 1 of SEQ ID NO: 69 | - |
| 534 | isomiR sequence 2 of SEQ ID NO: 69 | - |
| 535 | isomiR sequence 1 of SEQ ID NO: 71 | - |
| 536 | isomiR sequence 2 of SEQ ID NO: 71 | - |
| 537 | isomiR sequence 1 of SEQ ID NO: 72 | - |
| 538 | isomiR sequence 2 of SEQ ID NO: 72 | - |
| 539 | isomiR sequence 1 of SEQ ID NO: 74 | - |
| 540 | isomiR sequence 2 of SEQ ID NO: 74 | - |
| 541 | isomiR sequence 1 of SEQ ID NO: 75 | - |
| 542 | isomiR sequence 2 of SEQ ID NO: 75 | - |
| 543 | isomiR sequence 1 of SEQ ID NO: 76 | - |
| 544 | isomiR sequence 2 of SEQ ID NO: 76 | - |
| 545 | isomiR sequence 1 of SEQ ID NO: 77 | - |
| 546 | isomiR sequence 2 of SEQ ID NO: 77 | - |
| 547 | isomiR sequence 1 of SEQ ID NO: 79 | - |
| 548 | isomiR sequence 2 of SEQ ID NO: 79 | - |
| 549 | isomiR sequence 1 of SEQ ID NO: 80 | - |
| 550 | isomiR sequence 2 of SEQ ID NO: 80 | - |
| 551 | isomiR sequence 1 of SEQ ID NO: 81 | - |
| 552 | isomiR sequence 2 of SEQ ID NO: 81 | - |
| 553 | isomiR sequence 1 of SEQ ID NO: 82 | - |
| 554 | isomiR sequence 2 of SEQ ID NO: 82 | - |
| 555 | isomiR sequence 1 of SEQ ID NO: 83 | - |
| 556 | isomiR sequence 2 of SEQ ID NO: 83 | - |
| 557 | isomiR sequence 1 of SEQ ID NO: 84 | - |
| 558 | isomiR sequence 2 of SEQ ID NO: 84 | - |
| 559 | isomiR sequence 1 of SEQ ID NO: 85 | - |
| 560 | isomiR sequence 2 of SEQ ID NO: 85 | - |
| 561 | isomiR sequence 1 of SEQ ID NO: 86 | - |
| 562 | isomiR sequence 2 of SEQ ID NO: 86 | - |
| 563 | isomiR sequence 1 of SEQ ID NO: 87 | - |
| 564 | isomiR sequence 2 of SEQ ID NO: 87 | - |
| 565 | isomiR sequence 1 of SEQ ID NO: 88 | - |
| 566 | isomiR sequence 2 of SEQ ID NO: 88 | - |
| 567 | isomiR sequence 1 of SEQ ID NO: 89 | - |

(continued)

| SEQ ID NO: | Name of gene | miRBase Accession No. |
|---|---|---|
| 568 | isomiR sequence 2 of SEQ ID NO: 89 | - |
| 569 | isomiR sequence 1 of SEQ ID NO: 90 | - |
| 570 | isomiR sequence 2 of SEQ ID NO: 90 | - |
| 571 | isomiR sequence 1 of SEQ ID NO: 91 | - |
| 572 | isomiR sequence 2 of SEQ ID NO: 91 | - |
| 573 | isomiR sequence 1 of SEQ ID NO: 92 | - |
| 574 | isomiR sequence 2 of SEQ ID NO: 92 | - |
| 575 | isomiR sequence 1 of SEQ ID NO: 94 | - |
| 576 | isomiR sequence 2 of SEQ ID NO: 94 | - |
| 577 | isomiR sequence 1 of SEQ ID NO: 95 | - |
| 578 | isomiR sequence 2 of SEQ ID NO: 95 | - |
| 579 | isomiR sequence 1 of SEQ ID NO: 96 | - |
| 580 | isomiR sequence 2 of SEQ ID NO: 96 | - |
| 581 | isomiR sequence 1 of SEQ ID NO: 97 | - |
| 582 | isomiR sequence 2 of SEQ ID NO: 97 | - |
| 583 | isomiR sequence 1 of SEQ ID NO: 99 | - |
| 584 | isomiR sequence 2 of SEQ ID NO: 99 | - |
| 585 | isomiR sequence 1 of SEQ ID NO: 102 | - |
| 586 | isomiR sequence 2 of SEQ ID NO: 102 | - |
| 587 | isomiR sequence 1 of SEQ ID NO: 104 | - |
| 588 | isomiR sequence 2 of SEQ ID NO: 104 | - |
| 589 | isomiR sequence 1 of SEQ ID NO: 105 | - |
| 590 | isomiR sequence 2 of SEQ ID NO: 105 | - |
| 591 | isomiR sequence 1 of SEQ ID NO: 106 | - |
| 592 | isomiR sequence 2 of SEQ ID NO: 106 | - |
| 593 | isomiR sequence 1 of SEQ ID NO: 109 | - |
| 594 | isomiR sequence 2 of SEQ ID NO: 109 | - |
| 595 | isomiR sequence 1 of SEQ ID NO: 110 | - |
| 596 | isomiR sequence 2 of SEQ ID NO: 110 | - |
| 597 | isomiR sequence 1 of SEQ ID NO: 111 | - |
| 598 | isomiR sequence 2 of SEQ ID NO: 111 | - |
| 599 | isomiR sequence 1 of SEQ ID NO: 112 | - |
| 600 | isomiR sequence 2 of SEQ ID NO: 112 | - |
| 601 | isomiR sequence 1 of SEQ ID NO: 113 | - |
| 602 | isomiR sequence 2 of SEQ ID NO: 113 | - |
| 603 | isomiR sequence 1 of SEQ ID NO: 114 | - |
| 604 | isomiR sequence 2 of SEQ ID NO: 114 | - |
| 605 | isomiR sequence 1 of SEQ ID NO: 115 | - |

(continued)

| SEQ ID NO: | Name of gene | miRBase Accession No. |
|---|---|---|
| 606 | isomiR sequence 2 of SEQ ID NO: 115 | - |
| 607 | isomiR sequence 1 of SEQ ID NO: 116 | - |
| 608 | isomiR sequence 2 of SEQ ID NO: 116 | - |
| 609 | isomiR sequence 1 of SEQ ID NO: 117 | - |
| 610 | isomiR sequence 2 of SEQ ID NO: 117 | - |
| 611 | isomiR sequence 1 of SEQ ID NO: 118 | - |
| 612 | isomiR sequence 2 of SEQ ID NO: 118 | - |
| 613 | isomiR sequence 1 of SEQ ID NO: 119 | - |
| 614 | isomiR sequence 2 of SEQ ID NO: 119 | - |
| 615 | isomiR sequence 1 of SEQ ID NO: 120 | - |
| 616 | isomiR sequence 2 of SEQ ID NO: 120 | - |
| 617 | isomiR sequence 1 of SEQ ID NO: 121 | - |
| 618 | isomiR sequence 2 of SEQ ID NO: 121 | - |
| 619 | isomiR sequence 1 of SEQ ID NO: 122 | - |
| 620 | isomiR sequence 2 of SEQ ID NO: 122 | - |
| 621 | isomiR sequence 1 of SEQ ID NO: 123 | - |
| 622 | isomiR sequence 2 of SEQ ID NO: 123 | - |
| 623 | isomiR sequence 1 of SEQ ID NO: 124 | - |
| 624 | isomiR sequence 2 of SEQ ID NO: 124 | - |
| 625 | isomiR sequence 1 of SEQ ID NO: 125 | - |
| 626 | isomiR sequence 2 of SEQ ID NO: 125 | - |
| 627 | isomiR sequence 1 of SEQ ID NO: 127 | - |
| 628 | isomiR sequence 2 of SEQ ID NO: 127 | - |
| 629 | isomiR sequence 1 of SEQ ID NO: 128 | - |
| 630 | isomiR sequence 2 of SEQ ID NO: 128 | - |
| 631 | isomiR sequence 1 of SEQ ID NO: 130 | - |
| 632 | isomiR sequence 2 of SEQ ID NO: 130 | - |
| 633 | isomiR sequence 1 of SEQ ID NO: 132 | - |
| 634 | isomiR sequence 2 of SEQ ID NO: 132 | - |
| 635 | isomiR sequence 1 of SEQ ID NO: 133 | - |
| 636 | isomiR sequence 2 of SEQ ID NO: 133 | - |
| 637 | isomiR sequence 1 of SEQ ID NO: 134 | - |
| 638 | isomiR sequence 2 of SEQ ID NO: 134 | - |
| 639 | isomiR sequence 1 of SEQ ID NO: 135 | - |
| 640 | isomiR sequence 2 of SEQ ID NO: 135 | - |
| 641 | isomiR sequence 1 of SEQ ID NO: 136 | - |
| 642 | isomiR sequence 2 of SEQ ID NO: 136 | - |
| 643 | isomiR sequence 1 of SEQ ID NO: 137 | - |

(continued)

| SEQ ID NO: | Name of gene | miRBase Accession No. |
|---|---|---|
| 644 | isomiR sequence 2 of SEQ ID NO: 137 | - |
| 645 | isomiR sequence 1 of SEQ ID NO: 138 | - |
| 646 | isomiR sequence 2 of SEQ ID NO: 138 | - |
| 647 | isomiR sequence 1 of SEQ ID NO: 139 | - |
| 648 | isomiR sequence 2 of SEQ ID NO: 139 | - |
| 649 | isomiR sequence 1 of SEQ ID NO: 140 | - |
| 650 | isomiR sequence 2 of SEQ ID NO: 140 | - |
| 651 | isomiR sequence 1 of SEQ ID NO: 141 | - |
| 652 | isomiR sequence 2 of SEQ ID NO: 141 | - |
| 653 | isomiR sequence 1 of SEQ ID NO: 142 | - |
| 654 | isomiR sequence 2 of SEQ ID NO: 142 | - |
| 655 | isomiR sequence 1 of SEQ ID NO: 143 | - |
| 656 | isomiR sequence 2 of SEQ ID NO: 143 | - |
| 657 | isomiR sequence 1 of SEQ ID NO: 145 | - |
| 658 | isomiR sequence 2 of SEQ ID NO: 145 | - |
| 659 | isomiR sequence 1 of SEQ ID NO: 150 | - |
| 660 | isomiR sequence 2 of SEQ ID NO: 150 | - |
| 661 | isomiR sequence 1 of SEQ ID NO: 151 | - |
| 662 | isomiR sequence 2 of SEQ ID NO: 151 | - |
| 663 | isomiR sequence 1 of SEQ ID NO: 152 | - |
| 664 | isomiR sequence 2 of SEQ ID NO: 152 | - |
| 665 | isomiR sequence 1 of SEQ ID NO: 153 | - |
| 666 | isomiR sequence 2 of SEQ ID NO: 153 | - |
| 667 | isomiR sequence 1 of SEQ ID NO: 155 | - |
| 668 | isomiR sequence 2 of SEQ ID NO: 155 | - |
| 669 | isomiR sequence 1 of SEQ ID NO: 156 | - |
| 670 | isomiR sequence 2 of SEQ ID NO: 156 | - |
| 671 | isomiR sequence 1 of SEQ ID NO: 158 | - |
| 672 | isomiR sequence 2 of SEQ ID NO: 158 | - |
| 673 | isomiR sequence 1 of SEQ ID NO: 159 | - |
| 674 | isomiR sequence 2 of SEQ ID NO: 159 | - |
| 675 | isomiR sequence 1 of SEQ ID NO: 160 | - |
| 676 | isomiR sequence 2 of SEQ ID NO: 160 | - |
| 677 | isomiR sequence 1 of SEQ ID NO: 161 | - |
| 678 | isomiR sequence 2 of SEQ ID NO: 161 | - |
| 679 | isomiR sequence 1 of SEQ ID NO: 162 | - |
| 680 | isomiR sequence 2 of SEQ ID NO: 162 | - |
| 681 | isomiR sequence 1 of SEQ ID NO: 163 | - |

(continued)

| SEQ ID NO: | Name of gene | miRBase Accession No. |
|---|---|---|
| 682 | isomiR sequence 2 of SEQ ID NO: 163 | - |
| 683 | isomiR sequence 1 of SEQ ID NO: 164 | - |
| 684 | isomiR sequence 2 of SEQ ID NO: 164 | - |
| 685 | isomiR sequence 1 of SEQ ID NO: 165 | - |
| 686 | isomiR sequence 2 of SEQ ID NO: 165 | - |
| 687 | isomiR sequence 1 of SEQ ID NO: 166 | - |
| 688 | isomiR sequence 2 of SEQ ID NO: 166 | - |
| 689 | isomiR sequence 1 of SEQ ID NO: 167 | - |
| 690 | isomiR sequence 2 of SEQ ID NO: 167 | - |
| 691 | isomiR sequence 1 of SEQ ID NO: 168 | - |
| 692 | isomiR sequence 2 of SEQ ID NO: 168 | - |
| 693 | isomiR sequence 1 of SEQ ID NO: 169 | - |
| 694 | isomiR sequence 2 of SEQ ID NO: 169 | - |
| 695 | isomiR sequence 1 of SEQ ID NO: 170 | - |
| 696 | isomiR sequence 2 of SEQ ID NO: 170 | - |
| 697 | isomiR sequence 1 of SEQ ID NO: 173 | - |
| 698 | isomiR sequence 2 of SEQ ID NO: 173 | - |
| 699 | isomiR sequence 1 of SEQ ID NO: 174 | - |
| 700 | isomiR sequence 2 of SEQ ID NO: 174 | - |
| 701 | isomiR sequence 1 of SEQ ID NO: 175 | - |
| 702 | isomiR sequence 2 of SEQ ID NO: 175 | - |
| 703 | isomiR sequence 1 of SEQ ID NO: 176 | - |
| 704 | isomiR sequence 2 of SEQ ID NO: 176 | - |
| 705 | isomiR sequence 1 of SEQ ID NO: 178 | - |
| 706 | isomiR sequence 2 of SEQ ID NO: 178 | - |
| 707 | isomiR sequence 1 of SEQ ID NO: 180 | - |
| 708 | isomiR sequence 2 of SEQ ID NO: 180 | - |
| 709 | isomiR sequence 1 of SEQ ID NO: 182 | - |
| 710 | isomiR sequence 2 of SEQ ID NO: 182 | - |
| 711 | isomiR sequence 1 of SEQ ID NO: 183 | - |
| 712 | isomiR sequence 2 of SEQ ID NO: 183 | - |
| 713 | isomiR sequence 1 of SEQ ID NO: 184 | - |
| 714 | isomiR sequence 2 of SEQ ID NO: 184 | - |
| 715 | isomiR sequence 1 of SEQ ID NO: 187 | - |
| 716 | isomiR sequence 2 of SEQ ID NO: 187 | - |
| 717 | isomiR sequence 1 of SEQ ID NO: 188 | - |
| 718 | isomiR sequence 2 of SEQ ID NO: 188 | - |
| 719 | isomiR sequence 1 of SEQ ID NO: 190 | - |

(continued)

| SEQ ID NO: | Name of gene | miRBase Accession No. |
|---|---|---|
| 720 | isomiR sequence 2 of SEQ ID NO: 190 | - |
| 721 | isomiR sequence 1 of SEQ ID NO: 191 | - |
| 722 | isomiR sequence 2 of SEQ ID NO: 191 | - |
| 723 | isomiR sequence 1 of SEQ ID NO: 192 | - |
| 724 | isomiR sequence 2 of SEQ ID NO: 192 | - |
| 725 | isomiR sequence 1 of SEQ ID NO: 193 | - |
| 726 | isomiR sequence 2 of SEQ ID NO: 193 | - |
| 727 | isomiR sequence 1 of SEQ ID NO: 194 | - |
| 728 | isomiR sequence 2 of SEQ ID NO: 194 | - |
| 729 | isomiR sequence 1 of SEQ ID NO: 195 | - |
| 730 | isomiR sequence 2 of SEQ ID NO: 195 | - |
| 731 | isomiR sequence 1 of SEQ ID NO: 196 | - |
| 732 | isomiR sequence 2 of SEQ ID NO: 196 | - |
| 733 | isomiR sequence 1 of SEQ ID NO: 197 | - |
| 734 | isomiR sequence 2 of SEQ ID NO: 197 | - |
| 735 | isomiR sequence 1 of SEQ ID NO: 198 | - |
| 736 | isomiR sequence 2 of SEQ ID NO: 198 | - |
| 737 | isomiR sequence 1 of SEQ ID NO: 199 | - |
| 738 | isomiR sequence 2 of SEQ ID NO: 199 | - |
| 739 | isomiR sequence 1 of SEQ ID NO: 200 | - |
| 740 | isomiR sequence 2 of SEQ ID NO: 200 | - |

The present specification includes the contents disclosed in Japanese Patent Application No. 2020-064383 from which the present application claims priority.

Advantageous Effect of Invention

The present invention makes it possible to detect hippocampal atrophy with high accuracy (or AUC), sensitivity, and specificity, and thereby discriminating a hippocampal atrophy subject from a normal hippocampus subject (i.e., a hippocampal non-atrophy subject).

BRIEF DESCRIPTION OF THE DRAWINGS

[Fig. 1] Fig. 1 illustrates the relationship between the nucleotide sequences of a precursor hsa-mir-1915 shown by SEQ ID NO: 379, and hsa-miR-1915-5p shown by SEQ ID NO: 166 and hsa-miR-1915-3p shown by SEQ ID NO: 196, which are generated from the precursor.
[Fig. 2] Fig. 2A and Fig. 2B each show plots of discriminant scores in a training cohort (A) and a cross validation cohort (B) obtained in Example 6. Fig. 2C and Fig. 2D each show ROC curves for the training cohort (C) and the cross validation cohort (D).
[Fig. 3] Fig. 3 shows regression lines based on the quantified hippocampal volumes and the explanatory variables obtained in Example 7-(2). Fig. 3A indicates the training and cross validation cohort and Fig. 3B indicates the independent validation cohort.

DESCRIPTION OF EMBODIMENTS

Hereinafter, the present invention will be further described in detail.

1. Target Nucleic acids for hippocampal atrophy

The target nucleic acids as hippocampal atrophy markers for detecting hippocampal atrophy, or atrophy or non-atrophy of hippocampus by using the nucleic acids, such as nucleic acid probes or primers, for detection of hippocampal atrophy as defined above according to the present invention may be one or more miRNA polynucleotides selected from the group consisting of: miR-3131, miR-6757-5p, miR-4706, miR-5001-5p, miR-3180-3p, miR-642b-3p, miR-4655-5p, miR-6819-5p, miR-937-5p, miR-4688, miR-6741-5p, miR-7107-5p, miR-4271, miR-1229-5p, miR-4707-5p, miR-6808-5p, miR-4656, miR-6076, miR-6762-5p, miR-7109-5p, miR-6732-5p, miR-3195, miR-7150, miR-642a-3p, miR-1249-5p, miR-3185, miR-4689, miR-3141, miR-6840-3p, miR-3135b, miR-1914-3p, miR-4446-3p, miR-4433b-3p, miR-6877-5p, miR-6848-5p, miR-3620-5p, miR-6825-5p, miR-5739, miR-3663-3p, miR-4695-5p, miR-3162-5p, miR-3679-5p, miR-8059, miR-7110-5p, miR-1275, miR-6779-5p, miR-197-5p, miR-6845-5p, miR-4327, miR-4723-5p, miR-4530, miR-6771-5p, miR-614, miR-92a-2-5p, miR-6891-5p, miR-6124, miR-4687-3p, miR-4442, miR-7977, miR-6785-5p, miR-4497, miR-8071, miR-663b, miR-3180, miR-4251, miR-1285-3p, miR-6870-5p, miR-4484, miR-4476, miR-6749-5p, miR-4454, miR-6893-5p, miR-6085, miR-4787-5p, miR-149-3p, miR-7704, miR-6125, miR-6090, miR-3197, miR-6850-5p, miR-4467, miR-6885-5p, miR-6803-5p, miR-6798-5p, miR-6780b-5p, miR-6768-5p, miR-5100, miR-6724-5p, miR-6879-5p, miR-7108-5p, miR-4649-5p, miR-4739, miR-6089, miR-1908-5p, miR-4516, miR-2861, miR-4492, miR-4294, miR-6791-5p, miR-1469, miR-6752-5p, miR-4730, miR-6126, miR-6869-5p, miR-1268a, miR-6799-5p, miR-8069, miR-3621, and miR-4763-3p.

Examples of major target nucleic acids as such hippocampal atrophy markers include one or more miRNA polynucleotides selected from the group consisting of: hsa-miR-3131, hsa-miR-6757-5p, hsa-miR-4706, hsa-miR-5001-5p, hsa-miR-3180-3p, hsa-miR-642b-3p, hsa-miR-4655-5p, hsa-miR-6819-5p, hsa-miR-937-5p, hsa-miR-4688, hsa-miR-6741-5p, hsa-miR-7107-5p, hsa-miR-4271, hsa-miR-1229-5p, hsa-miR-4707-5p, hsa-miR-6808-5p, hsa-miR-4656, hsa-miR-6076, hsa-miR-6762-5p, hsa-miR-7109-5p, hsa-miR-6732-5p, hsa-miR-3195, hsa-miR-7150, hsa-miR-642a-3p, hsa-miR-1249-5p, hsa-miR-3185, hsa-miR-4689, hsa-miR-3141, hsa-miR-6840-3p, hsa-miR-3135b, hsa-miR-1914-3p, hsa-miR-4446-3p, hsa-miR-4433b-3p, hsa-miR-6877-5p, hsa-miR-6848-5p, hsa-miR-3620-5p, hsa-miR-6825-5p, hsa-miR-5739, hsa-miR-3663-3p, hsa-miR-4695-5p, hsa-miR-3162-5p, hsa-miR-3679-5p, hsa-miR-8059, hsa-miR-7110-5p, hsa-miR-1275, hsa-miR-6779-5p, hsa-miR-197-5p, hsa-miR-6845-5p, hsa-miR-4327, hsa-miR-4723-5p, hsa-miR-4530, hsa-miR-6771-5p, hsa-miR-614, hsa-miR-92a-2-5p, hsa-miR-6891-5p, hsa-miR-6124, hsa-miR-4687-3p, hsa-miR-4442, hsa-miR-7977, hsa-miR-6785-5p, hsa-miR-4497, hsa-miR-8071, hsa-miR-663b, hsa-miR-3180, hsa-miR-4251, hsa-miR-1285-3p, hsa-miR-6870-5p, hsa-miR-4484, hsa-miR-4476, hsa-miR-6749-5p, hsa-miR-4454, hsa-miR-6893-5p, hsa-miR-6085, hsa-miR-4787-5p, hsa-miR-149-3p, hsa-miR-7704, hsa-miR-6125, hsa-miR-6090, hsa-miR-3197, hsa-miR-6850-5p, hsa-miR-4467, hsa-miR-6885-5p, hsa-miR-6803-5p, hsa-miR-6798-5p, hsa-miR-6780b-5p, hsa-miR-6768-5p, hsa-miR-5100, hsa-miR-6724-5p, hsa-miR-6879-5p, hsa-miR-7108-5p, hsa-miR-4649-5p, hsa-miR-4739, hsa-miR-6089, hsa-miR-1908-5p, hsa-miR-4516, hsa-miR-2861, hsa-miR-4492, hsa-miR-4294, hsa-miR-6791-5p, hsa-miR-1469, hsa-miR-6752-5p, hsa-miR-4730, hsa-miR-6126, hsa-miR-6869-5p, hsa-miR-1268a, hsa-miR-6799-5p, hsa-miR-8069, hsa-miR-3621, and hsa-miR-4763-3p.

In combination with the miRNA(s) mentioned above, other hippocampal atrophy marker(s), i.e., one or more miRNA polynucleotides selected from the group consisting of: miR-1228-5p, miR-760, miR-187-5p, miR-7111-5p, miR-6088, miR-6805-3p, miR-4640-5p, miR-6721-5p, miR-6880-5p, miR-711, miR-128-1-5p, miR-4525, miR-486-3p, miR-6756-5p, miR-1260b, miR-3184-5p, miR-6075, miR-204-3p, miR-4728-5p, miR-4534, miR-4758-5p, miR-8063, miR-6836-3p, miR-6789-5p, miR-744-5p, miR-1909-3p, miR-887-3p, miR-4745-5p, miR-4433a-3p, miR-5090, miR-296-5p, miR-939-5p, miR-3648, miR-3196, miR-6722-3p, miR-6805-5p, miR-1202, miR-6775-5p, miR-6087, miR-6765-5p, miR-6875-5p, miR-4674, miR-1233-5p, miR-7114-5p, miR-5787, miR-8072, miR-3619-3p, miR-4632-5p, miR-6800-5p, miR-4634, miR-4486, miR-6727-5p, miR-4505, miR-4725-3p, miR-1538, miR-320b, miR-1915-5p, miR-328-5p, miR-6820-5p, miR-6726-5p, miR-3665, miR-638, miR-762, miR-4466, miR-3940-5p, miR-1237-5p, miR-575, miR-3656, miR-4488, miR-4281, miR-6781-5p, miR-4532, miR-4665-5p, miR-6816-5p, miR-4508, miR-6784-5p, miR-6786-5p, miR-4741, miR-1343-5p, miR-1227-5p, miR-4734, miR-3960, miR-128-2-5p, miR-6743-5p, miR-663a, miR-6729-5p, miR-1915-3p, miR-1268b, miR-4651, miR-3178, and miR-4463, can be favorably used as target nucleic acids.

Examples of the other hippocampal atrophy markers include one or more miRNA polynucleotides selected from the group consisting of: hsa-miR-1228-5p, hsa-miR-760, hsa-miR-187-5p, hsa-miR-7111-5p, hsa-miR-6088, hsa-miR-6805-3p, hsa-miR-4640-5p, hsa-miR-6721-5p, hsa-miR-6880-5p, hsa-miR-711, hsa-miR-128-1-5p, hsa-miR-4525, hsa-miR-486-3p, hsa-miR-6756-5p, hsa-miR-1260b, hsa-miR-3184-5p, hsa-miR-6075, hsa-miR-204-3p, hsa-miR-4728-5p, hsa-miR-4534, hsa-miR-4758-5p, hsa-miR-8063, hsa-miR-6836-3p, hsa-miR-6789-5p, hsa-miR-744-5p, hsa-miR-1909-3p, hsa-miR-887-3p, hsa-miR-4745-5p, hsa-miR-4433a-3p, hsa-miR-5090, hsa-miR-296-5p, hsa-miR-939-5p, hsa-miR-3648, hsa-miR-3196, hsa-miR-6722-3p, hsa-miR-6805-5p, hsa-miR-1202, hsa-miR-6775-5p, hsa-miR-6087, hsa-miR-6765-5p, hsa-miR-6875-5p, hsa-miR-4674, hsa-miR-1233-5p, hsa-miR-7114-5p, hsa-miR-5787, hsa-miR-8072, hsa-miR-3619-3p, hsa-miR-4632-5p, hsa-miR-6800-5p, hsa-miR-4634, hsa-miR-4486, hsa-miR-6727-5p, hsa-miR-4505, hsa-miR-4725-3p, hsa-miR-1538, hsa-miR-320b, hsa-miR-1915-5p, hsa-miR-328-5p, hsa-miR-6820-5p, hsa-miR-6726-5p, hsa-miR-3665, hsa-miR-638, hsa-miR-762, hsa-miR-4466, hsa-miR-3940-5p, hsa-miR-1237-5p, hsa-miR-575, hsa-miR-3656, hsa-miR-4488, hsa-miR-4281, hsa-miR-6781-5p, hsa-miR-4532, hsa-miR-4665-5p, hsa-miR-6816-5p, hsa-miR-4508, hsa-miR-6784-5p, hsa-miR-6786-5p, hsa-miR-4741, hsa-miR-1343-5p, hsa-miR-1227-5p, hsa-miR-4734, hsa-miR-3960, hsa-miR-128-2-5p, hsa-miR-6743-5p, hsa-miR-663a, hsa-miR-6729-5p, hsa-miR-1915-3p, hsa-miR-1268b, hsa-miR-4651, hsa-miR-3178, and hsa-miR-4463.

The above miRNAs include polynucleotides/genes comprising the nucleotide sequences shown by any of SEQ ID Nos: 1 to 200 corresponding to hsa-miR-3131, hsa-miR-6757-5p, hsa-miR-4706, hsa-miR-5001-5p, hsa-miR-3180-3p, hsa-miR-642b-3p, hsa-miR-4655-5p, hsa-miR-6819-5p, hsa-miR-937-5p, hsa-miR-4688, hsa-miR-6741-5p, hsa-miR-7107-5p, hsa-miR-4271, hsa-miR-1229-5p, hsa-miR-4707-5p, hsa-miR-6808-5p, hsa-miR-4656, hsa-miR-6076, hsa-miR-6762-5p, hsa-miR-7109-5p, hsa-miR-6732-5p, hsa-miR-3195, hsa-miR-7150, hsa-miR-642a-3p, hsa-miR-1249-5p, hsa-miR-3185, hsa-miR-4689, hsa-miR-3141, hsa-miR-6840-3p, hsa-miR-3135b, hsa-miR-1914-3p, hsa-miR-4446-3p, hsa-miR-4433b-3p, hsa-miR-6877-5p, hsa-miR-6848-5p, hsa-miR-3620-5p, hsa-miR-6825-5p, hsa-miR-5739, hsa-miR-3663-3p, hsa-miR-4695-5p, hsa-miR-3162-5p, hsa-miR-3679-5p, hsa-miR-8059, hsa-miR-7110-5p, hsa-miR-1275, hsa-miR-6779-5p, hsa-miR-197-5p, hsa-miR-6845-5p, hsa-miR-4327, hsa-miR-4723-5p, hsa-miR-4530, hsa-miR-6771-5p, hsa-miR-614, hsa-miR-92a-2-5p, hsa-miR-6891-5p, hsa-miR-6124, hsa-miR-4687-3p, hsa-miR-4442, hsa-miR-7977, hsa-miR-6785-5p, hsa-miR-4497, hsa-miR-8071, hsa-miR-663b, hsa-miR-3180, hsa-miR-4251, hsa-miR-1285-3p, hsa-miR-6870-5p, hsa-miR-4484, hsa-miR-4476, hsa-miR-6749-5p, hsa-miR-4454, hsa-miR-6893-5p, hsa-miR-6085, hsa-miR-4787-5p, hsa-miR-149-3p, hsa-miR-7704, hsa-miR-6125, hsa-miR-6090, hsa-miR-3197, hsa-miR-6850-5p, hsa-miR-4467, hsa-miR-6885-5p, hsa-miR-6803-5p, hsa-miR-6798-5p, hsa-miR-6780b-5p, hsa-miR-6768-5p, hsa-miR-5100, hsa-miR-6724-5p, hsa-miR-6879-5p, hsa-miR-7108-5p, hsa-miR-4649-5p, hsa-miR-4739, hsa-miR-6089, hsa-miR-1908-5p, hsa-miR-4516, hsa-miR-2861, hsa-miR-4492, hsa-miR-4294, hsa-miR-6791-5p, hsa-miR-1469, hsa-miR-6752-5p, hsa-miR-4730, hsa-miR-6126, hsa-miR-6869-5p, hsa-miR-1268a, hsa-miR-6799-5p, hsa-miR-8069, hsa-miR-3621, hsa-miR-4763-3p, hsa-miR-1228-5p, hsa-miR-760, hsa-miR-187-5p, hsa-miR-7111-5p, hsa-miR-6088, hsa-miR-6805-3p, hsa-miR-4640-5p, hsa-miR-6721-5p, hsa-miR-6880-5p, hsa-miR-711, hsa-miR-128-1-5p, hsa-miR-4525, hsa-miR-486-3p, hsa-miR-6756-5p, hsa-miR-1260b, hsa-miR-3184-5p, hsa-miR-6075, hsa-miR-204-3p, hsa-miR-4728-5p, hsa-miR-4534, hsa-miR-4758-5p, hsa-miR-8063, hsa-miR-6836-3p, hsa-miR-6789-5p, hsa-miR-744-5p, hsa-miR-1909-3p, hsa-miR-887-3p, hsa-miR-4745-5p, hsa-miR-4433a-3p, hsa-miR-5090, hsa-miR-296-5p, hsa-miR-939-5p, hsa-miR-3648, hsa-miR-3196, hsa-miR-6722-3p, hsa-miR-6805-5p, hsa-miR-1202, hsa-miR-6775-5p, hsa-miR-6087, hsa-miR-6765-5p, hsa-miR-6875-5p, hsa-miR-4674, hsa-miR-1233-5p, hsa-miR-7114-5p, hsa-miR-5787, hsa-miR-8072, hsa-miR-3619-3p, hsa-miR-4632-5p, hsa-miR-6800-5p, hsa-miR-4634, hsa-miR-4486, hsa-miR-6727-5p, hsa-miR-4505, hsa-miR-4725-3p, hsa-miR-1538, hsa-miR-320b, hsa-miR-1915-5p, hsa-miR-328-5p, hsa-miR-6820-5p, hsa-miR-6726-5p, hsa-miR-3665, hsa-miR-638, hsa-miR-762, hsa-miR-4466, hsa-miR-3940-5p, hsa-miR-1237-5p, hsa-miR-575, hsa-miR-3656, hsa-miR-4488, hsa-miR-4281, hsa-miR-6781-5p, hsa-miR-4532, hsa-miR-4665-5p, hsa-miR-6816-5p, hsa-miR-4508, hsa-miR-6784-5p, hsa-miR-6786-5p, hsa-miR-4741, hsa-miR-1343-5p, hsa-miR-1227-5p, hsa-miR-4734, hsa-miR-3960, hsa-miR-128-2-5p, hsa-miR-6743-5p, hsa-miR-663a, hsa-miR-6729-5p, hsa-miR-1915-3p, hsa-miR-1268b, hsa-miR-4651, hsa-miR-3178, and hsa-miR-4463, respectively, which are human miRNA polynucleotides/genes; and congeners thereof, transcripts thereof, and variants or derivatives thereof. Here, the gene, the congener, the transcript, the variant, and the derivative are as defined above.

Target nucleic acids are preferably human miRNA polynucleotides/genes comprising the nucleotide sequences shown by any of SEQ ID NOs: 1 to 200 or transcripts thereof, and more preferably the transcripts, i.e. miRNAs and precursor RNAs thereof, such as pri-miRNAs or pre-miRNAs.

The 1 st target gene is the hsa-miR-3131 gene, a congener thereof, a transcript thereof, or a variant or derivative thereof. None of the previously known reports show that change in the expression of the gene or the transcript thereof can serve as a marker for hippocampal atrophy.

The 2nd target gene is the hsa-miR-6757-5p gene, a congener thereof, a transcript thereof, or a variant or derivative thereof. None of the previously known reports show that change in the expression of the gene or the transcript thereof can serve as a marker for hippocampal atrophy.

The 3rd target gene is the hsa-miR-4706 gene, a congener thereof, a transcript thereof, or a variant or derivative thereof. None of the previously known reports show that change in the expression of the gene or the transcript thereof can serve as a marker for hippocampal atrophy.

The 4th target gene is the hsa-miR-5001-5p gene, a congener thereof, a transcript thereof, or a variant or derivative thereof. None of the previously known reports show that change in the expression of the gene or the transcript thereof can serve as a marker for hippocampal atrophy.

The 5th target gene is the hsa-miR-3180-3p gene, a congener thereof, a transcript thereof, or a variant or derivative thereof. None of the previously known reports show that change in the expression of the gene or the transcript thereof can serve as a marker for hippocampal atrophy.

The 6th target gene is the hsa-miR-642b-3p gene, a congener thereof, a transcript thereof, or a variant or derivative thereof. None of the previously known reports show that change in the expression of the gene or the transcript thereof can serve as a marker for hippocampal atrophy.

The 7th target gene is the hsa-miR-4655-5p gene, a congener thereof, a transcript thereof, or a variant or derivative thereof. None of the previously known reports show that change in the expression of the gene or the transcript thereof can serve as a marker for hippocampal atrophy.

The 8th target gene is the hsa-miR-6819-5p gene, a congener thereof, a transcript thereof, or a variant or derivative thereof. None of the previously known reports show that change in the expression of the gene or the transcript thereof can serve as a marker for hippocampal atrophy.

The 9th target gene is the hsa-miR-937-5p gene, a congener thereof, a transcript thereof, or a variant or derivative thereof. None of the previously known reports show that change in the expression of the gene or the transcript thereof can serve as a marker for hippocampal atrophy.

The 10th target gene is the hsa-miR-4688 gene, a congener thereof, a transcript thereof, or a variant or derivative thereof. None of the previously known reports show that change in the expression of the gene or the transcript thereof can serve as a marker for hippocampal atrophy.

The 11th target gene is the hsa-miR-6741-5p gene, a congener thereof, a transcript thereof, or a variant or derivative thereof. None of the previously known reports show that change in the expression of the gene or the transcript thereof can serve as a marker for hippocampal atrophy.

The 12th target gene is the hsa-miR-7107-5p gene, a congener thereof, a transcript thereof, or a variant or derivative thereof. None of the previously known reports show that change in the expression of the gene or the transcript thereof can serve as a marker for hippocampal atrophy.

The 13th target gene is the hsa-miR-4271 gene, a congener thereof, a transcript thereof, or a variant or derivative thereof. None of the previously known reports show that change in the expression of the gene or the transcript thereof can serve as a marker for hippocampal atrophy.

The 14th target gene is the hsa-miR-1229-5p gene, a congener thereof, a transcript thereof, or a variant or derivative thereof. None of the previously known reports show that change in the expression of the gene or the transcript thereof can serve as a marker for hippocampal atrophy.

The 15th target gene is the hsa-miR-4707-5p gene, a congener thereof, a transcript thereof, or a variant or derivative thereof. None of the previously known reports show that change in the expression of the gene or the transcript thereof can serve as a marker for hippocampal atrophy.

The 16th target gene is the hsa-miR-6808-5p gene, a congener thereof, a transcript thereof, or a variant or derivative thereof. None of the previously known reports show that change in the expression of the gene or the transcript thereof can serve as a marker for hippocampal atrophy.

The 17th target gene is the hsa-miR-4656 gene, a congener thereof, a transcript thereof, or a variant or derivative thereof. None of the previously known reports show that change in the expression of the gene or the transcript thereof can serve as a marker for hippocampal atrophy.

The 18th target gene is the hsa-miR-6076 gene, a congener thereof, a transcript thereof, or a variant or derivative thereof. None of the previously known reports show that change in the expression of the gene or the transcript thereof can serve as a marker for hippocampal atrophy.

The 19th target gene is the hsa-miR-6762-5p gene, a congener thereof, a transcript thereof, or a variant or derivative thereof. None of the previously known reports show that change in the expression of the gene or the transcript thereof can serve as a marker for hippocampal atrophy.

The 20th target gene is the hsa-miR-7109-5p gene, a congener thereof, a transcript thereof, or a variant or derivative thereof. None of the previously known reports show that change in the expression of the gene or the transcript thereof can serve as a marker for hippocampal atrophy.

The 21st target gene is the hsa-miR-6732-5p gene, a congener thereof, a transcript thereof, or a variant or derivative thereof. None of the previously known reports show that change in the expression of the gene or the transcript thereof can serve as a marker for hippocampal atrophy.

The 22nd target gene is the hsa-miR-3195 gene, a congener thereof, a transcript thereof, or a variant or derivative thereof. None of the previously known reports show that change in the expression of the gene or the transcript thereof can serve as a marker for hippocampal atrophy.

The 23rd target gene is the hsa-miR-7150 gene, a congener thereof, a transcript thereof, or a variant or derivative thereof. None of the previously known reports show that change in the expression of the gene or the transcript thereof can serve as a marker for hippocampal atrophy.

The 24th target gene is the hsa-miR-642a-3p gene, a congener thereof, a transcript thereof, or a variant or derivative thereof. None of the previously known reports show that change in the expression of the gene or the transcript thereof can serve as a marker for hippocampal atrophy.

The 25th target gene is the hsa-miR-1249-5p gene, a congener thereof, a transcript thereof, or a variant or derivative thereof. None of the previously known reports show that change in the expression of the gene or the transcript thereof can serve as a marker for hippocampal atrophy.

The 26th target gene is the hsa-miR-3185 gene, a congener thereof, a transcript thereof, or a variant or derivative thereof. None of the previously known reports show that change in the expression of the gene or the transcript thereof can serve as a marker for hippocampal atrophy.

The 27th target gene is the hsa-miR-4689 gene, a congener thereof, a transcript thereof, or a variant or derivative thereof. None of the previously known reports show that change in the expression of the gene or the transcript thereof can serve as a marker for hippocampal atrophy.

The 28th target gene is the hsa-miR-3141 gene, a congener thereof, a transcript thereof, or a variant or derivative thereof. None of the previously known reports show that change in the expression of the gene or the transcript thereof can serve as a marker for hippocampal atrophy.

The 29th target gene is the hsa-miR-6840-3p gene, a congener thereof, a transcript thereof, or a variant or derivative thereof. None of the previously known reports show that change in the expression of the gene or the transcript thereof can serve as a marker for hippocampal atrophy.

The 30th target gene is the hsa-miR-3135b gene, a congener thereof, a transcript thereof, or a variant or derivative thereof. None of the previously known reports show that change in the expression of the gene or the transcript thereof can serve as a marker for hippocampal atrophy.

The 31st target gene is the hsa-miR-1914-3p gene, a congener thereof, a transcript thereof, or a variant or derivative thereof. None of the previously known reports show that change in the expression of the gene or the transcript thereof can serve as a marker for hippocampal atrophy.

The 32nd target gene is the hsa-miR-4446-3p gene, a congener thereof, a transcript thereof, or a variant or derivative thereof. None of the previously known reports show that change in the expression of the gene or the transcript thereof can serve as a marker for hippocampal atrophy.

The 33rd target gene is the hsa-miR-4433b-3p gene, a congener thereof, a transcript thereof, or a variant or derivative thereof. None of the previously known reports show that change in the expression of the gene or the transcript thereof can serve as a marker for hippocampal atrophy.

The 34th target gene is the hsa-miR-6877-5p gene, a congener thereof, a transcript thereof, or a variant or derivative thereof. None of the previously known reports show that change in the expression of the gene or the transcript thereof can serve as a marker for hippocampal atrophy.

The 35th target gene is the hsa-miR-6848-5p gene, a congener thereof, a transcript thereof, or a variant or derivative thereof. None of the previously known reports show that change in the expression of the gene or the transcript thereof can serve as a marker for hippocampal atrophy.

The 36th target gene is the hsa-miR-3620-5p gene, a congener thereof, a transcript thereof, or a variant or derivative thereof. None of the previously known reports show that change in the expression of the gene or the transcript thereof can serve as a marker for hippocampal atrophy.

The 37th target gene is the hsa-miR-6825-5p gene, a congener thereof, a transcript thereof, or a variant or derivative thereof. None of the previously known reports show that change in the expression of the gene or the transcript thereof can serve as a marker for hippocampal atrophy.

The 38th target gene is the hsa-miR-5739 gene, a congener thereof, a transcript thereof, or a variant or derivative thereof. None of the previously known reports show that change in the expression of the gene or the transcript thereof can serve as a marker for hippocampal atrophy.

The 39th target gene is the hsa-miR-3663-3p gene, a congener thereof, a transcript thereof, or a variant or derivative thereof. None of the previously known reports show that change in the expression of the gene or the transcript thereof can serve as a marker for hippocampal atrophy.

The 40th target gene is the hsa-miR-4695-5p gene, a congener thereof, a transcript thereof, or a variant or derivative thereof. None of the previously known reports show that change in the expression of the gene or the transcript thereof can serve as a marker for hippocampal atrophy.

The 41st target gene is the hsa-miR-3162-5p gene, a congener thereof, a transcript thereof, or a variant or derivative thereof. None of the previously known reports show that change in the expression of the gene or the transcript thereof can serve as a marker for hippocampal atrophy.

The 42nd target gene is the hsa-miR-3679-5p gene, a congener thereof, a transcript thereof, or a variant or derivative thereof. None of the previously known reports show that change in the expression of the gene or the transcript thereof can serve as a marker for hippocampal atrophy.

The 43rd target gene is the hsa-miR-8059 gene, a congener thereof, a transcript thereof, or a variant or derivative thereof. None of the previously known reports show that change in the expression of the gene or the transcript thereof can serve as a marker for hippocampal atrophy.

The 44th target gene is the hsa-miR-7110-5p gene, a congener thereof, a transcript thereof, or a variant or derivative thereof. None of the previously known reports show that change in the expression of the gene or the transcript thereof can serve as a marker for hippocampal atrophy.

The 45th target gene is the hsa-miR-1275 gene, a congener thereof, a transcript thereof, or a variant or derivative thereof. None of the previously known reports show that change in the expression of the gene or the transcript thereof can serve as a marker for hippocampal atrophy.

The 46th target gene is the hsa-miR-6779-5p gene, a congener thereof, a transcript thereof, or a variant or derivative thereof. None of the previously known reports show that change in the expression of the gene or the transcript thereof can serve as a marker for hippocampal atrophy.

The 47th target gene is the hsa-miR-197-5p gene, a congener thereof, a transcript thereof, or a variant or derivative thereof. None of the previously known reports show that change in the expression of the gene or the transcript thereof can serve as a marker for hippocampal atrophy.

The 48th target gene is the hsa-miR-6845-5p gene, a congener thereof, a transcript thereof, or a variant or derivative thereof. None of the previously known reports show that change in the expression of the gene or the transcript thereof can serve as a marker for hippocampal atrophy.

The 49th target gene is the hsa-miR-4327 gene, a congener thereof, a transcript thereof, or a variant or derivative thereof. None of the previously known reports show that change in the expression of the gene or the transcript thereof can serve as a marker for hippocampal atrophy.

The 50th target gene is the hsa-miR-4723-5p gene, a congener thereof, a transcript thereof, or a variant or derivative thereof. None of the previously known reports show that change in the expression of the gene or the transcript thereof can serve as a marker for hippocampal atrophy.

The 51 st target gene is the hsa-miR-4530 gene, a congener thereof, a transcript thereof, or a variant or derivative thereof. None of the previously known reports show that change in the expression of the gene or the transcript thereof can serve as a marker for hippocampal atrophy.

The 52nd target gene is the hsa-miR-6771-5p gene, a congener thereof, a transcript thereof, or a variant or derivative thereof. None of the previously known reports show that change in the expression of the gene or the transcript thereof can serve as a marker for hippocampal atrophy.

The 53rd target gene is the hsa-miR-614 gene, a congener thereof, a transcript thereof, or a variant or derivative thereof. None of the previously known reports show that change in the expression of the gene or the transcript thereof can serve as a marker for hippocampal atrophy.

The 54th target gene is the hsa-miR-92a-2-5p gene, a congener thereof, a transcript thereof, or a variant or derivative thereof. None of the previously known reports show that change in the expression of the gene or the transcript thereof can serve as a marker for hippocampal atrophy.

The 55th target gene is the hsa-miR-6891-5p gene, a congener thereof, a transcript thereof, or a variant or derivative thereof. None of the previously known reports show that change in the expression of the gene or the transcript thereof can serve as a marker for hippocampal atrophy.

The 56th target gene is the hsa-miR-6124 gene, a congener thereof, a transcript thereof, or a variant or derivative thereof. None of the previously known reports show that change in the expression of the gene or the transcript thereof can serve as a marker for hippocampal atrophy.

The 57th target gene is the hsa-miR-4687-3p gene, a congener thereof, a transcript thereof, or a variant or derivative thereof. None of the previously known reports show that change in the expression of the gene or the transcript thereof can serve as a marker for hippocampal atrophy.

The 58th target gene is the hsa-miR-4442 gene, a congener thereof, a transcript thereof, or a variant or derivative thereof. None of the previously known reports show that change in the expression of the gene or the transcript thereof can serve as a marker for hippocampal atrophy.

The 59th target gene is the hsa-miR-7977 gene, a congener thereof, a transcript thereof, or a variant or derivative thereof. None of the previously known reports show that change in the expression of the gene or the transcript thereof can serve as a marker for hippocampal atrophy.

The 60th target gene is the hsa-miR-6785-5p gene, a congener thereof, a transcript thereof, or a variant or derivative thereof. None of the previously known reports show that change in the expression of the gene or the transcript thereof can serve as a marker for hippocampal atrophy.

The 61 st target gene is the hsa-miR-4497 gene, a congener thereof, a transcript thereof, or a variant or derivative thereof. None of the previously known reports show that change in the expression of the gene or the transcript thereof can serve as a marker for hippocampal atrophy.

The 62nd target gene is the hsa-miR-8071 gene, a congener thereof, a transcript thereof, or a variant or derivative thereof. None of the previously known reports show that change in the expression of the gene or the transcript thereof can serve as a marker for hippocampal atrophy.

The 63rd target gene is the hsa-miR-663b gene, a congener thereof, a transcript thereof, or a variant or derivative thereof. None of the previously known reports show that change in the expression of the gene or the transcript thereof can serve as a marker for hippocampal atrophy.

The 64th target gene is the hsa-miR-3180 gene, a congener thereof, a transcript thereof, or a variant or derivative thereof. None of the previously known reports show that change in the expression of the gene or the transcript thereof can serve as a marker for hippocampal atrophy.

The 65th target gene is the hsa-miR-4251 gene, a congener thereof, a transcript thereof, or a variant or derivative thereof. None of the previously known reports show that change in the expression of the gene or the transcript thereof can serve as a marker for hippocampal atrophy.

The 66th target gene is the hsa-miR-1285-3p gene, a congener thereof, a transcript thereof, or a variant or derivative thereof. None of the previously known reports show that change in the expression of the gene or the transcript thereof can serve as a marker for hippocampal atrophy.

The 67th target gene is the hsa-miR-6870-5p gene, a congener thereof, a transcript thereof, or a variant or derivative thereof. None of the previously known reports show that change in the expression of the gene or the transcript thereof can serve as a marker for hippocampal atrophy.

The 68th target gene is the hsa-miR-4484 gene, a congener thereof, a transcript thereof, or a variant or derivative thereof. None of the previously known reports show that change in the expression of the gene or the transcript thereof can serve as a marker for hippocampal atrophy.

The 69th target gene is the hsa-miR-4476 gene, a congener thereof, a transcript thereof, or a variant or derivative thereof. None of the previously known reports show that change in the expression of the gene or the transcript thereof can serve as a marker for hippocampal atrophy.

The 70th target gene is the hsa-miR-6749-5p gene, a congener thereof, a transcript thereof, or a variant or derivative thereof. None of the previously known reports show that change in the expression of the gene or the transcript thereof can serve as a marker for hippocampal atrophy.

The 71 st target gene is the hsa-miR-4454 gene, a congener thereof, a transcript thereof, or a variant or derivative thereof. None of the previously known reports show that change in the expression of the gene or the transcript thereof can serve as a marker for hippocampal atrophy.

The 72nd target gene is the hsa-miR-6893-5p gene, a congener thereof, a transcript thereof, or a variant or derivative thereof. None of the previously known reports show that change in the expression of the gene or the transcript thereof can serve as a marker for hippocampal atrophy.

The 73rd target gene is the hsa-miR-6085 gene, a congener thereof, a transcript thereof, or a variant or derivative thereof. None of the previously known reports show that change in the expression of the gene or the transcript thereof can serve as a marker for hippocampal atrophy.

The 74th target gene is the hsa-miR-4787-5p gene, a congener thereof, a transcript thereof, or a variant or derivative thereof. None of the previously known reports show that change in the expression of the gene or the transcript thereof can serve as a marker for hippocampal atrophy.

The 75th target gene is the hsa-miR-149-3p gene, a congener thereof, a transcript thereof, or a variant or derivative thereof. None of the previously known reports show that change in the expression of the gene or the transcript thereof can serve as a marker for hippocampal atrophy.

The 76th target gene is the hsa-miR-7704 gene, a congener thereof, a transcript thereof, or a variant or derivative thereof. None of the previously known reports show that change in the expression of the gene or the transcript thereof can serve as a marker for hippocampal atrophy.

The 77th target gene is the hsa-miR-6125 gene, a congener thereof, a transcript thereof, or a variant or derivative thereof. None of the previously known reports show that change in the expression of the gene or the transcript thereof can serve as a marker for hippocampal atrophy.

The 78th target gene is the hsa-miR-6090 gene, a congener thereof, a transcript thereof, or a variant or derivative thereof. None of the previously known reports show that change in the expression of the gene or the transcript thereof can serve as a marker for hippocampal atrophy.

The 79th target gene is the hsa-miR-3197 gene, a congener thereof, a transcript thereof, or a variant or derivative thereof. None of the previously known reports show that change in the expression of the gene or the transcript thereof can serve as a marker for hippocampal atrophy.

The 80th target gene is the hsa-miR-6850-5p gene, a congener thereof, a transcript thereof, or a variant or derivative thereof. None of the previously known reports show that change in the expression of the gene or the transcript thereof can serve as a marker for hippocampal atrophy.

The 81 st target gene is the hsa-miR-4467 gene, a congener thereof, a transcript thereof, or a variant or derivative thereof. None of the previously known reports show that change in the expression of the gene or the transcript thereof can serve as a marker for hippocampal atrophy.

The 82nd target gene is the hsa-miR-6885-5p gene, a congener thereof, a transcript thereof, or a variant or derivative thereof. None of the previously known reports show that change in the expression of the gene or the transcript thereof can serve as a marker for hippocampal atrophy.

The 83rd target gene is the hsa-miR-6803-5p gene, a congener thereof, a transcript thereof, or a variant or derivative thereof. None of the previously known reports show that change in the expression of the gene or the transcript thereof can serve as a marker for hippocampal atrophy.

The 84th target gene is the hsa-miR-6798-5p gene, a congener thereof, a transcript thereof, or a variant or derivative thereof. None of the previously known reports show that change in the expression of the gene or the transcript thereof can serve as a marker for hippocampal atrophy.

The 85th target gene is the hsa-miR-6780b-5p gene, a congener thereof, a transcript thereof, or a variant or derivative thereof. None of the previously known reports show that change in the expression of the gene or the transcript thereof can serve as a marker for hippocampal atrophy.

The 86th target gene is the hsa-miR-6768-5p gene, a congener thereof, a transcript thereof, or a variant or derivative thereof. None of the previously known reports show that change in the expression of the gene or the transcript thereof can serve as a marker for hippocampal atrophy.

The 87th target gene is the hsa-miR-5100 gene, a congener thereof, a transcript thereof, or a variant or derivative thereof. None of the previously known reports show that change in the expression of the gene or the transcript thereof can serve as a marker for hippocampal atrophy.

The 88th target gene is the hsa-miR-6724-5p gene, a congener thereof, a transcript thereof, or a variant or derivative thereof. None of the previously known reports show that change in the expression of the gene or the transcript thereof can serve as a marker for hippocampal atrophy.

The 89th target gene is the hsa-miR-6879-5p gene, a congener thereof, a transcript thereof, or a variant or derivative thereof. None of the previously known reports show that change in the expression of the gene or the transcript thereof can serve as a marker for hippocampal atrophy.

The 90th target gene is the hsa-miR-7108-5p gene, a congener thereof, a transcript thereof, or a variant or derivative thereof. None of the previously known reports show that change in the expression of the gene or the transcript thereof can serve as a marker for hippocampal atrophy.

The 91st target gene is the hsa-miR-4649-5p gene, a congener thereof, a transcript thereof, or a variant or derivative thereof. None of the previously known reports show that change in the expression of the gene or the transcript thereof can serve as a marker for hippocampal atrophy.

The 92nd target gene is the hsa-miR-4739 gene, a congener thereof, a transcript thereof, or a variant or derivative thereof. None of the previously known reports show that change in the expression of the gene or the transcript thereof can serve as a marker for hippocampal atrophy.

The 93rd target gene is the hsa-miR-6089 gene, a congener thereof, a transcript thereof, or a variant or derivative thereof. None of the previously known reports show that change in the expression of the gene or the transcript thereof can serve as a marker for hippocampal atrophy.

The 94th target gene is the hsa-miR-1908-5p gene, a congener thereof, a transcript thereof, or a variant or derivative thereof. None of the previously known reports show that change in the expression of the gene or the transcript thereof can serve as a marker for hippocampal atrophy.

The 95th target gene is the hsa-miR-4516 gene, a congener thereof, a transcript thereof, or a variant or derivative thereof. None of the previously known reports show that change in the expression of the gene or the transcript thereof can serve as a marker for hippocampal atrophy.

The 96th target gene is the hsa-miR-2861 gene, a congener thereof, a transcript thereof, or a variant or derivative thereof. None of the previously known reports show that change in the expression of the gene or the transcript thereof can serve as a marker for hippocampal atrophy.

The 97th target gene is the hsa-miR-4492 gene, a congener thereof, a transcript thereof, or a variant or derivative thereof. None of the previously known reports show that change in the expression of the gene or the transcript thereof can serve as a marker for hippocampal atrophy.

The 98th target gene is the hsa-miR-4294 gene, a congener thereof, a transcript thereof, or a variant or derivative thereof. None of the previously known reports show that change in the expression of the gene or the transcript thereof can serve as a marker for hippocampal atrophy.

The 99th target gene is the hsa-miR-6791-5p gene, a congener thereof, a transcript thereof, or a variant or derivative thereof. None of the previously known reports show that change in the expression of the gene or the transcript thereof can serve as a marker for hippocampal atrophy.

The 100th target gene is the hsa-miR-1469 gene, a congener thereof, a transcript thereof, or a variant or derivative thereof. None of the previously known reports show that change in the expression of the gene or the transcript thereof can serve as a marker for hippocampal atrophy.

The 101st target gene is the hsa-miR-6752-5p gene, a congener thereof, a transcript thereof, or a variant or derivative thereof. None of the previously known reports show that change in the expression of the gene or the transcript thereof can serve as a marker for hippocampal atrophy.

The 102nd target gene is the hsa-miR-4730 gene, a congener thereof, a transcript thereof, or a variant or derivative thereof. None of the previously known reports show that change in the expression of the gene or the transcript thereof can serve as a marker for hippocampal atrophy.

The 103rd target gene is the hsa-miR-6126 gene, a congener thereof, a transcript thereof, or a variant or derivative thereof. None of the previously known reports show that change in the expression of the gene or the transcript thereof can serve as a marker for hippocampal atrophy.

The 104th target gene is the hsa-miR-6869-5p gene, a congener thereof, a transcript thereof, or a variant or derivative thereof. None of the previously known reports show that change in the expression of the gene or the transcript thereof can serve as a marker for hippocampal atrophy.

The 105th target gene is the hsa-miR-1268a gene, a congener thereof, a transcript thereof, or a variant or derivative thereof. None of the previously known reports show that change in the expression of the gene or the transcript thereof can serve as a marker for hippocampal atrophy.

The 106th target gene is the hsa-miR-6799-5p gene, a congener thereof, a transcript thereof, or a variant or derivative thereof. None of the previously known reports show that change in the expression of the gene or the transcript thereof can serve as a marker for hippocampal atrophy.

The 107th target gene is the hsa-miR-8069 gene, a congener thereof, a transcript thereof, or a variant or derivative thereof. None of the previously known reports show that change in the expression of the gene or the transcript thereof can serve as a marker for hippocampal atrophy.

The 108th target gene is the hsa-miR-3621 gene, a congener thereof, a transcript thereof, or a variant or derivative thereof. None of the previously known reports show that change in the expression of the gene or the transcript thereof can serve as a marker for hippocampal atrophy.

The 109th target gene is the hsa-miR-4763-3p gene, a congener thereof, a transcript thereof, or a variant or derivative thereof. None of the previously known reports show that change in the expression of the gene or the transcript thereof can serve as a marker for hippocampal atrophy.

The 110th target gene is the hsa-miR-1228-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 111th target gene is the hsa-miR-760 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 5).

The 112th target gene is the hsa-miR-187-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 7).

The 113rd target gene is the hsa-miR-7111-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 114th target gene is the hsa-miR-6088 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 115th target gene is the hsa-miR-6805-3p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 116th target gene is the hsa-miR-4640-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 117th target gene is the hsa-miR-6721-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 118th target gene is the hsa-miR-6880-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 119th target gene is the hsa-miR-711 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 7).

The 120th target gene is the hsa-miR-128-1-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 7).

The 121st target gene is the hsa-miR-4525 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 122nd target gene is the hsa-miR-486-3p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 2).

The 123rd target gene is the hsa-miR-6756-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 124th target gene is the hsa-miR-1260b gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 125th target gene is the hsa-miR-3184-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 126th target gene is the hsa-miR-6075 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 127th target gene is the hsa-miR-204-3p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Non-Patent Literature 5).

The 128th target gene is the hsa-miR-4728-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 129th target gene is the hsa-miR-4534 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 130th target gene is the hsa-miR-4758-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 131st target gene is the hsa-miR-8063 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 132nd target gene is the hsa-miR-6836-3p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 133rd target gene is the hsa-miR-6789-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 134th target gene is the hsa-miR-744-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 135th target gene is the hsa-miR-1909-3p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 136th target gene is the hsa-miR-887-3p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 5).

The 137th target gene is the hsa-miR-4745-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 138th target gene is the hsa-miR-4433a-3p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 139th target gene is the hsa-miR-5090 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 140th target gene is the hsa-miR-296-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 141st target gene is the hsa-miR-939-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 142nd target gene is the hsa-miR-3648 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 143rd target gene is the hsa-miR-3196 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 144th target gene is the hsa-miR-6722-3p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Non-Patent Literature 4).

The 145th target gene is the hsa-miR-6805-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 3).

The 146th target gene is the hsa-miR-1202 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 147th target gene is the hsa-miR-6775-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 148th target gene is the hsa-miR-6087 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 149th target gene is the hsa-miR-6765-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 150th target gene is the hsa-miR-6875-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 151st target gene is the hsa-miR-4674 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Non-Patent Literature 4).

The 152nd target gene is the hsa-miR-1233-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 153rd target gene is the hsa-miR-7114-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 154th target gene is the hsa-miR-5787 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 155th target gene is the hsa-miR-8072 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 156th target gene is the hsa-miR-3619-3p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 157th target gene is the hsa-miR-4632-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 158th target gene is the hsa-miR-6800-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 159th target gene is the hsa-miR-4634 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 160th target gene is the hsa-miR-4486 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 161st target gene is the hsa-miR-6727-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 162nd target gene is the hsa-miR-4505 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 163rd target gene is the hsa-miR-4725-3p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 164th target gene is the hsa-miR-1538 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 165th target gene is the hsa-miR-320b gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 4).

The 166th target gene is the hsa-miR-1915-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 167th target gene is the hsa-miR-328-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 7).

The 168th target gene is the hsa-miR-6820-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 169th target gene is the hsa-miR-6726-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 170th target gene is the hsa-miR-3665 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 171 st target gene is the hsa-miR-63 8 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 172nd target gene is the hsa-miR-762 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 173rd target gene is the hsa-miR-4466 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 174th target gene is the hsa-miR-3940-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 175th target gene is the hsa-miR-1237-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 176th target gene is the hsa-miR-575 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 177th target gene is the hsa-miR-3656 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 178th target gene is the hsa-miR-4488 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 179th target gene is the hsa-miR-4281 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 180th target gene is the hsa-miR-6781-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 181st target gene is the hsa-miR-4532 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 182nd target gene is the hsa-miR-4665-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 183rd target gene is the hsa-miR-6816-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 184th target gene is the hsa-miR-4508 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 185th target gene is the hsa-miR-6784-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 186th target gene is the hsa-miR-6786-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 187th target gene is the hsa-miR-4741 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 188th target gene is the hsa-miR-1343-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 189th target gene is the hsa-miR-1227-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 190th target gene is the hsa-miR-4734 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 191st target gene is the hsa-miR-3960 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 192nd target gene is the hsa-miR-128-2-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 7).

The 193rd target gene is the hsa-miR-6743-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 194th target gene is the hsa-miR-663a gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Non-Patent Literature 3).

The 195th target gene is the hsa-miR-6729-5p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 196th target gene is the hsa-miR-1915-3p gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 197th target gene is the hsa-miR-1268b gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 198th target gene is the hsa-miR-4651 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 199th target gene is the hsa-miR-3178 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 1).

The 200th target gene is the hsa-miR-4463 gene, a congener thereof, a transcript thereof, or a variant or derivative thereof. The previously known report shows that change in the expression of the gene or the transcript thereof can serve as a marker for hippocampal atrophy or a disease characterized by hippocampal atrophy (Patent Literature 6).

2. Nucleic acids for detection of hippocampal atrophy

In the present invention, a nucleic acid(s) for detection of hippocampal atrophy, for example, a nucleic acid(s) that can be used for diagnosis of hippocampal atrophy, such as a nucleic acid probe(s) or primer(s), may be a nucleic acid(s) capable of specifically binding to, as a target nucleic acid(s) for hippocampal atrophy (or a hippocampal atrophy marker(s)), one or more miRNA polynucleotide(s) selected from the group consisting of: miR-3131, miR-6757-5p, miR-4706, miR-5001-5p, miR-3180-3p, miR-642b-3p, miR-4655-5p, miR-6819-5p, miR-937-5p, miR-4688, miR-6741-5p, miR-7107-5p, miR-4271, miR-1229-5p, miR-4707-5p, miR-6808-5p, miR-4656, miR-6076, miR-6762-5p, miR-7109-5p, miR-6732-5p, miR-3195, miR-7150, miR-642a-3p, miR-1249-5p, miR-3185, miR-4689, miR-3141, miR-6840-3p, miR-3135b, miR-1914-3p, miR-4446-3p, miR-4433b-3p, miR-6877-5p, miR-6848-5p, miR-3620-5p, miR-6825-5p, miR-5739, miR-3663-3p, miR-4695-5p, miR-3162-5p, miR-3679-5p, miR-8059, miR-7110-5p, miR-1275, miR-6779-5p, miR-197-5p, miR-6845-5p, miR-4327, miR-4723-5p, miR-4530, miR-6771-5p, miR-614, miR-92a-2-5p, miR-6891-5p, miR-6124, miR-4687-3p, miR-4442, miR-7977, miR-6785-5p, miR-4497, miR-8071, miR-663b, miR-3180, miR-4251, miR-1285-3p, miR-6870-5p, miR-4484, miR-4476, miR-6749-5p, miR-4454, miR-6893-5p, miR-6085, miR-4787-5p, miR-149-3p, miR-7704, miR-6125, miR-6090, miR-3197, miR-6850-5p, miR-4467, miR-6885-5p, miR-6803-5p, miR-6798-5p, miR-6780b-5p, miR-6768-5p, miR-5100, miR-6724-5p, miR-6879-5p, miR-7108-5p, miR-4649-5p, miR-4739, miR-6089, miR-1908-5p, miR-4516, miR-2861, miR-4492, miR-4294, miR-6791-5p, miR-1469, miR-6752-5p, miR-4730, miR-6126, miR-6869-5p, miR-1268a, miR-6799-5p, miR-8069, miR-3621, and miR-4763-3p, or to a complementary strand(s) of the polynucleotide(s).

In one embodiment, a nucleic acid(s), such as a nucleic acid probe(s) or primer(s), that can be used for detection of hippocampal atrophy or for diagnosis of hippocampal atrophy may specifically bind to human-derived one or more miRNA polynucleotide(s) selected from the group consisting of: hsa-miR-3131, hsa-miR-6757-5p, hsa-miR-4706, hsa-miR-5001-5p, hsa-miR-3180-3p, hsa-miR-642b-3p, hsa-miR-4655-5p, hsa-miR-6819-5p, hsa-miR-937-5p, hsa-miR-4688, hsa-miR-6741-5p, hsa-miR-7107-5p, hsa-miR-4271, hsa-miR-1229-5p, hsa-miR-4707-5p, hsa-miR-6808-5p, hsa-miR-4656, hsa-miR-6076, hsa-miR-6762-5p, hsa-miR-7109-5p, hsa-miR-6732-5p, hsa-miR-3195, hsa-miR-7150, hsa-miR-642a-3p, hsa-miR-1249-5p, hsa-miR-3185, hsa-miR-4689, hsa-miR-3141, hsa-miR-6840-3p, hsa-miR-3135b, hsa-miR-1914-3p, hsa-miR-4446-3p, hsa-miR-4433b-3p, hsa-miR-6877-5p, hsa-miR-6848-5p, hsa-miR-3620-5p, hsa-miR-6825-5p, hsa-miR-5739, hsa-miR-3663-3p, hsa-miR-4695-5p, hsa-miR-3162-5p, hsa-miR-3679-5p, hsa-miR-8059, hsa-miR-7110-5p, hsa-miR-1275, hsa-miR-6779-5p, hsa-miR-197-5p, hsa-miR-6845-5p, hsa-miR-4327, hsa-miR-4723-5p, hsa-miR-4530, hsa-miR-6771-5p, hsa-miR-614, hsa-miR-92a-2-5p, hsa-miR-6891-5p, hsa-miR-6124, hsa-miR-4687-3p, hsa-miR-4442, hsa-miR-7977, hsa-miR-6785-5p, hsa-miR-4497, hsa-miR-8071, hsa-miR-663b, hsa-miR-3180, hsa-miR-4251, hsa-miR-1285-3p, hsa-miR-6870-5p, hsa-miR-4484, hsa-miR-4476, hsa-miR-6749-5p, hsa-miR-4454, hsa-miR-6893-5p, hsa-miR-6085, hsa-miR-4787-5p, hsa-miR-149-3p, hsa-miR-7704, hsa-miR-6125, hsa-miR-6090, hsa-miR-3197, hsa-miR-6850-5p, hsa-miR-4467, hsa-miR-6885-5p, hsa-miR-6803-5p, hsa-miR-6798-5p, hsa-miR-6780b-5p, hsa-miR-6768-5p, hsa-miR-5100, hsa-miR-6724-5p, hsa-miR-6879-5p, hsa-miR-7108-5p, hsa-miR-4649-5p, hsa-miR-4739, hsa-miR-6089, hsa-miR-1908-5p, hsa-miR-4516, hsa-miR-2861, hsa-miR-4492, hsa-miR-4294, hsa-miR-6791-5p, hsa-miR-1469, hsa-miR-6752-5p, hsa-miR-4730, hsa-miR-6126, hsa-miR-6869-5p, hsa-miR-1268a, hsa-miR-6799-5p, hsa-miR-8069, hsa-miR-3621, and hsa-miR-4763-3p, or a complementary strand(s) of the polynucleotide(s). When the nucleic acid is a primer, the nucleic acid may be a nucleic acid that is capable of specifically binding to and amplifying the above-mentioned miRNA polynucleotide or a complementary stand of the polynucleotide. Primers may be a set of primers, such as a primer pair, that is capable of specifically binding to and amplifying the above-mentioned miRNA polynucleotide or a complementary stand of the polynucleotide.

The hippocampal atrophy marker miRNA(s) described above can be used for detection of hippocampal atrophy, in combination with a nucleic acid(s) capable of specifically binding to one or more polynucleotide(s) selected from the group consisting of other hippocampal atrophy markers: miR-1228-5p, miR-760, miR-187-5p, miR-7111-5p, miR-6088, miR-6805-3p, miR-4640-5p, miR-6721-5p, miR-6880-5p, miR-711, miR-128-1-5p, miR-4525, miR-486-3p, miR-6756-5p, miR-1260b, miR-3184-5p, miR-6075, miR-204-3p, miR-4728-5p, miR-4534, miR-4758-5p, miR-8063, miR-6836-3p, miR-6789-5p, miR-744-5p, miR-1909-3p, miR-887-3p, miR-4745-5p, miR-4433a-3p, miR-5090, miR-296-5p, miR-939-5p, miR-3648, miR-3196, miR-6722-3p, miR-6805-5p, miR-1202, miR-6775-5p, miR-6087, miR-6765-5p, miR-6875-5p, miR-4674, miR-1233-5p, miR-7114-5p, miR-5787, miR-8072, miR-3619-3p, miR-4632-5p, miR-6800-5p, miR-4634, miR-4486, miR-6727-5p, miR-4505, miR-4725-3p, miR-1538, miR-320b, miR-1915-5p, miR-328-5p, miR-6820-5p, miR-6726-5p, miR-3665, miR-638, miR-762, miR-4466, miR-3940-5p, miR-1237-5p, miR-575, miR-3656, miR-4488, miR-4281, miR-6781-5p, miR-4532, miR-4665-5p, miR-6816-5p, miR-4508, miR-6784-5p, miR-6786-5p, miR-4741, miR-1343-5p, miR-1227-5p, miR-4734, miR-3960, miR-128-2-5p, miR-6743-5p, miR-663a, miR-6729-5p, miR-1915-3p, miR-1268b, miR-4651, miR-3178, and miR-4463, or to a complementary strand(s) of the polynucleotide(s).

For example, a nucleic acid(s) capable of specifically binding to one or more polynucleotide(s) selected from the group consisting of: hsa-miR-1228-5p, hsa-miR-760, hsa-miR-187-5p, hsa-miR-7111-5p, hsa-miR-6088, hsa-miR-6805-3p, hsa-miR-4640-5p, hsa-miR-6721-5p, hsa-miR-6880-5p, hsa-miR-711, hsa-miR-128-1-5p, hsa-miR-4525, hsa-miR-486-3p, hsa-miR-6756-5p, hsa-miR-1260b, hsa-miR-3184-5p, hsa-miR-6075, hsa-miR-204-3p, hsa-miR-4728-5p, hsa-miR-4534, hsa-miR-4758-5p, hsa-miR-8063, hsa-miR-6836-3p, hsa-miR-6789-5p, hsa-miR-744-5p, hsa-miR-1909-3p, hsa-miR-887-3p, hsa-miR-4745-5p, hsa-miR-4433a-3p, hsa-miR-5090, hsa-miR-296-5p, hsa-miR-939-5p, hsa-miR-3648, hsa-miR-3196, hsa-miR-6722-3p, hsa-miR-6805-5p, hsa-miR-1202, hsa-miR-6775-5p, hsa-miR-6087, hsa-miR-6765-5p, hsa-miR-6875-5p, hsa-miR-4674, hsa-miR-1233-5p, hsa-miR-7114-5p, hsa-miR-5787, hsa-miR-8072, hsa-miR-3619-3p, hsa-miR-4632-5p, hsa-miR-6800-5p, hsa-miR-4634, hsa-miR-4486, hsa-miR-6727-5p, hsa-miR-4505, hsa-miR-4725-3p, hsa-miR-1538, hsa-miR-320b, hsa-miR-1915-5p, hsa-miR-328-5p, hsa-miR-6820-5p, hsa-miR-6726-5p, hsa-miR-3665, hsa-miR-638, hsa-miR-762, hsa-miR-4466, hsa-miR-3940-5p, hsa-miR-1237-5p, hsa-miR-575, hsa-miR-3656, hsa-miR-4488, hsa-miR-4281, hsa-miR-6781-5p, hsa-miR-4532, hsa-miR-4665-5p, hsa-miR-6816-5p, hsa-miR-4508, hsa-miR-6784-5p, hsa-miR-6786-5p, hsa-miR-4741, hsa-miR-1343-5p, hsa-miR-1227-5p, hsa-miR-4734, hsa-miR-3960, hsa-miR-128-2-5p, hsa-miR-6743-5p, hsa-miR-663a, hsa-miR-6729-5p, hsa-miR-1915-3p, hsa-miR-1268b, hsa-miR-4651, hsa-miR-3178, and hsa-miR-4463 or to a complementary strand(s) of the polynucleotide(s) can be used in combination with the hippocampal atrophy marker(s) described above, for detection of hippocampal atrophy.

The above-mentioned nucleic acids for detection of hippocampal atrophy, such as nucleic acid probes or primers, are capable of detection of hippocampal atrophy markers. The nucleic acids for detection of hippocampal atrophy can be used individually or in combinations of two or more thereof, to enable qualitative and/or quantitative measurement of the presence, expression levels, or amounts of the hippocampal atrophy marker miRNAs, such as polynucleotides/genes comprising nucleotide sequences shown by SEQ ID Nos: 1 to 200, or congeners thereof, transcripts thereof, or variants or derivatives thereof.

In one embodiment, the nucleic acid(s), such as a nucleic acid probe(s) or primer(s), that can be used in the present invention is a nucleic acid probe(s) capable of specifically binding to a polynucleotide(s) consisting of nucleotide sequence(s) shown by at least one selected from SEQ ID NOs: 1 to 109 or to a complementary strand(s) of the polynucleotide(s); or a primer(s) capable of amplifying a polynucleotide(s) consisting of a nucleotide sequence(s) shown by at least one selected from SEQ ID NOs: 1 to 109.

In one embodiment, the nucleic acids, such as nucleic acid probes or primers, that can be used in the present invention may further comprise a nucleic acid probe(s) capable of specifically binding to a polynucleotide(s) consisiting of a nucleotide sequence(s) shown by at least one selected from SEQ ID NOs: 110 to 200 or to a complementary strand(s) of the polynucleotide(s); or a primer(s) capable of amplifying a polynucleotide(s) consisting of a nucleotide sequence(s) shown by at least one selected from SEQ ID NOs: 110 to 200.

In one embodiment, a nucleic acid(s), such as a nucleic acid probe(s) or primer(s), that can be used in the present invention include any combination of one or more polynucleotides selected from a group of polynucleotides each comprising nucleotide sequences shown by any of SEQ ID NOs: 1 to 740 and nucleotide sequences derived from the nucleotide sequences by the replacement of u with t and a group of polynucleotides complementary thereto, a group of polynucleotides hybridizing under stringent conditions (described below) to DNAs comprising nucleotide sequences complementary to the former nucleotide sequences and a group of polynucleotides complementary thereto, and a group of polynucleotides comprising 15 or more, preferably 17 or more consecutive nucleotides in the nucleotide sequences of these polynucleotide groups. These polynucleotides can be used as nucleic acids, such as nucleic acid probes and primers, for detecting target nucleic acids, namely, the above-mentioned hippocampal atrophy marker miRNAs.

In one embodiment, examples of a nucleic acid(s), such as a nucleic acid probe(s) or primer(s), that can be used in the present invention include one or more polynucleotides selected from the group consisting of the following polynucleotides (a) to (e):

(a) a polynucleotide consisting of a nucleotide sequence shown by any of SEQ ID NOs: 1 to 109 or a nucleotide sequence derived from the nucleotide sequence by the replacement of u with t, a variant thereof, a derivative thereof, or a fragment thereof comprising 15 or more consecutive nucleotides;

(b) a polynucleotide comprising a nucleotide sequence shown by any of SEQ ID NOs: 1 to 109, a variant thereof, a derivative thereof, or a fragment thereof comprising 15 or more consecutive nucleotides;

(c) a polynucleotide consisting of a nucleotide sequence complementary to a nucleotide sequence shown by any of SEQ ID NOs: 1 to 109 or a nucleotide sequence derived from the nucleotide sequence by the replacement of u with t, a variant thereof, a derivative thereof, or a fragment thereof comprising 15 or more consecutive nucleotides;

(d) a polynucleotide comprising a nucleotide sequence complementary to a nucleotide sequence shown by any of SEQ ID NOs: 1 to 109 or a nucleotide sequence derived from the nucleotide sequence by the replacement of u with t, a variant thereof, a derivative thereof, or a fragment thereof comprising 15 or more consecutive nucleotides; and

(e) a polynucleotide hybridizing under stringent conditions to any of the polynucleotides (a) to (d).

These polynucleotides (a) to (e) may each consist of a nucleotide sequence shown by any of SEQ ID NOs: 1 to 109 or may be derived therefrom.

The nucleic acids, such as nucleic acid probe(s) or primer(s), that can be used in the present invention may further comprise, in addition to one or more polynucleotides selected from the group consisting of the above-mentioned polynucleotides (a) to (e), one or more polynucleotides selected from the group consisting of the following (f) to (j):

(f) a polynucleotide consisting of a nucleotide sequence shown by any of SEQ ID NOs: 110 to 200 or a nucleotide sequence derived from the nucleotide sequence by the replacement of u with t, a variant thereof, a derivative thereof, or a fragment thereof comprising 15 or more consecutive nucleotides;

(g) a polynucleotide comprising a nucleotide sequence shown by any of SEQ ID NOs: 110 to 200, a variant thereof, a derivative thereof, or a fragment thereof comprising 15 or more consecutive nucleotides;

(h) a polynucleotide consisting of a nucleotide sequence complementary to a nucleotide sequence shown by any of SEQ ID NOs: 110 to 200 or a nucleotide sequence derived from the nucleotide sequence by the replacement of u with t, a variant thereof, a derivative thereof, or a fragment thereof comprising 15 or more consecutive nucleotides;

(i) a polynucleotide comprising a nucleotide sequence complementary to a nucleotide sequence shown by any of SEQ ID NOs: 110 to 200 or a nucleotide sequence derived from the nucleotide sequence by the replacement of u with t, a variant thereof, a derivative thereof, or a fragment thereof comprising 15 or more consecutive nucleotides; and

(j) a polynucleotide hybridizing under stringent conditions to any of the polynucleotides (f) to (i).

These polynucleotides (f) to (j) may each consist of a nucleotide sequence shown by any of SEQ ID NOs: 110 to 200 or may be derived therefrom.

The above nucleic acid to be used in the present invention may each be DNA or RNA.

The nucleic acids that can be used in the present invention may be prepared using a general technique such as DNA recombination technology, a PCR method, or a method using an automated DNA/RNA synthesizer. As the DNA recombination technology or the PCR method, it is possible to use techniques described in, for example, Ausubel et al., Current Protocols in Molecular Biology, John Willey & Sons, US (1993); and Sambrook et al., Molecular Cloning: A Laboratory Manual, Cold Spring Harbor Laboratory Press, US (1989).

The human-derived hsa-miR-3131, hsa-miR-6757-5p, hsa-miR-4706, hsa-miR-5001-5p, hsa-miR-3180-3p, hsa-miR-642b-3p, hsa-miR-4655-5p, hsa-miR-6819-5p, hsa-miR-937-5p, hsa-miR-4688, hsa-miR-6741-5p, hsa-miR-7107-5p, hsa-miR-4271, hsa-miR-1229-5p, hsa-miR-4707-5p, hsa-miR-6808-5p, hsa-miR-4656, hsa-miR-6076, hsa-miR-6762-5p, hsa-miR-7109-5p, hsa-miR-6732-5p, hsa-miR-3195, hsa-miR-7150, hsa-miR-642a-3p, hsa-miR-1249-5p, hsa-miR-3185, hsa-miR-4689, hsa-miR-3141, hsa-miR-6840-3p, hsa-miR-3135b, hsa-miR-1914-3p, hsa-miR-4446-3p, hsa-miR-4433b-3p, hsa-miR-6877-5p, hsa-miR-6848-5p, hsa-miR-3620-5p, hsa-miR-6825-5p, hsa-miR-5739, hsa-miR-3663-3p, hsa-miR-4695-5p, hsa-miR-3162-5p, hsa-miR-3679-5p, hsa-miR-8059, hsa-miR-7110-5p, hsa-miR-1275, hsa-miR-6779-5p, hsa-miR-197-5p, hsa-miR-6845-5p, hsa-miR-4327, hsa-miR-4723-5p, hsa-miR-4530, hsa-miR-6771-5p, hsa-miR-614, hsa-miR-92a-2-5p, hsa-miR-6891-5p, hsa-miR-6124, hsa-miR-4687-3p, hsa-miR-4442, hsa-miR-7977, hsa-miR-6785-5p, hsa-miR-4497, hsa-miR-8071, hsa-miR-663b, hsa-miR-3180, hsa-miR-4251, hsa-miR-1285-3p, hsa-miR-6870-5p, hsa-miR-4484, hsa-miR-4476, hsa-miR-6749-5p, hsa-miR-4454, hsa-miR-6893-5p, hsa-miR-6085, hsa-miR-4787-5p, hsa-miR-149-3p, hsa-miR-7704, hsa-miR-6125, hsa-miR-6090, hsa-miR-3197, hsa-miR-6850-5p, hsa-miR-4467, hsa-miR-6885-5p, hsa-miR-6803-5p, hsa-miR-6798-5p, hsa-miR-6780b-5p, hsa-miR-6768-5p, hsa-miR-5100, hsa-miR-6724-5p, hsa-miR-6879-5p, hsa-miR-7108-5p, hsa-miR-4649-5p, hsa-miR-4739, hsa-miR-6089, hsa-miR-1908-5p, hsa-miR-4516, hsa-miR-2861, hsa-miR-4492, hsa-miR-4294, hsa-miR-6791-5p, hsa-miR-1469, hsa-miR-6752-5p, hsa-miR-4730, hsa-miR-6126, hsa-miR-6869-5p, hsa-miR-1268a, hsa-miR-6799-5p, hsa-miR-8069, hsa-miR-3621, hsa-miR-4763-3p, hsa-miR-1228-5p, hsa-miR-760, hsa-miR-187-5p, hsa-miR-7111-5p, hsa-miR-6088, hsa-miR-6805-3p, hsa-miR-4640-5p, hsa-miR-6721-5p, hsa-miR-6880-5p, hsa-miR-711, hsa-miR-128-1-5p, hsa-miR-4525, hsa-miR-486-3p, hsa-miR-6756-5p, hsa-miR-1260b, hsa-miR-3184-5p, hsa-miR-6075, hsa-miR-204-3p, hsa-miR-4728-5p, hsa-miR-4534, hsa-miR-4758-5p, hsa-miR-8063, hsa-miR-6836-3p, hsa-miR-6789-5p, hsa-miR-744-5p, hsa-miR-1909-3p, hsa-miR-887-3p, hsa-miR-4745-5p, hsa-miR-4433a-3p, hsa-miR-5090, hsa-miR-296-5p, hsa-miR-939-5p, hsa-miR-3648, hsa-miR-3196, hsa-miR-6722-3p, hsa-miR-6805-5p, hsa-miR-1202, hsa-miR-6775-5p, hsa-miR-6087, hsa-miR-6765-5p, hsa-miR-6875-5p, hsa-miR-4674, hsa-miR-1233-5p, hsa-miR-7114-5p, hsa-miR-5787, hsa-miR-8072, hsa-miR-3619-3p, hsa-miR-4632-5p, hsa-miR-6800-5p, hsa-miR-4634, hsa-miR-4486, hsa-miR-6727-5p, hsa-miR-4505, hsa-miR-4725-3p, hsa-miR-1538, hsa-miR-320b, hsa-miR-1915-5p, hsa-miR-328-5p, hsa-miR-6820-5p, hsa-miR-6726-5p, hsa-miR-3665, hsa-miR-638, hsa-miR-762, hsa-miR-4466, hsa-miR-3940-5p, hsa-miR-1237-5p, hsa-miR-575, hsa-miR-3656, hsa-miR-4488, hsa-miR-4281, hsa-miR-6781-5p, hsa-miR-4532, hsa-miR-4665-5p, hsa-miR-6816-5p, hsa-miR-4508, hsa-miR-6784-5p, hsa-miR-6786-5p, hsa-miR-4741, hsa-miR-1343-5p, hsa-miR-1227-5p, hsa-miR-4734, hsa-miR-3960, hsa-miR-128-2-5p, hsa-miR-6743-5p, hsa-miR-663a, hsa-miR-6729-5p, hsa-miR-1915-3p, hsa-miR-1268b, hsa-miR-4651, hsa-miR-3178, and hsa-miR-4463, comprising nucleotide sequences shown by SEQ ID NOs: 1 to 200, are known, and methods for obtaining them are also known, as described above. Therefore, a polynucleotide that can be used as a nucleic acid, such as a nucleic acid probe or primer, in the present invention can be produced on the basis of the nucleotide sequence of the miRNA/gene.

The nucleic acids, such as nucleic acid probes or primers, as described above may be chemically synthesized using an automated nucleic acid synthesizer. A phosphoramidite process is commonly used for this synthesis, and this process can be used to automatically synthesize a single-stranded DNA with up to about 100 nucleotides in length. The automated nucleic acid synthesizer is commercially available from, for example, Polygen, Inc., ABI, Inc., or Applied BioSystems, Inc.

Alternatively, the polynucleotides of the present invention may be prepared by cDNA cloning. For the cDNA cloning technology, a microRNA Cloning Kit Wako, for example, can be utilized.

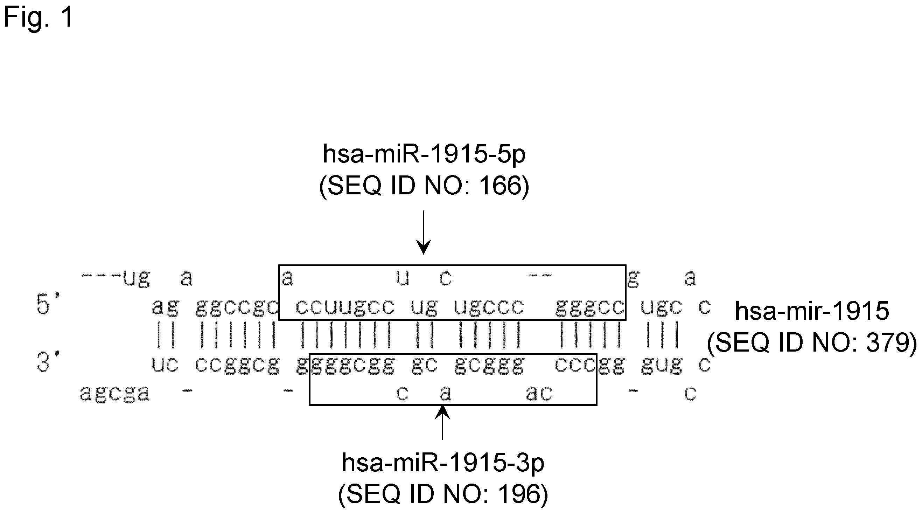

The sequence of a nucleic acid, such as a nucleic acid probe or primer, for detection of a polynucleotide consisting of the nucleotide sequence shown by any of SEQ ID NOs: 1 to 200, may not be present *in vivo* as an miRNA or a precursor thereof. For example, the nucleotide sequences shown by SEQ ID NOs: 166 and 196 are each generated from the precursor shown by SEQ ID NO: 379. This precursor has a hairpin-like structure as shown in Fig. 1, and the nucleotide sequences shown by SEQ ID NOs: 166 and 196 have mismatch nucleotides therebetween. Accordingly, a nucleotide sequence perfectly complementary to the nucleotide sequence shown by SEQ ID NO: 166 or 196 is not naturally occurring *in vivo.* Thus, the nucleic acid(s), such as a nucleic acid probe(s) or primer(s), for detecting a nucleotide sequence(s) shown by any of SEQ ID NOs: 1 to 200 may have an artificial nucleotide sequence(s) that are not present *in vivo.*

3. Kit or device for detection of hippocampal atrophy

The present invention provides a kit or device for detection of hippocampal atrophy, comprising one or more polynucleotides that can be used as nucleic acids, such as nucleic acid probes or primers, for detection of hippocampal atrophy for measuring target nucleic acids as hippocampal atrophy markers in the present invention.

A Target nucleic acid(s) as a hippocampal atrophy marker(s) according to the present invention is preferably at least one selected from the following group A.

Group A:

miR-3131, miR-6757-5p, miR-4706, miR-5001-5p, miR-3180-3p, miR-642b-3p, miR-4655-5p, miR-6819-5p, miR-937-5p, miR-4688, miR-6741-5p, miR-7107-5p, miR-4271, miR-1229-5p, miR-4707-5p, miR-6808-5p, miR-4656, miR-6076, miR-6762-5p, miR-7109-5p, miR-6732-5p, miR-3195, miR-7150, miR-642a-3p, miR-1249-5p, miR-3185, miR-4689, miR-3141, miR-6840-3p, miR-3135b, miR-1914-3p, miR-4446-3p, miR-4433b-3p, miR-6877-5p, miR-6848-5p, miR-3620-5p, miR-6825-5p, miR-5739, miR-3663-3p, miR-4695-5p, miR-3162-5p, miR-3679-5p, miR-8059, miR-7110-5p, miR-1275, miR-6779-5p, miR-197-5p, miR-6845-5p, miR-4327, miR-4723-5p, miR-4530, miR-6771-5p, miR-614, miR-92a-2-5p, miR-6891-5p, miR-6124, miR-4687-3p, miR-4442, miR-7977, miR-6785-5p, miR-4497, miR-8071, miR-663b, miR-3180, miR-4251, miR-1285-3p, miR-6870-5p, miR-4484, miR-4476, miR-6749-5p, miR-4454, miR-6893-5p, miR-6085, miR-4787-5p, miR-149-3p, miR-7704, miR-6125, miR-6090, miR-3197, miR-6850-5p, miR-4467, miR-6885-5p, miR-6803-5p, miR-6798-5p, miR-6780b-5p, miR-6768-5p, miR-5100, miR-6724-5p, miR-6879-5p, miR-7108-5p, miR-4649-5p, miR-4739, miR-6089, miR-1908-5p, miR-4516, miR-2861, miR-4492, miR-4294, miR-6791-5p, miR-1469, miR-6752-5p, miR-4730, miR-6126, miR-6869-5p, miR-1268a, miR-6799-5p, miR-8069, miR-3621, and miR-4763-3p.

In addition to the target nucleic acids selected from Group A, an additional target nucleic acid(s) that can be optionally used for the measurement are preferably selected from the following group B.

Group B:

miR-1228-5p, miR-760, miR-187-5p, miR-7111-5p, miR-6088, miR-6805-3p, miR-4640-5p, miR-6721-5p, miR-6880-5p, miR-711, miR-128-1-5p, miR-4525, miR-486-3p, miR-6756-5p, miR-1260b, miR-3184-5p, miR-6075, miR-204-3p, miR-4728-5p, miR-4534, miR-4758-5p, miR-8063, miR-6836-3p, miR-6789-5p, miR-744-5p, miR-1909-3p, miR-887-3p, miR-4745-5p, miR-4433a-3p, miR-5090, miR-296-5p, miR-939-5p, miR-3648, miR-3196, miR-6722-3p, miR-6805-5p, miR-1202, miR-6775-5p, miR-6087, miR-6765-5p, miR-6875-5p, miR-4674, miR-1233-5p, miR-7114-5p, miR-5787, miR-8072, miR-3619-3p, miR-4632-5p, miR-6800-5p, miR-4634, miR-4486, miR-6727-5p, miR-4505, miR-4725-3p, miR-1538, miR-320b, miR-1915-5p, miR-328-5p, miR-6820-5p, miR-6726-5p, miR-3665, miR-638, miR-762, miR-4466, miR-3940-5p, miR-1237-5p, miR-575, miR-3656, miR-4488, miR-4281, miR-6781-5p, miR-4532, miR-4665-5p, miR-6816-5p, miR-4508, miR-6784-5p, miR-6786-5p, miR-4741, miR-1343-5p, miR-1227-5p, miR-4734, miR-3960, miR-128-2-5p, miR-6743-5p, miR-663a, miR-6729-5p, miR-1915-3p, miR-1268b, miR-4651, miR-3178, and miR-4463.

The kit or device of the present invention comprises a nucleic acid(s) capable of specifically binding to the above target nucleic acid(s) as hippocampal atrophy marker(s) or a complementary strand(s) of the polynucleotide(s), and the kit or device preferably comprises one or more polynucleotides selected from the miRNA polynucleotides described in the above groups, or a variant(s) thereof.

The expression level of the above-mentioned target nucleic acid in samples from patients whose hippocampi have become atrophic (herein, also referred to as "patients with hippocampal atrophy," "subjects with hippocampal atrophy," or "hippocampal atrophy subjects") is increased or decreased depending on the type of the target nucleic acid, compared to samples from healthy subjects whose hippocampi have not become atrophic (herein, also referred to as "subjects with no hippocampal atrophy" or "hippocampal non-atrophy subjects") (hereinafter, which is referred to as "increase/decrease"). The kit or device of the present invention can be effectively used for detection of hippocampal atrophy by measuring the expression level(s) of the target nucleic acid(s) in body fluid from a subject to be tested for hippocampal atrophy, for example, a subject (e.g., a human) suspected of hippocampal atrophy, and in body fluid from subjects with no hippocampal atrophy, and then comparing the expression level(s) therebetween.

The kit or device of the present invention can comprise at least one of any of the above-mentioned nucleic acids, such as nucleic acid probes or primers, for detection of hippocampal atrophy.

In one embodiment, the kit or device of the present invention can comprise at least one of a polynucleotide comprising (or, consisting of) the nucleotide sequence shown by any of SEQ ID NOs: 1 to 109 or a nucleotide sequence derived from the nucleotide sequence by the replacement of u with t, a polynucleotide comprising (or, consisting of) a complementary sequence thereof, a polynucleotide hybridizing under stringent conditions to any of the polynucleotides, or a variant or fragment thereof comprising 15 or more consecutive nucleotides of any of the polynucleotide sequences.

In one embodiment, the kit or device of the present invention can further comprise at least one of a polynucleotide comprising (or, consisting of) a nucleotide sequence shown by any of SEQ ID NOs: 110 to 200 or a nucleotide sequence derived from the nucleotide sequence by the replacement of u with t, a polynucleotide comprising (or, consisting of) a complementary sequence thereof, a polynucleotide hybridizing under stringent conditions to any of the polynucleotides, or a variant or fragment thereof comprising 15 or more consecutive nucleotides of any of the polynucleotide sequences.

In one embodiment, a fragment(s) that can be comprised in the kit or device of the present invention may be, for example, one or more, preferably two or more polynucleotides selected from the group consisting of the following (1) and (2):

(1) a polynucleotide comprising a sequence of 15 or more consecutive nucleotides in a nucleotide sequence derived from the nucleotide sequence shown by any of SEQ ID NOs: 1 to 109 by the replacement of u with t or in a complementary sequence thereof;
(2) a polynucleotide comprising a sequence of 15 or more consecutive nucleotides in a nucleotide sequence derived from the nucleotide sequence shown by any of SEQ ID NOs: 110 to 200 by the replacement of u with t or in a complementary sequence thereof.

In one embodiment, the above-mentioned polynucleotide is a polynucleotide consisting of a nucleotide sequence shown by any of SEQ ID NOs: 1 to 109 or a nucleotide sequence derived from the nucleotide sequence by the replacement of u with t, a polynucleotide consisting of a complementary sequence thereof, a polynucleotide hybridizing under stringent conditions to any of the polynucleotides, or a variant comprising a sequence of 15 or more, preferably 17 or more, and more preferably 19 or more consecutive nucleotides of any of the polynucleotides.

In one embodiment, the above-mentioned polynucleotide is a polynucleotide consisting of a nucleotide sequence shown by any of SEQ ID NOs: 110 to 200 or a nucleotide sequence derived from the nucleotide sequence by the replacement of u with t, a polynucleotide consisting of a complementary sequence thereof, a polynucleotide hybridizing under stringent conditions to any of the polynucleotides, or a variant comprising a sequence of 15 or more, preferably 17 or more, and more preferably 19 or more consecutive nucleotides of any of the polynucleotides.

In a preferred embodiment, the above-mentioned fragment may be a polynucleotide comprising 15 or more, preferably 17 or more, and more preferably 19 or more consecutive nucleotides.

In the present invention, the polynucleotide fragment has a size of the number of nucleotides in a range of, for example, from 15 to less than the total number of consecutive nucleotides in the nucleotide sequence of each polynucleotide, from 17 to less than the total number of nucleotides in the sequence, or from 19 to less than the total number of nucleotides in the sequence.

The kit or device of the present invention may comprise one nucleic acid (or polynucleotide) specifically binding to the polynucleotide as the above-mentioned hippocampal atrophy marker (target nucleic acid) or to a complementary strand of such polynucleotide, or the kit or device may comprise two or more such nucleic acids in combination.

Specific examples of a combination of the above polynucleotides capable of specifically binding to the target nucleic acids in the kit or device of the present invention include any combinations of 1, 2, 3, 4, 5, 6, 7, 8, 9, 10 or more (e.g., 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50; or 70 or more, 80 or more, 90 or more, or 100 or more) polynucleotides selected from the above-mentioned polynucleotides consisting of the nucleotide sequences shown by SEQ ID Nos: 1 to 740 shown in Table 1 or derived therefrom. This is just an example, and all of various other possible combinations fall within the scope of the present invention.

In one embodiment, the target nucleic acids for the nucleic acids, such as nucleic acid probes or primers, in the kit or device of the present invention may be a combination of one or more polynucleotides selected from among the hippocampal atrophy markers of Group A above (for example, a polynucleotide of miR-3131, miR-6757-5p, miR-4706, miR-5001-5p, miR-3180-3p, miR-642b-3p, miR-4655-5p, miR-6819-5p, miR-937-5p, miR-4688, miR-6741-5p, miR-7107-5p, miR-4271, miR-1229-5p, miR-4707-5p, miR-6808-5p, miR-4656, miR-6076, miR-6762-5p, miR-7109-5p, miR-6732-5p, miR-3195, miR-7150, miR-642a-3p, miR-1249-5p, miR-3185, miR-4689, miR-3141, miR-6840-3p, miR-3135b, miR-1914-3p, miR-4446-3p, miR-4433b-3p, miR-6877-5p, miR-6848-5p, miR-3620-5p, miR-6825-5p, miR-5739, miR-3663-3p, miR-4695-5p, miR-3162-5p, miR-3679-5p, miR-8059, miR-7110-5p, miR-1275, miR-6779-5p, miR-197-5p, miR-6845-5p, miR-4327, miR-4723-5p, miR-4530, miR-6771-5p, miR-614, miR-92a-2-5p, miR-6891-5p, miR-6124, miR-4687-3p, miR-4442, miR-7977, miR-6785-5p, miR-4497, miR-8071, miR-663b, miR-3180, miR-4251, miR-1285-3p, miR-6870-5p, miR-4484, miR-4476, miR-6749-5p, miR-4454, miR-6893-5p, miR-6085, miR-4787-5p, miR-149-3p, miR-7704, miR-6125, miR-6090, miR-3197, miR-6850-5p, miR-4467, miR-6885-5p, miR-6803-5p, miR-6798-5p, miR-6780b-5p, miR-6768-5p, miR-5100, miR-6724-5p, miR-6879-5p, miR-7108-5p, miR-4649-5p, miR-4739, miR-6089, miR-1908-5p, miR-4516, miR-2861, miR-4492, miR-4294, miR-6791-5p, miR-1469, miR-6752-5p, miR-4730, miR-6126, miR-6869-5p, miR-1268a, miR-6799-5p, miR-8069, miR-3621, or miR-4763-3p, or any combination of two or more thereof) and one or more polynucleotides selected from among the hippocampal atrophy markers of Group A excluding the polynucleotides selected. In another embodiment, the target nucleic acids for the nucleic acids, such as nucleic acid probes or primers, in the kit or device of the present invention may be a combination of one or more polynucleotides selected from among the hippocampal atrophy markers of Group A, one or more polynucleotides selected from among the hippocampal atrophy markers of Group A excluding the polynucleotides selected, and one or more polynucleotides selected from among the hippocampal atrophy markers of Group B. In further another embodiment, the target nucleic acids for the nucleic acids, such as nucleic acid probes or primers, in the kit or device of the present invention may be a combination of one or more polynucleotides selected from among the hippocampal atrophy markers of Group A and one or more polynucleotides selected from among the hippocampal atrophy markers of Group B. In one embodiment, the target nucleic acids of the kit or device of the present invention may be a combination of one or more polynucleotides selected from among the hippocampal atrophy markers of Group A and one or more polynucleotides selected from the group consisting of the hippocampal atrophy markers of Group A excluding the polynucleotides selected and the hippocampal atrophy markers of Group B.

In one embodiment, as a combination of hippocampal atrophy markers (target nucleic acids) in the kit or device of the present invention for discriminating hippocampal atrophy subjects from hippocampal non-atrophy subjects, it is desirable to combine two or more polynucleotides selected from polynucleotides consisting of the nucleotide sequences shown by SEQ ID NOs: 1 to 740 listed in Table 1. The combinations of hippocampal atrophy markers (target nucleic acids) may be preferably 2 or more, 3 or more, 4 or more, 5 or more, 6 or more, 7 or more, 8 or more, 9 or more, or 10 or more (e.g., 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 4, 47, 48, 49, or 50; or 70 or more, 80 or more, 90 or more, or 100 or more) polynucleotides selected from the above-mentioned polynucleotides consisting of the nucleotide sequences shown by SEQ ID Nos listed in Tables 3 to 6.

Specifically, any two or more polynucleotides selected from the above-mentioned polynucleotides consisting of the nucleotide sequences shown by SEQ ID NOs: 1 to 200 may be used in combination as hippocampal atrophy markers (target nucleic acids). Such combination preferably comprises one or more polynucleotides selected from the group of polynucleotides consisting of the nucleotide sequences shown by SEQ ID NOs: 1 to 109 that have been newly found as hippocampal atrophy markers.

In one embodiment, as a combination of hippocampal atrophy markers (target nucleic acids) in the kit or device of the present invention for discriminating hippocampal atrophy subjects from hippocampal non-atrophy subjects, it is desirable to combine any two polynucleotides selected from the above-mentioned polynucleotides consisting of the nucleotide sequences shown by SEQ ID NOs: 1 to 3, 68, 110, 111, 114, 127, 135, 137, 142, 149, 152, and 155; or miRNAs or human miRNAs corresponding thereto. Such combination preferably comprises one or more polynucleotides selected from the group of polynucleotides consisting of the nucleotide sequences shown by SEQ ID NOs: 1 to 3 and 68 that have been newly found as hippocampal atrophy markers; or miRNAs or human miRNAs corresponding thereto.

In one embodiment, as a combination of hippocampal atrophy markers (target nucleic acids) in the kit or device of the present invention for discriminating hippocampal atrophy subjects from hippocampal non-atrophy subjects, it is desirable to combine any two polynucleotides selected from the above-mentioned polynucleotides consisting of the nucleotide sequences shown by SEQ ID NOs: 1, 26, 65, 66, 71, 110, 112, 114, 125 to 127, 162, and 164 to 166; or miRNAs or human miRNAs corresponding thereto. Such combination preferably comprises one or more polynucleotides selected from the group of polynucleotides consisting of the nucleotide sequences shown by SEQ ID NOs: 1, 26, 65, 66, and 71 that have been newly found as hippocampal atrophy markers; or miRNAs or human miRNAs corresponding thereto.

In one embodiment, as a combination of hippocampal atrophy markers (target nucleic acids) in the kit or device of the present invention for discriminating hippocampal atrophy subjects from hippocampal non-atrophy subjects, it is desirable to combine any two polynucleotides selected from the above-mentioned polynucleotides consisting of the nucleotide sequences shown by SEQ ID NOs: 1 to 7, 9 to 14, 16 to 21, 23 to 26, 30 to 32, 34, 35, 37 to 48, 50 to 56, 58 to 63, 67 to 73, 75, 77 to 93, 95 to 106, 108 to 111, 113 to 121, 124, 125, 127 to 129, 132 to 138, 140 to 143, 145, 146, 148 to 150, 152 to 155, 159 to 161, 163, 167 to 169, 171, 172, 174 to 189, 191 to 194, and 196 to 200; or miRNAs or human miRNAs corresponding thereto. Such combination preferably comprises one or more polynucleotides selected from the group of polynucleotides consisting of the nucleotide sequences shown by SEQ ID NOs: 1 to 7, 9 to 14, 16 to 21, 23 to 26, 30 to 32, 34, 35, 37 to 48, 50 to 56, 58 to 63, 67 to 73, 75, 77 to 93, 95 to 106, and 108 to 109 that have been newly found as hippocampal atrophy markers; or miRNAs or human miRNAs corresponding thereto.

In one embodiment, as a combination of hippocampal atrophy markers (target nucleic acids) in the kit or device of the present invention for discriminating hippocampal atrophy subjects from hippocampal non-atrophy subjects, it is desirable to combine any two polynucleotides selected from the above-mentioned polynucleotides consisting of the nucleotide sequences shown by SEQ ID NOs: 1 to 5, 9,10, 12, 13, 16 to 18, 20, 21, 24 to 26, 29 to 32, 37 to 39, 41, 42, 44, 45, 47 to 59, 61 to 63, 67 to 71, 73 to 75, 77 to 88, 90 to 94, 96 to 102, 104 to 111, 113, 114, 117, 118, 121, 123, 125, 127, 128, 133 to 138, 141 to 143, 145 to 150, 152 to 159, 161 to 163, 167 to 169, 171 to 180, 182, 183, 185 to 194, and 196 to 200; or miRNAs or human miRNAs corresponding thereto. Such combination preferably comprises one or more polynucleotides selected from the group of polynucleotides consisting of the nucleotide sequences shown by SEQ ID NOs: 1 to 5, 9,10, 12, 13, 16 to 18, 20, 21, 24 to 26, 29 to 32, 37 to 39, 41, 42, 44, 45, 47 to 59, 61 to 63, 67 to 71, 73 to 75, 77 to 88, 90 to 94, 96 to 102, and 104 to 109 that have been newly found as hippocampal atrophy markers; or miRNAs or human miRNAs corresponding thereto.

In one embodiment, as a combination of hippocampal atrophy markers (target nucleic acids) in the kit or device of the present invention for discriminating hippocampal atrophy subjects from hippocampal non-atrophy subjects, it is desirable to combine any two polynucleotides selected from the above-mentioned polynucleotides consisting of the nucleotide sequences shown by SEQ ID NOs: 155, 188, 127, 5, 198, 189, 110, 176, 143, 102, 148, 101, 75, 109, 199, 145, 54, 174, 106, 152, 133, 2, 20, 51, and 114; or miRNAs or human miRNAs corresponding thereto. Such combination preferably comprises one or more polynucleotides selected from the group of polynucleotides consisting of the nucleotide sequences shown by SEQ ID NOs: 2, 5, 20, 51, 54, 75, 101, 102, 106, and 109 that have been newly found as hippocampal atrophy markers; or miRNAs or human miRNAs corresponding thereto.

In one embodiment, as a combination of hippocampal atrophy markers (target nucleic acids) in the kit or device of the present invention for discriminating hippocampal atrophy subjects from hippocampal non-atrophy subjects, it is desirable to combine any two polynucleotides selected from the above-mentioned polynucleotides consisting of the nucleotide sequences shown by SEQ ID NOs: 1 to 6, 9, 17, 21, 27, 34, 39, 43, 47 to 48, 51, 53 to 54, 59, 61 to 63, 71, 73 to 78, 80, 82 to 88, 90 to 92, 94, 96 to 98, 100, 102, 104, 106, 109 to 110, 113 to 114, 121, 123 to 125, 127, 131 to 133, 135 to 137, 142 to 143, 145, 147 to 150, 152 to 153, 155, 159 to 162, 168 to 169, 174 to 176, 179, 182, 185, 187 to 189, 192 to 193, and 196 to 200; or miRNAs or human miRNAs corresponding thereto. Such combination preferably comprises one or more polynucleotides selected from the group of polynucleotides consisting of the nucleotide sequences shown by SEQ ID NOs: 1 to 6, 9, 17, 21, 27, 34, 39, 43, 47 to 48, 51, 53 to 54, 59, 61 to 63, 71, 73 to 78, 80, 82 to 88, 90 to 92, 94, 96 to 98, 100, 102, 104, 106, and 109 that have been newly found as hippocampal atrophy markers; or miRNAs or human miRNAs corresponding thereto.

The number of polynucleotides that are used in combination as the above-mentioned hippocampal atrophy markers (target nucleic acids) for discrimination of hippocampal atrophy may be 1, 2, 3, 4, 5, 6, 7, 8, 9, 10 or more (e.g., 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50; or 70 or more, 80 or more, 90 or more, or 100 or more). A combination of two or more polynucleotides is preferred.

The following shows, as non-limiting examples of combinations of hippocampal atrophy markers (target nucleic acids) to be used in the present invention, combinations of the polynucleotide consisting of the nucleotide sequence shown by SEQ ID NO: 110 and polynucleotide(s) of hippocampal atrophy marker(s) selected from among those shown in Tables 3 to 6. Combinations of hippocampal atrophy markers (target nucleic acids) may also be combinations of miRNAs or human miRNAs corresponding to the following combinations.

(1) a combination of SEQ ID NOs: 110 and 114;
(2) a combination of SEQ ID NOs: 110, 114, and 1;
(3) a combination of SEQ ID NOs: 110, 114, and 135;
(4) a combination of SEQ ID NOs: 110, 114, and 155;
(5) a combination of SEQ ID NOs: 110, 114, and 137;
(6) a combination of SEQ ID NOs: 110, 114, 155, and 135;
(7) a combination of SEQ ID NOs: 110, 114, 137, and 111;
(8) a combination of SEQ ID NOs: 110, 114, 155, and 2;
(9) a combination of SEQ ID NOs: 110, 114, 142, and 2;
(10) a combination of SEQ ID NOs: 110, 114, 1, and 149;
(11) a combination of SEQ ID NOs: 110, 114, 1, and 135;
(12) a combination of SEQ ID NOs: 110, 114, 155, and 68;
(13) a combination of SEQ ID NOs: 110, 114, 2, and 135;
(14) a combination of SEQ ID NOs: 110, 114, 155, 135, and 2;
(15) a combination of SEQ ID NOs: 110, 114, 155, 137, and 2;
(16) a combination of SEQ ID NOs: 110, 114, 3, 2, and 142;
(17) a combination of SEQ ID NOs: 110, 114, 142, 135, and 3;
(18) a combination of SEQ ID NOs: 110, 114, 127, 1, and 149;
(19) a combination of SEQ ID NOs: 110, 114, 127, 1, and 135;
(20) a combination of SEQ ID NOs: 110, 114, 155, 68, and 152;
(21) a combination of SEQ ID NOs: 110, 114, 68, 135, and 2;
(22) a combination of SEQ ID NOs: 110, 114, 142, 1, and 2;
(23) a combination of SEQ ID NOs: 127, 1, 162, 114, and 110;
(24) a combination of SEQ ID NOs: 1, 126, 125, 114, and 110;
(25) a combination of SEQ ID NOs: 66, 110, 165, 65, 166, and 164;
(26) a combination of SEQ ID NOs: 66, 110, 165, 26, 112, and 114; and
(27) a combination of SEQ ID NOs: 1, 110, 165, 65, 164, and 71.

The following shows, as non-limiting examples of combinations of hippocampal atrophy markers (target nucleic acids) to be used in the present invention, combinations of the polynucleotide consisting of the nucleotide sequence shown by SEQ ID NO: 39 and polynucleotides of hippocampal atrophy markers selected from among those shown in Tables 3 to 6. Combinations of hippocampal atrophy markers (target nucleic acids) may also be combinations of miRNAs or human miRNAs corresponding to the following combinations.

(1) a combination of SEQ ID NOs: 155, 87, 188, 127, 85, 5, 1, 110, 193, 175, 118, 152, 133, 39, 159, 149, 150, and 114;
(2) a combination of SEQ ID NOs: 115, 188, 127, 85, 1, 16, 110, 41, 24, 200, 26, 193, 175, 108, 152, 113, 39, 159, and 114;
(3) a combination of SEQ ID NOs: 155, 188, 127, 146, 5, 1, 16, 110, 200, 101, 193, 175, 145, 125, 152, 111, 39, and 114;
(4) a combination of SEQ ID NOs: 155, 87, 188, 127, 85, 1, 183, 196, 14, 189, 110, 24, 101, 193, 175, 118, 178, 152, 39, 150, and 114;
(5) a combination of SEQ ID NOs: 179, 155, 142, 188, 127, 146, 1, 189, 110, 4, 200, 193, 175, 118, 63, 152, 111, 180, 39, and 114;
(6) a combination of SEQ ID NOs: 87, 188, 127, 85, 5, 1, 16, 196, 14, 38, 110, 4, 200, 193, 175, 109, 145, 152, 39, 159, 149, and 114;
(7) a combination of SEQ ID NOs: 155, 87, 142, 188, 127, 85, 5, 1, 189, 154, 110, 41, 193, 175, 167, 10, 152, 111, 133, 39, 171, 150, and 114;
(8) a combination of SEQ ID NOs: 179, 188, 127, 5, 16, 154, 110, 193, 175, 118, 100, 109, 10, 88, 152, 39, 91, 159, 149, 82, 104, 182, and 114;
(9) a combination of SEQ ID NOs: 155, 188, 127, 1, 183, 187, 110, 185, 86, 193, 175, 109, 145, 88, 3, 120, 152, 133, 39, 149, 182, and 114;
(10) a combination of SEQ ID NOs: 155, 87, 142, 188, 127, 85, 1, 16, 110, 26, 193, 175, 118, 97, 174, 152, 111, 133, 113, 39, 159, and 114;

(11) a combination of SEQ ID NOs: 155, 87, 188, 127, 1, 110, 9, 24, 200, 193, 43, 109, 145, 125, 178, 135, 174, 152, 133, 39, 91, 149, 182, and 114;

(12) a combination of SEQ ID NOs: 155, 87, 127, 5, 1, 198, 189, 110, 41, 200, 193, 175, 75, 31, 109, 59, 174, 152, 111, 133, 113, 39, 91, and 114;

(13) a combination of SEQ ID NOs: 155, 87, 188, 127, 5, 1, 16, 187, 110, 176, 193, 175, 145, 125, 88, 152, 89, 141, 133, 39, 149, 150, and 114;

(14) a combination of SEQ ID NOs: 179, 95, 188, 127, 85, 5, 129, 16, 196, 154, 110, 200, 193, 175, 118, 63, 174, 108, 113, 39, 91, 159, 34, and 114;

(15) a combination of SEQ ID NOs: 155, 87, 188, 127, 85, 5, 1, 16, 196, 198, 38, 110, 24, 200, 193, 88, 96, 152, 111, 39, 159, 149, 182, and 114;

(16) a combination of SEQ ID NOs: 179, 121, 155, 87, 188, 127, 5, 187, 14, 189, 194, 110, 58, 200, 193, 118, 145, 125, 59, 152, 111, 113, 39, 159, 149, and 114;

(17) a combination of SEQ ID NOs: 121, 155, 87, 188, 127, 1, 189, 67, 110, 4, 176, 193, 75, 145, 125, 88, 59, 108, 152, 111, 39, 159, 149, 182, 114, and 23;

(18) a combination of SEQ ID NOs: 155, 137, 87, 168, 188, 196, 189, 110, 41, 4, 24, 58, 101, 193, 175, 145, 152, 111, 113, 39, 91, 61, 51, 55, 182, and 114;

(19) a combination of SEQ ID NOs: 155, 87, 188, 127, 85, 1, 16, 84, 187, 110, 4, 26, 193, 175, 118, 109, 88, 108, 152, 111, 39, 159, 149, 182, and 114;

(20) a combination of SEQ ID NOs: 179, 155, 132, 168, 188, 127, 5, 1, 14, 189, 110, 124, 4, 24, 200, 193, 175, 97, 145, 174, 108, 152, 133, 39, and 114;

(21) a combination of SEQ ID NOs: 142, 188, 85, 146, 1, 196, 187, 189, 110, 58, 101, 193, 175, 25, 105, 54, 120, 152, 111, 113, 39, 91, 159, 149, 61, 182, and 114;

(22) a combination of SEQ ID NOs: 121, 155, 95, 188, 127, 80, 198, 189, 110, 200, 101, 193, 175, 75, 145, 88, 120, 152, 133, 39, 91, 149, 34, 171, 140, 150, and 114;

(23) a combination of SEQ ID NOs: 155, 188, 127, 85, 1, 16, 196, 84, 53, 189, 110, 4, 68, 101, 193, 145, 178, 54, 108, 152, 111, 133, 39, 159, 182, and 114;

(24) a combination of SEQ ID NOs: 121, 115, 155, 137, 188, 127, 5, 1, 196, 35, 187, 110, 193, 175, 118, 97, 178, 48, 37, 152, 133, 39, 91, 149, 182, and 114;

(25) a combination of SEQ ID NOs: 155, 87, 188, 127, 85, 1, 16, 196, 187, 110, 24, 200, 185, 101, 175, 118, 192, 145, 88, 54, 108, 152, 133, 39, 91, 149, 182, and 114;

(26) a combination of SEQ ID NOs: 115, 155, 87, 188, 127, 146, 5, 16, 161, 53, 198, 110, 143, 148, 193, 175, 25, 100, 109, 97, 48, 108, 152, 133, 39, 149, and 114;

(27) a combination of SEQ ID NOs: 155, 87, 142, 188, 85, 1, 16, 196, 53, 110, 9, 41, 4, 185, 193, 175, 97, 135, 174, 73, 152, 111, 133, 113, 39, 149, 55, 182, and 114;

(28) a combination of SEQ ID NOs: 121, 155, 188, 127, 85, 1, 16, 198, 189, 110, 4, 86, 101, 193, 75, 199, 145, 108, 152, 111, 113, 39, 7, 91, 159, 149, 182, and 114;

(29) a combination of SEQ ID NOs: 155, 142, 188, 127, 5, 129, 16, 14, 154, 110, 153, 193, 175, 118, 75, 109, 169, 97, 21, 10, 59, 152, 111, 133, 39, 149, 18, and 114;

(30) a combination of SEQ ID NOs: 179, 155, 188, 127, 5, 196, 187, 14, 189, 154, 38, 110, 101, 175, 118, 25, 100, 174, 120, 108, 152, 133, 39, 91, 159, 149, 82, 104, 182, and 114;

(31) a combination of SEQ ID NOs: 155, 188, 127, 79, 16, 198, 110, 68, 200, 102, 193, 175, 43, 100, 109, 167, 10, 59, 191, 152, 111, 133, 39, 159, 149, 82, 55, 182, and 114;

(32) a combination of SEQ ID NOs: 155, 95, 87, 188, 127, 85, 1, 196, 198, 187, 189, 110, 200, 193, 175, 192, 109, 97, 145, 125, 59, 152, 133, 113, 39, 159, 149, 150, and 114;

(33) a combination of SEQ ID NOs: 121, 155, 188, 127, 1, 198, 187, 189, 154, 110, 193, 175, 118, 31, 109, 145, 59, 54, 174, 108, 152, 133, 113, 39, 91, 159, 149, 2, 55, 182, and 114;

(34) a combination of SEQ ID NOs: 121, 155, 87, 188, 127, 85, 1, 79, 16, 196, 187, 110, 176, 24, 101, 193, 175, 109, 199, 145, 178, 88, 152, 111, 133, 39, 149, 150, 182, 114, 23;

(35) a combination of SEQ ID NOs: 155, 188, 127, 1, 196, 189, 110, 32, 41, 143, 185, 193, 175, 97, 145, 178, 54, 174, 108, 152, 111, 133, 39, 91, 149, 61, 18, 55, 182, and 114;

(36) a combination of SEQ ID NOs: 121, 155, 95, 188, 127, 196, 53, 187, 189, 194, 110, 128, 101, 193, 175, 25, 109, 97, 145, 174, 152, 111, 113, 39, 91, 159, 149, 104, 182, and 114;

(37) a combination of SEQ ID NOs: 155, 137, 168, 188, 127, 16, 196, 187, 154, 110, 6, 44, 9, 200, 148, 101, 193, 175, 109, 145, 178, 48, 152, 133, 113, 39, 77, 55, 182, and 114;

(38) a combination of SEQ ID NOs: 155, 87, 188, 127, 85, 1, 16, 196, 84, 17, 14, 110, 176, 24, 200, 193, 175, 75, 109, 97, 145, 174, 108, 152, 141, 39, 159, 149, 182, and 114;

(39) a combination of SEQ ID NOs: 155, 87, 188, 127, 85, 1, 16, 196, 14, 110, 176, 185, 193, 175, 118, 109, 199, 145, 48, 108, 152, 141, 133, 39, 159, 149, 55, 182, 114, 23;

(40) a combination of SEQ ID NOs: 179, 121, 155, 95, 188, 127, 85, 1, 129, 196, 187, 110, 176, 193, 175, 31, 145, 178, 48, 88, 47, 54, 174, 120, 108, 133, 39, 91, 149, and 114;

(41) a combination of SEQ ID NOs: 179, 115, 155, 87, 188, 127, 85, 146, 5, 1, 45, 84, 187, 189, 110, 128, 56, 200, 185, 86, 193, 175, 118, 145, 48, 59, 152, 113, 39, 159, 149, and 114;

(42) a combination of SEQ ID NOs: 179, 95, 142, 188, 127, 5, 1, 129, 16, 187, 110, 193, 175, 118, 100, 109, 97, 125, 63, 92, 152, 133, 180, 39, 91, 159, 149, 104, 171, 150, 182, and 114;

(43) a combination of SEQ ID NOs: 155, 137, 142, 188, 127, 85, 196, 189, 110, 136, 41, 143, 148, 193, 175, 118, 199, 97, 145, 178, 88, 117, 108, 133, 39, 91, 159, 149, 18, 182, and 114;

(44) a combination of SEQ ID NOs: 13, 155, 137, 87, 188, 127,85, 196, 187, 110, 136, 41, 24, 200, 101, 193, 175, 118, 97, 145, 48, 152, 111, 133, 113, 39, 91, 149, 55, 182, and 114;

(45) a combination of SEQ ID NOs: 155, 188, 127, 85, 5, 1, 79, 84, 187, 67, 110, 41, 176, 175, 118, 109, 97, 145, 54, 108, 152, 111, 133, 113, 39, 91, 159, 149, 171, 182, and 114;

(46) a combination of SEQ ID NOs: 155, 87, 188, 127, 85, 146, 1, 16, 196, 187, 14, 110, 200, 26, 101, 193, 175, 97, 145, 48, 120, 108, 152, 141, 111, 39, 159, 149, 77, 182, and 114;

(47) a combination of SEQ ID NOs: 155, 168, 188, 127, 85, 5, 1, 16, 196, 84, 187, 189, 110, 128, 136, 176, 200, 193, 175, 118, 109, 145, 125, 152, 89, 133, 39, 149, 46, 182, and 114;

(48) a combination of SEQ ID NOs: 179, 121, 115, 155, 188, 127, 1, 16, 196, 161, 84, 67, 110, 124, 4, 176, 24, 148, 193, 175, 109, 145, 178, 48, 117, 152, 39, 159, 149, 182, and 114;

(49) a combination of SEQ ID NOs: 115, 95, 87, 188, 127, 85, 5, 1, 16, 196, 198, 187, 110, 176, 101, 193, 175, 118, 109, 97, 125, 178, 120, 108, 152, 141, 39, 159, 149, 182, and 114;

(50) a combination of SEQ ID NOs: 179, 87, 188, 127, 85, 30, 1, 84, 198, 187, 110, 193, 175, 109, 145, 125, 54, 174, 120, 163, 152, 133, 113, 39, 91, 159, 149, 82, 55, 182, and 114;

(51) a combination of SEQ ID NOs: 115, 155, 137, 168, 188, 127, 5, 1, 79, 196, 187, 189, 194, 110, 136, 193, 175, 118, 109, 199, 145, 88, 152, 133, 39, 91, 149, 18, 46, 182, and 114;

(52) a combination of SEQ ID NOs: 121, 155, 188, 127, 85, 1, 196, 187, 110, 176, 24, 200, 26, 101, 193, 175, 118, 25, 100, 169, 145, 125, 178, 54, 92, 152, 111, 113, 39, 91, 159, 55, and 114;

(53) a combination of SEQ ID NOs: 155, 95, 188, 127, 85, 1, 16, 196, 187, 189, 110, 136, 176, 69, 101, 193, 175, 118, 25, 75, 109, 97, 167, 145, 108, 152, 111, 39, 159, 149, 171, 182, and 114;

(54) a combination of SEQ ID NOs: 121, 115, 155, 168, 188, 127, 85, 5, 1, 84, 198, 187, 14, 189, 110, 193, 109, 97, 145, 54, 120, 152, 111, 113, 39, 91, 159, 149, 104, 150, 182, 114, 12;

(55) a combination of SEQ ID NOs: 155, 188, 127, 85, 146, 5, 1, 80, 183, 196, 189, 110, 177, 193, 175, 109, 169, 97, 167, 145, 59, 174, 152, 111, 133, 39, 91, 149, 82, 103, 52, and 114;

(56) a combination of SEQ ID NOs: 121, 168, 188, 127, 85, 16, 45, 198, 187, 189, 110, 4, 176, 200, 148, 86, 193, 175, 97, 145, 125, 174, 152, 113, 39, 91, 159, 149, 70, 82, 182, and 114;

(57) a combination of SEQ ID NOs: 121, 155, 188, 127, 85, 5, 1, 129, 187, 194, 110, 176, 24, 86, 26, 193, 118, 100, 125, 54, 174, 120, 152, 133, 39, 91, 149, 104, 171, 150, 182, and 114;

(58) a combination of SEQ ID NOs: 155, 142, 188, 127, 85, 1, 80, 183, 196, 187, 110, 124, 200, 101, 193, 25, 97, 145, 125, 59, 54, 174, 120, 108, 152, 133, 39, 91, 159, 82, 104, 61, 150, and 114;

(59) a combination of SEQ ID NOs: 121, 155, 95, 87, 188, 127, 85, 16, 196, 187, 194, 110, 136, 176, 200, 185, 101, 193, 175, 109, 145, 59, 108, 152, 111, 113, 39, 91, 159, 149, 46, 55, 182, and 114;

(60) a combination of SEQ ID NOs: 155, 87, 142, 188, 127, 85, 146, 5, 1, 16, 45, 196, 67, 110, 101, 193, 175, 118, 25, 109, 145, 178, 63, 54, 174, 108, 152, 39, 91, 159, 149, 55, 182, and 114;

(61) a combination of SEQ ID NOs: 155, 188, 127, 85, 5, 1, 138, 196, 84, 187, 110, 24, 148, 177, 101, 193, 175, 100, 145, 178, 88, 54, 71, 152, 133, 39, 91, 149, 82, 61, 51, 55, 182, and 114;

(62) a combination of SEQ ID NOs: 115, 155, 87, 168, 188, 127, 85, 1, 16, 196, 187, 110, 124, 41, 176, 24, 148, 153, 193, 175, 109, 145, 10, 88, 59, 120, 152, 133, 39, 91, 149, 2, 104, 182, and 114;

(63) a combination of SEQ ID NOs: 87, 188, 127, 1, 79, 183, 196, 187, 189, 110, 6, 32, 24, 148, 86, 193, 175, 118, 100, 109, 88, 135, 108, 92, 152, 111, 113, 39, 149, 55, 150, 182, and 114;

(64) a combination of SEQ ID NOs: 121, 155, 142, 188, 127, 85, 1, 16, 172, 53, 187, 110, 101, 193, 118, 75, 109, 199, 97, 145, 125, 63, 88, 54, 152, 42, 39, 159, 149, 82, 55, 114, 23;

(65) a combination of SEQ ID NOs: 155, 188, 127, 85, 146, 5, 1, 196, 84, 187, 110, 200, 193, 175, 118, 75, 97, 145, 125, 178, 59, 54, 106, 163, 152, 133, 39, 91, 159, 82, 34, 182, and 114;

(66) a combination of SEQ ID NOs: 121, 95, 188, 127, 85, 5, 79, 84, 187, 189, 110, 4, 193, 175, 119, 25, 97, 145, 10, 88, 108, 152, 111, 133, 113, 39, 91, 159, 149, 171, 150, 182, and 114;

(67) a combination of SEQ ID NOs: 115, 155, 188, 127, 85, 1, 16, 196, 161, 187, 189, 110, 176, 24, 101, 193, 175, 118, 31, 199, 145, 48, 54, 152, 111, 133, 39, 149, 82, 104, 51, 182, and 114;

(68) a combination of SEQ ID NOs: 179, 13, 155, 87, 188, 127, 85, 1, 79, 183, 189, 110, 81, 24, 193, 175, 199, 167,

125, 88, 59, 174, 108, 152, 111, 113, 39, 159, 149, 70, 150, 114, 23;

(69) a combination of SEQ ID NOs: 155, 87, 188, 127, 85, 1, 16, 196, 161, 84, 187, 14, 38, 124, 109, 145, 178, 48, 54, 96, 174, 120, 108, 152, 141, 39, 91, 159, 149, 82, 77, 55, 182, 93, and 114;

(70) a combination of SEQ ID NOs: 155, 87, 168, 188, 127, 85, 1, 196, 187, 110, 136, 24, 200, 148, 193, 175, 118, 109, 178, 120, 108, 152, 111, 133, 39, 91, 159, 149, 82, 171, 18, 55, 182, and 114;

(71) a combination of SEQ ID NOs: 155, 87, 142, 188, 127, 85, 80, 161, 84, 187, 189, 194, 110, 101, 193, 175, 25, 199, 97, 145, 120, 152, 111, 133, 39, 91, 159, 149, 103, 77, 184, 182, 114, and 23;

(72) a combination of SEQ ID NOs: 155, 95, 87, 188, 85, 5, 1, 196, 84, 53, 187, 110, 90, 176, 148, 193, 175, 118, 25, 145, 48, 88, 96, 174, 108, 152, 133, 39, 91, 149, 171, 55, 182, and 114;

(73) a combination of SEQ ID NOs: 155, 95, 188, 127, 85, 146, 1, 45, 196, 187, 189, 194, 110, 128, 68, 200, 86, 177, 101, 193, 175, 118, 109, 199, 37, 92, 152, 133, 39, 159, 149, 104, 182, and 114;

(74) a combination of SEQ ID NOs: 121, 155, 95, 168, 188, 127, 5, 16, 196, 172, 53, 187, 14, 189, 38, 67, 110, 68, 193, 175, 145, 178, 174, 152, 133, 39, 91, 159, 149, 82, 20, 182, 114, and 12;

(75) a combination of SEQ ID NOs: 95, 188, 127, 85, 1, 16, 196, 161, 14, 110, 32, 24, 175, 118, 100, 109, 97, 145, 178, 10, 59, 54, 108, 92, 111, 133, 180, 113, 39, 91, 159, 149, 184, 182, 114, and 40;

(76) a combination of SEQ ID NOs: 155, 95, 87, 142, 188, 127, 85, 5, 1, 196, 84, 198, 187, 110, 41, 68, 200, 177, 101, 193, 175, 118, 25, 109, 97, 145, 152, 111, 133, 113, 39, 159, 149, 55, 182, and 114;

(77) a combination of SEQ ID NOs: 121, 155, 188, 127, 85, 1, 79, 16, 196, 187, 189, 110, 124, 193, 175, 75, 169, 97, 145, 178, 21, 63, 54, 108, 71, 152, 111, 133, 39, 91, 159, 149, 55, 114, and 23;

(78) a combination of SEQ ID NOs: 121, 155, 188, 127, 85, 5, 1, 79, 160, 84, 187, 14, 110, 32, 176, 200, 193, 175, 118, 109, 97, 145, 178, 63, 10, 88, 152, 111, 133, 39, 91, 149, 77, 182, and 114;

(79) a combination of SEQ ID NOs: 13, 155, 87, 168, 188, 127, 85, 196, 187, 189, 194, 110, 78, 24, 200, 177, 101, 193, 175, 118, 109, 125, 174, 108, 73, 152, 111, 133, 39, 159, 149, 171, 52, 182, and 114;

(80) a combination of SEQ ID NOs: 121, 137, 142, 188, 127, 85, 5, 1, 196, 84, 187, 189, 38, 110, 4, 185, 177, 101, 193, 199, 145, 88, 3, 174, 152, 111, 133, 180, 113, 39, 91, 159, 149, 171, 140, 182, and 114;

(81) a combination of SEQ ID NOs: 121, 95, 188, 127, 85, 5, 1, 196, 187, 189, 194, 110, 128, 4, 26, 177, 101, 193, 175, 31, 109, 97, 145, 62, 88, 108, 152, 111, 133, 39, 91, 159, 149, 82, 171, 182, and 114;

(82) a combination of SEQ ID NOs: 121, 188, 127, 85, 30, 1, 16, 196, 187, 189, 11, 154, 194, 110, 58, 185, 101, 193, 175, 118, 25, 100, 88, 54, 152, 111, 180, 39, 91, 159, 61, 77, 20, 55, 182, 114, and 23;

(83) a combination of SEQ ID NOs: 155, 87, 188, 127, 1, 138, 16, 196, 187, 154, 38, 110, 200, 101, 193, 175, 25, 100, 199, 145, 178, 96, 174, 92, 152, 111, 133, 113, 39, 91, 159, 149, 82, 77, 182, 114, and 23;

(84) a combination of SEQ ID NOs: 87, 142, 188, 127, 85, 146, 5, 1, 196, 35, 189, 38, 67, 110, 9, 4, 177, 101, 193, 175, 118, 109, 145, 63, 88, 54, 108, 133, 39, 91, 159, 149, 72, 82, 51, 182, and 114;

(85) a combination of SEQ ID NOs: 155, 137, 188, 127, 1, 16, 196, 161, 35, 187, 189, 38, 67, 110, 181, 200, 148, 101, 193, 175, 118, 109, 145, 125, 174, 120, 152, 141, 111, 133, 39, 91, 159, 82, 55, and 114;

(86) a combination of SEQ ID NOs: 155, 132, 87, 188, 127, 1, 196, 161, 53, 198, 187, 189, 110, 181, 136, 4, 176, 143, 200, 26, 193, 175, 75, 97, 145, 54, 174, 37, 163, 152, 111, 39, 70, 55, 182, and 114;

(87) a combination of SEQ ID NOs: 179, 121, 155, 188, 127, 5, 1, 79, 16, 196, 187, 14, 67, 110, 176, 24, 143, 86, 175, 109, 145, 48, 88, 47, 54, 174, 120, 152, 133, 39, 91, 159, 149, 82, 182, and 114;

(88) a combination of SEQ ID NOs: 121, 87, 142, 188, 127, 85, 45, 196, 187, 189, 194, 110, 176, 24, 102, 86, 101, 193, 118, 100, 109, 145, 88, 174, 108, 71, 99, 152, 141, 111, 133, 39, 91, 159, 149, 182, 114, and 23;

(89) a combination of SEQ ID NOs: 121, 155, 87, 188, 127, 1, 16, 198, 187, 189, 38, 67, 194, 110, 4, 176, 24, 200, 185, 193, 175, 118, 192, 109, 167, 145, 125, 63, 120, 108, 152, 133, 180, 39, 91, 159, 149, and 182;

(90) a combination of SEQ ID NOs: 121, 115, 155, 137, 168, 188, 127, 85, 1, 79, 138, 16, 196, 187, 67, 110, 136, 4, 101, 193, 175, 118, 109, 97, 145, 48, 54, 120, 108, 152, 133, 39, 91, 159, 149, 18, 182, and 114;

(91) a combination of SEQ ID NOs: 137, 188, 127, 85, 146, 1, 16, 196, 161, 189, 110, 56, 58, 148, 193, 175, 118, 25, 100, 109, 145, 178, 48, 174, 120, 108, 152, 141, 133, 39, 91, 149, 82, 77, 55, 150, and 114;

(92) a combination of SEQ ID NOs: 155, 87, 168, 188, 127, 85, 1, 196, 84, 189, 110, 124, 176, 24, 200, 185, 69, 175, 25, 169, 145, 125, 178, 96, 120, 117, 108, 152, 111, 133, 39, 91, 159, 149, 82, 182, and 114;

(93) a combination of SEQ ID NOs: 155, 95, 188, 127, 85, 5, 1, 16, 187, 194, 110, 9, 41, 4, 153, 193, 175, 100, 109, 145, 62, 21, 48, 10, 54, 152, 111, 133, 39, 91, 197, 159, 149, 104, 171, 182, and 114;

(94) a combination of SEQ ID NOs: 87, 188, 127, 85, 5, 1, 80, 196, 189, 154, 110, 124, 78, 186, 90, 177, 193, 175, 118, 31, 109, 97, 145, 88, 59, 54, 120, 108, 134, 152, 111, 133, 39, 91, 149, 104, 140, 182, and 114;

(95) a combination of SEQ ID NOs: 179, 155, 95, 188, 127, 146, 5, 129, 16, 196, 84, 187, 14, 189, 110, 32, 68, 26, 193, 175, 109, 145, 178, 59, 54, 174, 120, 152, 133, 39, 91, 149, 82, 104, 61, 182, 93, 114, and 23;

(96) a combination of SEQ ID NOs: 121, 115, 155, 87, 188, 127, 85, 1, 80, 161, 187, 14, 110, 4, 24, 200, 193, 109, 97, 145, 125, 178, 63, 54, 174, 152, 111, 133, 39, 91, 159, 149, 70, 82, 61, 55, 182, and 114;

(97) a combination of SEQ ID NOs: 155, 137, 95, 87, 188, 127, 85, 146, 1, 196, 189, 38, 194, 110, 32, 176, 193, 175, 118, 109, 125, 178, 63, 59, 54, 96, 191, 174, 120, 152, 141, 133, 39, 91, 149, 82, 55, 182, and 114;

(98) a combination of SEQ ID NOs: 179, 155, 87, 142, 188, 127, 85, 1, 80, 189, 110, 6, 56, 185, 153, 26, 193, 175, 109, 62, 178, 10, 88, 59, 135, 54, 108, 92, 152, 111, 133, 180, 113, 39, 91, 159, 149, 150, 114, and 60;

(99) a combination of SEQ ID NOs: 179, 115, 188, 127, 85, 1, 79, 183, 196, 53, 187, 189, 194, 110, 200, 185, 193, 175, 119, 105, 97, 145, 125, 88, 54, 120, 152, 113, 39, 91, 116, 159, 149, 34, 61, 150, 182, 114, and 23;

(100) a combination of SEQ ID NOs: 87, 188, 127, 85, 16, 196, 84, 14, 189, 67, 110, 90, 24, 193, 175, 43, 100, 109, 145, 48, 63, 10, 88, 59, 54, 174, 108, 152, 111, 133, 39, 91, 159, 149, 82, 104, 61, 182, and 114;

(101) a combination of SEQ ID NOs: 155, 137, 142, 188, 127, 85, 5, 1, 16, 196, 84, 53, 187, 38, 67, 110, 4, 176, 24, 177, 101, 193, 175, 100, 109, 145, 48, 88, 54, 71, 152, 89, 133, 39, 91, 159, 149, 104, 182, 93, and 114;

(102) a combination of SEQ ID NOs: 87, 188, 127, 1, 80, 183, 45, 187, 189, 110, 128, 4, 200, 102, 26, 177, 101, 193, 175, 145, 178, 88, 96, 174, 117, 92, 152, 111, 133, 39, 91, 159, 149, 82, 171, 51, 55, 150, 182, 93, 114, and 12;

(103) a combination of SEQ ID NOs: 121, 155, 188, 127, 85, 146, 196, 53, 187, 189, 110, 32, 56, 26, 101, 193, 175, 100, 109, 199, 97, 145, 178, 48, 63, 10, 88, 59, 54, 108, 152, 111, 133, 39, 7, 197, 159, 149, 82, 182, and 114;

(104) a combination of SEQ ID NOs: 121, 155, 87, 188, 127, 85, 1, 80, 84, 198, 187, 189, 194, 110, 200, 102, 86, 193, 175, 118, 75, 97, 145, 125, 178, 88, 59, 37, 92, 134, 152, 111, 39, 159, 149, 82, 20, 51, 150, 182, and 114;

(105) a combination of SEQ ID NOs: 137, 95, 87, 142, 188, 127, 85, 1, 79, 196, 187, 189, 67, 110, 136, 41, 4, 24, 19, 177, 101, 193, 118, 100, 105, 97, 48, 54, 191, 174, 108, 152, 111, 133, 39, 159, 149, 171, 18, 182, and 114;

(106) a combination of SEQ ID NOs: 95, 87, 188, 127, 146, 5, 196, 161, 84, 187, 14, 189, 154, 194, 110, 128, 176, 24, 68, 200, 185, 193, 175, 43, 109, 97, 145, 178, 48, 54, 174, 92, 152, 113, 39, 159, 149, 82, 55, 114, and 23;

(107) a combination of SEQ ID NOs: 121, 155, 137, 87, 188, 127, 85, 79, 129, 16, 84, 14, 189, 67, 110, 124, 136, 41, 176, 24, 68, 185, 193, 175, 109, 97, 145, 178, 120, 73, 152, 111, 133, 113, 39, 91, 159, 149, 150, 182, and 114;

(108) a combination of SEQ ID NOs: 155, 137, 168, 188, 127, 85, 1, 80, 79, 183, 196, 172, 84, 187, 189, 110, 41, 68, 185, 175, 75, 100, 109, 97, 145, 178, 48, 98, 88, 135, 54, 108, 92, 73, 111, 133, 39, 91, 149, 82, 150, and 114;

(109) a combination of SEQ ID NOs: 13, 155, 132, 87, 168, 188, 127, 5, 1, 196, 84, 110, 6, 124, 41, 90, 176, 81, 24, 143, 68, 200, 101, 193, 175, 100, 109, 169, 178, 47, 174, 152, 111, 133, 39, 91, 149, 82, 61, 18, 182, and 114;

(110) a combination of SEQ ID NOs: 121, 115, 155, 168, 142, 188, 127, 85, 1, 16, 196, 161, 187, 14, 189, 67, 110, 4, 176, 200, 193, 175, 25, 100, 167, 145, 178, 59, 54, 174, 108, 152, 111, 133, 113, 39, 91, 159, 82, 104, 182, 114, and 23;

(111) a combination of SEQ ID NOs: 179, 115, 142, 188, 127, 146, 79, 196, 161, 187, 189, 194, 110, 4, 68, 177, 101, 193, 175, 100, 109, 145, 48, 59, 174, 108, 99, 152, 133, 113, 39, 149, 70, 82, 104, 140, 61, 103, 77, 55, 182, 114, and 50;

(112) a combination of SEQ ID NOs: 13, 121, 155, 132, 95, 87, 168, 188, 127, 1, 16, 196, 84, 198, 189, 38, 110, 124, 4, 176, 24, 200, 102, 148, 185, 193, 175, 25, 109, 145, 125, 178, 10, 59, 37, 108, 141, 111, 133, 39, 159, 149, 82, 150, 182, and 114;

(113) a combination of SEQ ID NOs: 179, 121, 95, 188, 127, 85, 1, 79, 16, 84, 53, 198, 187, 38, 194, 110, 136, 78, 4, 176, 193, 175, 75, 100, 109, 97, 145, 48, 88, 59, 54, 37, 152, 111, 113, 39, 91, 159, 149, 82, 171, 46, 150, 182, and 114; and

(114) a combination of SEQ ID NOs: 179, 115, 155, 87, 168, 188, 127, 85, 1, 16, 196, 198, 14, 110, 176, 24, 143, 68, 200, 193, 175, 118, 109, 97, 125, 178, 88, 83, 59, 47, 54, 120, 108, 163, 111, 133, 39, 91, 159, 149, 82, 46, 55, 182, and 114.

The following shows, as non-limiting examples of combinations of hippocampal atrophy markers (target nucleic acids) to be used in the present invention, combinations of the polynucleotide consisting of the nucleotide sequence shown by SEQ ID NO: 1 and polynucleotides of hippocampal atrophy markers selected from among those shown in Tables 3 to 6. Combinations of hippocampal atrophy markers (target nucleic acids) may also be combinations of miRNAs or human miRNAs corresponding to the following combinations.

(1) a combination of SEQ ID NOs: 127, 1, and 110;

(2) a combination of SEQ ID NOs: 1, 110, 3, and 114;

(3) a combination of SEQ ID NOs: 137, 1, 110, 111, and 114;

(4) a combination of SEQ ID NOs: 155, 127, 5, 1, 154, 110;

(5) a combination of SEQ ID NOs: 155, 87, 1, 154, 110, 73, 133, 149, and 114;

(6) a combination of SEQ ID NOs: 30, 1, 110, 128, 145, 3, 159, and 114;

(7) a combination of SEQ ID NOs: 127, 5, 1, 154, 110, 3, 73, and 114;

(8) a combination of SEQ ID NOs: 155, 5, 1, 110, 100, 145, 54, 133, and 114;

(9) a combination of SEQ ID NOs: 188, 5, 1, 38, 110, 152, 133, 159, and 114;

(10) a combination of SEQ ID NOs: 155, 1, 110, 100, 145, 54, 152, 133, 159, and 114;

(11) a combination of SEQ ID NOs: 137, 188, 127, 5, 1, 110, 145, 3, 152, 180, and 114;

(12) a combination of SEQ ID NOs: 155, 188, 1, 110, 75, 3, 71, 152, 133, 159, 149, and 114;

(13) a combination of SEQ ID NOs: 179, 121, 137, 5, 1, 183, 110, 185, 3, 54, 152, and 114;

(14) a combination of SEQ ID NOs: 155, 188, 127, 30, 5, 1, 53, 110, 109, 145, 152, and 159;

(15) a combination of SEQ ID NOs: 155, 188, 127, 1, 196, 189, 110, 109, 71, 152, 133, 149, and 114;

(16) a combination of SEQ ID NOs: 155, 137, 127, 1, 110, 193, 175, 109, 3, 152, 133, 180, and 114;

(17) a combination of SEQ ID NOs: 155, 142, 1, 154, 110, 100, 109, 145, 152, 133, 39, 104, and 114;

(18) a combination of SEQ ID NOs: 155, 137, 188, 127, 5, 1, 187, 189, 110, 26, 109, 71, 152, and 114;

(19) a combination of SEQ ID NOs: 155, 188, 127, 1, 189, 110, 78, 185, 100, 71, 152, 133, 149, and 114;

(20) a combination of SEQ ID NOs: 121, 155, 188, 1, 79, 198, 154, 110, 100, 109, 54, 133, 104, and 114;

(21) a combination of SEQ ID NOs: 155, 142, 188, 127, 1, 196, 189, 110, 175, 100, 109, 145, 3, 174, and 114;

(22) a combination of SEQ ID NOs: 121, 155, 188, 127, 1, 110, 4, 100, 109, 145, 71, 152, 133, 82, and 114;

(23) a combination of SEQ ID NOs: 155, 142, 188, 127, 1, 189, 110, 100, 109, 174, 71, 133, 57, 51, and 114;

(24) a combination of SEQ ID NOs: 155, 188, 127, 1, 154, 110, 58, 100, 109, 54, 71, 73, 133, 149, and 114;

(25) a combination of SEQ ID NOs: 155, 142, 188, 127, 1, 189, 110, 100, 145, 174, 152, 133, 149, 51, and 114;

(26) a combination of SEQ ID NOs: 179, 155, 137, 188, 127, 1, 138, 110, 193, 100, 145, 10, 152, 51, and 114;

(27) a combination of SEQ ID NOs: 155, 142, 188, 30, 1, 53, 110, 109, 145, 54, 96, 174, 152, 2, 51, and 114;

(28) a combination of SEQ ID NOs: 155, 188, 127, 30, 1, 189, 194, 110, 100, 109, 145, 3, 71, 152, 133, 82, and 114;

(29) a combination of SEQ ID NOs: 155, 137, 188, 127, 85, 30, 1, 110, 175, 100, 145, 54, 71, 152, 159, and 114;

(30) a combination of SEQ ID NOs: 155, 188, 127, 1, 110, 78, 185, 175, 100, 145, 152, 133, 149, 82, and 51;

(31) a combination of SEQ ID NOs: 155, 188, 127, 1, 198, 110, 86, 175, 100, 109, 174, 152, 133, 159, 149, 93, and 114;

(32) a combination of SEQ ID NOs: 155, 188, 127, 1, 110, 175, 145, 3, 152, 133, 180, 39, 159, 149, 77, and 114;

(33) a combination of SEQ ID NOs: 155, 188, 127, 5, 1, 196, 53, 154, 110, 58, 54, 152, 133, 82, and 114;

(34) a combination of SEQ ID NOs: 179, 188, 127, 1, 110, 102, 175, 75, 100, 109, 3, 54, 152, 190, 133, 159, and 2;

(35) a combination of SEQ ID NOs: 155, 137, 188, 127, 1, 196, 189, 110, 109, 145, 3, 73, 152, 133, 2, 82, and 114;

(36) a combination of SEQ ID NOs: 155, 188, 127, 1, 198, 110, 68, 175, 75, 100, 54, 71, 73, 133, 149, 77, and 114;

(37) a combination of SEQ ID NOs: 121, 155, 87, 188, 127, 30, 1, 161, 110, 193, 109, 145, 135, 71, 152, 82, and 114;

(38) a combination of SEQ ID NOs: 155, 87, 188, 127, 1, 198, 154, 110, 100, 174, 73, 152, 133, 159, 82, 51, and 114;

(39) a combination of SEQ ID NOs: 155, 137, 87, 188, 127, 1, 198, 110, 68, 101, 100, 109, 145, 54, 152, 149, 51, and 114;

(40) a combination of SEQ ID NOs: 121, 155, 188, 127, 1, 198, 110, 75, 109, 145, 54, 152, 133, 82, 104, and 114;

(41) a combination of SEQ ID NOs: 155, 137, 87, 142, 188, 127, 1, 110, 185, 175, 100, 145, 174, 152, 141, 133, and 180;

(42) a combination of SEQ ID NOs: 155, 142, 188, 127, 1, 189, 154, 110, 71, 73, 152, 159, 149, 82, 51, 182, and 114;

(43) a combination of SEQ ID NOs: 155, 188, 127, 5, 1, 79, 154, 110, 75, 3, 54, 108, 71, 73, 152, 133, and 114;

(44) a combination of SEQ ID NOs: 155, 142, 127, 1, 189, 154, 110, 109, 3, 174, 108, 71, 133, 91, 159, 82, 104, and 114;

(45) a combination of SEQ ID NOs: 121, 137, 188, 127, 1, 196, 189, 110, 175, 109, 3, 54, 191, 152, 133, 149, 82, and 114;

(46) a combination of SEQ ID NOs: 142, 188, 127, 1, 198, 110, 175, 100, 109, 145, 133, 180, 91, 159, 149, 82, 182, and 114;

(47) a combination of SEQ ID NOs: 121, 155, 137, 188, 127, 85, 5, 1, 110, 100, 109, 3, 54, 73, 152, 133, 159, and 114;

(48) a combination of SEQ ID NOs: 155, 188, 127, 1, 138, 198, 110, 68, 100, 109, 48, 3, 71, 152, 141, 133, 91, and 182;

(49) a combination of SEQ ID NOs: 155, 137, 142, 188, 127, 1, 110, 109, 174, 71, 152, 111, 133, 180, 159, 149, 51, and 114;

(50) a combination of SEQ ID NOs: 155, 137, 188, 127, 30, 1, 161, 154, 194, 110, 178, 10, 152, 111, 133, 180, and 114;

(51) a combination of SEQ ID NOs: 179, 155, 188, 127, 1, 161, 110, 175, 109, 62, 125, 10, 54, 71, 152, 133, 180, and 114;

(52) a combination of SEQ ID NOs: 155, 188, 127, 1, 110, 193, 175, 145, 48, 10, 71, 152, 141, 133, 180, 182, and 114;

(53) a combination of SEQ ID NOs: 155, 137, 87, 188, 127, 1, 189, 194, 110, 193, 100, 10, 111, 133, 180, 159, 149, and 114;

(54) a combination of SEQ ID NOs: 155, 188, 5, 1, 154, 110, 109, 3, 54, 71, 73, 163, 152, 133, 180, 82, 104, and 114;

(55) a combination of SEQ ID NOs: 155, 137, 188, 127, 30, 1, 17, 110, 193, 145, 71, 152, 111, 133, 180, 77, 150, and 114;

(56) a combination of SEQ ID NOs: 155, 137, 87, 142, 188, 127, 1, 198, 110, 58, 175, 109, 10, 152, 133, 149, 82, 93, and 114;

(57) a combination of SEQ ID NOs: 155, 188, 127, 5, 1, 196, 110, 68, 193, 109, 10, 54, 152, 133, 180, 82, and 114;

(58) a combination of SEQ ID NOs: 155, 142, 188, 127, 1, 110, 75, 109, 145, 10, 3, 71, 152, 133, 82, 51, and 114;

(59) a combination of SEQ ID NOs: 121, 155, 142, 188, 127, 1, 161, 154, 110, 175, 109, 71, 73, 152, 133, 149, 51, and 114;

(60) a combination of SEQ ID NOs: 155, 137, 188, 127, 5, 1, 198, 154, 110, 193, 109, 3, 71, 152, 180, 159, 82, 104, and 114;

(61) a combination of SEQ ID NOs: 121, 155, 137, 142, 188, 127, 1, 154, 110, 175, 109, 108, 133, 159, 149, 82, 77, and 114;

(62) a combination of SEQ ID NOs: 155, 188, 127, 1, 189, 110, 26, 193, 175, 109, 145, 73, 152, 141, 133, 149, 82, 77, and 114;

(63) a combination of SEQ ID NOs: 155, 188, 127, 1, 162, 198, 189, 154, 110, 75, 100, 109, 54, 71, 152, 133, 82, 104, and 114;

(64) a combination of SEQ ID NOs: 121, 155, 137, 188, 127, 1, 110, 185, 69, 100, 109, 3, 174, 71, 133, 180, 159, 82, and 114;

(65) a combination of SEQ ID NOs: 179, 155, 188, 127, 1, 154, 110, 86, 193, 175, 75, 109, 145, 71, 133, 159, 82, and 114;

(66) a combination of SEQ ID NOs: 155, 87, 188, 127, 1, 84, 110, 193, 175, 100, 109, 71, 152, 133, 149, 82, 104, 77, and 114;

(67) a combination of SEQ ID NOs: 155, 137, 188, 127, 1, 110, 175, 100, 145, 10, 135, 3, 71, 152, 133, 180, 149, 77, and 114;

(68) a combination of SEQ ID NOs: 155, 142, 188, 127, 1, 189, 154, 110, 175, 109, 145, 125, 174, 71, 152, 133, 91, 82, and 114;

(69) a combination of SEQ ID NOs: 121, 142, 188, 127, 5, 1, 189, 154, 110, 100, 109, 145, 125, 174, 108, 71, 152, 82, and 114;

(70) a combination of SEQ ID NOs: 155, 137, 87, 188, 127, 1, 198, 110, 185, 175, 100, 109, 145, 92, 152, 133, 180, 149, and 114;

(71) a combination of SEQ ID NOs: 155, 137, 87, 188, 127, 1, 161, 198, 110, 100, 109, 73, 152, 141, 133, 149, 182, and 114;

(72) a combination of SEQ ID NOs: 155, 142, 188, 1, 79, 198, 154, 110, 78, 185, 175, 100, 10, 152, 133, 180, 159, 51, and 114;

(73) a combination of SEQ ID NOs: 155, 137, 142, 188, 127, 1, 196, 189, 110, 78, 143, 200, 185, 145, 152, 133, 180, and 159;

(74) a combination of SEQ ID NOs: 155, 137, 188, 127, 1, 198, 110, 41, 101, 109, 145, 174, 73, 152, 159, 82, 51, 182, and 114;

(75) a combination of SEQ ID NOs: 155, 142, 188, 127, 1, 80, 189, 110, 185, 153, 109, 145, 178, 174, 111, 133, 82, 104, and 114;

(76) a combination of SEQ ID NOs: 179, 155, 142, 188, 127, 1, 198, 194, 110, 68, 185, 101, 100, 109, 96, 152, 133, 180, and 149;

(77) a combination of SEQ ID NOs: 121, 155, 87, 188, 127, 5, 1, 198, 194, 110, 68, 193, 145, 152, 180, 159, 149, 182, and 114;

(78) a combination of SEQ ID NOs: 155, 137, 127, 1, 198, 110, 175, 100, 109, 54, 152, 133, 180, 39, 91, 149, 2, 82, and 114;

(79) a combination of SEQ ID NOs: 121, 155, 137, 87, 142, 188, 127, 1, 198, 110, 56, 193, 109, 10, 3, 152, 180, 104, 182, and 114;

(80) a combination of SEQ ID NOs: 121, 137, 87, 188, 127, 1, 187, 189, 110, 193, 109, 145, 135, 3, 152, 133, 159, 2, 82, and 114;

(81) a combination of SEQ ID NOs: 155, 137, 188, 127, 1, 79, 198, 110, 86, 100, 109, 3, 174, 71, 152, 133, 91, 149, 82, and 114;

(82) a combination of SEQ ID NOs: 155, 137, 188, 127, 1, 198, 110, 193, 100, 109, 145, 98, 3, 71, 152, 133, 159, 82, 150, and 114;

(83) a combination of SEQ ID NOs: 155, 137, 188, 127, 1, 110, 153, 193, 75, 109, 88, 174, 71, 152, 133, 180, 149, 82, 51, and 114;

(84) a combination of SEQ ID NOs: 155, 87, 188, 127, 1, 161, 198, 110, 175, 109, 169, 145, 178, 10, 92, 133, 159, 82, 104, and 114;

(85) a combination of SEQ ID NOs: 155, 87, 142, 188, 127, 1, 154, 194, 110, 101, 75, 100, 109, 145, 152, 133, 180, 82, and 114;

(86) a combination of SEQ ID NOs: 155, 137, 87, 127, 1, 110, 185, 175, 173, 109, 145, 133, 180, 91, 159, 149, 104, 77, and 114;

(87) a combination of SEQ ID NOs: 155, 142, 188, 127, 1, 196, 110, 58, 185, 175, 75, 100, 109, 145, 125, 3, 91, 2, 82, and 114;

(88) a combination of SEQ ID NOs: 121, 137, 142, 188, 127, 1, 154, 110, 175, 100, 109, 145, 88, 3, 54, 174, 152, 133, 82, 182, and 114;

(89) a combination of SEQ ID NOs: 121, 137, 87, 188, 127, 1, 53, 110, 185, 175, 109, 152, 133, 91, 159, 149, 82, 104, 77, 150, and 114;

(90) a combination of SEQ ID NOs: 155, 142, 188, 127, 30, 1, 196, 198, 189, 154, 110, 109, 145, 10, 174, 152, 133, 159, 82, and 114;

(91) a combination of SEQ ID NOs: 179, 155, 188, 127, 1, 198, 110, 101, 175, 118, 109, 145, 3, 37, 73, 133, 91, 149, 82, 77, and 114;

(92) a combination of SEQ ID NOs: 155, 137, 87, 188, 127, 1, 196, 154, 110, 68, 26, 178, 71, 152, 133, 180, 91, 149, 82, and 114;

(93) a combination of SEQ ID NOs: 121, 155, 137, 188, 127, 1, 79, 198, 110, 86, 75, 100, 109, 3, 152, 133, 149, 2, 82, 182, and 114;

(94) a combination of SEQ ID NOs: 155, 137, 188, 127, 5, 1, 84, 198, 194, 110, 75, 100, 109, 145, 48, 71, 152, 133, 82, and 114;

(95) a combination of SEQ ID NOs: 155, 142, 188, 127, 1, 79, 196, 154, 110, 41, 31, 109, 145, 48, 54, 174, 108, 71, 133, 82, and 114;

(96) a combination of SEQ ID NOs: 121, 155, 188, 127, 1, 161, 198, 189, 110, 78, 100, 178, 71, 152, 133, 159, 149, 104, 182, and 114;

(97) a combination of SEQ ID NOs: 155, 188, 127, 1, 79, 189, 110, 176, 175, 109, 145, 48, 71, 73, 152, 133, 91, 159, 82, 77, and 114;

(98) a combination of SEQ ID NOs: 155, 87, 142, 188, 127, 1, 196, 17, 189, 110, 153, 101, 175, 100, 109, 133, 180, 149, 51, and 114;

(99) a combination of SEQ ID NOs: 121, 155, 188, 127, 85, 1, 198, 189, 154, 110, 109, 145, 125, 178, 71, 152, 141, 133, 180, 159, and 114;

(100) a combination of SEQ ID NOs: 155, 188, 127, 1, 198, 187, 110, 68, 175, 100, 109, 145, 125, 10, 3, 71, 152, 133, 82, 182, and 114;

(101) a combination of SEQ ID NOs: 121, 155, 87, 142, 188, 127, 1, 84, 110, 26, 75, 100, 71, 106, 152, 180, 159, 149, 2, and 114;

(102) a combination of SEQ ID NOs: 155, 142, 188, 127, 1, 189, 154, 110, 78, 185, 153, 177, 109, 178, 54, 174, 92, 133, 82, 57, and 114;

(103) a combination of SEQ ID NOs: 155, 142, 188, 127, 146, 1, 189, 154, 110, 185, 153, 175, 100, 109, 145, 92, 152, 133, 82, 104, and 114;

(104) a combination of SEQ ID NOs: 155, 137, 188,5, 1, 196, 198, 154, 110, 193, 109, 145, 135, 3, 152, 159, 104, 171, 182, and 114;

(105) a combination of SEQ ID NOs: 121, 155, 188, 127, 1, 198, 110, 175, 75, 109, 145, 3, 96, 71, 152, 133, 180, 104, 51, 182, and 114;

(106) a combination of SEQ ID NOs: 121, 155, 137, 188, 127, 30, 1, 79, 110, 58, 193, 109, 145, 156, 71, 152, 133, 149, 182, and 114;

(107) a combination of SEQ ID NOs: 121, 155, 87, 127, 1, 158, 161, 84, 110, 175, 100, 109, 48, 3, 54, 190, 133, 91, 159, 82, and 114;

(108) a combination of SEQ ID NOs: 155, 188, 127, 1, 196, 198, 110, 185, 175, 75, 100, 109, 145, 152, 133, 39, 91, 149, 82, 182, and 114;

(109) a combination of SEQ ID NOs: 155, 137, 142, 188, 127, 1, 196, 161, 154, 110, 185, 109, 199, 145, 178, 3, 71, 133, 82, 114, and 12;

(110) a combination of SEQ ID NOs: 121, 155, 137, 87, 188, 127, 5, 1, 198, 189, 194, 110, 75, 100, 109, 145, 3, 152, 159, 82, and 114;

(111) a combination of SEQ ID NOs: 155, 137, 87, 188, 127, 85, 1, 198, 154, 110, 128, 185, 101, 145, 10, 3, 152, 180, 159, 149, 51, and 114;

(112) a combination of SEQ ID NOs: 155, 137, 142, 188, 127, 1, 161, 194, 110, 101, 100, 145, 178, 3, 54, 152, 133, 180, 149, 51, 182, and 114;

(113) a combination of SEQ ID NOs: 155, 87, 188, 127, 1, 161, 198, 189, 110, 185, 193, 175, 109, 145, 73, 133, 159, 149, 82, 61, 18, and 114;

(114) a combination of SEQ ID NOs: 121, 155, 87, 188, 127, 1, 16, 161, 198, 154, 110, 109, 145, 178, 10, 174, 71, 133, 159, 2, 82, and 114;

(115) a combination of SEQ ID NOs: 121, 155, 188, 127, 1, 161, 189, 110, 185, 177, 100, 109, 125, 71, 133, 159, 149, 82, 104, 182, and 114;

(116) a combination of SEQ ID NOs: 121, 137, 188, 127, 30, 1, 110, 128, 193, 175, 100, 199, 145, 10, 152, 133, 180, 159, 149, 104, 51, and 114;

(117) a combination of SEQ ID NOs: 155, 188, 127, 85, 1, 161, 198, 154, 110, 175, 100, 109, 178, 54, 92, 152, 133,

82, 104, 77, 51, and 114;

(118) a combination of SEQ ID NOs: 155, 142, 188, 127, 146, 1, 198, 189, 38, 110, 68, 193, 192, 100, 109, 145, 10, 152, 133, 159, 149, 82, and 114;

(119) a combination of SEQ ID NOs: 155, 137, 142, 188, 127, 1, 198, 189, 154, 110, 68, 192, 100, 109, 145, 152, 133, 159, 149, 82, and 114;

(120) a combination of SEQ ID NOs: 155, 142, 188, 127, 1, 194, 110, 9, 200, 148, 86, 109, 145, 125, 174, 92, 163, 152, 159, 149, 82, and 114;

(121) a combination of SEQ ID NOs: 121, 155, 137, 188, 127, 1, 161, 198, 110, 86, 109, 135, 3, 71, 152, 133, 180, 149, 82, 104, 182, and 114;

(122) a combination of SEQ ID NOs: 155, 142, 188, 127, 146, 1, 189, 110, 143, 101, 175, 75, 109, 145, 135, 174, 152, 133, 159, 82, 182, and 114;

(123) a combination of SEQ ID NOs: 155, 137, 87, 142, 188, 127, 30, 1, 196, 198, 110, 58, 193, 175, 109, 145, 73, 133, 180, 149, 182, and 114;

(124) a combination of SEQ ID NOs: 155, 137, 188, 127, 1, 189, 110, 78, 185, 175, 100, 109, 145, 178, 152, 133, 180, 159, 149, 82, 182, and 114;

(125) a combination of SEQ ID NOs: 155, 87, 142, 188, 127, 1, 53, 198, 110, 193, 175, 109, 97, 3, 152, 133, 39, 91, 159, 149, 82, 93, and 114;

(126) a combination of SEQ ID NOs: 121, 155, 137, 87, 188, 127, 1, 84, 198, 110, 102, 175, 75, 100, 109, 145, 54, 152, 133, 149, 77, and 114;

(127) a combination of SEQ ID NOs: 121, 155, 137, 188, 127, 5, 1, 84, 189, 154, 194, 110, 75, 3, 152, 133, 180, 159, 149, 104, 77, and 114;

(128) a combination of SEQ ID NOs: 155, 137, 87, 188, 127, 1, 196, 161, 110, 143, 86, 175, 100, 109, 63, 71, 133, 39, 91, 159, 149, 82, and 114;

(129) a combination of SEQ ID NOs: 121, 155, 137, 87, 188, 127, 1, 196, 110, 9, 193, 100, 109, 135, 54, 71, 73, 111, 133, 149, 104, 182, and 114;

(130) a combination of SEQ ID NOs: 155, 137, 87, 188, 127, 1, 53, 198, 189, 110, 153, 175, 100, 109, 145, 10, 88, 190, 133, 180, 91, 149, and 114;

(131) a combination of SEQ ID NOs: 121, 155, 87, 142, 188, 127, 1, 196, 198, 110, 128, 41, 101, 175, 100, 109, 145, 54, 152, 150, 182, 93, and 114;

(132) a combination of SEQ ID NOs: 179, 155, 87, 142, 188, 127, 5, 1, 198, 154, 194, 110, 193, 175, 199, 145, 3, 71, 152, 133, 159, 82, and 114;

(133) a combination of SEQ ID NOs: 121, 155, 87, 127, 1, 189, 154, 110, 193, 100, 109, 10, 3, 152, 133, 180, 159, 2, 82, 104, 182, and 114;

(134) a combination of SEQ ID NOs: 155, 142, 188, 127, 1, 162, 196, 154, 110, 200, 101, 193, 175, 109, 125, 71, 133, 180, 149, 82, 77, and 114;

(135) a combination of SEQ ID NOs: 155, 137, 188, 127, 1, 161, 198, 154, 194, 110, 100, 145, 178, 3, 71, 73, 152, 133, 159, 149, 82, and 114;

(136) a combination of SEQ ID NOs: 179, 155, 87, 188, 127, 5, 1, 154, 110, 200, 193, 175, 100, 109, 145, 3, 133, 91, 159, 149, 82, 182, and 114;

(137) a combination of SEQ ID NOs: 179, 121, 155, 87, 188, 127, 1, 161, 198, 154, 143, 193, 175, 192, 100, 109, 71, 133, 149, 104, 182, and 114;

(138) a combination of SEQ ID NOs: 155, 137, 87, 188, 127, 1, 198, 189, 38, 194, 110, 175, 100, 109, 145, 10, 152, 133, 149, 82, 57, and 114;

(139) a combination of SEQ ID NOs: 155, 137, 87, 142, 188, 127, 1, 154, 194, 110, 193, 175, 145, 156, 54, 152, 133, 180, 159, 149, 77, 57, and 114;

(140) a combination of SEQ ID NOs: 155, 137, 188, 127, 5, 1, 84, 154, 110, 175, 192, 100, 109, 145, 10, 108, 152, 133, 180, 159, 149, 82, and 114;

(141) a combination of SEQ ID NOs: 155, 188, 127, 146, 1, 196, 189, 194, 110, 200, 86, 193, 100, 109, 152, 39, 159, 149, 82, 77, 182, and 114;

(142) a combination of SEQ ID NOs: 155, 188, 127, 146, 1, 196, 161, 154, 38, 194, 110, 56, 78, 68, 175, 100, 73, 152, 133, 159, 149, 182, and 114;

(143) a combination of SEQ ID NOs: 155, 137, 87, 142, 188, 127, 5, 1, 84, 189, 154, 194, 110, 175, 75, 145, 152, 133, 180, 159, 77, and 114;

(144) a combination of SEQ ID NOs: 121, 137, 87, 142, 188, 127, 1, 154, 194, 110, 128, 185, 175, 100, 83, 71, 73, 152, 133, 149, 82, 182, and 114;

(145) a combination of SEQ ID NOs: 155, 188, 127, 5, 1, 84, 154, 110, 176, 58, 102, 148, 109, 145, 135, 3, 54, 191, 152, 133, 180, 82, and 114;

(146) a combination of SEQ ID NOs: 155, 137, 142, 188, 127, 5, 1, 196, 84, 189, 154, 110, 109, 145, 135, 96, 174, 152, 133, 180, 91, 159, 82, and 114;

(147) a combination of SEQ ID NOs: 121, 155, 137, 87, 188, 127, 1, 198, 189, 110, 26, 177, 175, 100, 109, 145, 48, 98, 54, 108, 133, 159, 82, 93, and 114;

(148) a combination of SEQ ID NOs: 179, 155, 142, 188, 127, 1, 154, 194, 110, 56, 175, 109, 145, 88, 3, 174, 73, 152, 133, 82, 171, 150, and 114;

(149) a combination of SEQ ID NOs: 179, 155, 137, 142, 188, 127, 1, 84, 189, 110, 148, 193, 109, 145, 88, 3, 71, 152, 180, 159, 82, 150, and 114;

(150) a combination of SEQ ID NOs: 179, 155, 137, 87, 142, 188, 127, 5, 1, 161, 198, 110, 41, 100, 109, 145, 174, 71, 152, 159, 149, 82, 182, and 114;

(151) a combination of SEQ ID NOs: 155, 107, 188, 127, 1, 79, 198, 110,58, 100, 109, 145, 125, 10, 54, 174, 106, 152, 133, 82, 57, 51, and 114;

(152) a combination of SEQ ID NOs: 155, 137, 87, 142, 188, 127, 1, 53, 198, 110, 41, 185, 175, 100, 109, 169, 49, 3, 54, 71, 133, 149, 182, and 114;

(153) a combination of SEQ ID NOs: 121, 155, 137, 87, 188, 127, 1, 198, 110, 58, 100, 109, 3, 54, 191, 71, 133, 180, 91, 149, 2, 77, 182, and 114;

(154) a combination of SEQ ID NOs: 155, 137, 87, 142, 188, 127, 1, 198, 110, 26, 101, 175, 109, 145, 178, 152, 133, 180, 149, 61, 150, 182, and 114;

(155) a combination of SEQ ID NOs: 121, 155, 87, 188, 127, 1, 162, 110, 86, 193, 175, 109, 145, 3, 71, 152, 133, 159, 149, 82, 77, 150, and 114;

(156) a combination of SEQ ID NOs: 121, 142, 188, 127, 1, 196, 154, 110, 56, 153, 177, 109, 21, 10, 3, 54, 174, 71, 163, 159, 82, 104, 182, and 114;

(157) a combination of SEQ ID NOs: 155, 137, 188, 127, 85, 1, 138, 110, 86, 26, 193, 175, 100, 145, 156, 10, 88, 3, 152, 111, 2, 51, 182, and 114;

(158) a combination of SEQ ID NOs: 155, 137, 188, 127, 1, 189, 110, 136, 68, 26, 193, 175, 100, 105, 145, 3, 71, 152, 111, 133, 82, 104, 77, and 114;

(159) a combination of SEQ ID NOs: 155, 137, 87, 188, 127, 1, 161, 198, 154, 110, 68, 185, 175, 100, 109, 178, 10, 54, 108, 71, 133, 82, 104, and 114;

(160) a combination of SEQ ID NOs: 121, 155, 137, 87, 188, 127, 1, 161, 189, 194, 110, 186, 200, 100, 145, 3, 191, 108, 133, 91, 159, 149, 82, and 114;

(161) a combination of SEQ ID NOs: 121, 155, 188, 127, 1, 198, 154, 110, 185, 86, 193, 175, 109, 145, 178, 135, 3, 73, 152, 133, 159, 82, 182, and 114;

(162) a combination of SEQ ID NOs: 121, 155, 137, 188, 127, 1, 161, 53, 198, 110, 102, 175, 100, 109, 178, 54, 174, 71, 133, 82, 150, 182, and 114;

(163) a combination of SEQ ID NOs: 155, 142, 188, 127, 85, 1, 161, 189, 154, 110, 185, 175, 109, 145, 178, 54, 174, 71, 133, 91, 82, 57, 93, and 114;

(164) a combination of SEQ ID NOs: 121, 155, 137, 142, 188, 127, 1, 198, 110, 193, 175, 100, 145, 174, 71, 106, 152, 159, 149, 82, 55, 182, 93, and 114;

(165) a combination of SEQ ID NOs: 121, 155, 188, 127, 146, 30, 1, 189, 38, 110, 68, 193, 175, 192, 100, 145, 152, 133, 180, 159, 149, 150, and 114;

(166) a combination of SEQ ID NOs: 155, 137, 87, 188, 127, 1, 84, 198, 154,38, 110, 176, 109, 48, 71, 152, 111, 190, 133, 91, 159, 149, 182, 93, and 114;

(167) a combination of SEQ ID NOs: 121, 155, 137, 87, 142, 188, 127, 1, 198, 110, 9, 175, 100, 109, 145, 54, 174, 152, 133, 149, 82, 104, 182, and 114;

(168) a combination of SEQ ID NOs: 121, 155, 188, 127, 1, 196, 172, 198, 154, 110, 145, 48, 135, 3, 152, 133, 39, 159, 82, 104, 51, 182, and 114;

(169) a combination of SEQ ID NOs: 155, 188, 127, 146, 5, 1, 189, 110,200, 185, 26, 193, 100, 109, 145, 96, 71, 152, 133, 159, 82, 77, and 114;

(170) a combination of SEQ ID NOs: 121, 155, 188, 127, 1, 198, 110, 123, 41, 58, 175, 100, 109, 145, 125, 21, 54, 147, 152, 133, 149, 82, 182, and 114;

(171) a combination of SEQ ID NOs: 155, 137, 87, 142, 188, 127, 1, 198, 110, 193, 175, 100, 109, 145, 178, 10, 3, 54, 174, 152, 133, 91, 149, and 114;

(172) a combination of SEQ ID NOs: 155, 87, 188, 127, 1, 196, 161, 198, 154, 110, 186, 68, 200, 175, 100, 109, 48, 73, 152, 133, 159, 77, 182, and 114;

(173) a combination of SEQ ID NOs: 121, 155, 87, 188, 127, 1, 196, 189, 110, 200, 175, 109, 145, 48, 3, 54, 191, 71, 133, 159, 149, 82, 61, 77, and 114;

(174) a combination of SEQ ID NOs: 155, 137, 142, 188, 127, 1, 79, 198, 154, 110, 75, 100, 109, 145, 178, 96, 174, 92, 152, 133, 180, 91, 159, 182, and 114;

(175) a combination of SEQ ID NOs: 121, 155, 137, 87, 142, 188, 127, 1, 161, 154, 110, 175, 100, 109, 145, 62, 3, 174, 133, 180, 91, 159, 82, and 114;

(176) a combination of SEQ ID NOs: 155, 137, 188, 127, 1, 183, 196, 198, 189, 154, 38, 194, 110, 68, 175, 100, 109, 145, 152, 133, 159, 149, 82, 77, and 114;

(177) a combination of SEQ ID NOs: 155, 142, 188, 127, 1, 138, 196, 189, 154, 110, 185, 101, 175, 75, 100, 109, 145, 62, 178, 3, 133, 149, 82, and 114;

(178) a combination of SEQ ID NOs: 121, 155, 137, 87, 188, 127, 1, 196, 198, 110, 175, 100, 109, 145, 3, 71, 152, 133, 159, 82, 77, 150, 182, 93, and 114;

(179) a combination of SEQ ID NOs: 155, 87, 142, 188, 127, 1, 189, 110, 193, 175, 109, 145, 62, 178, 3, 71, 133, 180, 91, 159, 149, 82, 104, 182, 93, and 114;

(180) a combination of SEQ ID NOs: 179, 155, 137, 87, 188, 127, 30, 1, 161, 198, 189, 154, 110, 175, 100, 109, 145, 178, 71, 133, 39, 91, 159, 82, and 114;

(181) a combination of SEQ ID NOs: 179, 121, 155, 142, 188, 127, 1, 161, 172, 53, 198, 110, 175, 100, 109, 105, 3, 54, 133, 149, 82, 77, 182, 93, and 114;

(182) a combination of SEQ ID NOs: 179, 121, 155, 188, 127, 5, 1, 154, 110, 68, 193, 175, 100, 109, 145, 10, 3, 108, 152, 133, 91, 159, 149, 82, and 114;

(183) a combination of SEQ ID NOs: 179, 121, 155, 142, 188, 127, 1, 110, 128, 175, 100, 109, 125, 178, 10, 135, 3, 174, 92, 133, 42, 149, 82, 182, and 114;

(184) a combination of SEQ ID NOs: 121, 155, 188, 127, 85, 5, 1, 79, 198, 154, 110, 145, 135, 54, 71, 73, 152, 133, 39, 82, 104, 51, 182, and 114;

(185) a combination of SEQ ID NOs: 121, 155, 87, 142, 188, 127, 1, 161, 198, 154, 110, 185, 193, 109, 178, 48, 174, 152, 133, 159, 2, 82, 104, and 114;

(186) a combination of SEQ ID NOs: 155, 142, 188, 127, 1, 198, 189, 110, 193, 175, 100, 109, 62, 48, 191, 174, 71, 152, 133, 91, 159, 149, 82, 104, 182, and 114;

(187) a combination of SEQ ID NOs: 155, 87, 142, 188, 127, 85, 30, 1, 198, 194, 110, 78, 58, 148, 105, 145, 3, 174, 152, 133, 180, 159, 149, 57, and 114;

(188) a combination of SEQ ID NOs: 121, 155, 137, 87, 188, 127, 1, 161, 53, 198, 194, 110, 102, 175, 173, 100, 109, 3, 73, 133, 149, 82, 104, 93, and 114;

(189) a combination of SEQ ID NOs: 121, 155, 137, 87, 188, 127, 1, 196, 198, 189, 154, 110, 185, 109, 178, 3, 54, 174, 71, 152, 133, 149, 82, and 114;

(190) a combination of SEQ ID NOs: 155, 87, 188, 127, 5, 1, 196, 161, 198, 110, 68, 185, 100, 109, 145, 63, 54, 37, 108, 133, 159, 2, 82, 104, and 114;

(191) a combination of SEQ ID NOs: 155, 142, 188, 85, 1, 138, 196, 53, 198, 189, 110, 44, 101, 175, 100, 145, 152, 180, 159, 149, 2, 82, 51, 182, 93, and 114;

(192) a combination of SEQ ID NOs: 155, 137, 87, 188, 127, 1, 161, 198, 189, 154, 194, 110, 143, 193, 175, 109, 145, 3, 111, 133, 39, 91, 149, 104, 93, and 114;

(193) a combination of SEQ ID NOs: 179, 121, 155, 188, 127, 1, 198, 110, 200, 148, 175, 75, 100, 109, 145, 125, 63, 54, 174, 92, 190, 133, 91, 82, and 104;

(194) a combination of SEQ ID NOs: 121, 155, 142, 188, 94, 1, 161, 154, 110, 185, 175, 75, 100, 109, 3, 96, 174, 108, 92, 152, 133, 180, 91, 2, 82, and 114;

(195) a combination of SEQ ID NOs: 121, 155, 137, 87, 188, 127, 1, 84, 198, 110, 148, 175, 75, 100, 109, 145, 178, 3, 54, 174, 152, 133, 180, 91, 149, and 114;

(196) a combination of SEQ ID NOs: 121, 155, 87, 188, 127, 1, 196, 198, 110, 148, 185, 75, 109, 145, 3, 54, 96, 174, 71, 152, 133, 91, 159, 2, 82, and 114;

(197) a combination of SEQ ID NOs: 155, 142, 188, 127, 94, 1, 84, 189, 194, 110, 9, 185, 175, 75, 109, 145, 88, 135, 71, 152, 190, 133, 180, 82, 182, and 114;

(198) a combination of SEQ ID NOs: 155, 87, 142, 188, 127, 1, 79, 161, 198, 154, 110, 100, 109, 178, 174, 92, 152, 133, 91, 159, 149, 82, 55, 93, and 114;

(199) a combination of SEQ ID NOs: 179, 155, 137, 87, 188, 127, 1, 196, 161, 198, 189, 110, 86, 175, 100, 109, 108, 152, 133, 39, 159, 149, 82, 77, 93, and 114;

(200) a combination of SEQ ID NOs: 155, 188, 127, 1, 45, 194, 110, 157, 68, 200, 86, 193, 75, 109, 145, 125, 3, 152, 133, 159, 149, 2, 82, 51, 150, and 114;

(201) a combination of SEQ ID NOs: 121, 155, 137, 87, 127, 1, 84, 198, 189, 110, 185, 177, 193, 100, 109, 62, 10, 88, 191, 133, 91, 159, 82, 104, 182, and 114;

(202) a combination of SEQ ID NOs: 121, 155, 87, 142, 188, 127, 1, 196, 172, 198, 110, 102, 26, 193, 175, 109, 145, 62, 71, 73, 133, 149, 82, 51, and 114;

(203) a combination of SEQ ID NOs: 13, 121, 87, 142, 188, 127, 30, 1, 198, 154, 110, 175, 192, 109, 145, 178, 10, 174, 152, 180, 91, 159, 149, 82, 182, and 114;

(204) a combination of SEQ ID NOs: 155, 137, 87, 142, 188, 127, 1, 79, 196, 84, 198, 110, 175, 100, 109, 145, 63, 152, 190, 133, 180, 91, 159, 149, 93, and 114;

(205) a combination of SEQ ID NOs: 155, 87, 188, 127, 1, 196, 84, 198, 110, 128, 56, 185, 175, 109, 178, 3, 174, 133, 180, 39, 159, 149, 82, 77, 182, and 114;

(206) a combination of SEQ ID NOs: 155, 87, 142, 188, 127, 5, 1, 79, 196, 161, 154, 110, 41, 58, 102, 175, 100, 109, 145, 48, 71, 133, 91, 82, 93, and 114;

(207) a combination of SEQ ID NOs: 155, 188, 127, 1, 194, 110, 200, 86, 193, 175, 100, 109, 145, 125, 88, 3, 71, 152, 133, 180, 39, 159, 149, 77, 114, 50;

(208) a combination of SEQ ID NOs: 179, 155, 137, 87, 188, 127, 85, 1, 161, 84, 110, 148, 153, 101, 175, 109, 178, 63, 71, 152, 133, 91, 159, 149, 77, and 114;

(209) a combination of SEQ ID NOs: 155, 137, 87, 188, 127, 1, 161, 198, 110, 193, 175, 100, 109, 97, 178, 48, 54, 174, 108, 152, 133, 159, 82, 61, and 114;

(210) a combination of SEQ ID NOs: 179, 155, 137, 142, 188, 127, 1, 161, 198, 189, 110, 58, 175, 100, 109, 10, 3, 108, 73, 152, 133, 159, 82, 77, 182, and 114;

(211) a combination of SEQ ID NOs: 121, 155, 87, 142, 188, 127, 1, 161, 198, 154, 110, 193, 175, 192, 100, 109, 169, 73, 163, 133, 159, 149, 82, 77, 182, 93, and 114;

(212) a combination of SEQ ID NOs: 179, 155, 87, 188, 127, 1, 161, 198, 189, 154, 194, 110, 68, 86, 175, 25, 199, 152, 133, 180, 149, 2, 104, 77, and 114;

(213) a combination of SEQ ID NOs: 155, 137, 87, 142, 188, 127, 1, 198, 154, 110, 68, 86, 75, 100, 54, 174, 92, 152, 111, 190, 133, 180, 159, 57, and 114;

(214) a combination of SEQ ID NOs: 155, 87, 188, 127, 1, 196, 161, 198, 110, 26, 193, 175, 100, 109, 145, 178, 48, 71, 73, 152, 133, 149, 82, 104, 182, and 114;

(215) a combination of SEQ ID NOs: 155, 87, 188, 127, 1, 161, 198, 110, 185, 175, 25, 100, 109, 178, 54, 108, 152, 141, 133, 91, 149, 82, 104, 182, and 114;

(216) a combination of SEQ ID NOs: 155, 87, 142, 188, 127, 5, 1, 198, 154, 110, 148, 101, 193, 175, 109, 178, 48, 135, 73, 152, 133, 91, 159, 82, 51, 182, and 114;

(217) a combination of SEQ ID NOs: 155, 87, 188, 127, 146, 30, 1, 196, 198, 154, 38, 110, 193, 175, 100, 167, 178, 54, 133, 180, 159, 2, 77, 18, 20, 182, and 114;

(218) a combination of SEQ ID NOs: 121, 155, 87, 142, 188, 127, 146, 1, 196, 198, 189, 154, 38, 110, 177, 100, 109, 178, 174, 152, 133, 91, 159, 82, 182, and 114;

(219) a combination of SEQ ID NOs: 87, 188, 127, 1, 196, 161, 84, 53, 198, 110, 200, 193, 175, 109, 48, 135, 54, 174, 71, 133, 180, 91, 159, 82, 104, 182, and 114;

(220) a combination of SEQ ID NOs: 155, 142, 188, 127, 1, 53, 110, 81, 185, 153, 101, 175, 173, 109, 117, 152, 133, 180, 39, 91, 159, 149, 2, 82, 182, and 114;

(221) a combination of SEQ ID NOs: 121, 155, 87, 188, 127, 1, 196, 161, 198, 154, 110, 58, 175, 109, 145, 125, 178, 3, 191, 92, 152, 133, 149, 82, 93, and 114;

(222) a combination of SEQ ID NOs: 155, 137, 87, 142, 188, 127, 1, 198, 154, 110, 175, 100, 109, 145, 178, 3, 47, 54, 133, 39, 91, 149, 82, 104, 182, and 114;

(223) a combination of SEQ ID NOs: 179, 155, 142, 188, 127, 5, 1, 161, 198, 154, 110, 143, 69, 193, 175, 173, 109, 145, 178, 10, 174, 71, 159, 82, 93, and 114;

(224) a combination of SEQ ID NOs: 179, 155, 137, 87, 188, 127, 1, 161, 84, 194, 110, 185, 175, 109, 178, 63, 71, 152, 133, 180, 91, 159, 149, 82, 77, 182, and 114;

(225) a combination of SEQ ID NOs: 155, 87, 142, 188, 127, 30, 1, 198, 154, 110, 176, 100, 109, 145, 48, 135, 3, 71, 152, 180, 39, 149, 82, 51, 93, and 114;

(226) a combination of SEQ ID NOs: 121, 155, 137, 87, 188, 127, 1, 198, 194, 110, 193, 175, 100, 109, 199, 145, 10, 3, 71, 133, 149, 2, 82, 77, 93, and 114;

(227) a combination of SEQ ID NOs: 121, 155, 137, 87, 127, 1, 79, 198, 154, 110, 185, 175, 109, 145, 178, 10, 3, 152, 91, 159, 149, 82, 104, 52, 182, and 114;

(228) a combination of SEQ ID NOs: 121, 155, 137, 87, 142, 127, 1, 194, 110, 86, 26, 193, 175, 25, 75, 109, 145, 3, 174, 152, 133, 180, 39, 159, 82, 182, and 114;

(229) a combination of SEQ ID NOs: 155, 137, 142, 188, 127, 1, 196, 161, 198, 110, 143, 185, 153, 175, 109, 145, 62, 178, 48, 174, 73, 152, 133, 159, 82, 104, and 114;

(230) a combination of SEQ ID NOs: 155, 137, 142, 188, 127, 30, 1, 198, 194, 110, 9, 58, 86, 175, 100, 167, 145, 98, 152, 133, 180, 159, 82, 104, 51, 150, and 114;

(231) a combination of SEQ ID NOs: 121, 155, 87, 188, 127, 1, 79, 161, 198, 154, 110, 78, 175, 100, 109, 54, 174, 71, 152, 133, 180, 149, 82, 182, 93, and 114;

(232) a combination of SEQ ID NOs: 155, 87, 142, 188, 127, 1, 80, 161, 53, 187, 189, 154, 110, 175, 75, 31, 109, 62, 48, 54, 191, 92, 71, 133, 149, 82, and 114;

(233) a combination of SEQ ID NOs: 155, 137, 87, 188, 127, 5, 1, 161, 84, 198, 194, 110, 175, 100, 109, 145, 3,

133, 180, 159, 149, 82, 104, 150, 182, and 114;

(234) a combination of SEQ ID NOs: 155, 137, 87, 142, 188, 127, 1, 53, 198, 189, 154, 110, 175, 192, 100, 109, 145, 125, 178, 54, 174, 133, 159, 82, 182, and 114;

(235) a combination of SEQ ID NOs: 155, 142, 188, 127, 146, 1, 53, 189, 154, 38, 194, 110, 185, 175, 100, 109, 145, 135, 3, 96, 174, 71, 133, 159, 82, 182, and 114;

(236) a combination of SEQ ID NOs: 155, 188, 127, 5, 1, 198, 110, 176, 68, 175, 145, 125, 98, 10, 3, 71, 152, 111, 133, 39, 159, 149, 82, 104, 51, 150, and 114;

(237) a combination of SEQ ID NOs: 155, 137, 142, 188, 127, 146, 1, 189, 194, 110, 143, 200, 193, 175, 100, 109, 145, 156, 10, 152, 180, 91, 159, 82, 77, 182, and 114;

(238) a combination of SEQ ID NOs: 179, 155, 142, 188, 127, 1, 196, 198, 189, 110, 44, 78, 148, 185, 175, 100, 109, 105, 167, 145, 62, 152, 133, 39, 82, and 114;

(239) a combination of SEQ ID NOs: 179, 87, 142, 188, 127, 1, 196, 161, 53, 198, 154, 110, 58, 153, 175, 109, 10, 3, 141, 133, 180, 91, 149, 82, 182, and 114;

(240) a combination of SEQ ID NOs: 121, 155, 87, 188, 127, 1, 79, 158, 196, 161, 198, 38, 110, 102, 175, 100, 109, 135, 54, 108, 71, 133, 149, 82, 104, and 114;

(241) a combination of SEQ ID NOs: 121, 155, 137, 87, 188, 127, 94, 1, 84, 194, 110, 148, 193, 175, 75, 100, 109, 145, 178, 3, 54, 152, 133, 149, 2, 82, 77, and 114;

(242) a combination of SEQ ID NOs: 155, 137, 87, 188, 127, 1, 161, 198, 154, 110, 185, 175, 109, 145, 178, 63, 108, 71, 152, 133, 91, 159, 149, 82, 104, 77, 150, and 114;

(243) a combination of SEQ ID NOs: 155, 142, 188, 127, 30, 1, 161, 189, 110, 153, 193, 175, 109, 145, 62, 178, 98, 3, 191, 71, 152, 133, 39, 149, 82, 51, 182, and 114;

(244) a combination of SEQ ID NOs: 121, 155, 137, 142, 188, 127, 30, 1, 196, 161, 198, 110, 193, 175, 100, 109, 10, 174, 152, 133, 180, 91, 149, 82, 51, 182, and 114;

(245) a combination of SEQ ID NOs: 155, 137, 87, 142, 188, 127, 1, 196, 161, 198, 110, 78, 90, 193, 175, 100, 109, 145, 71, 73, 133, 159, 149, 82, 51, 182, and 114;

(246) a combination of SEQ ID NOs: 155, 137, 87, 188, 127, 1, 79, 196, 161, 189, 110, 153, 175, 100, 109, 145, 62, 54, 191, 108, 71, 73, 133, 91, 159, 149, 82, and 114;

(247) a combination of SEQ ID NOs: 179, 155, 142, 188, 127, 1, 79, 158, 189, 154, 110, 4, 185, 175, 100, 109, 145, 54, 174, 71, 152, 133, 159, 82, 51, 93, and 114;

(248) a combination of SEQ ID NOs: 179, 155, 142, 188, 127, 1, 80, 198, 187, 189, 110, 148, 175, 100, 109, 97, 145, 135, 54, 174, 92, 152, 133, 149, 82, 51, and 114;

(249) a combination of SEQ ID NOs: 179, 121, 155, 142, 188, 127, 5, 1, 194, 110, 9, 4, 75, 100, 109, 145, 125, 178, 3, 54, 92, 152, 133, 91, 82, 182, and 114;

(250) a combination of SEQ ID NOs: 179, 155, 188, 127, 1, 161, 198, 154, 110, 58, 153, 26, 175, 192, 109, 97, 145, 178, 10, 174, 152, 133, 91, 159, 104, 93, and 114;

(251) a combination of SEQ ID NOs: 155, 188, 127, 1, 79, 84, 189, 110, 78, 176, 58, 185, 175, 75, 100, 109, 145, 48, 3, 152, 141, 133, 39, 159, 82, 77, 51, and 114;

(252) a combination of SEQ ID NOs: 179, 155, 87, 188, 127, 1, 79, 196, 198, 189, 110, 153, 193, 175, 100, 109, 3, 152, 190, 133, 39, 91, 159, 149, 82, 61, 182, and 114;

(253) a combination of SEQ ID NOs: 155, 87, 142, 188, 127, 30, 1, 198, 189, 154, 194, 110, 68, 86, 193, 175, 100, 109, 97, 145, 10, 152, 133, 159, 77, 57, 182, and 114;

(254) a combination of SEQ ID NOs: 121, 155, 137, 87, 188, 127, 1, 198, 110, 56, 193, 175, 192, 100, 109, 145, 178, 48, 10, 83, 71, 152, 133, 180, 159, 149, 182, and 114;

(255) a combination of SEQ ID NOs: 121, 155, 87, 188, 127, 1, 161, 198, 189, 154, 38, 110, 175, 109, 48, 108, 71, 73, 152, 133, 91, 159, 82, 77, 182, 114, 12;

(256) a combination of SEQ ID NOs: 121, 155, 137, 87, 188, 127, 1, 198, 110, 176, 185, 175, 109, 48, 10, 3, 54, 73, 133, 180, 91, 159, 149, 82, 51, 182, 93, and 114;

(257) a combination of SEQ ID NOs: 179, 155, 87, 142, 188, 127, 1, 183, 53, 198, 189, 110, 68, 148, 175, 100, 109, 3, 99, 152, 133, 180, 39, 159, 149, 2, 82, 182, and 114;

(258) a combination of SEQ ID NOs: 155, 137, 87, 142, 188, 127, 1, 79, 196, 198, 189, 38, 110, 75, 109, 145, 10, 174, 92, 71, 152, 190, 133, 180, 91, 104, 182, and 114;

(259) a combination of SEQ ID NOs: 121, 155, 137, 87, 188, 127, 1, 196, 172, 84, 194, 110, 200, 102, 86, 193, 75, 109, 3, 71, 133, 159, 149, 2, 82, 77, 182, and 114;

(260) a combination of SEQ ID NOs: 121, 155, 87, 142, 188, 127, 94, 1, 196, 161, 198, 189, 110, 102, 175, 100, 109, 145, 48, 54, 174, 39, 91, 159, 82, 77, 182, and 114;

(261) a combination of SEQ ID NOs: 155, 87, 188, 127, 30, 1, 79, 196, 161, 154, 38, 194, 110, 86, 26, 100, 145, 3, 54, 174, 71, 152, 133, 39, 159, 149, 104, 57, and 114;

(262) a combination of SEQ ID NOs: 155, 137, 142, 188, 127, 1, 196, 161, 53, 198, 189, 154, 38, 110, 68, 102, 175, 100, 109, 97, 98, 54, 133, 91, 149, 82, 182, and 114;

(263) a combination of SEQ ID NOs: 155, 137, 87, 142, 188, 127, 85, 1, 79, 198, 189, 154, 110, 148, 185, 100, 109, 145, 135, 174, 71, 152, 133, 91, 159, 149, 82, 182, and 114;

(264) a combination of SEQ ID NOs: 155, 137, 87, 142, 188, 127, 146, 30, 1, 79, 53, 189, 154, 110, 192, 100, 109, 145, 48, 174, 71, 152, 133, 159, 82, 150, 182, 93, and 114;

(265) a combination of SEQ ID NOs: 121, 155, 137, 87, 142, 188, 127, 1, 110, 177, 193, 175, 192, 100, 109, 178, 54, 73, 152, 111, 133, 180, 91, 159, 149, 104, 51, 150, and 114;

(266) a combination of SEQ ID NOs: 155, 137, 87, 142, 188, 127, 1, 198, 110, 41, 185, 193, 175, 109, 97, 167, 62, 178, 10, 54, 71, 152, 190, 133, 91, 149, 104, 182, and 114;

(267) a combination of SEQ ID NOs: 121, 155, 137, 142, 188, 127, 1, 189, 194, 110, 58, 193, 175, 109, 169, 145, 125, 178, 54, 174, 133, 180, 91, 159, 82, 104, 51, 182, and 114;

(268) a combination of SEQ ID NOs: 155, 137, 87, 142, 188, 127, 1, 80, 196, 198, 189, 154, 194, 110, 41, 148, 177, 175, 100, 109, 145, 178, 48, 174, 133, 91, 2, 82, 93, and 114;

(269) a combination of SEQ ID NOs: 121, 155, 137, 87, 142, 188, 127, 1, 84, 198, 194, 110, 153, 193, 175, 100, 109, 48, 135, 3, 96, 174, 133, 39, 91, 149, 2, 82, 93, and 114;

(270) a combination of SEQ ID NOs: 155, 142, 188, 127, 1, 79, 198, 189, 154, 67, 110, 175, 75, 100, 109, 145, 62, 125, 174, 108, 190, 133, 39, 91, 159, 149, 82, 55, and 114;

(271) a combination of SEQ ID NOs: 155, 137, 188, 127, 1, 79, 53, 154, 110, 186, 143, 58, 86, 69, 100, 145, 178, 3, 54, 152, 133, 180, 91, 159, 149, 77, 57, 182, and 114;

(272) a combination of SEQ ID NOs: 121, 155, 137, 87, 142, 188, 127, 85, 1, 189, 110, 128, 157, 101, 193, 175, 118, 75, 31, 100, 145, 178, 88, 3, 54, 152, 133, 2, 82, and 114;

(273) a combination of SEQ ID NOs: 179, 121, 155, 142, 188, 127, 1, 161, 53, 154, 110, 148, 193, 175, 31, 100, 109, 62, 48, 156, 88, 54, 71, 152, 111, 133, 149, 82, 182, and 114;

(274) a combination of SEQ ID NOs: 121, 155, 87, 188, 127, 1, 161, 198, 110, 128, 200, 148, 185, 175, 192, 75, 100, 109, 145, 48, 174, 152, 133, 180, 149, 82, 104, 182, and 114;

(275) a combination of SEQ ID NOs: 155, 137, 188, 127, 5, 1, 196, 53, 198, 154, 110, 74, 68, 101, 175, 192, 109, 145, 10, 3, 108, 71, 133, 159, 149, 82, 77, 150, 182, 93, and 114;

(276) a combination of SEQ ID NOs: 155, 137, 87, 127, 5, 1, 84, 198, 110, 185, 153, 175, 75, 100, 109, 145, 178, 10, 174, 71, 141, 190, 133, 180, 39, 91, 149, 182, 93, and 114;

(277) a combination of SEQ ID NOs: 121, 155, 87, 142, 188, 127, 94, 1, 161, 198, 187, 110, 185, 175, 109, 167, 145, 178, 54, 152, 133, 39, 91, 159, 149, 2, 82, 61, 51, and 114;

(278) a combination of SEQ ID NOs: 121, 155, 137, 142, 188, 127, 5, 1, 161, 198, 189, 154, 110, 185, 153, 177, 175, 100, 109, 178, 10, 54, 174, 152, 133, 91, 159, 149, 82, 104, and 114;

(279) a combination of SEQ ID NOs: 179, 155, 137, 142, 188, 127, 1, 198, 187, 154, 110, 176, 143, 200, 175, 100, 109, 97, 145, 62, 178, 48, 108, 71, 152, 111, 133, 91, 159, 104, and 114;

(280) a combination of SEQ ID NOs: 155, 142, 188, 127, 1, 161, 198, 189, 154, 110, 78, 143, 175, 100, 109, 178, 10, 3, 191, 174, 108, 71, 73, 152, 133, 149, 2, 82, 104, 182, and 114;

(281) a combination of SEQ ID NOs: 121, 155, 87, 188, 127, 1, 29, 161, 53, 198, 187, 110, 185, 153, 175, 100, 109, 145, 178, 48, 59, 152, 141, 133, 180, 91, 149, 82, 104, and 114;

(282) a combination of SEQ ID NOs: 155, 142, 188, 127, 1, 189, 154, 110, 78, 193, 175, 75, 100, 109, 145, 62, 178, 96, 174, 108, 92, 71, 134, 152, 133, 159, 82, 104, 93, and 114;

(283) a combination of SEQ ID NOs: 121, 155, 87, 142, 188, 127, 1, 161, 198, 189, 194, 110, 185, 193, 175, 75, 100, 109, 125, 54, 191, 174, 152, 190, 133, 91, 159, 82, 77, 182, 93, and 114;

(284) a combination of SEQ ID NOs: 155, 137, 87, 188, 127, 1, 79, 198, 189, 110, 193, 175, 75, 109, 145, 178, 48, 98, 135, 3, 71, 133, 39, 91, 159, 149, 2, 82, 77, 93, and 114;

(285) a combination of SEQ ID NOs: 179, 121, 155, 137, 87, 188, 127, 1, 79, 196, 161, 198, 154, 110, 148, 86, 175, 100, 109, 145, 174, 92, 152, 133, 159, 149, 2, 82, 182, 93, and 114;

(286) a combination of SEQ ID NOs: 179, 121, 87, 142, 188, 127, 5, 1, 183, 161, 154, 110, 153, 175, 109, 145, 125, 178, 156, 3, 54, 174, 71, 133, 159, 149, 82, 104, 150, 93, and 114;

(287) a combination of SEQ ID NOs: 121, 155, 137, 87, 188, 127, 1, 196, 198, 189, 194, 110, 68, 193, 175, 100, 109, 169, 145, 10, 3, 174, 152, 133, 39, 91, 159, 149, 82, 77, 57, 182, and 114;

(288) a combination of SEQ ID NOs: 155, 137, 87, 188, 127, 1, 80, 161, 198, 189, 110, 176, 102, 185, 175, 100, 109, 97, 145, 54, 92, 152, 133, 180, 91, 149, 104, 61, 18, 57, 93, and 114;

(289) a combination of SEQ ID NOs: 121, 155, 137, 87, 188, 127, 1, 161, 198, 189, 38, 110, 185, 175, 100, 109, 145, 62, 125, 178, 48, 10, 3, 108, 71, 152, 133, 91, 159, 104, 77, 182, 93, and 114;

(290) a combination of SEQ ID NOs: 179, 121, 155, 87, 188, 127, 1, 79, 161, 172, 198, 110, 148, 185, 153, 175, 109, 145, 178, 48, 54, 191, 174, 71, 73, 152, 133, 39, 91, 149, 82, 51, 93, and 114; and

(291) a combination of SEQ ID NOs: 121, 155, 137, 87, 188, 127, 1, 79, 198, 189, 38, 194, 110, 143, 68, 26, 177,

101, 175, 25, 192, 100, 109, 63, 135, 54, 108, 71, 152, 133, 91, 82, 104, 171, 77, 182, and 114.

The following shows, as non-limiting examples of combinations of hippocampal atrophy markers (target nucleic acids) to be used in the present invention, combinations of the polynucleotide consisting of the nucleotide sequence shown by SEQ ID NO: 1, and polynucleotides of hippocampal atrophy markers selected from among those shown in Tables 3 to 6 or polynucleotides consisting of complementary sequences thereof. Combinations of hippocampal atrophy markers (target nucleic acids) may also be combinations of miRNAs or human miRNAs corresponding to the following combinations.

(1) a combination of SEQ ID NOs: 155, 142, 188, 127, 198, 154, 110, 75, 100, 3, 133, 149, 82, and 114;

(2) a combination of SEQ ID NOs: 155, 5, 198, 110, 75, 109, 54, 174, 71, 152, 133, 91, 82, and 114;

(3) a combination of SEQ ID NOs: 155, 188, 127, 110, 9, 101, 175, 54, 71, 111, 133, 2, 82, and 114;

(4) a combination of SEQ ID NOs: 155, 142, 188, 127, 154, 110, 100, 109, 96, 174, 71, 133, 2, 82, and 114;

(5) a combination of SEQ ID NOs: 155, 142, 188, 127, 198, 154, 110, 68, 109, 3, 54, 71, 111, 82, and 114;

(6) a combination of SEQ ID NOs: 155, 188, 127, 110, 68, 200, 109, 145, 10, 83, 71, 152, 180, 159, 82, and 114;

(7) a combination of SEQ ID NOs: 155, 137, 188, 127, 5, 194, 110, 100, 109, 156, 3, 71, 152, 133, 82, 150, and 114;

(8) a combination of SEQ ID NOs: 155, 142, 188, 127, 154, 110, 75, 31, 100, 109, 3, 71, 152, 133, 2, 82, and 114;

(9) a combination of SEQ ID NOs: 121, 155, 188, 127, 198, 110, 68, 175, 109, 135, 3, 174, 71, 133, 180, 149, 82, and 114;

(10) a combination of SEQ ID NOs: 121, 155, 188, 127, 154, 110, 41, 100, 145, 71, 152, 133, 159, 2, 82, 51, 150, and 114;

(11) a combination of SEQ ID NOs: 155, 142, 188, 127, 154, 110, 177, 100, 109, 125, 54, 71, 152, 2, 82, 104, 182, and 114;

(12) a combination of SEQ ID NOs: 155, 188, 127, 30, 198, 189, 110, 193, 175, 100, 3, 71, 133, 159, 2, 82, 182, and 114;

(13) a combination of SEQ ID NOs: 155, 137, 142, 188, 127, 154, 110, 193, 175, 109, 88, 135, 3, 54, 152, 133, 82, and 114;

(14) a combination of SEQ ID NOs: 155, 137, 188, 127, 110, 101, 193, 25, 109, 145, 10, 3, 152, 133, 180, 159, 2, 82, and 114;

(15) a combination of SEQ ID NOs: 155, 87, 188, 127, 161, 198, 38, 110, 101, 175, 100, 109, 10, 54, 71, 133, 91, 82, and 114;

(16) a combination of SEQ ID NOs: 121, 188, 127, 189, 154, 110, 102, 177, 100, 109, 3, 54, 174, 133, 159, 2, 82, 104, and 114;

(17) a combination of SEQ ID NOs: 137, 142, 188, 127, 161, 110, 75, 109, 145, 178, 10, 96, 174, 133, 39, 159, 82, 104, and 114;

(18) a combination of SEQ ID NOs: 87, 188, 127, 196, 161, 154, 110, 100, 109, 135, 71, 73, 152, 159, 2, 82, 182, and 114;

(19) a combination of SEQ ID NOs: 155, 87, 188, 127, 196, 194, 110, 4, 193, 175, 109, 145, 178, 48, 10, 59, 71, 159, 82, and 114;

(20) a combination of SEQ ID NOs: 155, 142, 188, 127, 196, 198, 154, 110, 101, 75, 109, 3, 174, 71, 152, 133, 159, 2, 82, and 114;

(21) a combination of SEQ ID NOs: 155, 137, 188, 127, 5, 198, 189, 154, 110, 128, 193, 100, 109, 3, 152, 133, 82, 182, and 114;

(22) a combination of SEQ ID NOs: 121, 155, 188, 127, 5, 79, 198, 110, 100, 145, 59, 54, 108, 73, 152, 133, 159, 2, 82, and 114;

(23) a combination of SEQ ID NOs: 121, 155, 87, 188, 127, 196, 110, 175, 75, 145, 48, 3, 152, 159, 82, 104, 77, 57, and 114;

(24) a combination of SEQ ID NOs: 155, 87, 142, 188, 127, 198, 154, 110, 185, 109, 178, 54, 174, 152, 133, 159, 82, 77, 51, and 114;

(25) a combination of SEQ ID NOs: 87, 142, 188, 127, 161, 198, 187, 110, 175, 100, 109, 145, 178, 174, 133, 159, 2, 82, 93, and 114;

(26) a combination of SEQ ID NOs: 155, 137, 87, 188, 127, 189, 154, 194, 110, 200, 148, 86, 193, 3, 152, 159, 82, 104, 77, 93, and 114;

(27) a combination of SEQ ID NOs: 121, 155, 142, 188, 127, 196, 110, 185, 26, 175, 100, 109, 145, 88, 3, 71, 152, 2, 82, and 114;

(28) a combination of SEQ ID NOs: 179, 155, 137, 142, 188, 127, 110, 109, 145, 125, 10, 88, 3, 174, 152, 180, 113, 82, 51, 150, and 114;

(29) a combination of SEQ ID NOs: 155, 87, 142, 188, 127, 198, 194, 110, 136, 68, 175, 109, 145, 3, 71, 73, 152, 159, 82, 77, and 114;

(30) a combination of SEQ ID NOs: 121, 155, 142, 188, 127, 53, 189, 110, 200, 193, 75, 109, 145, 3, 152, 133, 180, 82, 104, and 114;

(31) a combination of SEQ ID NOs: 155, 137, 87, 188, 127, 5, 198, 154, 110, 193, 175, 100, 145, 178, 152, 91, 159, 2, 82, 77, and 114;

(32) a combination of SEQ ID NOs: 155, 137, 87, 188, 127, 196, 189, 110, 193, 100, 109, 48, 71, 73, 152, 133, 91, 159, 2, 82, and 114;

(33) a combination of SEQ ID NOs: 155, 142, 188, 127, 53, 189, 154, 110, 78, 185, 75, 109, 145, 174, 152, 133, 2, 82, 93, and 114;

(34) a combination of SEQ ID NOs: 155, 188, 127, 196, 154, 110, 176, 102, 69, 175, 75, 100, 109, 145, 54, 108, 73, 133, 159, 82, and 114;

(35) a combination of SEQ ID NOs: 121, 155, 137, 87, 142, 188, 127, 30, 196, 154, 110, 100, 109, 145, 135, 71, 152, 133, 149, 2, 82, and 114;

(36) a combination of SEQ ID NOs: 155, 137, 87, 188, 127, 196, 187, 110, 102, 193, 175, 100, 109, 145, 125, 152, 149, 2, 82, 104, and 114;

(37) a combination of SEQ ID NOs: 155, 137, 87, 188, 127, 196, 189, 194, 110, 68, 200, 175, 100, 145, 10, 152, 133, 180, 159, 82, 104, and 114;

(38) a combination of SEQ ID NOs: 121, 155, 188, 127, 198, 110, 193, 75, 100, 109, 145, 48, 152, 133, 180, 159, 2, 82, 104, 182, and 114;

(39) a combination of SEQ ID NOs: 155, 87, 188, 127, 110, 185, 193, 109, 145, 10, 3, 71, 73, 180, 91, 159, 149, 82, 51, 182, 93, and 114;

(40) a combination of SEQ ID NOs: 155, 87, 188, 127, 196, 161, 198, 110, 193, 175, 109, 178, 174, 152, 133, 91, 159, 82, 51, 93, and 114;

(41) a combination of SEQ ID NOs: 155, 87, 188, 127, 198, 110, 175, 109, 145, 178, 48, 10, 3, 152, 133, 91, 159, 2, 82, 51, and 114;

(42) a combination of SEQ ID NOs: 121, 155, 188, 127, 196, 189, 110, 68, 193, 100, 109, 145, 135, 3, 71, 152, 133, 180, 159, 82, 182, and 114;

(43) a combination of SEQ ID NOs: 155, 137, 87, 188, 127, 53, 189, 194, 110, 101, 193, 175, 100, 109, 145, 3, 152, 133, 149, 82, and 114;

(44) a combination of SEQ ID NOs: 155, 142, 188, 127, 154, 110, 68, 148, 193, 175, 145, 156, 10, 152, 133, 180, 159, 149, 82, 77, 182, and 114;

(45) a combination of SEQ ID NOs: 121, 155, 87, 188, 127, 198, 194, 110, 109, 135, 3, 191, 152, 133, 39, 159, 149, 82, 104, 51, 150, and 114;

(46) a combination of SEQ ID NOs: 121, 155, 87, 142, 188, 127, 196, 198, 154, 110, 4, 175, 109, 167, 191, 73, 113, 159, 149, 2, 82, 182, and 114;

(47) a combination of SEQ ID NOs: 179, 155, 137, 87, 188, 127, 196, 198, 194, 110, 86, 75, 100, 109, 152, 133, 180, 91, 149, 2, 82, 182, and 114;

(48) a combination of SEQ ID NOs: 155, 87, 188, 127, 161, 198, 110, 153, 175, 192, 109, 145, 178, 10, 152, 133, 91, 149, 82, 104, 182, and 114;

(49) a combination of SEQ ID NOs: 155, 137, 87, 142, 188, 127, 189, 154, 110, 175, 100, 109, 145, 178, 10, 3, 174, 152, 133, 91, 82, 93, and 114;

(50) a combination of SEQ ID NOs: 155, 87, 142, 188, 127, 94, 138, 189, 110, 193, 109, 145, 10, 135, 3, 71, 152, 133, 159, 2, 82, 182, and 114;

(51) a combination of SEQ ID NOs: 142, 188, 127, 196, 189, 154, 110, 185, 193, 109, 145, 178, 88, 3, 152, 133, 180, 159, 82, 150, 182, and 114;

(52) a combination of SEQ ID NOs: 121, 155, 87, 188, 127, 85, 198, 154,38, 110, 185, 193, 109, 145, 125, 178, 3, 152, 133, 159, 82, 182, and 114;

(53) a combination of SEQ ID NOs: 179, 121, 155, 87, 142, 188, 127, 161, 172, 198, 110, 193, 175, 145, 54, 73, 152, 159, 149, 82, 93, and 114;

(54) a combination of SEQ ID NOs: 179, 155, 142, 188, 127, 138, 198, 110, 9, 56, 175, 100, 109, 145, 10, 54, 174, 141, 133, 82, 182, and 114;

(55) a combination of SEQ ID NOs: 121, 155, 87, 142, 188, 127, 198, 110, 109, 167, 145, 21, 48, 3, 174, 71, 152, 133, 149, 2, 82, 182, and 114;

(56) a combination of SEQ ID NOs: 179, 155, 87, 142, 188, 127, 79, 198, 110, 102, 175, 100, 109, 97, 145, 3, 174, 141, 91, 159, 149, 2, 82, and 114;

(57) a combination of SEQ ID NOs: 121, 155, 87, 142, 188, 127, 5, 196, 198, 110, 102, 69, 175, 192, 109, 145, 88, 54, 174, 2, 82, 52, and 114;

(58) a combination of SEQ ID NOs: 155, 142, 188, 127, 183, 198, 110, 128, 200, 185, 175, 75, 100, 178, 152, 133, 180, 159, 149, 82, 77, 51, and 114;

(59) a combination of SEQ ID NOs: 155, 137, 87, 142, 188, 127, 79, 53, 198, 110, 193, 100, 109, 62, 135, 174, 152, 133, 39, 91, 82, 51, 182, and 114;

(60) a combination of SEQ ID NOs: 155, 137, 188, 127, 196, 198, 110, 68, 86, 193, 100, 109, 145, 88, 73, 152, 39, 159, 149, 2, 82, 182, and 114;

(61) a combination of SEQ ID NOs: 155, 87, 188, 127, 30, 198, 110, 9, 175, 109, 48, 10, 3, 96, 73, 152, 133, 91, 159, 149, 2, 82, 51, 93, and 114;

(62) a combination of SEQ ID NOs: 121, 155, 87, 142, 188, 127, 79, 196, 198, 110, 101, 175, 109, 178, 3, 54, 152, 133, 39, 159, 149, 82, 150, 182, and 114;

(63) a combination of SEQ ID NOs: 155, 87, 188, 127, 196, 161, 198, 110, 68, 185, 86, 193, 175, 109, 145, 178, 10, 152, 141, 133, 149, 82, 51, 182, and 114;

(64) a combination of SEQ ID NOs: 179, 155, 137, 188, 127, 5, 79, 198, 189, 154, 110, 177, 193, 100, 109, 145, 178, 3, 152, 133, 149, 82, 104, 150, and 114;

(65) a combination of SEQ ID NOs: 155, 87, 188, 127, 5, 196, 161, 84, 198, 38, 110, 68, 175, 109, 145, 3, 73, 133, 180, 91, 159, 149, 82, 104, and 114;

(66) a combination of SEQ ID NOs: 121, 155, 137, 87, 142, 188, 127, 183, 196, 84, 189, 154, 110, 143, 145, 135, 71, 152, 91, 159, 2, 82, 182, and 114;

(67) a combination of SEQ ID NOs: 121, 155, 137, 87, 188, 127, 161, 198, 154, 110, 69, 175, 100, 3, 71, 73, 152, 133, 91, 159, 82, 182, 93, and 114;

(68) a combination of SEQ ID NOs: 179, 121, 155, 188, 127, 84, 198, 110, 78, 68, 148, 185, 175, 100, 109, 174, 108, 152, 141, 133, 91, 149, 82, 182, 93, and 114;

(69) a combination of SEQ ID NOs: 155, 137, 87, 142, 127, 196, 198, 189, 38, 110, 68, 175, 100, 109, 145, 174, 71, 152, 133, 91, 159, 2, 82, 77, 182, and 114;

(70) a combination of SEQ ID NOs: 155, 137, 87, 188, 127, 198, 110, 68, 185, 177, 175, 100, 145, 63, 3, 174, 71, 152, 91, 159, 2, 82, 77, 51, and 114;

(71) a combination of SEQ ID NOs: 155, 137, 142, 188, 127, 198, 189, 110, 56, 176, 193, 175, 75, 100, 97, 145, 174, 73, 152, 133, 159, 82, 77, 182, and 114;

(72) a combination of SEQ ID NOs: 179, 121, 155, 137, 87, 188, 127, 196, 198, 154, 110, 193, 175, 25, 100, 109, 178, 48, 3, 152, 133, 91, 82, 182, and 114;

(73) a combination of SEQ ID NOs: 155, 137, 87, 188, 127, 196, 84, 198, 154, 194, 110, 68, 175, 73, 152, 133, 180, 159, 149, 2, 82, 104, 77, 182, and 114;

(74) a combination of SEQ ID NOs: 155, 137, 142, 188, 127, 138, 53, 198, 110, 41, 175, 75, 109, 145, 3, 174, 71, 152, 133, 180, 159, 149, 2, 82, 51, 182, and 114;

(75) a combination of SEQ ID NOs: 179, 155, 137, 87, 188, 127, 161, 198, 189, 110, 4, 100, 109, 145, 178, 10, 3, 54, 174, 133, 39, 91, 159, 149, 82, 51, and 114;

(76) a combination of SEQ ID NOs: 121, 155, 137, 87, 142, 188, 127, 80, 198, 110, 101, 193, 175, 100, 109, 145, 54, 152, 111, 133, 159, 149, 82, 182, 93, and 114;

(77) a combination of SEQ ID NOs: 121, 155, 137, 87, 188, 127, 161, 189, 38, 110, 56, 193, 175, 109, 62, 178, 3, 191, 133, 180, 91, 159, 149, 82, 77, and 114;

(78) a combination of SEQ ID NOs: 121, 155, 142, 188, 127, 5, 196, 161, 198, 154, 110, 26, 175, 100, 109, 145, 63, 10, 54, 152, 133, 159, 2, 82, 104, and 114;

(79) a combination of SEQ ID NOs: 155, 87, 142, 188, 127, 146, 158, 196, 53, 198, 110, 68, 101, 25, 100, 109, 145, 3, 54, 152, 180, 91, 159, 82, 182, 114, and 12;

(80) a combination of SEQ ID NOs: 155, 137, 87, 188, 127, 30, 79, 161, 53, 198, 194, 110, 185, 175, 100, 109, 97, 174, 71, 133, 159, 149, 2, 82, 77, 182, and 114;

(81) a combination of SEQ ID NOs: 121, 155, 137, 142, 188, 127, 161, 198, 187, 194, 110, 148, 26, 177, 175, 109, 145, 178, 10, 71, 152, 133, 91, 159, 149, 82, 182, and 114;

(82) a combination of SEQ ID NOs: 121, 155, 87, 142, 188, 127, 196, 161, 198, 189, 38, 110, 200, 175, 75, 100, 109, 169, 125, 71, 152, 133, 91, 149, 82, 182, and 114;

(83) a combination of SEQ ID NOs: 121, 155, 87, 142, 188, 127, 30, 161, 198, 187, 110, 101, 193, 175, 100, 109, 145, 178, 10, 152, 111, 133, 39, 149, 82, 51, 182, and 114;

(84) a combination of SEQ ID NOs: 155, 142, 188, 127, 53, 198, 154, 110, 176, 69, 193, 192, 100, 109, 145, 48, 10, 152, 133, 39, 91, 159, 149, 82, 77, 182, and 114;

(85) a combination of SEQ ID NOs: 121, 155, 87, 142, 188, 127, 198, 110, 102, 101, 175, 100, 109, 169, 145, 3, 174, 108, 71, 152, 133, 91, 159, 2, 82, 57, 182, and 114;

(86) a combination of SEQ ID NOs: 121, 155, 137, 87, 142, 188, 127, 196, 161, 198, 110, 41, 193, 175, 173, 75, 100, 109, 97, 71, 73, 133, 91, 159, 149, 82, 77, and 114;

(87) a combination of SEQ ID NOs: 155, 137, 142, 188, 127, 196, 198, 189, 110, 78, 185, 175, 100, 109, 145, 3,

174, 73, 152, 141, 133, 91, 159, 149, 82, 51, 182, and 114;

(88) a combination of SEQ ID NOs: 179, 155, 87, 188, 127, 85, 79, 196, 198, 187, 154, 110, 86, 175, 109, 145, 88, 3, 174, 71, 134, 152, 39, 159, 82, 57, and 114;

(89) a combination of SEQ ID NOs: 121, 155, 87, 188, 127, 198, 110, 102, 185, 86, 175, 109, 145, 178, 96, 71, 190, 133, 180, 91, 159, 149, 82, 20, 93, 114, and 12;

(90) a combination of SEQ ID NOs: 179, 155, 137, 188, 127, 5, 198, 154, 110, 68, 200, 148, 86, 175, 109, 145, 71, 152, 190, 180, 39, 159, 149, 2, 82, 150, 182, and 114;

(91) a combination of SEQ ID NOs: 155, 87, 142, 188, 127, 161, 84, 189, 110, 186, 24, 58, 175, 100, 109, 3, 96, 174, 133, 39, 91, 159, 2, 82, 104, 182, 93, and 114;

(92) a combination of SEQ ID NOs: 155, 137, 87, 168, 188, 127, 161, 53, 198, 187, 110, 101, 193, 175, 100, 109, 10, 152, 141, 111, 133, 42, 91, 159, 82, 51, 93, and 114;

(93) a combination of SEQ ID NOs: 155, 87, 188, 127, 85, 161, 84, 198, 154, 110, 68, 86, 193, 175, 109, 3, 191, 73, 152, 180, 39, 91, 159, 149, 82, 77, 182, 93, and 114;

(94) a combination of SEQ ID NOs: 155, 87, 188, 127, 161, 53, 198, 189, 110, 193, 175, 109, 145, 48, 135, 174, 71, 152, 111, 133, 180, 39, 91, 159, 82, 104, 77, 182, and 114;

(95) a combination of SEQ ID NOs: 121, 155, 87, 142, 188, 127, 94, 79, 196, 198, 189, 154, 110, 102, 175, 75, 109, 145, 62, 48, 3, 174, 108, 133, 197, 159, 82, 77, 150, and 114;

(96) a combination of SEQ ID NOs: 179, 155, 142, 188, 127, 79, 196, 161, 53, 198, 189, 154, 110, 148, 69, 100, 109, 145, 54, 174, 92, 152, 180, 113, 91, 159, 149, 2, 82, 77, 57, and 114;

(97) a combination of SEQ ID NOs: 155, 137, 87, 142, 188, 127, 146, 196, 161, 17, 198, 187, 194, 110, 56, 200, 148, 86, 193, 109, 54, 73, 152, 159, 149, 2, 82, 104, 77, 150, 182, and 114; and

(98) a combination of SEQ ID NOs: 179, 155, 137, 87, 142, 188, 127, 172, 84, 53, 154, 38, 110, 86, 153, 193, 175, 75, 100, 109, 145, 125, 156, 3, 54, 133, 180, 39, 91, 159, 149, 2, 82, 77, 93, and 114.

The following shows, as non-limiting examples of combinations of hippocampal atrophy markers (target nucleic acids) to be used in the present invention, combinations of the polynucleotide consisting of the nucleotide sequence shown by SEQ ID NO: 100 and polynucleotides of hippocampal atrophy markers selected from among those shown in Tables 3 to 6. Combinations of hippocampal atrophy markers (target nucleic acids) may also be combinations of miRNAs or human miRNAs corresponding to the following combinations.

(1) a combination of SEQ ID NOs: 155, 87, 188, 127, 110, 68, 185, 100, 145, 152, 180, and 114;

(2) a combination of SEQ ID NOs: 155, 137, 188, 127, 196, 154, 110, 128, 100, 152, 133, 159, and 114;

(3) a combination of SEQ ID NOs: 121, 155, 188, 196, 198, 110, 193, 100, 145,3, 152, 133, 39, 182, and 114;

(4) a combination of SEQ ID NOs: 121, 155, 137, 188, 194, 110, 100, 109, 145, 21, 71, 152, 180, 2, 182, and 114;

(5) a combination of SEQ ID NOs: 155, 188, 127, 196, 154, 110, 193, 100, 109, 145, 62, 3, 54, 152, 133, 180, and 114;

(6) a combination of SEQ ID NOs: 155, 137, 188, 127, 85, 154, 110, 68, 100, 109, 199, 145, 3, 152, 133, and 114;

(7) a combination of SEQ ID NOs: 155, 137, 188, 5, 198, 110, 41, 100, 109, 145, 125, 96, 92, 71, 152, 133, 91, and 114;

(8) a combination of SEQ ID NOs: 155, 142, 188, 127, 5, 154, 110, 175, 25, 100, 109, 54, 133, 180, 91, 159, 2, and 114;

(9) a combination of SEQ ID NOs: 155, 137, 188, 127, 110, 68, 75, 100, 109, 145, 135, 152, 111, 133, 113, 182, and 114;

(10) a combination of SEQ ID NOs: 121, 155, 142, 188, 127, 5, 198, 154, 110, 100, 109, 145, 54, 71, 163, 152, 2, and 114;

(11) a combination of SEQ ID NOs: 155, 137, 142, 188, 127, 85, 5, 154, 110, 128, 193, 175, 100, 145, 152, 133, 51, 182, and 114;

(12) a combination of SEQ ID NOs: 155, 87, 142, 188, 127, 196, 154, 110, 193, 100, 109, 145, 3, 174, 71, 152, 159, and 114;

(13) a combination of SEQ ID NOs: 155, 87, 188, 127, 198, 110, 68, 101, 25, 192, 100, 145, 3, 152, 180, 159, 149, and 114;

(14) a combination of SEQ ID NOs: 155, 188, 127, 189, 110, 68, 86, 193, 192, 100, 135, 3, 71, 152, 133, 180, 159, 77, and 114;

(15) a combination of SEQ ID NOs: 121, 155, 87, 188, 127, 79, 53, 154, 110, 185, 175, 100, 3, 54, 73, 152, 133, 93, and 114;

(16) a combination of SEQ ID NOs: 155, 137, 188, 127, 198, 189, 194, 110, 200, 175, 75, 100, 10, 152, 133, 180, 159, 149, 2, 182, and 114;

(17) a combination of SEQ ID NOs: 121, 155, 137, 188, 127, 194, 110, 148, 100, 109, 145, 125, 88, 135, 3, 152, 91, 159, 149, 77, and 114;

(18) a combination of SEQ ID NOs: 121, 155, 188, 127, 154, 194, 110, 176, 200, 69, 100, 145, 174, 71, 73, 152, 133, 159, 149, 77, 51, and 114;

(19) a combination of SEQ ID NOs: 142, 188, 127, 138, 189, 154, 110, 26, 193, 100, 109, 178, 10, 88, 152, 133, 159, 104, 171, 77, 182, and 114;

(20) a combination of SEQ ID NOs: 155, 137, 188, 127, 146, 183, 196, 198, 110, 200, 193, 25, 100, 109, 152, 133, 180, 91, 159, 149, 77, and 114;

(21) a combination of SEQ ID NOs: 121, 155, 188, 127, 198, 154, 110, 26, 193, 175, 100, 109, 145, 88, 3, 54, 174, 71, 152, 159, 182, and 114;

(22) a combination of SEQ ID NOs: 179, 155, 137, 142, 188, 127, 79, 196, 198, 154, 110, 102, 148, 101, 75, 100, 109, 145, 3, 54, 152, 2, and 114;

(23) a combination of SEQ ID NOs: 155, 137, 87, 188, 127, 85, 162, 196, 198, 110, 68, 175, 75, 100, 145, 3, 54, 71, 152, 133, 159, 55, 114, and 12;

(24) a combination of SEQ ID NOs: 155, 137, 188, 127, 183, 196, 110, 68, 86, 193, 175, 100, 145, 135, 71, 73, 152, 133, 180, 159, 149, 2, 77, 51, and 182;

(25) a combination of SEQ ID NOs: 155, 142, 188, 127, 5, 194, 110, 68, 86, 26, 193, 175, 75, 100, 145, 10, 3, 71, 152, 111, 133, 149, 77, 150, and 114;

(26) a combination of SEQ ID NOs: 121, 155, 87, 142, 188, 127, 138, 196, 161, 198, 189, 110, 56, 185, 101, 100, 10, 73, 152, 133, 180, 149, 70, 51, 182, and 114;

(27) a combination of SEQ ID NOs: 155, 137, 142, 188, 127, 146, 196, 53, 154, 110, 41, 68, 200, 175, 100, 109, 97, 145, 63, 152, 190, 180, 91, 159, 182, and 114;

(28) a combination of SEQ ID NOs: 179, 155, 188, 127, 146, 198, 187, 38, 110, 200, 193, 175, 100, 109, 199, 178, 71, 152, 133, 180, 91, 159, 149, 77, 51, 93, and 114;

(29) a combination of SEQ ID NOs: 155, 137, 142, 188, 127, 53, 189, 110, 41, 200, 175, 25, 75, 31, 100, 125, 156, 54, 71, 106, 152, 133, 180, 159, 2, 77, 182, and 114;

(30) a combination of SEQ ID NOs: 121, 155, 188, 127, 5, 196, 84, 198, 187, 189, 110, 78, 102, 148, 86, 177, 175, 100, 109, 145, 3, 174, 133, 180, 149, 2, 182, and 114; and

(31) a combination of SEQ ID NOs: 179, 121, 155, 137, 87, 142, 188, 127, 198, 189, 110, 148, 192, 100, 109, 145, 135, 174, 152, 133, 180, 113, 91, 159, 149, 77, 18, 182, and 114.

The following shows, as non-limiting examples of combinations of hippocampal atrophy markers (target nucleic acids) to be used in the present invention, combinations of the polynucleotide consisting of the nucleotide sequence shown by SEQ ID NO: 3 and polynucleotides of hippocampal atrophy markers selected from among those shown in Tables 3 to 6. Combinations of hippocampal atrophy markers (target nucleic acids) may also be combinations of miRNAs or human miRNAs corresponding to the following combinations.

(1) a combination of SEQ ID NOs: 110, 3, and 114;

(2) a combination of SEQ ID NOs: 188, 5, 110, 3, and 114;

(3) a combination of SEQ ID NOs: 155, 142, 110, 68, 3, 71, and 114;

(4) a combination of SEQ ID NOs: 155, 188, 127, 110, 145, 3, 152, 180, 159, and 114;

(5) a combination of SEQ ID NOs: 155, 188, 127, 110, 148, 193, 145, 3, 71, 152, 180, 159, 2, and 114;

(6) a combination of SEQ ID NOs: 155, 137, 87, 188, 127, 196, 194, 110, 101, 145, 3, 152, 133, 149, 182, and 114;

(7) a combination of SEQ ID NOs: 155, 142, 188, 127, 5, 196, 154, 110,68, 193, 109, 3, 71, 133, 180, 77, and 114;

(8) a combination of SEQ ID NOs: 155, 137, 142, 188, 127, 85, 30, 5, 196, 110, 193, 145, 135, 3, 152, 2, 182, and 114;

(9) a combination of SEQ ID NOs: 155, 137, 87, 107, 188, 127, 198, 154, 110, 41, 185, 193, 109, 178, 3, 152, 133, 91, 149, 104, 51, 93, and 114;

(10) a combination of SEQ ID NOs: 155, 87, 188, 127, 183, 198, 189, 154, 194, 110, 56, 58, 175, 75, 109, 199, 145, 3, 174, 73, 152, 133, 159, 149, 57, 93, and 114;

(11) a combination of SEQ ID NOs: 121, 155, 87, 142, 188, 79, 53, 198, 154, 110, 185, 101, 175, 75, 109, 169, 125, 3, 174, 92, 71, 152, 141, 133, 91, 149, 104, and 114; and

(12) a combination of SEQ ID NOs: 155, 137, 87, 142, 188, 127, 146, 196, 198, 189, 154, 38, 110, 148, 177, 109, 135, 3, 108, 152, 180, 39, 91, 159, 149, 2, 182, and 114.

Further, the following shows, as non-limiting examples, combinations of the polynucleotide consisting of the nucleotide sequence shown by SEQ ID NO: 5 or a complementary sequence thereof, and polynucleotides selected from among those shown in Tables 3 to 6 or polynucleotides consisting of complementary sequences thereof.

(1) a combination of SEQ ID NOs: 137, 127, 5, 110, 145, 152, and 114;

(2) a combination of SEQ ID NOs: 155, 137, 87, 188, 5, 194, 110, 68, 109, 145, 71, 152, 2, and 114;

(3) a combination of SEQ ID NOs: 155, 137, 188, 127, 5, 110, 68, 145, 71, 73, 152, 159, 149, and 114;

(4) a combination of SEQ ID NOs: 155, 137, 188, 127, 30, 5, 110, 193, 71, 152, 180, 159, 149, 2, 51, 182, and 114;

(5) a combination of SEQ ID NOs: 155, 142, 188, 127, 5, 154, 194, 110, 32, 41, 152, 159, 2, 51, 150, 182, and 114;

(6) a combination of SEQ ID NOs: 155, 188, 127, 5, 84, 198, 110, 185, 75, 109, 145, 71, 152, 180, 159, 2, 51, and 114; and

(7) a combination of SEQ ID NOs: 179, 121, 155, 142, 188, 127, 5, 84, 198, 189, 154, 194, 110, 78, 143, 175, 75, 145, 135, 47, 174, 71, 73, 152, 133, 104, 182, and 114.

Further, the following shows, as non-limiting examples, combinations of the polynucleotide consisting of the nucleotide sequence shown by SEQ ID NO: 77 or a complementary sequence thereof, and polynucleotides selected from among those shown in Tables 3 to 6 or polynucleotides consisting of complementary sequences thereof.

(1) a combination of SEQ ID NOs: 155, 137, 188, 127, 196, 110, 75, 145, 71, 73, 152, 133, 2, 77, and 114;

(2) a combination of SEQ ID NOs: 155, 142, 188, 127, 94, 196, 189, 110, 143, 68, 75, 71, 152, 133, 2, 77, and 114; and

(3) a combination of SEQ ID NOs: 155, 87, 188, 127, 161, 198, 38, 110, 185, 109, 10, 152, 180, 91, 159, 104, 77, 182, and 114.

The following further shows, as non-limiting examples of combinations of hippocampal atrophy markers (target nucleic acids) to be used in the present invention, combinations of the polynucleotide consisting of the nucleotide sequence shown by SEQ ID NO: 2 and polynucleotides of hippocampal atrophy markers selected from among those shown in Tables 3 to 6. A combination of hippocampal atrophy markers (target nucleic acids) may also be a combination of miRNAs or human miRNAs corresponding to the following combination.

(1) a combination of SEQ ID NOs: 155, 188, 127, 5, 198, 189, 110, 176, 143, 102, 148, 101, 75, 109, 199, 145, 54, 174, 106, 152, 133, 2, 20, 51, and 114.

The following shows, as non-limiting examples of combinations of hippocampal atrophy markers (target nucleic acids) to be used in the present invention, combinations of the polynucleotide consisting of the nucleotide sequence shown by SEQ ID NO: 4 and polynucleotides of hippocampal atrophy markers selected from among those shown in Tables 3 to 6. A combination of hippocampal atrophy markers (target nucleic acids) may also be a combination of miRNAs or human miRNAs corresponding to the following combination.

(1) a combination of SEQ ID NOs: 179, 121, 155, 137, 132, 87, 168, 142, 188, 127, 85, 94, 5, 1, 162, 80, 196, 161, 160, 84, 53, 17, 198, 187, 189, 110, 6, 124, 9, 123, 136, 74, 78, 4, 90, 176, 143, 200, 102, 148, 185, 86, 76, 153, 193, 175, 192, 43, 75, 100, 109, 169, 199, 97, 145, 62, 125, 21, 48, 63, 98, 88, 83, 59, 135, 3, 47, 54, 96, 174, 131, 92, 71, 73, 106, 147, 152, 27, 133, 113, 39, 91, 197, 159, 149, 2, 82, 34, 104, 61, 77, 51, 150, 182, and 114.

In one embodiment, the kit or device of the present invention comprises a nucleic acid capable of specifically binding to the above-mentioned target nucleic acid as the hippocampal atrophy marker, and the kit or device preferably comprises a nucleic acid(s) capable of specifically binding to one or more polynucleotides selected from among the polynucleotides of the hippocampal atrophy markers of Group A and Group B as listed above.

The kit or device of the present invention may comprise a reagent, e.g., other known polynucleotides or polynucleotides that would be found in the future that enable detection of hippocampal atrophy, in addition to the nucleic acid(s) capable of specifically binding to the above-described polynucleotide(s) as the hippocampal atrophy marker(s) of the present invention (i.e., the nucleic acid(s) for detection of hippocampal atrophy).

The kit or device of the present invention may comprise a known marker for detection of dementia, such as an amyloid β-protein or tau protein, in addition to the nucleic acid(s) capable of specifically binding to the above-described polynucleotide(s) as the hippocampal atrophy marker(s) of the present invention. In that case, an imaging test, such as MRI, CT, SPECT, PET, or MIBG myocardial scintigraphy, which can visualize such markers, can be used in combination with the kit or device of the present invention.

The kit or device of the present invention may be used in combination with a neuropsychological examination or test such as MMSE.

The nucleic acids for detection of hippocampal atrophy contained in the kit of the present invention may be packaged in different containers either individually or in any combination.

The kit of the present invention may comprise one or more components selected from among a reagent for extracting nucleic acids (e.g., total RNA) from samples such as body fluids, cells, or tissues, a fluorescent material for labeling, an enzyme and a medium for nucleic acid amplification, an instruction manual, etc.

The device of the present invention may be a device for measuring or detecting hippocampal atrophy marker(s), in which the nucleic acid(s) for detection of hippocampal atrophy according to the present invention are, for example, bonded or attached onto a solid phase. Examples of the material for the solid phase may be plastics, paper, glass, silicon, or the like. A preferred material for the solid phase may be a plastic from the viewpoint of easy processability. The solid phase has any shape and is, for example, square, round, reed-shaped, or film-shaped. The device of the present invention may be, for example, a device for measurement by a hybridization technique. Specific examples thereof include blotting devices and nucleic acid arrays (e.g., microarrays, DNA chips, and RNA chips).

Nucleic acid arrays can be produced by bonding or attaching the nucleic acids one by one by onto a solid phase by, for example, a method of spotting the nucleic acids (single-stranded or double-stranded nucleic acid probes) using a high-density dispenser called spotter or arrayer onto the surface of the solid phase (substrate) surface that is treated, if necessary, by coating with L-lysine or the introduction of a functional group such as an amino group or a carboxyl group, a method of spraying the nucleic acids (single-stranded or double-stranded nucleic acid probes) onto a solid phase using an inkjet which injects very small liquid droplets by a piezoelectric element or the like from a nozzle, or a method of sequentially synthesizing nucleotides on a solid phase. Target nucleic acids can be measured via hybridization with the use of the nucleic acid arrays.

The kit or device of the present invention comprises nucleic acid(s) capable of specifically binding to at least one, preferably at least two, more preferably at least three, most preferably at least five to all polynucleotides selected from the above-mentioned hippocampal atrophy marker miRNAs of Group A, or to a complementary strand(s) of the polynucleotide(s). The kit or device of the present invention may optionally further comprise nucleic acids capable of specifically binding to at least one, preferably at least two, more preferably at least three, and most preferably at least five to all polynucleotides selected from the above-mentioned hippocampal atrophy marker miRNAs of Group B, or to a complementary strand(s) of the polynucleotide(s).

The kit or device of the present invention can be favorably used, for example, in the method for detecting hippocampal atrophy described in Section 4 below. Thus, the present invention also provides a kit and a device for use in detection or diagnosis of hippocampal atrophy.

The hippocampal atrophy markers (target nucleic acids) and combinations thereof as described above with regard to the kit or device of the present invention are also applied to the method, use, etc. according to the present invention.

4. Method for detecting hippocampal atrophy

The present invention provides a method for detecting hippocampal atrophy comprising measuring an expression level(s) of a polynucleotide(s) as the hippocampal atrophy marker(s) according to the present invention in a sample from a subject and evaluating whether or not the subj ect's hippocampus has become atrophic by using the measured expression level(s).

The present invention also provides a method for detecting hippocampal atrophy, a method of assisting the detection of hippocampal atrophy, and a method of obtaining an indicator for hippocampal atrophy, of which each comprises measuring an expression level(s) of a polynucleotide(s) as the hippocampal atrophy marker(s) according to the present invention in a sample from a subject and evaluating whether or not the subject's hippocampus has become atrophic or obtaining an indicator for hippocampal atrophy or non-atrophy, by using the measured expression level(s).

The present invention provides a method for diagnosing hippocampal atrophy comprising measuring an expression level(s) of a polynucleotide(s) as the hippocampal atrophy marker(s) according to the present invention in a sample from a subject and evaluating whether or not the subj ect's hippocampus has become atrophic by using the measured expression level(s).

The present invention relates to a method for detecting hippocampal atrophy, comprising measuring an expression level(s) of one or more hippocampal atrophy marker gene(s)/miRNA(s) selected from Group A and optionally the expression level(s) of one or more hippocampal atrophy marker gene(s) or miRNA(s) selected from Group B in a sample, and evaluating (e.g., evaluating *in vitro*) the measured expression level(s) as to whether or not the subject's hippocampus has become atrophic.

The method for detecting hippocampal atrophy according to the present invention may also serve as a method for detecting neuronal cell death in the hippocampus.

Preferably, the method of the present invention enables quantitative and objective diagnosis of hippocampal atrophy less invasively with high sensitivity and specificity, thereby leading to an early medical intervention and suppression of disease progression, and further enabling monitoring of disease exacerbation and monitoring of effectiveness of surgical treatment or medication.

In the method of the present invention, the expression levels of the hippocampal atrophy marker genes or miRNAs can be measured using, for example, nucleic acid(s) (e.g., probe(s) or primer(s)) capable of specifically binding to the polynucleotide(s) as the hippocampal atrophy marker(s) according to the present invention, or the kit or device of the present invention, although the method is not limited thereto.

The kit or device that can be used in the method of the present invention may comprise any one alone or any possible combination of the nucleic acids (e.g., probes or primers) for detection of hippocampal atrophy of the present invention as described above.

The present invention also provides use of the nucleic acid(s) capable of specifically binding to the polynucleotide(s) being hippocampal atrophy marker(s) according to the present invention (the nucleic acid(s) for detection of hippocampal atrophy, e.g., probe(s) or primer(s)) or the kit or device of the present invention, for *in vitro* detection of the target nucleic acid(s) of the hippocampal atrophy marker(s) in a sample from a subject (e.g., miRNA precursor(s) as expression product(s) of miRNA gene(s), or miRNA(s) derived therefrom).

The method of the present invention is useful for diagnosis of an extent of hippocampal atrophy or detection of the presence or absence of hippocampal atrophy. In the present invention, detection of hippocampal atrophy can be performed by detecting *in vitro* the expression level(s) of the hippocampal atrophy marker gene(s)/miRNA(s) in a sample obtained from a subject to be examined for hippocampal atrophy, e.g., a subject suspected of hippocampal atrophy, using the nucleic acid(s) for detection of hippocampal atrophy (e.g., nucleic acid probe(s) or primer(s)), e.g., the nucleic acids for detection of hippocampal atrophy (e.g., nucleic acid probe(s) or primer(s)) in the kit or device of the present invention.

The method for detecting hippocampal atrophy according to the present invention can also be used to evaluate or diagnose the presence or absence of amelioration of the hippocampal atrophy or disease or the degree of amelioration thereof, for example, in a subject with hippocampal atrophy, when a therapeutic drug which is known in the art or on a development stage (including Aricept, GE Aricept, memantine, galantamine, and rivastigmine, and combination drugs thereof, as non-limiting examples) is administered to the subject, for the purpose of treatment or amelioration of hippocampal atrophy or a disease accompanied thereby.

In one embodiment, the method of the present invention may comprise, for example, the following steps (a), (b), and (c):

(a) contacting *in vitro* a sample from a subject with a nucleic acid(s) for detection of hippocampal atrophy in the kit or device of the present invention;
(b) measuring expression level(s) of hippocampal atrophy marker(s) (target nucleic acid(s)) in the sample using the nucleic acid(s) as a nucleic acid probe(s) or primer(s); and
(c) evaluating the presence or absence of hippocampal atrophy (neuronal cell death) in the subject, or obtaining an indicator for the evaluation, on the basis of the measurement results in step (b).

The present invention provides a method for detecting hippocampal atrophy comprising measuring an expression level(s) of one or more polynucleotide(s) (e.g., 1, or 2 or more, preferably 3 or more, 10 or more polynucleotides, and more preferably 3 to 30 polynucleotides, i.e., target nucleic acids) selected from the group consisting of the hippocampal atrophy markers: miR-3131, miR-6757-5p, miR-4706, miR-5001-5p, miR-3180-3p, miR-642b-3p, miR-4655-5p, miR-6819-5p, miR-937-5p, miR-4688, miR-6741-5p, miR-7107-5p, miR-4271, miR-1229-5p, miR-4707-5p, miR-6808-5p, miR-4656, miR-6076, miR-6762-5p, miR-7109-5p, miR-6732-5p, miR-3195, miR-7150, miR-642a-3p, miR-1249-5p, miR-3185, miR-4689, miR-3141, miR-6840-3p, miR-3135b, miR-1914-3p, miR-4446-3p, miR-4433b-3p, miR-6877-5p, miR-6848-5p, miR-3620-5p, miR-6825-5p, miR-5739, miR-3663-3p, miR-4695-5p, miR-3162-5p, miR-3679-5p, miR-8059, miR-7110-5p, miR-1275, miR-6779-5p, miR-197-5p, miR-6845-5p, miR-4327, miR-4723-5p, miR-4530, miR-6771-5p, miR-614, miR-92a-2-5p, miR-6891-5p, miR-6124, miR-4687-3p, miR-4442, miR-7977, miR-6785-5p, miR-4497, miR-8071, miR-663b, miR-3180, miR-4251, miR-1285-3p, miR-6870-5p, miR-4484, miR-4476, miR-6749-5p, miR-4454, miR-6893-5p, miR-6085, miR-4787-5p, miR-149-3p, miR-7704, miR-6125, miR-6090, miR-3197, miR-6850-5p, miR-4467, miR-6885-5p, miR-6803-5p, miR-6798-5p, miR-6780b-5p, miR-6768-5p, miR-5100, miR-6724-5p, miR-6879-5p, miR-7108-5p, miR-4649-5p, miR-4739, miR-6089, miR-1908-5p, miR-4516, miR-2861, miR-4492, miR-4294, miR-6791-5p, miR-1469, miR-6752-5p, miR-4730, miR-6126, miR-6869-5p, miR-1268a, miR-6799-5p, miR-8069, miR-3621, and miR-4763-3p, in a sample from a subject, and evaluating whether or not the subject's hippocampus has become atrophic by using the measured expression level(s).

In a preferred embodiment, the method of the present invention may comprise measuring an expression level(s) of a target nucleic acid(s) in a sample from a subject using a nucleic acid(s) (a nucleic acid(s) for detection of hippocampal atrophy) capable of specifically binding to one or more polynucleotides (target nucleotides), for example, 1, or preferably 2 or more, 3 or more, 10 or more polynucleotides, and more preferably 3 to 30 polynucleotides, selected from the group consisting of: miR-3131, miR-6757-5p, miR-4706, miR-5001-5p, miR-3180-3p, miR-642b-3p, miR-4655-5p, miR-6819-5p, miR-937-5p, miR-4688, miR-6741-5p, miR-7107-5p, miR-4271, miR-1229-5p, miR-4707-5p, miR-6808-5p, miR-4656, miR-6076, miR-6762-5p, miR-7109-5p, miR-6732-5p, miR-3195, miR-7150, miR-642a-3p, miR-1249-5p, miR-3185, miR-4689, miR-3141, miR-6840-3p, miR-3135b, miR-1914-3p, miR-4446-3p, miR-4433b-3p, miR-6877-5p, miR-6848-5p, miR-3620-5p, miR-6825-5p, miR-5739, miR-3663-3p, miR-4695-5p, miR-3162-5p, miR-3679-5p, miR-8059, miR-7110-5p, miR-1275, miR-6779-5p, miR-197-5p, miR-6845-5p, miR-4327, miR-4723-5p, miR-4530, miR-6771-5p, miR-614, miR-92a-2-5p, miR-6891-5p, miR-6124, miR-4687-3p, miR-4442, miR-7977, miR-6785-5p, miR-4497, miR-8071, miR-663b, miR-3180, miR-4251, miR-1285-3p, miR-6870-5p, miR-4484, miR-4476, miR-6749-5p, miR-4454, miR-6893-5p, miR-6085, miR-4787-5p, miR-149-3p, miR-7704, miR-6125, miR-6090, miR-3197, miR-6850-5p, miR-4467, miR-6885-5p, miR-6803-5p, miR-6798-5p, miR-6780b-5p, miR-6768-5p, miR-5100, miR-6724-5p, miR-6879-5p, miR-7108-5p, miR-4649-5p, miR-4739, miR-6089, miR-1908-5p, miR-4516, miR-2861, miR-4492, miR-4294, miR-6791-5p, miR-1469, miR-6752-5p, miR-4730, miR-6126, miR-6869-5p, miR-1268a, miR-6799-5p, miR-8069, miR-3621, and miR-4763-3p, or to a complementary strand(s) of the polynucleotide(s).

In a preferred embodiment of the method of the present invention, miR-3131 may be hsa-miR-3131, miR-6757-5p may be hsa-miR-6757-5p, miR-4706 may be hsa-miR-4706, miR-5001-5p may be hsa-miR-5001-5p, miR-3180-3p may be hsa-miR-3180-3p, miR-642b-3p may be hsa-miR-642b-3p, miR-4655-5p may be hsa-miR-4655-5p, miR-6819-5p may be hsa-miR-6819-5p, miR-937-5p may be hsa-miR-937-5p, miR-4688 may be hsa-miR-4688, miR-6741-5p may be hsa-miR-6741-5p, miR-7107-5p may be hsa-miR-7107-5p, miR-4271 may be hsa-miR-4271, miR-1229-5p may be hsa-miR-1229-5p, miR-4707-5p may be hsa-miR-4707-5p, miR-6808-5p may be hsa-miR-6808-5p, miR-4656 may be hsa-miR-4656, miR-6076 may be hsa-miR-6076, miR-6762-5p may be hsa-miR-6762-5p, miR-7109-5p may be hsa-miR-7109-5p, miR-6732-5p may be hsa-miR-6732-5p, miR-3195 may be hsa-miR-3195, miR-7150 may be hsa-miR-7150, miR-642a-3p may be hsa-miR-642a-3p, miR-1249-5p may be hsa-miR-1249-5p, miR-3185 may be hsa-miR-3185, miR-4689 may be hsa-miR-4689, miR-3141 may be hsa-miR-3141, miR-6840-3p may be hsa-miR-6840-3p, miR-3135b may be hsa-miR-3135b, miR-1914-3p may be hsa-miR-1914-3p, miR-4446-3p may be hsa-miR-4446-3p, miR-4433b-3p may be hsa-miR-4433b-3p, miR-6877-5p may be hsa-miR-6877-5p, miR-6848-5p may be hsa-miR-6848-5p, miR-3620-5p may be hsa-miR-3620-5p, miR-6825-5p may be hsa-miR-6825-5p, miR-5739 may be hsa-miR-5739, miR-3663-3p may be hsa-miR-3663-3p, miR-4695-5p may be hsa-miR-4695-5p, miR-3162-5p may be hsa-miR-3162-5p, miR-3679-5p may be hsa-miR-3679-5p, miR-8059 may be hsa-miR-8059, miR-7110-5p may be hsa-miR-7110-5p, miR-1275 may be hsa-miR-1275, miR-6779-5p may be hsa-miR-6779-5p, miR-197-5p may be hsa-miR-197-5p, miR-6845-5p may be hsa-miR-6845-5p, miR-4327 may be hsa-miR-4327, miR-4723-5p may be hsa-miR-4723-5p, miR-4530 may be hsa-miR-4530, miR-6771-5p may be hsa-miR-6771-5p, miR-614 may be hsa-miR-614, miR-92a-2-5p may be hsa-miR-92a-2-5p, miR-6891-5p may be hsa-miR-6891-5p, miR-6124 may be hsa-miR-6124, miR-4687-3p may be hsa-miR-4687-3p, miR-4442 may be hsa-miR-4442, miR-7977 may be hsa-miR-7977, miR-6785-5p may be hsa-miR-6785-5p, miR-4497 may be hsa-miR-4497, miR-8071 may be hsa-miR-8071, miR-663b may be hsa-miR-663b, miR-3180 may be hsa-miR-3180, miR-4251 may be hsa-miR-4251, miR-1285-3p may be hsa-miR-1285-3p, miR-6870-5p may be hsa-miR-6870-5p, miR-4484 may be hsa-miR-4484, miR-4476 may be hsa-miR-4476, miR-6749-5p may be hsa-miR-6749-5p, miR-4454 may be hsa-miR-4454, miR-6893-5p may be hsa-miR-6893-5p, miR-6085 may be hsa-miR-6085, miR-4787-5p may be hsa-miR-4787-5p, miR-149-3p may be hsa-miR-149-3p, miR-7704 may be hsa-miR-7704, miR-6125 may be hsa-miR-6125, miR-6090 may be hsa-miR-6090, miR-3197 may be hsa-miR-3197, miR-6850-5p may be hsa-miR-6850-5p, miR-4467 may be hsa-miR-4467, miR-6885-5p may be hsa-miR-6885-5p, miR-6803-5p may be hsa-miR-6803-5p, miR-6798-5p may be hsa-miR-6798-5p, miR-6780b-5p may be hsa-miR-6780b-5p, miR-6768-5p may be hsa-miR-6768-5p, miR-5100 may be hsa-miR-5100, miR-6724-5p may be hsa-miR-6724-5p, miR-6879-5p may be hsa-miR-6879-5p, miR-7108-5p may be hsa-miR-7108-5p, miR-4649-5p may be hsa-miR-4649-5p, miR-4739 may be hsa-miR-4739, miR-6089 may be hsa-miR-6089, miR-1908-5p may be hsa-miR-1908-5p, miR-4516 may be hsa-miR-4516, miR-2861 may be hsa-miR-2861, miR-4492 may be hsa-miR-4492, miR-4294 may be hsa-miR-4294, miR-6791-5p may be hsa-miR-6791-5p, miR-1469 may be hsa-miR-1469, miR-6752-5p may be hsa-miR-6752-5p, miR-4730 may be hsa-miR-4730, miR-6126 may be hsa-miR-6126, miR-6869-5p may be hsa-miR-6869-5p, miR-1268a may be hsa-miR-1268a, miR-6799-5p may be hsa-miR-6799-5p, miR-8069 may be hsa-miR-8069, miR-3621 may be hsa-miR-3621, and miR-4763-3p may be hsa-miR-4763-3p.

In a preferred embodiment of the method of the present invention, the nucleic acid(s) for detection of hippocampal atrophy (e.g., probe(s) or primer(s)) used for measurement of expression level(s) may be a polynucleotide(s) selected from the group consisting of the following polynucleotides (a) to (e):

(a) a polynucleotide consisting of a nucleotide sequence shown by any of SEQ ID NOs: 1 to 109 or a nucleotide sequence derived from the nucleotide sequence by the replacement of u with t, a variant thereof, a derivative thereof, or a fragment thereof comprising 15 or more consecutive nucleotides;

(b) a polynucleotide comprising a nucleotide sequence shown by any of SEQ ID NOs: 1 to 109, a variant thereof, a derivative thereof, or a fragment thereof comprising 15 or more consecutive nucleotides;

(c) a polynucleotide consisting of a nucleotide sequence complementary to a nucleotide sequence shown by any of SEQ ID NOs: 1 to 109 or a nucleotide sequence derived from the nucleotide sequence by the replacement of u with t, a variant thereof, a derivative thereof, or a fragment thereof comprising 15 or more consecutive nucleotides;

(d) a polynucleotide comprising a nucleotide sequence complementary to a nucleotide sequence shown by any of SEQ ID NOs: 1 to 109 or a nucleotide sequence derived from the nucleotide sequence by the replacement of u with t, a variant thereof, a derivative thereof, or a fragment thereof comprising 15 or more consecutive nucleotides; and

(e) a polynucleotide hybridizing under stringent conditions to any of the polynucleotides (a) to (d).

In one embodiment, the nucleic acid(s) for detection of hippocampal atrophy (e.g., probe(s) or primer(s)) used for measurement of expression levels in the method of the present invention may further comprise a nucleic acid(s) capable of specifically binding to one or more polynucleotide(s) selected from the group consisting of: miR-1228-5p, miR-760, miR-187-5p, miR-7111-5p, miR-6088, miR-6805-3p, miR-4640-5p, miR-6721-5p, miR-6880-5p, miR-711, miR-128-1-5p, miR-4525, miR-486-3p, miR-6756-5p, miR-1260b, miR-3184-5p, miR-6075, miR-204-3p, miR-4728-5p, miR-4534, miR-4758-5p, miR-8063, miR-6836-3p, miR-6789-5p, miR-744-5p, miR-1909-3p, miR-887-3p, miR-4745-5p, miR-4433a-3p, miR-5090, miR-296-5p, miR-939-5p, miR-3648, miR-3196, miR-6722-3p, miR-6805-5p, miR-1202, miR-6775-5p, miR-6087, miR-6765-5p, miR-6875-5p, miR-4674, miR-1233-5p, miR-7114-5p, miR-5787, miR-8072, miR-3619-3p, miR-4632-5p, miR-6800-5p, miR-4634, miR-4486, miR-6727-5p, miR-4505, miR-4725-3p, miR-1538, miR-320b, miR-1915-5p, miR-328-5p, miR-6820-5p, miR-6726-5p, miR-3665, miR-638, miR-762, miR-4466, miR-3940-5p, miR-1237-5p, miR-575, miR-3656, miR-4488, miR-4281, miR-6781-5p, miR-4532, miR-4665-5p, miR-6816-5p, miR-4508, miR-6784-5p, miR-6786-5p, miR-4741, miR-1343-5p, miR-1227-5p, miR-4734, miR-3960, miR-128-2-5p, miR-6743-5p, miR-663a, miR-6729-5p, miR-1915-3p, miR-1268b, miR-4651, miR-3178, and miR-4463, or to a complementary strand(s) of the polynucleotide(s).

In one embodiment, the additional nucleic acid(s) for detection of hippocampal atrophy may be a polynucleotide(s) selected from the group consisting of the following polynucleotides (f) to (j):

(f) a polynucleotide consisting of a nucleotide sequence shown by any of SEQ ID NOs: 110 to 200 or a nucleotide sequence derived from the nucleotide sequence by the replacement of u with t, a variant thereof, a derivative thereof, or a fragment thereof comprising 15 or more consecutive nucleotides;
(g) a polynucleotide comprising a nucleotide sequence shown by any of SEQ ID NOs: 110 to 200, a variant thereof, a derivative thereof, or a fragment thereof comprising 15 or more consecutive nucleotides;
(h) a polynucleotide consisting of a nucleotide sequence complementary to a nucleotide sequence shown by any of SEQ ID NOs: 110 to 200 or a nucleotide sequence derived from the nucleotide sequence by the replacement of u with t, a variant thereof, a derivative thereof, or a fragment thereof comprising 15 or more consecutive nucleotides;
(i) a polynucleotide comprising a nucleotide sequence complementary to a nucleotide sequence shown by any of SEQ ID NOs: 110 to 200 or a nucleotide sequence derived from the nucleotide sequence by the replacement of u with t, a variant thereof, a derivative thereof, or a fragment thereof comprising 15 or more consecutive nucleotides; and
(j) a polynucleotide hybridizing under stringent conditions to any of the polynucleotides (f) to (i).

The sample used in the method of the present invention may be any sample from the subject. Examples of the sample include living tissues (preferably brain tissues or nerve tissues), body fluids, or cells obtained from the subject, or samples prepared therefrom. Examples of the samples include an RNA-containing sample prepared from the living tissues, a polynucleotide-containing sample further prepared therefrom; a body fluid such as blood, serum, plasma, or urine, a portion or the whole of a living tissue collected from the subject by biopsy or the like, or a living tissue excised by surgery. A sample to be subjected to measurement can be prepared from the samples indicated above with a conventional technique. Total RNA can be prepared from the samples, or various polynucleotides, such as cDNA, can be prepared from RNA, such as total RNA, and they may be subjected to measurement of the expression levels.

In the method of the present invention, the hippocampal atrophy marker genes/miRNAs may be extracted from the samples. In the present invention, it is particularly preferred that hippocampal atrophy marker genes/miRNAs be extracted from the samples, such as blood, serum, or plasma samples, with the use of an RNA extraction reagent in the 3D-Gene(R) RNA extraction reagent from liquid sample kit (Toray Industries, Inc.). Alternatively, a common acidic phenol method (acid guanidinium-phenol-chloroform (AGPC) method) may be adopted, an RNA extraction reagent including acidic phenol, such as Trizol(R) (Life Technologies Corp.) or Isogen (Nippon Gene Co., Ltd., Japan), may be used or a kit such as miRNeasy(R) Mini Kit (Qiagen N.V.) may be used, for the extraction. It should be noted that means for extraction is not limited thereto.

The subject to which the method of the present invention is applied is a mammal as defined above, and may be, for example, but not limited to, a human, monkey, mouse, rat or the like, and preferably a human.

The steps of the method of the present invention can be changed depending on the type of the polynucleotide(s) to be measured.

When an analyte polynucleotide is RNA, the method for detecting hippocampal atrophy (neuronal cell death) may comprise, for example, the following steps (a), (b), and (c):

(a) contacting RNA prepared from a sample from a subject (wherein, for example, for quantitative RT-PCR in step (b), the 3' end of the RNA may be polyadenylated, or any sequence may be added to one or both ends of the RNA by ligation) or a polynucleotide consisting of a complementary sequence thereof synthesized from the RNA via reverse transcription (cDNA) with and binding to the nucleic acid(s) for detection of hippocampal atrophy, e.g., in the kit or device of the present invention;

(b) measuring (e.g., quantifying) the sample-derived RNA or the cDNA synthesized from the RNA, which has been bound to the above nucleic acid(s), via hybridization using the nucleic acid(s) as a nucleic acid probe(s) or by nucleic acid amplification, e.g., quantitative RT-PCR, using the nucleic acid(s) as primer(s); and

(c) evaluating the presence or absence of hippocampal atrophy (or marker gene(s)/miRNA(s) for hippocampal atrophy/neuronal cell death) on the basis of the measurement results of step (b).

For example, various hybridization techniques can be used for measuring the expression levels in the present invention. Examples of hybridization techniques include, but are not limited to, Northern blot (Northern hybridization), Southern blot (Southern hybridization), nucleic acid array methods such as DNA chip analysis, and *in situ* hybridization. PCR such as quantitative RT-PCR, nucleic acid amplification techniques such as LAMP, or next-generation sequencing can be used in combination with the hybridization technique or as an alternative thereto.

When using Northern blot, for example, the nucleic acid probe(s) that can be used in the present invention may be used to detect or measure the presence or absence of the expression of the hippocampal atrophy marker miRNA(s) or gene(s) corresponding to the nucleic acid probe(s), or the expression level(s) thereof. A specific example of such method may comprise labeling the nucleic acid probe(s) according to the present invention (e.g., a complementary strand(s) to the hippocampal atrophy marker miRNA(s)) with a label such as a radioisotope ($^{32}$P, $^{33}$P $^{35}$S, etc.) or a fluorescent material, hybridizing the labeled probe(s) with a sample-derived RNA from a subject, which is transferred to a membrane such as a nylon membrane according to a conventional method, and then detecting and measuring a signal from the label (e.g., a radioisotope or fluorescent material) on the formed DNA/RNA duplex using a detector such as a radiation detector (e.g., BAS-1800 II (FUJIFII,M Corp.)) or a fluorescence detector (e.g., STORM 865 (GE Healthcare Japan Corp.)).

When using quantitative RT-PCR, for example, the primers that can be used in the present invention may be used to detect or measure the presence or absence of the expression of the hippocampal atrophy marker miRNA(s) or gene(s) corresponding to the primer(s), or the expression level(s) thereof. A specific example of such method may comprise recovering a sample-derived RNA from a subject, polyadenylating the 3'-end, preparing cDNA from the polyadenylated RNA according to a conventional method, hybridizing a primer pair comprising the primer(s) of the present invention (preferably, of which one primer consists of a plus strand sequence and another primer consists of a reverse strand sequence and each of them specifically binds to the cDNA), which may be contained in the kit or device for detection of the present invention, with the cDNA as a template and performing PCR according to a conventional method, and then detecting the resulting single-stranded or double-stranded DNA. Examples of the techniques for detecting single-stranded or double-stranded DNA can include a method by performing the above-mentioned PCR using primers labeled in advance with a radioisotope, a fluorescent material or the like and detecting a labeling signal, a method for detection by electrophoresing the PCR product on an agarose gel and staining the double-stranded DNA with ethidium bromide or the like, and a method by transferring the resulting single-stranded or double-stranded DNA to a membrane such as a nylon membrane according to a conventional method and hybridizing the single-stranded or double-stranded DNA with a nucleic acid probe for detection (Northern blot method).

The nucleic acid array methods use, for example, an nucleic acid array in which the nucleic acid(s) for detection of hippocampal atrophy of the present invention, such as nucleic acid probe(s) (single-stranded or double-stranded probe(s)) in the kit or device of the present invention are immobilized on a solid phase (substrate). Regions of the array on which the nucleic acid probe(s) are immobilized are referred to as "probe spots", and regions on which no nucleic acid probe(s) are immobilized are referred to as "blank spots". An array in which a set of nucleic acids are immobilized on a solid-phase (substrate) is generally called a nucleic acid chip (a DNA chip or an RNA chip), a nucleic acid array (a DNA array or an RNA array), a microarray (a DNA microarray or an RNA microarray), or the like. In the context of the present invention, the term "nucleic acid array" includes all of such chips and arrays. The DNA or RNA array includes a DNA or RNA macroarray and a DNA or RNA microarray. The nucleic acid array methods can use, for example, but not limited to, 3D-Gene$^{(R)}$ Human miRNA Oligo chip (Toray Industries, Inc., Japan) as a DNA chip.

Examples of the measurement using a DNA chip include, but are not limited to, a method of detecting and measuring a signalfrom the labeled nucleic acids for detection of hippocampal atrophy using an image detector (e.g., Typhoon 9410 (GE Healthcare) or a 3D-Gene$^{(R)}$ scanner (Toray Industries, Inc., Japan)).

The "stringent conditions" used herein, are conditions under which a nucleic acid of interest (e.g., the nucleic acid for detection of hippocampal atrophy of the present invention) hybridizes to its target nucleic acid at a detectable higher extent (e.g., at a measurement value equal to or larger than "(a mean of background measurement values) + (a standard error of the background measurement values) x 2)") than that to other nucleic acids.

The stringent conditions are defined by reaction conditions of hybridization and subsequent washing. The hybridization conditions may be, for example, but not limited to, conditions in which the reaction is carried out at 30°C to 60°C for 1 to 24 hours in a solution containing SSC, a surfactant, formamide, dextran sulfate, a blocking agent, etc. In this context, 1 x SSC is an aqueous solution (pH 7.0) containing 150 mM sodium chloride and 15 mM sodium citrate. The surfactant includes, for example, SDS (sodium dodecyl sulfate), Triton, or Tween. The hybridization conditions more preferably comprise 3-10 x SSC and 0.1-1% SDS. Examples of the conditions for the washing, following the hybridization, which also define the stringent conditions, include conditions comprising sequential washings at 30°C with a solution containing 0.5 x SSC and 0.1% SDS, at 30°C with a solution containing 0.2 x SSC and 0.1% SDS, and at 30°C with a 0.05 x SSC solution. It is preferred that the complementary polynucleotide maintain its hybridized state with a target plus strand even when washed under such conditions. Specifically, examples of such a complementary polynucleotide include a polynucleotide consisting of a nucleotide sequence in a completely complementary relationship with the nucleotide sequence of the target plus strand, and a polynucleotide consisting of a nucleotide sequence having at least 80%, preferably at least 85%, more preferably at least 90%, or at least 95% homology to the former polynucleotide.

Other examples of the "stringent conditions" for the hybridization are described in, for example, Sambrook, J. & Russel, D., Molecular Cloning, A LABORATORY MANUAL, Cold Spring Harbor Laboratory Press, published on January 15, 2001, Vol. 1, 7.42 to 7.45 and Vol. 2, 8.9 to 8.17, and can be used in the present invention.

Any nucleic acid amplification techniques such as PCR and LAMP can be used herein. A specific example may be a method of nucleic acid amplification using TaqMan[R] MicroRNA Assays (Life Technologies), miScript PCR System (Qiagen), LAMP primers, or the like, although the method is not limited thereto.

Examples of the conditions for carrying out PCR using primers being the nucleic acids for detection of hippocampal atrophy of the present invention include those involving treatment for approximately 15 seconds to 1 minute at 5 to 10°C plus a Tm value calculated from the nucleotide sequences of the primers, using a PCR buffer having composition such as a composition of 10 mM Tris-HCL (pH 8.3), 50 mM KCl, and 1 to 2 mM $MgCl_2$. The calculation of the Tm value can be carried out, for example, with the following: Tm value = 2 x (the number of adenine residues + the number of thymine residues) + 4 x (the number of guanine residues + the number of cytosine residues).

When using quantitative RT-PCR, a commercially available kit for measurement specially designed for quantitatively measuring miRNA, such as TaqMan[R] MicroRNA Assays (Life Technologies Corp.), LNA[R]-based MicroRNA PCR (Exiqon), or Ncode[R] miRNA qRT-PCT kit (Invitrogen Corp.), may be used.

In the method of the present invention, the measurement of the expression levels may be performed with a sequencer, in addition to hybridization methods as described above or as an alternative thereto. As a sequencer, any of DNA sequencers of the first generation based on Sanger method, the second generation with shorter read size, and the third generation with longer read size can be used. Herein, these sequencers of the second generation and the third generation are collectively referred to as "next-generation sequencers". For example, Miseq, Hiseq, or NexSeq (Illumina, Inc.); Ion Proton, Ion PGM, or Ion S5/S5 XL (Thermo Fisher Scientific Inc.); PacBio RS II or Sequel (Pacific Biosciences of California, Inc.); or MinION (Oxford Nanopore Technologies Ltd.) or the like in the case of using e.g., a Nanopore sequencer; may be used, together with a commercially available measurement kit specifically designed for measuring miRNA.

Next-generation sequencing is a technique of determining nucleotide sequence using a next-generation sequencer, and characterized by being capable of simultaneously performing a huge number of sequencing reactions over Sanger method (see, e.g., Rick Kamps et al., Int. J. Mol. Sci., 2017, 18(2), p.308 and Int. Neurourol. J., 2016, 20 (Suppl.2), S76-83). A non-limiting example of next-generation sequencing for miRNA comprises a step of adding adaptor sequences having given nucleotide sequences to the both ends of miRNA from a sample or cDNA thereof and a step of performing reverse transcription of total RNA (whole RNA) from the sample to cDNA before or after the adding the adaptor sequences. After the reverse transcription, cDNA from target miRNA(s) of interest may be amplified by a nucleic acid amplification method such as PCR or enriched with a probe or the like before the step of sequencing. While the details of the subsequent step of sequencing may vary depending on the type of a next-generation sequencer, typically, cDNA is linked to a substrate via an adaptor sequence, a sequencing reaction is performed using the adaptor sequence as a priming site of a sequencing primer, thereby determining and analysing nucleotide sequences. See details of the sequencing reaction, etc., for example, in Rick Kamps et al. (supra). Finally, the data are outputted. This provides a collection of sequence information (reads) obtained by the sequencing reactions and analysis data thereof. For example, next-generation sequencing can identify a target miRNA based on the resulting sequence information, and determine the expression level thereof based on the number of reads having the nucleotide sequence of the target miRNA.

Examples of the method of determining expression levels (e.g., miRNA or gene expression levels) in the presemt invention include, but not limited to, methods involving statistical treatment as described in, for example, "Statistical Analysis of Gene Expression Microarray Data" (Speed T., Chapman and Hall/CRC), and "A Beginner's guide Microarray Gene Expression data Analysis" (Causton H. C. et al., Blackwell publishing). For example, twice, preferably 3 times, and more preferably 6 times the standard deviation of the measurement values of the blank spots are added to the average measurement value of the blank spots on the nucleic acid array, such as a DNA chip, and probe spots having a signal value equal to or higher than the resulting value can be regarded as detected spots. Further, the average measurement value of the blank spots is regarded as a background and can be subtracted from the measurement values of the probe spots to determine the expression levels. A missing value for an expression level can be excluded from the data to be analyzed, or preferably replaced with the smallest value of the expression levels obtained on the individual nucleic acid array, such as DNA chip, or more preferably replaced with a value obtained by subtracting 0.1 from a logarithmic value of the smallest value of the expression levels. In order to exclude low-signal genes/miRNAs, only genes/miRNAs that have exhibited the expression levels of $2^6$ or higher, preferably $2^8$ or higher, and more preferably $2^{10}$ or higher in 20% or more, preferably 50% or more, and more preferably 80% or more of the number of measurement samples may be selected for analysis. Examples of the normalization of the expression levels include, but are not limited to, global normalization and quantile normalization (such as methods described in Bolstad, B.M. et al., 2003, Bioinformatics, Vol. 19, pp. 185-193).

In one embodiment, the method of the present invention can be a method for detecting hippocampal atrophy comprising evaluating whether or not a subject's hippocampus has become atrophic using the expression levels measured as described above and control expression levels as measured in the same manner of subjects with no hippocampal atrophy. The method for detecting hippocampal atrophy comprises measuring the gene/miRNA expression level of the hippocampal atrophy marker (target nucleic acid) of interest of the present invention in the samples (e.g., blood, serum, or plasma samples) obtained from, for example, subjects to be tested for hippocampal atrophy, such as subjects suspected of hippocampal atrophy, and subjects with no hippocampal atrophy (control), and comparing the measued expression levels therebetween. In the method, if there is a difference (e.g., a statistically significant difference) between the expression levels in the subjects to be tested for hippocampal atrophy, for example, subjects suspected of hippocampal atrophy, and the control expression levels in the subjects with no hippocampal atrophy (control), it indicates hippocampal atrophy in the subjects tested. In that case, the subjects' hippocampus can be evaluated to have become atrophic.

In the present invention, the subjects to be tested for hippocampal atrophy, such as subjects suspected of hippocampal atrophy, and the subjects with no hippocampal atrophy (control) to be compared to each other are preferably of the same organism species.

Herein, the "evaluating" whether or not a hippocampus has become atrophic may be an evaluation support based on results of *in vitro* examination, not physician's decision.

In one embodiment of the method for detecting hippocampal atrophy of the present invention, if the expression level(s) of polynucleotide(s) consisting of nucleotide sequences shown by one or more selected from among SEQ ID NOs: 1 to 109 and optionally nucleotide sequences shown by one or more selected from among SEQ ID NOs: 110 to 200 in a sample such as blood, serum, plasma, or urine of a subject to be tested for hippocampal atrophy, such as a subject suspected of hippocampal atrophy, are higher or lower (preferably statistically significantly higher or lower) than expression level(s) thereof in a sample such as blood, serum, or plasma, or urine of a subject with no hippocampal atrophy (see, for example, the difference (fold change) of the hippocampal non-atrophy group relative to the hippocampal atrophy group shown in Tables 3 to 6), the subject to be tested for hippocampal atrophy, such the subject suspected of hippocampal atrophy, can be evaluated to have hippocampal atrophy.

In one embodiment, provided is a method for detecting hippocampal atrophy in a subject, comprising measuring the expression level(s) of the hippocampal atrophy marker(s) in a sample from the subject, and assigning the measured expression level(s) to a discriminant (discriminant function) capable of distinctively discriminating between atrophy and non-atrophy of hippocampus, which is prepared using expression level(s) of the marker(s) in samples from subjects (or patients) known to have hippocampal atrophy and expression level(s) of the marker(s) in samples from subjects known to have no hippocampal atrophy as training samples, and thereby evaluating whether there is atrophy or non-atrophy of hippocampus (whether or not the subject's hippocampus has become atrophic), or obtaining an indicator for hippocampal atrophy or non-atrophy. The indicator for hippocampal atrophy or non-atrophy may be a discriminant score correlated with an extent of hippocampal atrophy (e.g., an extent based on the volume ratio (%) of the hippocampus to the whole brain). A discriminant (discriminant function) can be generated by selecting a marker candidate based on, for example, but not limited to, LASSO method, difference (fold change), or a statistically significant difference such as a p value, and constructing a discriminant using logistic regression analysis or Fisher's discriminant analysis for the marker candidate, although the method is not limited thereto.

In one embodiment, the present invention provides a method for determining an extent of hippocampal atrophy in a subject, comprising measuring the expression level(s) of the hippocampal atrophy marker(s) in a sample from the subject, and assigning the measured expression level(s) to a discriminant (discriminant function) capable of distinctively discriminating between atrophy and non-atrophy of hippocampus, which is prepared using expression level(s) of the marker(s) in samples from subjects (or patients) known to have hippocampal atrophy (e.g., by the icobrain measurement) and samples from subjects known to have no hippocampal atrophy as training samples, and thereby evaluating the extent of hippocampal atrophy. The present invention also provides a method for supporting determination of an extent of hippocampal atrophy in a subject or for obtaining an indicator for an extent of hippocampal atrophy in a subject, comprising measuring the expression level(s) of the hippocampal atrophy marker(s) in a sample from the subject, and assigning the measured expression level(s) to a discriminant (discriminant function) capable of distinctively discriminating between atrophy and non-atrophy of hippocampus, which is prepared using expression level(s) of the marker(s) in samples from subjects (or patients) known to have hippocampal atrophy and expression level(s) of the markers in samples from subjects known to have no hippocampal atrophy as a training sample, and thereby obtaining the indicator for the extent of hippocampal atrophy. The indicator for an extent of hippocampal atrophy may be the volume ratio (%) of the hippocampus to the whole brain.

In one embodiment, the present invention provides a method for estimating a hippocampal volume of a subject comprising measuring the expression level(s) of the hippocampal atrophy marker(s) in a sample from the subject, and assigning the measured expression level(s) to a regression equation capable of estimating hippocampal volume, which is prepared using expression level(s) of the markers insamples from subjects (or patients) known to have hippocampal atrophy and expression level(s) of the markers in samples from subjects known to have no hippocampal atrophy as training samples, and thereby evaluating the hippocampal volume. A regression equation can be prepared by, for example, but not limited to, LASSO regression analysis or regression analysis using partial least squares.

The method of the present invention may comprise: a step (1st step) of measuring *in vitro* the expression level(s) of the hippocampal atrophy marker(s) in samples from subjects known to have hippocampal atrophy and in samples from subjects known to have no hippocampal atrophy; a step (2nd step) of preparing a discriminant using the measured expression level(s) of the hippocampal atrophy marker(s) obtained in the 1st step as training samples; a step (3rd step) of measuring *in vitro* the expression level(s) of the hippocampal atrophy marker(s) in a sample from a subject in the same manner as in the 1st step; and a step (4th step) of assigning the measured expression level(s) of the hippocampal atrophy marker(s) obtained in the 3rd step to the discriminant prepared in the 2nd step, thereby evaluating whether or not the hippocampus has become atrophic in the subject subjected to the measurement of the expression level(s) in the 3rd step, on the basis of results of the assigning to the discriminant.

The discriminant capable of distinctively discriminating between atrophy and non-atrophy of hippocampus in the present invention can be prepared with any discriminant analysis method, for example, but not limited to, linear discriminant analysis such as Fisher's discriminant analysis, nonlinear discriminant analysis based on the Mahalanobis' distance, neural network, Support Vector Machine (SVM) such as C-SVC, logistic regression analysis (especially, logistic regression analysis using the LASSO (Least Absolute Shrinkage and Selection Operator) method, ridge regression, or logistic regression using elastic net, and multiple logistic regression using principal component analysis), k-nearest neighbor method, decision tree, or partial least square (PLS) regression.

When a clustering boundary is a straight line or a hyperplane, the linear discriminant analysis is a method for determining the belonging of a cluster using Formula 1 as a discriminant. In Formula 1, x represents an explanatory variable, w represents a coefficient of the explanatory variable, and $w_0$ represents a constant term.

$$f(x) = w_0 + \sum_{i=1}^{n} w_i x_i \qquad \text{Formula 1}$$

Values obtained from the discriminant are referred to as discriminant scores. The measurement values of a newly offered data set can be assigned as explanatory variables to the discriminant to determine clusters by the signs of the discriminant scores.

The Fisher's discriminant analysis, one type of linear discriminant analysis, is a dimensionality reduction method for selecting a dimension suitable for discriminating classes, and constructs a highly discriminating synthetic variable by focusing on the variance of the synthetic variables and minimizing the variance of data having the same label (Venables, W. N. et al., Modern Applied Statistics with S, Fourth edition, Springer, 2002). In the Fisher's discriminant analysis, direction w of projection is determined so as to maximize Formula 2. In this formula, $\mu$ represents an average input, $n_g$ represents the number of data belonging to class g, and $\mu_g$ represents an average input of the data belonging to class g. The numerator and the denominator are the interclass variance and the intraclass variance, respectively, when each of data is projected in the direction of the vector w. Discriminant coefficient wi is * determined by maximizing this ratio (Takafumi Kanamori et al., "Pattern Recognition," KYORITSU SHUPPAN CO., LTD. (Tokyo, Japan) (2009); Richard O. et al., Pattern Classification, Second Edition., Wiley-Interscience, 2000).

$$J(w) = \frac{\sum_{g=1}^{G} n_g \left( w^T \mu_g - w^T \mu \right)\left( w^T \mu_g - w^T \mu \right)^T}{\sum_{g=1}^{G} \sum_{i:y_i=g} \left( w^T x_i - w^T \mu_g \right)\left( w^T x_i - w^T \mu_g \right)} \qquad \text{Formula 2}$$

$$\text{subject to} \quad \mu = \sum_{i=1}^{n} \frac{x_i}{n}, \quad \mu_g = \sum_{i:u_i=g}^{n} \frac{x_i}{n_g} \quad .$$

The Mahalanobis' distance is calculated according to Formula 3 in consideration of data correlation and can be used as nonlinear discriminant analysis for determining a cluster in which a data point belongs to, based on a short Mahalanobis' distance from the data point to that cluster. In Formula 3, $\mu$ represents a central vector of each cluster, and $S^{-1}$ represents an inverse matrix of the variance-covariance matrix of the cluster. The central vector is calculated from explanatory variable x, and an average vector, a median value vector, or the like can be used.

$$D(x,\mu) = \left\{ (x-\mu)' S^{-1} (x-\mu) \right\}^{\frac{1}{2}} \qquad \text{Formula 3}$$

SVM is a discriminant analysis method devised by V. Vapnik (The Nature of Statistical Leaning Theory, Springer, 1995). Particular data points of a data set having known classes are defined as explanatory variables, and classes are defined as objective variables. A boundary plane called hyperplane for correctly classifying the data set into the known classes is determined, and a discriminant for data classification is determined using the boundary plane. Then, the measurement values of a newly offered data set can be assigned as explanatory variables to the discriminant to determine classes. In this respect, the result of the discriminant analysis may be classes, may be a probability of being classified into correct classes, or may be the distance from the hyperplane. In SVM, a method of nonlinearly converting a feature vector to a high dimension and performing linear discriminant analysis in the space is known as a method for tackling nonlinear problems. An expression in which an inner product of two factors in a nonlinearly mapped space is expressed only by inputs in their original spaces is called kernel. Examples of the kernel can include a linear kernel, an RBF (Radial Basis Function) kernel, and a Gaussian kernel. While highly dimensional mapping is performed according to the kernel, the optimum discriminant, i.e., a discriminant, can be actually constructed by mere calculation according to the kernel, which avoids calculating features in the mapped space (e.g., Hideki Aso et al., Frontier of Statistical Science 6 "Statistics of pattern recognition and learning - New concepts and approaches," Iwanami Shoten, Publishers (Tokyo, Japan) (2004); Nello Cristianini et al., Introduction to SVM, Kyoritsu Shuppan Co., Ltd. (Tokyo, Japan) (2008)).

C-support vector classification (C-SVC), a type of SVM, comprises preparing a hyperplane by training a data set with the explanatory variables of two groups and classifying an unknown data set into either of the groups (C. Cortes et al., 1995, Machine Learning, Vol. 20, pp. 273-297).

Exemplary calculation of the C-SVC discriminant that can be used in the method of the present invention will be given below. First, all subjects are divided into two groups, i.e., a group of patients with hippocampal atrophy and a group of subjects with no hippocampal atrophy. A criterion for determination as to whether the subject's hippocampus has become atrophic or has not become atrophic may be based on, for example, values quantified from the results of diagnostic brain imaging (MRI) using icobrain.

Next, a data set of comprehensive expression levels of samples (e.g., samples of serum) from the two divided groups is prepared (hereinafter, this data set is referred to as a training cohort), and a C-SVC discriminant is determined by using the hippocampal atrophy markers found to differ clearly in their expression levels between the two groups as an explanatory variable and the grouping as an objective variable (e.g., -1 or +1). An optimizing objective function is represented by Formula 4 wherein e represents all input vectors, y represents an objective variable, a represents a Lagrange's undetermined multiplier vector, Q represents a positive definite matrix, and C represents a parameter for adjusting constrained conditions.

$$\min_{a} \quad \frac{1}{2}a^T Q a - e^T a \qquad\qquad \text{Formula 4}$$

$$subject\ to \quad y^T a = 0, \quad 0 \le a_i \le C, \quad i = 1,...,l,$$

Formula 5 is a finally obtained discriminant, and a group in which the data point belongs to can be determined on the basis of the sign of a value obtained according to the discriminant. In this formula, x represents a support vector, y represents a label indicating the belonging of a group, a represents the corresponding coefficient, b represents a constant term, and K represents a kernel function.

$$f(x) = \mathrm{sgn}\left(\sum_{i=1}^{l} y_i a_i K(x_i, x) + b\right) \qquad \text{Formula 5}$$

For example, an RBF kernel defined by Formula 6 can be used as the kernel function. In this formula, x represents a support vector, and $\gamma$ represents a kernel parameter for adjusting the complexity of the hyperplane.

$$K(x_i, x_j) = \exp\left(-r\|x_i - x_j\|^2\right) \quad r < 0 \qquad \text{Formula 6}$$

Logistic regression is a multivariate analysis method in which one category variable (binary variable) is used as an objective variable to predict the probability of occurrence by using multiple explanatory variables, and is expressed in the following formula 7.

$$\mathrm{logit}(\mathrm{prob}(y_i = 1)) = \beta_0 + \sum_{j=1}^{p} \beta_j x_j \qquad \text{Formula 7}$$

The LASSO (Least Absolute Shrinkage and Selection Operator) method is one of techniques for selecting and adjusting variables when multiple observed variables are present, and was proposed by Tibshirani (Tibshirani R., 1996, J R Stat Soc Ser B, vol. 58, pp267-88). Ridge regression is the oldest regularization technique, and was proposed by Hoerl (Hoerl, A.E., 1970, Technometrics., vol. 12, pp.55-67). Elastic net refers to a model in which the LASSO method and ridge regression is linearly combined and was proposed by Zou (Zou, H., 2005, J R Stat Soc Ser B, vol. 67, pp.301-320). The LASSO method is characterized in that, when regression coefficients are estimated, penalties are imposed, so that overfitting to a model is reduced and some of the regression coefficients are then estimated as 0. In ridge regression, coefficients in a model can be estimated and even if the explanatory variable is larger than the number of samples, the variable larger than the number of samples can be selected. When there is a strong correlation between explanatory variables, only one of the variables with a high correlation remains in the LASSO method and the rest is estimated to 0. By contrast, in ridge regression, both can be selected. In elastic net, explanatory variables that have a high correlation and are difficult to be selected in the LASSO method can be properly selected to create a model with reduced dimensions. In logistic regression using the LASSO method, regression coefficients are estimated so as to maximize a log-likelihood function expressed in Formula 8.

$$\frac{1}{N}\sum_{i=1}^{N}\left(y_i(\beta_0 + x_j^T\beta) - \log\left(1 + e^{(\beta_0 + x_j^T\beta)}\right)\right) - \lambda\sum_{j=1}^{p}|\beta_j| \qquad \text{Formula 8}$$

The principal component analysis method is a technique in which correlation between variables of quantitative data described using many variables is excluded and analysis is conducted, with as small information loss as possible, by converging into a small number of synthetic variables without any correlation, and was proposed by Pearson (PEARSON, K., 1901, Philosophical Magazine, series 6, vol. 2 (11), pp. 559-572) and Hotelling (HOTELLING, H., 1933, Journal of Educational Psychology, vol. 24, pp. 417-441, pp. 498-520). The degree of contribution to a certain data point can be expressed by principal component scores obtained as a result of the principal component analysis.

The method of the present invention may comprise, for example, the following steps (a), (b), and (c):

(a) measuring an expression level(s) of the hippocampal atrophy marker(s) in samples from subjects known to have hippocampal atrophy and samples from subjects known to have no hippocampal atrophy using a nucleic acid(s) (e.g., probe(s) or primer(s)), or kit, or device for detection of hippocampal atrophy according to the present invention;

(b) preparing the discriminants of Formulas 1 to 3, 5, and 6 described above from the measurement values of the expression level(s) measured in step (a); and

(c) measuring expression level(s) of the hippocampal atrophy marker(s) in a sample from a subject to be tested (unknown) for hippocampal atrophy using the nucleic acid(s) (e.g., probe(s) or primer(s)), kit, or device for detection of hippocampal atrophy according to the present invention, assigning the obtained measurement value(s) to the discriminants prepared in step (b), and on the basis of the obtained results, determining or evaluating whether the subject's hippocampus has become atrophic or has not become atrophic, or comparing the marker expression level(s) from the subject suspected of and unknown for hippocampal atrophy with control expression level(s) from subjects with no hippocampal atrophy for evaluation. In the discriminants of Formulas 1 to 3, 5 and 6, x represents an explanatory variable and includes measurements of expression levels of the hippocampal atrophy markers. Specifically, the explanatory variable for discriminating between a subject with hippocampal atrophy and a subject with no hippocampal atrophy may be a measurement of an expression level of the hippocampal atrophy marker. For example, the explanatory variable may be the following expression level: the expression level in a sample (e.g., a serum sample) from a subject with hippocampal atrophy or from a subject with no hippocampal atrophy as measured with the use of a nucleic acid for detection of hippocampal atrophy, which is a polynucleotide consisting of a nucleotide sequence shown by any of SEQ ID NOs: 1 to 109 and 110 to 200, a nucleotide sequence derived from the nucleotide sequence by the replacement of u with t, or a nucleotide sequence complementary thereto; a variant thereof, a derivative thereof, or a fragment thereof (e.g., DNA) comprising 15 or more consecutive nucleotides.

In the method described above, a discriminant using expression level(s) of one or more of the hippocampal atrophy markers described above as an explanatory variable(s) is necessary in order to determine or evaluate whether or not the subject has hippocampal atrophy (whether or not the subject's hippocampus has become atrophic) using a sample from a subject. In particular, for improving the accuracy of the discriminant using only the expression level of a single hippocampal atrophy marker, it is preferred to use a hippocampal atrophy marker exhibiting a clear difference in the expression level between two groups consisting of a group of patients with hippocampal atrophy (group of subjects with hippocampal atrophy) and a group of subjects with no hippocampal atrophy (group of healthy subjects/group of subjects with no hippocampal atrophy), in a discriminant.

Specifically, the hippocampal atrophy marker that is used for an explanatory variable of a discriminant is preferably determined as follows. First, using comprehensive expression levels in a group of patients with hippocampal atrophy and comprehensive expression levels in a group of subjects with no hippocampal atrophy, both of which are in a training cohort, as a data set, the degree of difference in the expression level of each hippocampal atrophy marker between the two groups is shown in view of, for example a P value of the parametric analysis t-test, a P value of the nonparametric analysis Mann-Whitney's U test, or a P value of Wilcoxon test.

The difference may be regarded as being statistically significant when a P value, critical probability (significance level), as determined by a statistical test is smaller than, for example, 5%, 1%, or 0.01%.

In order to correct an increased probability of type I error attributed to the repetition of a statistical test, a known method therefor in the art, for example, the Bonferroni or Holm method, can be used (e.g., Yasushi Nagata et al., "Basics of statistical multiple comparison methods," Scientist Press Co., Ltd. (Tokyo, Japan) (2007)). As an example of the Bonferroni correction, for example, the P value by a statistical test is multiplied by the number of repetitions of the test, i.e., the number of the hippocampal atrophy markers used in the analysis, and the obtained value can be compared with a desired significance level to suppress a probability of causing type I error in the whole test.

Instead of the test, the absolute value of a ratio (expression ratio; fold change) of median expression levels of each hippocampal atrophy marker between the expression levels of a group of patients with hippocampal atrophy and the expression levels of a group of subjects with no hippocampal atrophy may be calculated for selecting a hippocampal atrophy marker that is used for an explanatory variable in a discriminant. Alternatively, ROC curves may be prepared using the expression levels of a group of patients with hippocampal atrophy and a group of subjects with no hippocampal atrophy and then a hippocampal atrophy marker that is used for an explanatory variable in a discriminant may be selected on the basis of an AUROC value.

Next, a discriminant capable of calculation by any of various methods described above is prepared using any number of hippocampal atrophy markers exhibiting large differences in their expression levels as selected above. Examples of the method for constructing a discriminant that produces the maximum discrimination accuracy include a method of constructing a discriminant in every combination of hippocampal atrophy markers that satisfy the significance level of the P value, and a method of repetitively evaluating the hippocampal atrophy markers used in the preparation of a discriminant while increasing the number of the markers one by one in a descending order of differences in expression levels (Furey TS. et al., 2000, Bioinformatics., Vol. 16, pp. 906-14). To the prepared discriminant, an expression level of another independent patient with hippocampal atrophy or a subject with no hippocampal atrophy is assigned as an explanatory variable to calculate a discrimination result indicating the group to which the independent patient with hippocampal atrophy or the subject with no hippocampal atrophy belongs. Specifically, the hippocampal atrophy marker set for diagnosis as found and the discriminant constructed using the hippocampal atrophy marker set for diagnosis can be evaluated with an independent sample cohort to find a hippocampal atrophy marker set for diagnosis and a method for discrimination of hippocampal atrophy which can be universally used to detect hippocampal atrophy.

In preparing a discriminant using expression levels of a plurality of hippocampal atrophy markers as explanatory variables, it is not always necessary to select a hippocampal atrophy marker having a clear difference in the expression level between the group of patients with hippocampal atrophy and the group of subjects with no hippocampal atrophy as described above. Specifically, a discriminant having high discrimination performance may be obtained by using expression levels of a plurality of markers in combination, even if the expression levels of individual markers do not clearly differ between the groups. Therefore, it is also possible to use a method of directly searching for a discriminant having high discrimination performance without selecting the hippocampal atrophy markers to be employed for the discriminant in advance.

When preparing a discriminant, as an explanatory variable, one or more hippocampal atrophy-associated factors other than the hippocampal atrophy marker(s) described above, such as one or more factors selected from among the age, the gender, and the number of ApoE4 alleles (e.g., the age; the age and the gender; the age and the number of ApoE4 alleles; or the age, the gender, and the number of ApoE4 alleles), may be also included. As described above, it is known that a hippocampus decreases in size or becomes atrophic approximately by 1% every other year with age. In addition, it has been reported that there is a gender difference in a size of each brain region and that the hippocampus size is larger in the brain of a female. Further, it has been reported that the presence of ApoE4, which is an Alzheimer's disease risk gene, would lead to early development of hippocampal atrophy, and the phenomenon depends on the number of the ApoE4 alleles. Accordingly, such factors associated with an extent of hippocampal atrophy in combination with the miRNAs can provide markers that can more effectively determine the hippocampal volume of a patient.

The split-sample method is preferably used for evaluating the discrimination performance (generality) of the discriminant. Specifically, a data set is divided into a training cohort and a validation cohort, and hippocampal atrophy marker selection by a statistical test and discriminant preparation are performed using the training cohort, and then a validation cohort is discriminated by using the discriminant. Based on the results of discriminating the validation cohort by using the discriminant as well as a true grouping of the validation cohort, accuracy, sensitivity, and specificity are calculated for evaluating the discrimination performance of the discriminant. Alternatively, instead of dividing a data set, all of samples may be used to select hippocampal atrophy marker(s) and prepare a discriminant by a statistical test, and then newly prepared samples may be discriminated by the discriminant for calculating accuracy, sensitivity, and specificity, thereby evaluating the discrimination performance of the discriminant.

The present invention also provides use of one or more miRNA polynucleotides selected from among Group A above and optionally one or more miRNA polynucleotides selected from among Group B above, as hippocampal atrophy marker(s) for use in detection of hippocampal atrophy.

Examples

The present invention is described in greater detail with reference to the following Examples, although the scope of the present invention is not limited to these Examples.

[Example 1]

< Collection of samples >

Sera were corrected from the total of 1,126 human subjects (Table 2) from which informed consent was obtained at the National Center for Geriatrics and Gerontology, using BD Vacutainer blood collection tube 219AFBZX00109000 (Becton, Dickinson and Company, (Japan)).

Table 2

| Subject's disease type | Number of samples |
|---|---|
| Patients with Alzheimer' disease | 359 |
| Patients with mild dementia | 428 |
| Subjects with normal cognitive functions | 339 |
| Total | 1,126 |

< Extraction of Total RNA >

Total RNA was obtained, using a reagent for RNA extraction in 3D-Gene(R) RNA extraction reagent from liquid sample kit (Toray Industries, Inc. (Japan)) according to the protocol provided by the manufacturer, from 300 $\mu$L of the serum sample obtained from each of the total of 1, 126 subjects.

< Measurement of Expression Level >

miRNA in the total RNA, which was obtained from the serum sample of each of the total of 1,126 subjects, was fluorescently labeled with the use of 3D-Gene(R) miRNA Labeling kit (Toray Industries, Inc.) according to the protocol provided by the manufacturer. The oligo DNA chip used was 3D-Gene(R) Human miRNA Oligo chip (Toray Industries, Inc.) with attached probes having sequences complementary to 2,565 miRNAs among the miRNAs registered in miRBase Release 21. Hybridization under stringent conditions and washing following the hybridization were performed according to the protocol provided by the manufacturer. The oligo DNA chip was scanned using the 3D-Gene(R) scanner (Toray Industries, Inc.) to obtain images. Fluorescence intensity on the image was digitized using 3D-Gene(R) Extraction (Toray Industries, Inc.). The digitized fluorescence intensity was converted to a logarithmic value having a base of 2 and used as an expression level. A blank value was subtracted therefrom. A missing value was replaced with a signal value of 0.1. As a result, the comprehensive miRNA expression levels from the serum samples were obtained for the above 1,126 subjects.

In accordance with the standard operation procedure designated in advance for the present study, 271 serum samples were selected from among the 1,126 samples. As described above, the hippocampus is known to become atrophic as aging, and thus by limiting the age of the subjects in the range of 60-year-old to 90-year-old in order to avoid outliers in respect of the age, the total of 264 samples were further selected for discriminant analysis of hippocampal atrophy.

When performing discriminant analysis, each sample group was divided into a training cohort, a cross validation cohort, and an independent validation cohort, as described in the Examples below. Calculation and statistical analysis using the digitized miRNA expression levels were carried out using R language 3.5.2 (R Core Team, 2018; R: A language and environment for statistical computing, R Foundation for Statistical Computing, Vienna, Austria, URL https://www.R-project.org/.) and MASS package 7.3.45 (Venables, W.N. & Ripley, B.D. (2002) Modern Applied Statistics with S. Fourth Edition. Springer, New York. ISBN 0-387-95457-0).

< Calculation of the volume ratio of the hippocampus to the whole brain >

Brain MRI images of the 264 subjects were obtained and digitized using the brain image analysis program icobrain (Icometrix) to obtain a quantified value of the normalized volume of each region of the brain. On the basis of the quantified value, the volume ratio of the hippocampus to the whole brain was calculated.

< Determination of the number of ApoE4 alleles >

DNAs were extracted from peripheral blood cells of the 264 subjects and the full-length sequences thereof were analyzed using a next-generation sequencer. The APOE gene polymorphism was analyzed and, in particular, the number of ApoE4 alleles was determined.

[Example 2]

< Comparison between the hippocampal atrophy group and the hippocampal non-atrophy group based on a single miRNA >

In this Example, two groups with different extents of hippocampal atrophy (based on the volume ratio of the hippocampus to the whole brain) were compared therebetween, and a single miRNA exhibiting a significant difference in the expression level between the groups was selected as the hippocampal atrophy marker. In Examples 2-(1) to 2-(4) below, the thresholds for an extent of hippocampal atrophy were designated at 4 different levels for trial, and markers were selected via classification into the hippocampal atrophy groups and the hippocampal non-atrophy groups at each threshold.

Specifically, miRNA expression levels of the markers determined in Example 1 were corrected using endogenous controls (miR-149-3p, miR-2861, and miR-4463). In order to obtain a more highly reliable diagnostic marker, in addition, 210 miRNAs exhibiting the expression levels of $2^4$ or more in 90% or more samples of the hippocampal atrophy group or the hippocampal non-atrophy group were selected and subjected to analysis.

(1) Comparison between the hippocampal atrophy group 1 with the volume ratio of the hippocampus to the whole brain of 0.60% or less and the hippocampal non-atrophy group 1 with the volume ratio of the hippocampus to the whole brain of 0.63% or more

In order to evaluate miRNAs/genes exhibiting a statistically significant difference in expression level between two groups: i.e., the hippocampal atrophy group 1 with the volume ratio of the hippocampus to the whole brain of 0.60% or less (132 samples) and the hippocampal non-atrophy group 1 with the volume ratio of the hippocampus to the whole brain of 0.63% or more (132 samples), and an equal-variance-assumed two-sided t test was conducted and the P value was calculated. As markers, miRNAs/genes with the P value of less than 0.1 or the absolute value of the difference (fold change) of the logarithmically converted gene expression level of the hippocampal atrophy group and of the hippocampal non-atrophy group of more than 0.2, which is deduced as a measurement error, were selected. The results are shown in Table 3.

Thus, hsa-miR-3131, hsa-miR-6757-5p, hsa-miR-4706, hsa-miR-5001-5p, hsa-miR-3180-3p, hsa-miR-642b-3p, hsa-miR-4655-5p, hsa-miR-6819-5p, hsa-miR-937-5p, hsa-miR-4688, hsa-miR-6741-5p, hsa-miR-7107-5p, hsa-miR-4271, hsa-miR-1229-5p, hsa-miR-4707-5p, hsa-miR-6808-5p, hsa-miR-4656, hsa-miR-6076, hsa-miR-6762-5p, hsa-miR-7109-5p, hsa-miR-6732-5p, hsa-miR-3195, hsa-miR-7150, hsa-miR-642a-3p, hsa-miR-1249-5p, hsa-miR-3185, hsa-miR-4689, hsa-miR-3141, hsa-miR-6840-3p, hsa-miR-3135b, hsa-miR-1914-3p, hsa-miR-4446-3p, hsa-miR-4433b-3p, hsa-miR-6877-5p, hsa-miR-6848-5p, hsa-miR-3620-5p, hsa-miR-6825-5p, hsa-miR-5739, hsa-miR-3663-3p, hsa-miR-4695-5p, hsa-miR-3162-5p, hsa-miR-3679-5p, hsa-miR-8059, hsa-miR-7110-5p, hsa-miR-1275, hsa-miR-6779-5p, hsa-miR-197-5p, hsa-miR-6845-5p, hsa-miR-4327, hsa-miR-4723-5p, hsa-miR-4530, hsa-miR-6771-5p, hsa-miR-614, hsa-miR-92a-2-5p, hsa-miR-6891-5p, hsa-miR-6124, hsa-miR-4687-3p, hsa-miR-4442, hsa-miR-7977, hsa-miR-6785-5p, hsa-miR-4497, hsa-miR-8071, hsa-miR-663b, hsa-miR-3180, hsa-miR-1228-5p, hsa-miR-760, hsa-miR-187-5p, hsa-miR-7111-5p, hsa-miR-6088, hsa-miR-6805-3p, hsa-miR-4640-5p, hsa-miR-6721-5p, hsa-miR-6880-5p, hsa-miR-711, hsa-miR-128-1-5p, hsa-miR-4525, hsa-miR-486-3p, hsa-miR-6756-5p, hsa-miR-1260b, hsa-miR-3184-5p, hsa-miR-6075, hsa-miR-204-3p, hsa-miR-4728-5p, hsa-miR-4534, hsa-miR-4758-5p, hsa-miR-8063, hsa-miR-6836-3p, hsa-miR-6789-5p, hsa-miR-744-5p, hsa-miR-1909-3p, hsa-miR-887-3p, hsa-miR-4745-5p, hsa-miR-4433a-3p, hsa-miR-5090, hsa-miR-296-5p, hsa-miR-939-5p, hsa-miR-3648, hsa-miR-3196, hsa-miR-6722-3p, hsa-miR-6805-5p, hsa-miR-1202, hsa-miR-6775-5p, hsa-miR-6087, hsa-miR-6765-5p, hsa-miR-6875-5p, hsa-miR-4674, hsa-miR-1233-5p, hsa-miR-7114-5p, hsa-miR-5787, hsa-miR-8072, hsa-miR-3619-3p, hsa-miR-4632-5p, hsa-miR-6800-5p, hsa-miR-4634, hsa-miR-4486, hsa-miR-6727-5p, hsa-miR-4505, and hsa-miR-4725-3p were selected. miRNAs thereof are polynucleotides consisting of the nucleotide sequences shown by SEQ ID NOs: 1 to 64 and 110 to 163, respectively.

Among them, miRNAs that were newly found as markers for detection of the presence or absence of hippocampal atrophy are polynucleotides consisting of the nucleotide sequences shown by SEQ ID NOs: 1 to 64.

Table 3

| SEQ ID NO: | Name of gene | P value | Difference of hippocampal atrophy group relative to hippocampal non-atrophy group (Fold change) |
|---|---|---|---|
| 1 | hsa-miR-3131 | 1.44E-06 | -1.35 |

(continued)

| SEQ ID NO: | Name of gene | P value | Difference of hippocampal atrophy group relative to hippocampal non-atrophy group (Fold change) |
|---|---|---|---|
| 2 | hsa-miR-6757-5p | 1.26E-05 | -1.12 |
| 3 | hsa-miR-4706 | 1.42E-05 | -1.11 |
| 4 | hsa-miR-5001-5p | 3.21E-05 | -0.81 |
| 5 | hsa-miR-3180-3p | 3.67E-05 | -0.10 |
| 6 | hsa-miR-642b-3p | 9.68E-05 | -0.81 |
| 7 | hsa-miR-4655-5p | 0.000128 | -0.80 |
| 8 | hsa-miR-6819-5p | 0.000136 | -0.78 |
| 9 | hsa-miR-937-5p | 0.000212 | -0.67 |
| 10 | hsa-miR-4688 | 0.000221 | -1.04 |
| 11 | hsa-miR-6741-5p | 0.000234 | -0.77 |
| 12 | hsa-miR-7107-5p | 0.000373 | -0.82 |
| 13 | hsa-miR-4271 | 0.000498 | -0.78 |
| 14 | hsa-miR-1229-5p | 0.000601 | -0.77 |
| 15 | hsa-miR-4707-5p | 0.000629 | -0.70 |
| 16 | hsa-miR-6808-5p | 0.000645 | -0.93 |
| 17 | hsa-miR-4656 | 0.000707 | -0.87 |
| 18 | hsa-miR-6076 | 0.000741 | -0.82 |
| 19 | hsa-miR-6762-5p | 0.000914 | -0.58 |
| 20 | hsa-miR-7109-5p | 0.000938 | -0.78 |
| 21 | hsa-miR-6732-5p | 0.000939 | -0.42 |
| 22 | hsa-miR-3195 | 0.001008 | -0.55 |
| 23 | hsa-miR-7150 | 0.001066 | -0.77 |

(continued)

| SEQ ID NO: | Name of gene | P value | Difference of hippocampal atrophy group relative to hippocampal non-atrophy group (Fold change) |
|---|---|---|---|
| 24 | hsa-miR-642a-3p | 0.001138 | -0.90 |
| 25 | hsa-miR-1249-5p | 0.001187 | -0.88 |
| 26 | hsa-miR-3185 | 0.001309 | -0.75 |
| 27 | hsa-miR-4689 | 0.001455 | -0.43 |
| 28 | hsa-miR-3141 | 0.001638 | -0.71 |
| 29 | hsa-miR-6840-3p | 0.001859 | -0.68 |
| 30 | hsa-miR-3135b | 0.001865 | -0.63 |
| 31 | hsa-miR-1914-3p | 0.002234 | -0.74 |
| 32 | hsa-miR-4446-3p | 0.002261 | -0.56 |
| 33 | hsa-miR-4433b-3p | 0.002597 | -0.54 |
| 34 | hsa-miR-6877-5p | 0.002994 | -0.78 |
| 35 | hsa-miR-6848-5p | 0.003043 | -0.74 |
| 36 | hsa-miR-3620-5p | 0.003078 | -0.50 |
| 37 | hsa-miR-6825-5p | 0.004465 | -0.74 |
| 38 | hsa-miR-5739 | 0.004499 | -0.79 |
| 39 | hsa-miR-3663-3p | 0.005218 | -0.15 |
| 40 | hsa-miR-4695-5p | 0.005936 | -0.41 |
| 41 | hsa-miR-3162-5p | 0.006461 | -0.76 |
| 42 | hsa-miR-3679-5p | 0.010099 | -0.71 |
| 43 | hsa-miR-8059 | 0.01382 | -0.47 |
| 44 | hsa-miR-7110-5p | 0.014164 | -0.58 |
| 45 | hsa-miR-1275 | 0.016412 | -0.63 |

(continued)

| SEQ ID NO: | Name of gene | P value | Difference of hippocampal atrophy group relative to hippocampal non-atrophy group (Fold change) |
|---|---|---|---|
| 46 | hsa-miR-6779-5p | 0.017319 | -0.61 |
| 47 | hsa-miR-197-5p | 0.020443 | -0.59 |
| 48 | hsa-miR-6845-5p | 0.02053 | -0.12 |
| 49 | hsa-miR-4327 | 0.027884 | -0.29 |
| 50 | hsa-miR-4723-5p | 0.030133 | -0.24 |
| 51 | hsa-miR-4530 | 0.032121 | 0.14 |
| 52 | hsa-miR-6771-5p | 0.03329 | -0.15 |
| 53 | hsa-miR-614 | 0.034917 | -0.61 |
| 54 | hsa-miR-92a-2-5p | 0.035431 | -0.23 |
| 55 | hsa-miR-6891-5p | 0.037723 | -0.60 |
| 56 | hsa-miR-6124 | 0.03973 | -0.59 |
| 57 | hsa-miR-4687-3p | 0.053084 | -0.14 |
| 58 | hsa-miR-4442 | 0.064082 | -0.17 |
| 59 | hsa-miR-7977 | 0.067554 | -0.54 |
| 60 | hsa-miR-6785-5p | 0.068641 | -0.19 |
| 61 | hsa-miR-4497 | 0.068733 | -0.10 |
| 62 | hsa-miR-8071 | 0.080023 | -0.46 |
| 63 | hsa-miR-663b | 0.083048 | -0.41 |
| 64 | hsa-miR-3180 | 0.086421 | -0.18 |
| 110 | hsa-miR-1228-5p | 2.07E-08 | 0.15 |
| 111 | hsa-miR-760 | 1.11E-06 | -0.92 |
| 112 | hsa-miR-187-5p | 8.61E-05 | -0.81 |

(continued)

| SEQ ID NO: | Name of gene | P value | Difference of hippocampal atrophy group relative to hippocampal non-atrophy group (Fold change) |
|---|---|---|---|
| 113 | hsa-miR-7111-5p | 0.000115 | -0.89 |
| 114 | hsa-miR-6088 | 0.00015 | -0.14 |
| 115 | hsa-miR-6805-3p | 0.000246 | -0.59 |
| 116 | hsa-miR-4640-5p | 0.000341 | -0.77 |
| 117 | hsa-miR-6721-5p | 0.000502 | -0.89 |
| 118 | hsa-miR-6880-5p | 0.000639 | -0.72 |
| 119 | hsa-miR-711 | 0.000646 | -0.58 |
| 120 | hsa-miR-128-1-5p | 0.00065 | -0.77 |
| 121 | hsa-miR-4525 | 0.000698 | -0.65 |
| 122 | hsa-miR-486-3p | 0.000796 | -0.53 |
| 123 | hsa-miR-6756-5p | 0.000926 | -0.48 |
| 124 | hsa-miR-1260b | 0.001009 | -0.84 |
| 125 | hsa-miR-3184-5p | 0.001403 | -0.45 |
| 126 | hsa-miR-6075 | 0.001517 | -0.51 |
| 127 | hsa-miR-204-3p | 0.001527 | 0.24 |
| 128 | hsa-miR-4728-5p | 0.001564 | -0.93 |
| 129 | hsa-miR-4534 | 0.001621 | -0.81 |
| 130 | hsa-miR-4758-5p | 0.001631 | -0.43 |
| 131 | hsa-miR-8063 | 0.001716 | -0.85 |
| 132 | hsa-miR-6836-3p | 0.002121 | -0.38 |
| 133 | hsa-miR-6789-5p | 0.00237 | -0.12 |
| 134 | hsa-miR-744-5p | 0.002462 | -0.73 |

(continued)

| SEQ ID NO: | Name of gene | P value | Difference of hippocampal atrophy group relative to hippocampal non-atrophy group (Fold change) |
|---|---|---|---|
| 135 | hsa-miR-1909-3p | 0.003815 | -0.09 |
| 136 | hsa-miR-887-3p | 0.005512 | -0.70 |
| 137 | hsa-miR-4745-5p | 0.006232 | -0.20 |
| 138 | hsa-miR-4433a-3p | 0.006426 | -0.75 |
| 139 | hsa-miR-5090 | 0.006751 | -0.54 |
| 140 | hsa-miR-296-5p | 0.007901 | -0.77 |
| 141 | hsa-miR-939-5p | 0.008515 | -0.72 |
| 142 | hsa-miR-3648 | 0.00972 | -0.21 |
| 143 | hsa-miR-3196 | 0.011181 | -0.07 |
| 144 | hsa-miR-6722-3p | 0.011562 | -0.32 |
| 145 | hsa-miR-6805-5p | 0.012652 | -0.07 |
| 146 | hsa-miR-1202 | 0.019697 | -0.70 |
| 147 | hsa-miR-6775-5p | 0.021518 | -0.32 |
| 148 | hsa-miR-6087 | 0.027508 | -0.11 |
| 149 | hsa-miR-6765-5p | 0.031912 | 0.05 |
| 150 | hsa-miR-6875-5p | 0.033864 | -0.10 |
| 151 | hsa-miR-4674 | 0.040809 | -0.12 |
| 152 | hsa-miR-1233-5p | 0.04355 | 0.08 |
| 153 | hsa-miR-7114-5p | 0.050684 | -0.44 |
| 154 | hsa-miR-5787 | 0.052306 | 0.06 |
| 155 | hsa-miR-8072 | 0.059608 | 0.07 |
| 156 | hsa-miR-3619-3p | 0.06347 | -0.26 |

(continued)

| SEQ ID NO: | Name of gene | P value | Difference of hippocampal atrophy group relative to hippocampal non-atrophy group (Fold change) |
|---|---|---|---|
| 157 | hsa-miR-4632-5p | 0.06537 | -0.21 |
| 158 | hsa-miR-6800-5p | 0.075364 | -0.19 |
| 159 | hsa-miR-4634 | 0.07605 | -0.08 |
| 160 | hsa-miR-4486 | 0.079349 | -0.44 |
| 161 | hsa-miR-6727-5p | 0.083474 | -0.08 |
| 162 | hsa-miR-4505 | 0.086658 | -0.19 |
| 163 | hsa-miR-4725-3p | 0.089039 | -0.16 |

(2) Comparison between the hippocampal atrophy group 2 with the volume ratio of the hippocampus to the whole brain of 0.59% or less and the hippocampal non-atrophy group 2 with the volume ratio of the hippocampus to the whole brain of 0.64% or more

By comparing two groups with more extremely different extents of hippocampal atrophy, analysis was performed more precisely than in Example 2-(1). Comparison was performed between two groups: the hippocampal atrophy group 2 with the volume ratio of the hippocampus to the whole brain of 0.59% or less (105 samples), for which the lower threshold was designated, and the hippocampal non-atrophy group 2 with the volume ratio of the hippocampus to the whole brain of 0.64% or more (105 samples), for which the higher threshold was designated.

As in the case of Example 2-(1), miRNAs/genes found to have the P value of less than 0.1 or the absolute value of the fold change of 0.2 or more as a result of comparison between the two groups were selected. The results are shown in Table 4.

As such, in addition to the miRNAs/genes selected in Example 2-(1), hsa-miR-4251, hsa-miR-1285-3p, hsa-miR-6870-5p, hsa-miR-4484, hsa-miR-4476, hsa-miR-4454, hsa-miR-1538, hsa-miR-320b, hsa-miR-1915-5p, hsa-miR-328-5p, and hsa-miR-6820-5p were newly selected. miRNAs thereof are polynucleotides consisting of the nucleotide sequences shown by SEQ ID NOs: 65 to 69, 71, and 164 to 168, respectively.

Among them, miRNAs that were newly found as markers for detection of the presence or absence of hippocampal atrophy are polynucleotides consisting of the nucleotide sequences shown by SEQ ID NOs: 65 to 69 and 71.

Table 4

| SEQ ID NO: | Name of gene | P value | Difference of hippocampal atrophy group relative to hippocampal non-atrophy group (Fold change) |
|---|---|---|---|
| 65 | hsa-miR-4251 | 6.25.E-11 | 1.27 |
| 66 | hsa-miR-1285-3p | 9.65.E-08 | 1.08 |
| 67 | hsa-miR-6870-5p | 5.24.E-02 | -0.62 |
| 68 | hsa-miR-4484 | 7.74.E-02 | -0.08 |
| 69 | hsa-miR-4476 | 1.47.E-01 | -0.44 |
| 71 | hsa-miR-4454 | 2.89.E-01 | -0.25 |
| 164 | hsa-miR-1538 | 9.01.E-12 | 1.47 |

(continued)

| SEQ ID NO: | Name of gene | P value | Difference of hippocampal atrophy group relative to hippocampal non-atrophy group (Fold change) |
|---|---|---|---|
| 165 | hsa-miR-320b | 9.69.E-11 | 1.27 |
| 166 | hsa-miR-1915-5p | 2.07.E-07 | 1.25 |
| 167 | hsa-miR-328-5p | 9.11.E-02 | 0.05 |
| 168 | hsa-miR-6820-5p | 1.25.E-01 | -0.39 |

(3) Comparison between the hippocampal atrophy group 3 with the volume ratio of the hippocampus to the whole brain of 0.57% or less and the hippocampal non-atrophy group 3 with the volume ratio of the hippocampus to the whole brain of 0.68% or more

By comparing two groups with more extremely different extents of hippocampal atrophy, analysis was performed more precisely than in Example 2-(2). Comparison was performed between two groups: the hippocampal atrophy group 3 with the volume ratio of the hippocampus to the whole brain of 0.57% or less (88 samples), for which the lower threshold was designated, and the hippocampal non-atrophy group 3 with the volume ratio of the hippocampus to the whole brain of 0.68% or more (88 samples), for which the higher threshold was designated.

As in the case of Example 2-(1) and Example 2-(2), miRNAs/genes found to have the P value of less than 0.1 or the absolute value of the fold change of 0.2 or more as a result of comparison between the two groups were selected. The results are shown in Table 5.

As such, in addition to the miRNAs/genes selected in Example 2-(1) and Example 2-(2), hsa-miR-6749-5p, hsa-miR-6893-5p, hsa-miR-6085, hsa-miR-4787-5p, hsa-miR-149-3p, hsa-miR-7704, hsa-miR-6125, hsa-miR-6090, hsa-miR-3197, hsa-miR-6850-5p, hsa-miR-4467, hsa-miR-6885-5p, hsa-miR-6803-5p, hsa-miR-6798-5p, hsa-miR-6780b-5p, hsa-miR-6768-5p, hsa-miR-5100, hsa-miR-6724-5p, hsa-miR-6879-5p, hsa-miR-7108-5p, hsa-miR-4649-5p, hsa-miR-4739, hsa-miR-6089, hsa-miR-1908-5p, hsa-miR-4516, hsa-miR-2861, hsa-miR-4492, hsa-miR-4294, hsa-miR-6791-5p, hsa-miR-1469, hsa-miR-6752-5p, hsa-miR-4730, hsa-miR-6126, hsa-miR-6869-5p, hsa-miR-1268a, hsa-miR-6799-5p, hsa-miR-8069, hsa-miR-6726-5p, hsa-miR-3665, hsa-miR-638, hsa-miR-762, hsa-miR-4466, hsa-miR-3940-5p, hsa-miR-1237-5p, hsa-miR-575, hsa-miR-3656, hsa-miR-4488, hsa-miR-4281, hsa-miR-6781-5p, hsa-miR-4532, hsa-miR-4665-5p, hsa-miR-6816-5p, hsa-miR-4508, hsa-miR-6784-5p, hsa-miR-6786-5p, hsa-miR-4741, hsa-miR-1343-5p, hsa-miR-1227-5p, hsa-miR-4734, hsa-miR-3960, hsa-miR-128-2-5p, hsa-miR-6743-5p, hsa-miR-663a, hsa-miR-6729-5p, hsa-miR-1915-3p, hsa-miR-1268b, hsa-miR-4651, hsa-miR-3178, and hsa-miR-4463 were newly selected. miRNAs thereof are polynucleotides consisting of the nucleotide sequences shown by SEQ ID NOs: 70, 72 to 107, and 169 to 200, respectively.

Among them, miRNAs that were newly found as markers for detection of the presence or absence of hippocampal atrophy are polynucleotides consisting of the nucleotide sequences shown by SEQ ID NOs: 70 and 72 to 107.

Table 5

| SEQ ID NO: | Name of gene | P value | Difference of hippocampal atrophy group relative to hippocampal non-atrophy group (Fold change) |
|---|---|---|---|
| 70 | hsa-miR-6749-5p | 0.029 | -0.43 |
| 72 | hsa-miR-6893-5p | 0.043 | 0.59 |
| 73 | hsa-miR-6085 | 0.051 | -0.79 |
| 74 | hsa-miR-4787-5p | 0.656 | -4.03 |

(continued)

| SEQ ID NO: | Name of gene | P value | Difference of hippocampal atrophy group relative to hippocampal non-atrophy group (Fold change) |
|---|---|---|---|
| 75 | hsa-miR-149-3p | 0.508 | -3.67 |
| 76 | hsa-miR-7704 | 0.523 | -3.09 |
| 77 | hsa-miR-6125 | 0.197 | -3.00 |
| 78 | hsa-miR-6090 | 0.647 | -2.82 |
| 79 | hsa-miR-3197 | 0.746 | -2.45 |
| 80 | hsa-miR-6850-5p | 0.652 | -2.39 |
| 81 | hsa-miR-4467 | 0.768 | -2.34 |
| 82 | hsa-miR-6885-5p | 0.915 | -2.33 |
| 83 | hsa-miR-6803-5p | 0.666 | -2.31 |
| 84 | hsa-miR-6798-5p | 0.800 | -2.28 |
| 85 | hsa-miR-6780b-5p | 0.798 | -2.19 |
| 86 | hsa-miR-6768-5p | 0.477 | 2.08 |
| 87 | hsa-miR-5100 | 0.551 | -1.92 |
| 88 | hsa-miR-6724-5p | 0.164 | -1.75 |
| 89 | hsa-miR-6879-5p | 0.158 | -1.73 |
| 90 | hsa-miR-7108-5p | 0.178 | -1.66 |
| 91 | hsa-miR-4649-5p | 0.365 | -1.65 |
| 92 | hsa-miR-4739 | 0.276 | -1.60 |
| 93 | hsa-miR-6089 | 0.727 | -1.57 |
| 94 | hsa-miR-1908-5p | 0.810 | -1.48 |
| 95 | hsa-miR-4516 | 0.602 | -1.41 |
| 96 | hsa-miR-2861 | 0.579 | 1.41 |

(continued)

| SEQ ID NO: | Name of gene | P value | Difference of hippocampal atrophy group relative to hippocampal non-atrophy group (Fold change) |
|---|---|---|---|
| 97 | hsa-miR-4492 | 0.926 | -1.37 |
| 98 | hsa-miR-4294 | 0.732 | -1.25 |
| 99 | hsa-miR-6791-5p | 0.795 | -0.75 |
| 100 | hsa-miR-1469 | 0.952 | -0.68 |
| 101 | hsa-miR-6752-5p | 0.462 | -0.62 |
| 102 | hsa-miR-4730 | 0.819 | 0.47 |
| 103 | hsa-miR-6126 | 0.430 | 0.42 |
| 104 | hsa-miR-6869-5p | 0.182 | -0.38 |
| 105 | hsa-miR-1268a | 0.591 | -0.37 |
| 106 | hsa-miR-6799-5p | 0.282 | 0.26 |
| 107 | hsa-miR-8069 | 0.474 | -0.24 |
| 169 | hsa-miR-6726-5p | 0.068 | -1.18 |
| 170 | hsa-miR-3665 | 0.384 | -3.99 |
| 171 | hsa-miR-638 | 0.938 | -3.82 |
| 172 | hsa-miR-762 | 0.477 | -3.39 |
| 173 | hsa-miR-4466 | 0.786 | -3.07 |
| 174 | hsa-miR-3940-5p | 0.224 | -2.87 |
| 175 | hsa-miR-1237-5p | 0.289 | -2.72 |
| 176 | hsa-miR-575 | 0.380 | -2.59 |
| 177 | hsa-miR-3656 | 0.212 | -2.50 |
| 178 | hsa-miR-4488 | 0.348 | -2.42 |
| 179 | hsa-miR-4281 | 0.443 | -2.34 |
| 180 | hsa-miR-6781-5p | 0.272 | -2.33 |

(continued)

| SEQ ID NO: | Name of gene | P value | Difference of hippocampal atrophy group relative to hippocampal non-atrophy group (Fold change) |
|---|---|---|---|
| 181 | hsa-miR-4532 | 0.802 | -2.22 |
| 182 | hsa-miR-4665-5p | 0.381 | 2.16 |
| 183 | hsa-miR-6816-5p | 0.191 | -2.12 |
| 184 | hsa-miR-4508 | 0.780 | -2.00 |
| 185 | hsa-miR-6784-5p | 0.442 | -1.70 |
| 186 | hsa-miR-6786-5p | 0.756 | -1.68 |
| 187 | hsa-miR-4741 | 0.513 | -1.66 |
| 188 | hsa-miR-1343-5p | 0.819 | 1.55 |
| 189 | hsa-miR-1227-5p | 0.380 | -1.53 |
| 190 | hsa-miR-4734 | 0.195 | -1.52 |
| 191 | hsa-miR-3960 | 0.349 | -1.48 |
| 192 | hsa-miR-128-2-5p | 0.778 | -1.31 |
| 193 | hsa-miR-6743-5p | 0.426 | 1.25 |
| 194 | hsa-miR-663a | 0.186 | 0.79 |
| 195 | hsa-miR-6729-5p | 0.840 | -0.72 |
| 196 | hsa-miR-1915-3p | 0.475 | 0.70 |
| 197 | hsa-miR-1268b | 0.522 | -0.63 |
| 198 | hsa-miR-4651 | 0.857 | -0.45 |
| 199 | hsa-miR-3178 | 0.657 | -0.43 |
| 200 | hsa-miR-4463 | 0.864 | -0.22 |

(4) Comparison between the hippocampal atrophy group 4 with the volume ratio of the hippocampus to the whole brain of 0.38% or less and the hippocampal non-atrophy group 4 with the volume ratio of the hippocampus to the whole brain of 0.86% or more

By comparing two groups with more extremely different extents of hippocampal atrophy, analysis was performed more precisely than in Example 2-(3). Comparison was performed between two groups: the hippocampal atrophy group 4 with the volume ratio of the hippocampus to the whole brain of 0.38% or less (5 samples), for which the extremely low threshold was designated, and the hippocampal non-atrophy group 4 with the volume ratio of the hippocampus to the whole brain of 0.86% or more (5 samples), for which the extremely high threshold was designated.

As in the case of Examples 2-(1) to 2-(3), miRNAs/genes found to have the P value of less than 0.1 or the absolute value of the fold change of 0.2 or more as a result of comparison between the two groups were selected. The results are shown in Table 6.

As such, in addition to the miRNAs/genes selected in Examples 2-(1) to 2-(3), hsa-miR-3621 and hsa-miR-4763-3p were newly selected. miRNAs thereof are polynucleotides consisting of the nucleotide sequences shown by SEQ ID NOs: 108 and 109, respectively, and were newly found as markers for detection of the presence or absence of hippocampal atrophy (the hippocampal atrophy markers).

Table 6

| SEQ ID NO: | Name of gene | P value | Difference of hippocampal atrophy group relative to hippocampal non-atrophy group (Fold change) |
|---|---|---|---|
| 108 | hsa-miR-3621 | 0.00430 | 0.29 |
| 109 | hsa-miR-4763-3p | 0.263 | 0.23 |

All the polynucleotides consisting of the nucleotide sequences shown by SEQ ID NOs: 1 to 200 as indicated above are each independently capable of discriminating subjects with hippocampal atrophy from subjects with no hippocampal atrophy.

[Example 3]

< Discrimination of patients with hippocampal atrophy with the use of a small number (2 to 5) of miRNA markers in combination (discrimination by logistic regression analysis) >

In this Example, discriminants with combinations of 2 to 5 miRNA markers were prepared, discrimination performance thereof was then evaluated in an independent validation cohort (Table 7), and miRNAs/genes used for a discriminant with higher performance were extracted to obtain a combination of miRNA markers capable of detection of hippocampal atrophy.

More specifically, the hippocampal atrophy group 3 (88 samples with the volume ratio of the hippocampus to the whole brain of 0.57% or less) and the hippocampal non-atrophy group 3 (88 samples with the volume ratio of the hippocampus to the whole brain of 0.68% or more) used in Example 2-(3) were used to search for miRNA markers capable of determining an extent of hippocampal atrophy. From each of the hippocampal atrophy group and the hippocampal non-atrophy group, 73 samples were allocated to the training and cross validation cohort and the remaining 15 samples were allocated to the independent validation cohort (Table 7).

Table 7

| Subject's disease type | Number of all samples | Training and cross validation cohort | Independent validation cohort |
|---|---|---|---|
| Hippocampal atrophy group | 88 | 73 | 15 |
| Hippocampal non-atrophy group | 88 | 73 | 15 |
| Total | 176 | 146 | 30 |

Concerning the training and cross validation cohort, the samples of the hippocampal atrophy group and the samples of the hippocampal non-atrophy group were each divided into 3 groups, 2/3 thereof were allocated to the training cohort, and the other 1/3 was allocated to the cross validation cohort. The cross validation cohort was shuffled 3 times, and discriminants were constructed using the training cohort every instance of shuffling of the cross validation cohort. Further, a pattern of the shuffling was changed 10 times.

The combinations of 2 to 5 miRNA markers were subjected to logistic regression analysis using the training cohort by the LASSO method to construct discriminants for determining hippocampal atrophy. Among the constructed discriminants, combinations of miRNA markers (shown by SEQ ID NOs:) used in discriminants exhibiting the Area Under the Curve (AUC) of 0.8 or more in the cross validation cohort and the independent validation cohort, and the AUC, the sensitivity, and the specificity calculated for each group are shown in Table 8. Regarding a discriminant using two markers, the expression levels of miRNAs consisting of the nucleotide sequences shown by SEQ ID NOs: 110 and 114 were used to calculate the probability of correct classification for hippocampal atrophy with the threshold (0.5) to discriminate the groups (i.e., the hippocampal atrophy group and the hippocampal non-atrophy group) designated in the training and cross validation cohort. As a result, the AUC of 0.862, the sensitivity of 80.0%, and the specificity of 86.7% were shown as the discrimination performance in the independent validation cohort. Regarding a discriminant using three markers, for example, the expression levels of miRNAs consisting of the nucleotide sequences shown by SEQ ID NOs: 1, 110, and 114 were used, and the AUC of 0.898, the sensitivity of 73.3%, and the specificity of 93.3% were shown as the discrimination performance in the independent validation cohort. Regarding a discriminant using four markers, for example, the expression levels of miRNAs consisting of the nucleotide sequences shown by SEQ ID NOs: 1, 110, 114, and 135 were used, and the AUC of 0.92, the sensitivity of 80.0%, and the specificity of 86.7% were shown as the discrimination performance in the independent validation cohort. Regarding a discriminant using five markers, for example, the expression levels of miRNAs consisting of the nucleotide sequences shown by SEQ ID NOs: 1, 110, 114, 127, and 135 were used, and the AUC of 0.902, the sensitivity of 86.7%, and the specificity of 73.3% were shown as the discrimination performance in the independent validation cohort.

Table 8

| Number of markers | SEQ ID NO: | Training and cross validation cohort | | | Independent validation cohort | | |
|---|---|---|---|---|---|---|---|
| | | AUC | Sensitivity (%) | Specificity (%) | AUC | Sensitivity (%) | Specificity (%) |
| 2 | 110_114 | 0.826 | 72.0 | 72.0 | 0.862 | 80.0 | 86.7 |
| 3 | 110_114_1 | 0.828 | 64.0 | 83.3 | 0.898 | 73.3 | 93.3 |
| | 110_114_135 | 0.861 | 68.0 | 80.0 | 0.876 | 73.3 | 86.7 |
| | 110_114_155 | 0.825 | 87.5 | 52.0 | 0.867 | 86.7 | 66.7 |
| | 110_114_137 | 0.812 | 83.3 | 66.7 | 0.867 | 86.7 | 86.7 |
| 4 | 110_114_155_135 | 0.878 | 76.0 | 84.0 | 0.853 | 73.3 | 80.0 |
| | 110_114_137_111 | 0.845 | 79.2 | 79.2 | 0.876 | 86.7 | 86.7 |
| | 110_114_155_2 | 0.833 | 83.3 | 64.0 | 0.822 | 80.0 | 80.0 |
| | 110_114_142_2 | 0.824 | 80.0 | 72.0 | 0.876 | 80.0 | 86.7 |
| | 110_114_1_149 | 0.82 | 72.0 | 75.0 | 0.889 | 80.0 | 86.7 |
| | 110_114_1_135 | 0.812 | 79.2 | 66.7 | 0.92 | 80.0 | 86.7 |
| | 110_114_155_68 | 0.809 | 50.0 | 83.3 | 0.818 | 66.7 | 86.7 |
| | 110_114_2_135 | 0.804 | 62.5 | 70.8 | 0.88 | 73.3 | 86.7 |

(continued)

| Number of markers | SEQ ID NO: | Training and cross validation cohort | | | Independent validation cohort | | |
|---|---|---|---|---|---|---|---|
| | | AUC | Sensitivity (%) | Specificity (%) | AUC | Sensitivity (%) | Specificity (%) |
| 5 | 110_114_155_135_2 | 0.888 | 80.0 | 80.0 | 0.844 | 66.7 | 86.7 |
| | 110_114_155_137_2 | 0.852 | 83.3 | 64.0 | 0.84 | 66.7 | 80.0 |
| | 110_114_3_2_142 | 0.826 | 80.0 | 76.0 | 0.88 | 80.0 | 80.0 |
| | 110_114_142_135_3 | 0.822 | 66.7 | 80.0 | 0.88 | 73.3 | 86.7 |
| | 110_114_127_1_149 | 0.815 | 72.0 | 70.8 | 0.889 | 86.7 | 86.7 |
| | 110_114_127_1_135 | 0.814 | 75.0 | 70.8 | 0.902 | 86.7 | 73.3 |
| | 110_114_155_68_152 | 0.809 | 54.2 | 87.5 | 0.853 | 60.0 | 86.7 |
| | 110_114_68_135_2 | 0.809 | 66.7 | 83.3 | 0.88 | 73.3 | 86.7 |
| | 110_114_142_1_2 | 0.808 | 76.0 | 75.0 | 0.893 | 73.3 | 86.7 |

As described above, at least, polynucleotides consisting of the nucleotide sequences shown by SEQ ID NOs: 1 to 3, 68, 110, 111, 114, 127, 135, 137, 142, 149, 152, and 155 were shown to be capable of discriminating a subject with hippocampal atrophy from a subject with no hippocampal atrophy when used in combinations of two or more.

[Example 4]

< Discrimination of patients with hippocampal atrophy with the use of a small number (5 or 6) of miRNA markers in combination (discrimination by Fisher's discriminant analysis) >

In Example 3, discriminants were prepared for a combination of 2 to 5 miRNA markers by the LASSO method. In this Example, in order to further improve performance of discriminating between two groups, discrimination was performed by Fisher's discriminant analysis.

In Examples 4-(1) and 4-(2) below, the hippocampal atrophy group 1 (132 samples with the volume ratio of the hippocampus to the whole brain of 0.60% or less) and the hippocampal non-atrophy group (132 samples with the volume ratio of the hippocampus to the whole brain of 0.64% or more) used in Example 2-(1) were used to search for miRNA markers capable of determining an extent of hippocampal atrophy. From each of the hippocampal atrophy group and the hippocampal non-atrophy group, 111 samples accounting for 5/6 of total samples were allocated to the training and cross validation cohort, and the remaining 22 samples as the other 1/6 were allocated to the independent validation cohort (Table 9).

Table 9

| Subject's disease type | Total number of samples | Example 4 | |
|---|---|---|---|
| | | Training and cross validation cohort | Independent validation cohort |
| Hippocampal atrophy group | 132 | 110 | 22 |
| Hippocampal non-atrophy group | 132 | 110 | 22 |
| Total | 264 | 220 | 44 |

The division of the training and cross validation cohortinto the training cohort and the cross validation cohort, and the construction of the discriminants were performed in the same manner as in Example 3.

(1) When a combination of five markers is used

With the use of the training and cross validation cohort, similar markers that are highly correlated between the miRNA expression levels were eliminated by the LASSO method, and miRNA markers with the P values ranked in the top 20 were selected. Of the selected 20 miRNA markers, 5 miRNA markers were used in all possible combinations to construct discriminants for hippocampal atrophy by Fisher's discriminant analysis. In order to evaluate discrimination performance of the discriminants constructed using the training and cross validation cohort, accuracy, sensitivity, and specificity were calculated, and further, the discrimination performance of the discriminants was validated using the independent validation cohort.

Table 10 shows combinations of miRNA markers (shown by SEQ ID NOs:) used in the discriminants exhibiting the AUC of 0.8 or more in the cross validation cohort and the independent validation cohort among the constructed discriminants, and the AUC, the sensitivity, and the specificity calculated for each group. As an example for the use of five markers, a discriminant was constructed using the expression levels of miRNAs consisting of the nucleotide sequences shown by SEQ ID NOs: 1, 110, 114, 125, and 126 in the training and cross validation cohort, of which the threshold discriminant score to discriminate the hippocampal atrophy group from the hippocampal non-atrophy group was set at 0 (zero). When the probability of correct classification for hippocampal atrophy is calculated using the discriminant, the AUC of 0.855, the sensitivity of 80.0%, and the specificity of 80.0% were shown as the discrimination performance in the independent validation cohort.

(2) When a combination of six markers is used

Whether or not discrimination performance of the discriminant would be improved when the number of markers is increased to 6 was examined. With the use of the training and cross validation cohort, similar markers that are highly correlated between the miRNA expression levels were eliminated by the LASSO method, and miRNA markers with the P values ranked in the top 40 were selected. Other procedures were performed in the same manner as in Example 4-(1).

Table 10 shows combinations of miRNA markers (shown by SEQ ID NOs:) used in the discriminants exhibiting AUC of 0.8 or more in the cross validation cohort and the independent validation cohort among the constructed discriminants, and AUC, sensitivity, and specificity calculated for each group. As an example for the use of six markers, a discriminant was constructed using the expression levels of miRNAs consisting of the nucleotide sequences shown by SEQ ID NOs: 26, 66, 110, 112, 114, and 165 in the training and cross validation cohort, of which the threshold discriminant score to discriminate the hippocampal atrophy group from the hippocampal non-atrophy group was set at 0 (zero). When the probability of correct classification for hippocampal atrophy is calculated using the discriminant, the AUC of 0.945, the sensitivity of 80.0%, and the specificity of 95.0%, were shown as the discrimination performance in the independent validation cohort.

Table 10

| Number of markers | SEQ ID NO: | Training and cross validation cohort | | | Independent validation cohort | | |
|---|---|---|---|---|---|---|---|
| | | AUC | Sensitivity (%) | Specificity (%) | AUC | Sensitivity (%) | Specificity (%) |
| 5 | 127_1_162_114_110 | 0.802 | 75.0 | 70.0 | 0.812 | 75.0 | 75.0 |
| | 1_126_125_114_110 | 0.818 | 75.0 | 70.0 | 0.855 | 80.0 | 80.0 |
| 6 | 66_110_165_65_166_164 | 0.942 | 85.0 | 85.0 | 0.898 | 70.0 | 90.0 |
| | 66_110_165_26_112_114 | 0.902 | 75.0 | 95.0 | 0.945 | 80.0 | 95.0 |
| | 1_110_165_65_164_71 | 0.935 | 85.0 | 95.0 | 0.92 | 60.0 | 100.0 |

As described above, at least, polynucleotides consisting of the nucleotide sequences shown by SEQ ID NOs: 1, 26, 65, 66, 71, 110, 112, 114, 125 to 127, 162, and 164 to 166 were shown to be capable of discriminating a subject with hippocampal atrophy from a subject with no hippocampal atrophy when used in combinations of five or more.

[Example 5]

< Discrimination of patients with hippocampal atrophy with the use of 5 or more miRNA markers in combination with hippocampal atrophy-associated factors (discrimination by logistic regression analysis) >

In this Example, logistic regression analysis was performed by the LASSO method in order to examine a variation of the method in which the number of markers to be used for discrimination was not limited. As hippocampal atrophy-associated factors, the age, the gender, and the number of ApoE4 alleles, were added as explanatory variables to the discriminant, thereby considering improving the discrimination performance.

The same samples used in Example 4 were divided into the same sample groups having the same sample numbersas shown in Table 9. The samples were divided and allocated to the training and cross validation cohort and the independent validation cohort in 30 different ways while randomly shuffling the samples. Logistic regression analysis was performed by the LASSO method using the measured expression levels of 210 miRNAs analyzed in Example 2 in addition to the age, the gender, and the number of ApoE4 alleles of the subjects as explanatory variables to construct discriminants to determine whether or not the hippocampus has become atrophic. The discrimination performance of the constructed discriminants was evaluated by calculating accuracy, sensitivity, and specificity using the training and cross validation cohort. The discrimination performance of the discriminants was further validated using the independent validation cohort.

Table 11 shows combinations of miRNA markers (shown by SEQ ID NOs:) used in the discriminants exhibiting AUC of 0.9 or more in the cross validation cohort and the independent validation cohort among the constructed discriminants, and AUC, sensitivity, and specificity calculated for each group. For example, the discriminant was constructed using, as explanatory variables, the age, the gender, and the number of ApoE4 alleles, as well as the expression levels of miRNAs consisting of the nucleotide sequences shown by SEQ ID NOs: 179, 155, 142, 188, 127, 146, 1, 189, 110, 4, 200, 193, 175, 118, 63, 152, 111, 180, 39, and 114 in the training and cross validation cohort, of which the threshold discriminant score to discriminate the hippocampal atrophy group from the hippocampal non-atrophy group was set at 0 (zero). When the probability of correct classification for hippocampal atrophy is calculated using the discriminant, the AUC of 0.986, the sensitivity of 86.4%, and the specificity of 95.5%, were shown as the discrimination performance in the independent validation cohort.

Table 11

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 20 | 155_87_188_127_85 _5_1_110_193_175_ 118_152_133_39_15 9_149_150_114 | 0.922 | 88.9 | 81.1 | 0.909 | 81.8 | 86.4 | Age, Number of ApoE4 alleles |
| 21 | 115_188_127_85_1_ 16_110_41_24_200_ 26_193_175_108_15 2_113_39_159_114 | 0.925 | 89.2 | 78.4 | 0.911 | 72.7 | 95.5 | Age, number of ApoE4 alleles |
| 21 | 155_188_127_146_5 _1_16_110_200_101 _193_175_145_125_ 152_111_39_114 | 0.903 | 89.2 | 81.1 | 0.913 | 86.4 | 77.3 | Age, gender, number of ApoE4 alleles |
| 23 | 155_87_188_127_85 _1_183_196_14_189 _110_24_101_193_1 75_118_178_152_39 _150_114 | 0.91 | 81.1 | 86.1 | 0.938 | 81.8 | 90.9 | Age, number of ApoE4 alleles |
| 23 | 179_155_142_188_1 27_146_1_189_110_ 4_200_193_175_118 _63_152_111_180_3 9_114 | 0.908 | 91.7 | 80.6 | 0.986 | 86.4 | 95.5 | Age, gender, number of ApoE4 alleles |
| 24 | 87_188_127_85_5_1 _16_196_14_38_110 _4_200_193_175_10 9_145_152_39_159_ 149_114 | 0.947 | 83.8 | 94.6 | 0.932 | 81.8 | 86.4 | Age, number of ApoE4 alleles |
| 25 | 155_87_142_188_12 7_85_5_1_189_154_ 110_41_193_175_16 7_10_152_111_133_ 39_171_150_114 | 0.912 | 89.2 | 78.4 | 0.911 | 86.4 | 86.4 | Age, ApoE4 allele number |
| 25 | 179_188_127_5_16_ 154_110_193_175_1 18_100_109_10_88_ 152_39_91_159_149 _82_104_182_114 | 0.939 | 91.9 | 70.3 | 0.901 | 81.8 | 81.8 | Age, number of ApoE4 alleles |
| 25 | 155_188_127_1_183 _187_110_185_86_1 93_175_109_145_88 _3_120_152_133_39 _149_182_114 | 0.924 | 81.1 | 89.2 | 0.926 | 81.8 | 90.9 | Age, gender, number of ApoE4 alleles |
| 25 | 155_87_142_188_12 7_85_1_16_110_26_ 193_175_118_97_17 4_152_111_133_113 _39_159_114 | 0.932 | 89.2 | 91.7 | 0.903 | 86.4 | 77.3 | Age, gender, number of ApoE4 alleles |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 26 | 155_87_188_127_1_ 110_9_24_200_193_ 43_109_145_125_17 8_135_174_152_133 _39_91_149_182_11 4 | 0.908 | 77.8 | 75.7 | 0.932 | 86.4 | 86.4 | Age, number of ApoE4 alleles |
| 26 | 155_87_127_5_1_19 8_189_110_41_200 _ 193_175_75_31_109 _59_174_152_111_1 33_113_39_91_114 | 0.914 | 80.6 | 86.5 | 0.903 | 90.9 | 81.8 | Age, number of ApoE4 alleles |
| 26 | 155_87_188_127_5_ 1_16_187_110_176_ 193_175_145_125_8 8_152_89_141_133_ 39_149_150_114 | 0.901 | 81.1 | 75.7 | 0.924 | 81.8 | 81.8 | Age, gender, number of ApoE4 alleles |
| 27 | 179_95_188_127_85 _5_129_16_196_154 _110_200_193_175_ 118_63_174_108_11 3_39_91_159_34_11 4 | 0.911 | 62.2 | 97.2 | 0.963 | 90.9 | 90.9 | Age, gender, number of ApoE4 alleles |
| 27 | 155_87_188_127_85 _5_1_16_196_198_3 8_110_24_200_193_ 88_96_152_111_39_ 159_149_182_114 | 0.915 | 83.3 | 91.9 | 0.913 | 77.3 | 86.4 | Age, gender, number of ApoE4 alleles |
| 28 | 179_121_155_87_18 8_127_5_187_14_18 9_194_110_58_200_ 193_118_145_125_5 9_152_111_113_39_ 159_149_114 | 0.941 | 91.7 | 78.4 | 0.919 | 81.8 | 86.4 | Age, number of ApoE4 alleles |
| 28 | 121_155_87_188_12 7_1_189_67_110_4_ 176_193_75_145_12 5_88_59_108_152_1 11_39_159_149_182 _114_23 | 0.917 | 80.6 | 80.6 | 0.913 | 81.8 | 90.9 | Age, number of ApoE4 alleles |
| 28 | 155_137_87_168_18 8_196_189_110_41_ 4_24_58_101_193_1 75_145_152_111_11 3_39_91_61_51_55_ 182_114 | 0.91 | 73.0 | 94.6 | 0.921 | 77.3 | 95.5 | Age, number of ApoE4 alleles |
| 28 | 155_87_188_127_85 _1_16_84_187_110_ 4_26_193_175_118_ 109_88_108_152_11 1_39_159_149_182_ 114 | 0.908 | 83.8 | 86.1 | 0.907 | 77.3 | 95.5 | Age, gender, number of ApoE4 alleles |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 28 | 179_155_132_168_1 88_127_5_1_14_189 _110_124_4_24_200 _193_175_97_145_1 74_108_152_133_39 _114 | 0.927 | 89.2 | 72.2 | 0.907 | 90.9 | 77.3 | Age, gender, number of ApoE4 alleles |
| 29 | 142_188_85_146_1_ 196 187 189 110 5 8_101_193_175_25_ 105_54_120_152_11 1_113_39_91_159_1 49_61_182_114 | 0.918 | 86.1 | 83.8 | 0.934 | 95.5 | 63.6 | Age, number of ApoE4 alleles |
| 29 | 121_155_95_188_12 7_80_198_189_110_ 200_101_193_175_7 5_145_88_120_152_ 133_39_91_149_34_ 171_140_150_114 | 0.911 | 83.8 | 81.1 | 0.924 | 90.9 | 81.8 | Age, number of ApoE4 alleles |
| 29 | 155_188_127_85_1_ 16_196_84_53_189_ 110_4_68_101_193_ 145_178_54_108_15 2_111_133_39_159_ 182_114 | 0.944 | 86.1 | 91.9 | 0.909 | 77.3 | 81.8 | Age, gender, number of ApoE4 alleles |
| 29 | 121_115_155_137_1 88_127_5_1_196_35 _187_110_193_175_ 118_97_178_48_37_ 152_133_39_91_149 _182_114 | 0.938 | 86.5 | 83.3 | 0.901 | 77.3 | 90.9 | Age, gender, number of ApoE4 alleles |
| 30 | 155_87_188_127_85 _1_16_196_187_110 _24_200_185_101_1 75_118_192_145_88 _54_108_152_133_3 9_91_149_182_114 | 0.921 | 81.1 | 89.2 | 0.94 | 86.4 | 90.9 | Age, number of ApoE4 alleles |
| 30 | 115_155_87_188_12 7_146_5_16_161_53 _198_110_143_148_ 193_175_25_100_10 9_97_48_108_152_1 33 39 149 114 | 0.915 | 91.9 | 78.4 | 0.905 | 72.7 | 77.3 | Age, gender, number of ApoE4 alleles |
| 31 | 155_87_142_188_85 _1_16_196_53_110_ 9_41_4_185_193_17 5_97_135_174_73_1 52_111_133_113_39 _149_55_182_114 | 0.908 | 88.9 | 78.4 | 0.917 | 86.4 | 81.8 | Age, number of ApoE4 alleles |
| 31 | 121_155_188_127_8 5_1_16_198_189_11 0_4_86_101_193_75 _199_145_108_152_ 111_113_39_7_91_1 59_149_182_114 | 0.954 | 83.3 | 89.2 | 0.911 | 86.4 | 86.4 | Age, gender, number of ApoE4 alleles |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 31 | 155_142_188_127_5 _129_16_14_154_11 0_153_193_175_118 _75_109_169_97_21 _10_59_152_111_13 3_39_149_18_114 | 0.904 | 91.7 | 78.4 | 0.905 | 81.8 | 81.8 | Age, gender, number of ApoE4 alleles |
| 32 | 179_155_188_127_5 _196_187_14_189_1 54_38_110_101_175 _118_25_100_174_1 20_108_152_133_39 _91_159_149_82_10 4_182_114 | 0.954 | 88.9 | 83.8 | 0.911 | 72.7 | 95.5 | Age, number of ApoE4 alleles |
| 32 | 155_188_127_79_16 _198_110_68_200_1 02_193_175_43_100 _109_167_10_59_19 1_152_111_133_39_ 159_149_82_55_182 _114 | 0.903 | 86.5 | 75.7 | 0.911 | 90.9 | 77.3 | Age, gender, number of ApoE4 alleles |
| 32 | 155_95_87_188_127 _85_1_196_198_187 _189_110_200_193_ 175_192_109_97_14 5_125_59_152_133_ 113_39_159_149_15 0_114 | 0.925 | 81.1 | 83.3 | 0.903 | 81.8 | 77.3 | Age, gender, number of ApoE4 alleles |
| 33 | 121_155_188_127_1 _198_187_189_154_ 110_193_175_118_3 1_109_145_59_54_1 74_108_152_133_11 3_39_91_159_149_2 _55_182_114 | 0.957 | 81.1 | 94.6 | 0.907 | 68.2 | 90.9 | Age, number of ApoE4 alleles |
| 33 | 121_155_87_188_12 7_85_1_79_16_196_ 187_110_176_24_10 1_193_175_109_199 _145_178_88_152_1 11_133_39_149_150 _182_114_23 | 0.914 | 80.6 | 83.8 | 0.917 | 81.8 | 77.3 | Age, number of ApoE4 alleles |
| 33 | 155_188_127_1_196 _189_110_32_41_14 3_185_193_175_97_ 145_178_54_174_10 8_152_111_133_39_ 91_149_61_18_55_1 82_114 | 0.937 | 89.2 | 80.6 | 0.959 | 81.8 | 90.9 | Age, gender, number of ApoE4 alleles |
| 33 | 121_155_95_188_12 7_196_53_187_189_ 194_110_128_101_1 93_175_25_109_97_ 145_174_152_111_1 13_39_91_159_149_ 104_182_114 | 0.923 | 83.8 | 91.9 | 0.919 | 81.8 | 95.5 | Age, gender, number of ApoE4 alleles |
| 33 | 155_137_168_188_1 27_16_196_187_154 _110_6_44_9_200_1 48_101_193_175_10 9_145_178_48_152_ 133_113_39_77_55_ 182_114 | 0.901 | 75.7 | 86.5 | 0.901 | 77.3 | 81.8 | Age, gender, number of ApoE4 alleles |

(continued)

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 33 | 155_87_188_127_85_1_16_196_84_17_14_110_176_24_200_193_175_75_109_97_145_174_108_152_141_39_159_149_182_114 | 0.907 | 70.3 | 86.5 | 0.926 | 68.2 | 100.0 | Age, gender, number of ApoE4 alleles |
| 33 | 155_87_188_127_85_1_16_196_14_110_176_185_193_175_118_109_199_145_48_108_152_141_133_39_159_149_55_182_114_23 | 0.93 | 86.5 | 91.9 | 0.955 | 86.4 | 90.9 | Age, gender, number of ApoE4 alleles |
| 33 | 179_121_155_95_188_127_85_1_129_196_187_110_176_193_175_31_145_178_48_88_47_54_174_120_108_133_39_91_149_114 | 0.936 | 86.5 | 88.9 | 0.905 | 77.3 | 77.3 | Age, gender, number of ApoE4 alleles |
| 34 | 179_115_155_87_188_127_85_146_5_1_45_84_187_189_110_128_56_200_185_86_193_175_118_145_48_59_152_113_39_159_149_114 | 0.953 | 86.1 | 91.9 | 0.917 | 77.3 | 81.8 | Age, number of ApoE4 alleles |
| 34 | 179_95_142_188_127_5_1_129_16_187_110_193_175_118_100_109_97_125_63_92_152_133_180_39_91_159_149_104_171_150_182_114 | 0.903 | 77.8 | 81.1 | 0.928 | 81.8 | 90.9 | Age, number of ApoE4 alleles |
| 34 | 155_137_142_188_127_85_196_189_110_136_41_143_148_193_175_118_199_97_145_178_88_117_108_133_39_91_159_149_18_182_114 | 0.928 | 81.1 | 83.3 | 0.917 | 68.2 | 90.9 | Age, gender, number of ApoE4 alleles |
| 34 | 13_155_137_87_188_127_85_196_187_110_136_41_24_200_101_193_175_118_97_14548_152_111_133_113_39_91_149_55_182_114 | 0.936 | 81.1 | 94.6 | 0.905 | 72.7 | 95.5 | Age, gender, number of ApoE4 alleles |
| 34 | 155_188_127_85_5_1_79_84_187_67_110_41_176_175_118_109_97_145_54_108_152_111_133_113_39_91_159_149_171_182_114 | 0.963 | 86.5 | 91.9 | 0.915 | 72.7 | 95.5 | Age, gender, number of ApoE4 alleles |
| 34 | 155_87_188_127_85_146_1_16_196_187_14_110_200_26_101_193_175_97_145_48_120_108_152_141_111_39_159_149_77_182_114 | 0.925 | 86.5 | 86.5 | 0.921 | 81.8 | 72.7 | Age, gender, number of ApoE4 alleles |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 34 | 155_168_188_127_8 5_5_1_16_196_84_1 87_189_110_128_13 6_176_200_193_175 _118_109_145_125_ 152_89_133_39_149 _46_182_114 | 0.955 | 83.3 | 94.6 | 0.903 | 72.7 | 90.9 | Age, gender, number of ApoE4 alleles |
| 34 | 179_121_115_155_1 88_127_1_16_196_1 61_84_67_110_124_ 4_176_24_148_193_ 175_109_145_178_4 8_117_152_39_159_ 149_182_114 | 0.92 | 89.2 | 86.1 | 0.946 | 81.8 | 86.4 | Age, gender, number of ApoE4 alleles |
| 34 | 115_95_87_188_127 _85_5_1_16_196_19 8_187_110_176_101 _193_175_118_109_ 97_125_178_120_10 8_152_141_39_159_ 149_182_114 | 0.9 | 78.4 | 81.1 | 0.961 | 77.3 | 95.5 | Age, gender, number of ApoE4 alleles |
| 34 | 179_87_188_127_85 _30_1_84_198_187_ 110_193_175_109_1 45_125_54_174_120 _163_152_133_113_ 39_91_159_149_82_ 55_182_114 | 0.955 | 86.5 | 91.9 | 0.911 | 81.8 | 81.8 | Age, gender, number of ApoE4 alleles |
| 34 | 115_155_137_168_1 88_127_5_1_79_196 _187_189_194_110_ 136_193_175_118_1 09_199_145_88_152 _133_39_91_149_18 _46_182_114 | 0.911 | 73.0 | 97.2 | 0.95 | 72.7 | 90.9 | Age, gender, number of ApoE4 alleles |
| 35 | 121_155_188_127_8 0.95 5_1_196_187_110_1 76_24_200_26_101_ 193_175_118_25_10 0_169_145_125_178 _54_92_152_111_11 3_39_91_159_55_11 4 | 0.95 | 86.1 | 91.9 | 0.94 | 86.4 | 86.4 | Age, number of ApoE4 alleles |
| 35 | 155_95_188_127_85 _1_16_196_187_189 _110_136_176_69_1 01_193_175_118_25 _75_109_97_167_14 5_108_152_111_39_ 159_149_171_182_1 14 | 0.924 | 83.8 | 83.8 | 0.926 | 86.4 | 86.4 | Age, number of ApoE4 alleles |
| 35 | 121_115_155_168_1 88_127_85_5_1_84_ 198_187_14_189_11 0_193_109_97_145_ 54_120_152_111_11 3_39_91_159_149_1 04_150_182_114_12 | 0.914 | 78.4 | 89.2 | 0.901 | 81.8 | 86.4 | Age, number of ApoE4 alleles |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 35 | 155_188_127_85_14 6_5_1_80_183_196_ 189_110_177_193_1 75_109_169_97_167 _145_59_174_152_1 11_133_39_91_149_ 82 103 52 114 | 0.935 | 91.9 | 83.3 | 0.936 | 86.4 | 90.9 | Age, gender, number of ApoE4 alleles |
| 35 | 121_168_188_127_8 5_16_45_198_187_1 89_110_4_176_200_ 148_86_193_175_97 _145_125_174_152_ 113_39_91_159_149 _70_82_182_114 | 0.962 | 91.7 | 91.9 | 0.915 | 90.9 | 90.9 | Age, gender, number of ApoE4 alleles |
| 35 | 121_155_188_127_8 5_5_1_129_187_194 _110_176_24_86_26 _193_118_100_125_ 54_174_120_152_13 3_39_91_149_104_1 71_150_182_114 | 0.908 | 83.3 | 83.8 | 0.915 | 81.8 | 86.4 | Age, gender, number of ApoE4 alleles |
| 36 | 155_142_188_127_8 5_1_80_183_196_18 7_110_124_200_101 _193_25_97_145_12 5_59_54_174_120_1 08_152_133_39_91_ 159_82_104_61_150 _114 | 0.924 | 89.2 | 86.1 | 0.957 | 86.4 | 90.9 | Age, number of ApoE4 alleles |
| 36 | 121_155_95_87_188 _127_85_16_196_18 7_194_110_136_176 _200_185_101_193_ 175_109_145_59_10 8_152_111_113_39_ 91_159_149_46_55_ 182_114 | 0.902 | 86.1 | 83.8 | 0.903 | 77.3 | 86.4 | Age, number of ApoE4 alleles |
| 36 | 155_87_142_188_12 7_85_146_5_1_16_4 5_196_67_110_101_ 193_175_118_25_10 9_145_178_63_54_1 74_108_152_39_91_ 159_149_55_182_11 4 | 0.944 | 86.5 | 91.9 | 0.946 | 68.2 | 86.4 | Age, number of ApoE4 alleles |
| 36 | 155_188_127_85_5_ 1_138_196_84_187_ 110_24_148_177_10 1_193_175_100_145 _178_88_54_71_152 _133_39_91_149_82 _61_51_55_182_114 | 0.938 | 86.5 | 88.9 | 0.907 | 81.8 | 90.9 | Age, number of ApoE4 alleles |

123

EP 4 130 259 A1

(continued)

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 36 | 115_155_87_168_18 8_127_85_1_16_196 _187_110_124_41_1 76_24_148_153_193 _175_109_145_10_8 8_59_120_152_133_ 39_91_149_2_104_1 82_114 | 0.902 | 83.8 | 77.8 | 0.917 | 81.8 | 81.8 | Age |
| 36 | 87_188_127_1_79_1 83_196_187_189_11 0_6_32_24_148_86_ 193_175_118_100_1 09_88_135_108_92_ 152_111_113_39_14 9_55_150_182_114 | 0.9 | 91.9 | 73.0 | 0.901 | 81.8 | 90.9 | Age, gender, number of ApoE4 alleles |
| 36 | 121_155_142_188_1 27_85_1_16_172_53 _187_110_101_193_ 118_75_109_199_97 _145_125_63_88_54 _152_42_39_159_14 9_82_55_114_23 | 0.901 | 83.8 | 83.8 | 0.932 | 86.4 | 90.9 | Age, gender, number of ApoE4 alleles |
| 36 | 155_188_127_85_14 6_5_1_196_84_187_ 110_200_193_175_1 18_75_97_145_125_ 178_59_54_106_163 _152_133_39_91_15 9_82_34_182_114 | 0.934 | 88.9 | 81.1 | 0.965 | 81.8 | 95.5 | Age, gender, number of ApoE4 alleles |
| 36 | 121_95_188_127_85 _5_79_84_187_189_ 110_4_193_175_119 _25_97_145_10_88_ 108_152_111_133_1 13_39_91_159_149_ 171_150_182_114 | 0.913 | 81.1 | 83.3 | 0.905 | 81.8 | 81.8 | Age, gender, number of ApoE4 alleles |
| 36 | 115_155_188_127_8 5_1_16_196_161_18 7_189_110_176_24_ 101_193_175_118_3 1_199_145_48_54_1 52_111_133_39_149 _82_104_51_182_11 4 | 0.947 | 83.8 | 91.7 | 0.903 | 72.7 | 86.4 | Age, gender, number of ApoE4 alleles |
| 36 | 179_13_155_87_188 _127_85_1_79_183_ 189_110_81_24_193 _175_199_167_125_ 88_59_174_108_152 _111_113_39_159_1 49_70_150_114_23 | 0.904 | 81.1 | 86.1 | 0.907 | 77.3 | 95.5 | Age, gender, number of ApoE4 alleles |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 37 | 155_87_188_127_85 _1_16_196_161_84_ 187_14_38_124_109 _145_178_48_54_96 _174_120_108_152_ 141_39_91_159_149 _82_77_55_182_93_ 114 | 0.912 | 83.8 | 86.5 | 0.928 | 90.9 | 81.8 | Age, number of ApoE4 alleles |
| 37 | 155_87_168_188_12 7_85 _ 1_ 196 _187 _ 11 0_136_24_200_148_ 193_175_118_109_1 - 78_120_108_152_11 1_133_39_91_159_1 49_82_171_18_55_1 82_114 | 0.951 | 83.8 | 89.2 | 0.94 | 72.7 | 100.0 | Age, gender, number of ApoE4 alleles |
| 37 | 155_87_142_188_12 7_85_80_161_84_18 7_189_194_110_101 _193_175_25_199_9 7_145_120_152_111 _133_39_91_159_14 9_103_77_184_182_ 114_23 | 0.905 | 75.7 | 86.1 | 0.901 | 95.5 | 72.7 | Age, gender, number of ApoE4 alleles |
| 37 | 155_95_87_188_85_ 5_1_196_84_53_187 _110_90_176_148_1 93_175_118_25_145 _48_88_96_174_108 _152_133_39_91_14 9_171_55_182_114 | 0.932 | 78.4 | 77.8 | 0.911 | 77.3 | 90.9 | Age, gender, number of ApoE4 alleles |
| 37 | 155_95_188_127_85 _146_1_45_196_187 _189_194_110_128_ 68_200_86_177_101 _193_175_118_109_ 199_37_92_152_133 _39_159_149_104_1 82_114 | 0.914 | 83.8 | 94.6 | 0.932 | 77.3 | 95.5 | Age, gender, number of ApoE4 alleles |
| 37 | 121_155_95_168_18 8_127_5_16_196_17 2_53_187_14_189_3 8_67_110_68_193_1 75_145_178_174_15 2_133_39_91_159_1 49_82_20_182_114_ 12 | 0.936 | 81.1 | 91.7 | 0.928 | 77.3 | 90.9 | Age, gender, number of ApoE4 alleles |
| 38 | 95_188_127_85_1_1 6_196_161_14_110_ 32_24_175_118_100 _109_97_145_178_1 0_59_54_108_92_11 1_133_180_113_39_ 91_159_149_184_18 2_114_40 | 0.909 | 81.1 | 86.5 | 0.934 | 86.4 | 81.8 | Age, number of ApoE4 alleles |

(continued)

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 38 | 155_95_87_142_188 _127_85_5_1_196_8 4_198_187_110_41_ 68_200_177_101_19 3_175_118_25_109_ 97_145_152_111_13 3_113_39_159_149_ 55_182_114 | 0.936 | 81.1 | 86.5 | 0.919 | 77.3 | 90.9 | Age, number of ApoE4 alleles |
| 38 | 121_155_188_127_8 5_1_79_16_196_187 _189_110_124_193_ 175_75_169_97_145 _178_21_63_54_108 _71_152_111_133_3 9_91_159_149_55_1 14_23 | 0.941 | 83.8 | 91.7 | 0.977 | 86.4 | 95.5 | Age, gender, number of ApoE4 alleles |
| 38 | 121_155_188_127_8 5_5_1_79_160_84_1 87_14_110_32_176_ 200_193_175_118_1 09_97_145_178_63_ 10_88_152_111_133 _39_91_149_77_182 _114 | 0.944 | 86.5 | 91.9 | 0.919 | 90.9 | 86.4 | Age, gender, number of ApoE4 alleles |
| 38 | 13_155_87_168_188 _127_85_196_187_1 89_194_110_78_24_ 200_177_101_193_1 75_118_109_125_17 4_108_73_152_111 133_39_159_149_17 1_52_182_114 | 0.929 | 75.7 | 94.4 | 0.921 | 68.2 | 95.5 | Age, gender, number of ApoE4 alleles |
| 39 | 121_137_142_188_1 27_85_5_1_196_84_ 187_189_38_110_4_ 185_177_101_193_1 99_145_88_3_174_1 52_111_133_180_11 3_39_91_159_149_1 71_140_182_114 | 0.917 | 78.4 | 91.7 | 0.903 | 77.3 | 90.9 | Age, number of ApoE4 alleles |
| 39 | 121_95_188_127_85 _5_1_196_187_189_ 194_110_128_4_26_ 177_101_193_175_3 1_109_97_145_62_8 8_108_152_111_133 _39_91_159_149_82 _171_182_114 | 0.918 | 83.8 | 86.1 | 0.955 | 77.3 | 95.5 | Age, number of ApoE4 alleles |
| 39 | 121_188_127_85_30 _1_16_196_187_189 _11_154_194_110_5 8_185_101_193_175 _118_25_100_88_54 _152_111_180_39_9 1_159_61_77_20_55 _182_114_23 | 0.912 | 77.8 | 89.2 | 0.93 | 95.5 | 63.6 | Age, number of ApoE4 alleles |

EP 4 130 259 A1

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 39 | 155_87_188_127_1_ 138_16_196_187_15 4_38_110_200_101_ 193_175_25_100_19 9_145_178_96_174_ 92_152_111_133_11 3_39_91_159_149_8 2_77_182_114_23 | 0.911 | 86.1 | 86.5 | 0.963 | 95.5 | 81.8 | Age, number of ApoE4 alleles |
| 39 | 87_142_188_127_85 _146_5_1_196_35_1 89_38_67_110_9_4_ 177_101_193_175_1 18_109_145_63_88_ 54_108_133_39_91_ 159_149_72_82_51_ 182_114 | 0.932 | 89.2 | 86.5 | 0.921 | 72.7 | 95.5 | Age, number of ApoE4 alleles |
| 39 | 155_137_188_127_1 _16_196_161_35_18 7_189_38_67_110_1 81_200_148_101_19 3 _ 175 _118 _109 _ 145 _125_174_120_152_ 141_111_133_39_91 _159_82_55_114 | 0.921 | 81.1 | 86.1 | 0.965 | 95.5 | 90.9 | Age, gender, number of ApoE4 alleles |
| 39 | 155_132_87_188_12 7_1_196_161_53_19 8_187_189_110_181 _136_4_176_143_20 0_26_193_175_75_9 7_145_54_174_37_1 63_152_111_39_70_ 55_182_114 | 0.91 | 86.5 | 75.0 | 0.905 | 86.4 | 68.2 | Age, gender, number of ApoE4 alleles |
| 39 | 179_121_155_188_1 27_5_1_79_16_196_ 187_14_67_110_176 _24_143_86_175_10 9_145_48_88_47_54 _174_120_152_133_ 39_91_159_149_82_ 182_114 | 0.907 | 81.1 | 86.5 | 0.938 | 77.3 | 86.4 | Age, gender, number of ApoE4 alleles |
| 40 | 121_87_142_188_12 7_85_45_196_187_1 89_194_110_176_24 _102_86_101_193_1 18_100_109_145_88 _174_108_71_99_15 2_141_111_133_39_ 91_159_149_182_11 4_23 | 0.944 | 86.5 | 91.9 | 0.903 | 81.8 | 86.4 | Age, number of ApoE4 alleles |
| 40 | 121_155_87_188_12 7_1_16_198_187_18 9_38_67_194_110_4 _176_24_200_185_1 93_175_118_192_10 9_167_145_125_63_ 120_108_152_133_1 80_39_91_159_149_ 182 | 0.918 | 78.4 | 86.5 | 0.944 | 81.8 | 95.5 | Age, number of ApoE4 alleles |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 40 | 121_115_155_137_1 68_188_127_85_1_ 7 9_138_16_196_187 _ 67_110_136_4_101_ 193_175_118_109_9 7_145_48_54_120_1 08_152_133_39_91_ 159_149_18_182_11 4 | 0.915 | 81.1 | 91.9 | 0.94 | 81.8 | 86.4 | Age, number of ApoE4 alleles |
| 40 | 137_188_127_85_14 6_1_16_196_161_18 9_110__ 56_58_148_1 93_175_118_25_100 _109_145_178_48_ 1 74_120_108_152_14 1_133_39_91_149_8 2_77_55_150_114 | 0.936 | 86.5 | 83.8 | 0.957 | 81.8 | 90.9 | Age, gender, number of ApoE4 alleles |
| 40 | 155_87_168_188_12 7_85_1_196_84_189 _110_124_176_24_2 00_185_69_175_25_ 169_145_125_178_9 6_120_117_108_152 _111_133_39_91_15 9_149_82_182_114 | 0.944 | 88.9 | 91.9 | 0.932 | 86.4 | 95.5 | Age, gender, number of ApoE4 alleles |
| 40 | 155_95_188_127_85 _5_1_16_187_194_1 10_9_41_4_153_193 _175_100_109_145_ 62_21_48_10_54_15 2_111_133_39_91_1 97_159_149_104_17 1_182_114 | 0.938 | 83.8 | 91.7 | 0.909 | 77.3 | 86.4 | Age, gender, number of ApoE4 alleles |
| 41 | 87_188_127_85_5_1 _80_196_189_154_1 10_124_78_186_90_ 177 193 175 118 3 1_109_97_145_88_5 9_54_120_108_134_ 152_111_133_39_91 _149_104_140_182_ 114 | 0.908 | 86.5 | 66.7 | 0.942 | 95.5 | 77.3 | Age, number of ApoE4 alleles |
| 41 | 179_155_95_188_12 7_146_5_129_16_19 6_84_187_14_189_1 10_32_68_26_193_1 75_109_145_178_59 _54_174_120_152_1 33_39_91_149_82_1 04_61_182_93_114_ 23 | 0.904 | 81.1 | 83.8 | 0.911 | 86.4 | 81.8 | Age, number of ApoE4 alleles |
| 41 | 121_115_155_87_18 8_127_85_1_80_161 _187_14_110_4_24_ 200_193_109_97_14 5 125 178 63 54 1 74_152_111_133_39 _91_159_149_70_82 _61_55_182_114 | 0.926 | 75.0 | 89.2 | 0.983 | 90.9 | 95.5 | Age, gender, number of ApoE4 alleles |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 41 | 155_137_95_87_188 _127_85_146_1_196 _189_38_194_110_3 2_176_193_175_118 _109_125_178_63_5 9_54_96_191_174_1 20_152_141_133_39 _91_149_82_55_182 _114 | 0.913 | 80.6 | 86.5 | 0.955 | 86.4 | 90.9 | Age, gender |
| 42 | 179_155_87_142_18 8_127_85_1_80_189 _110_6_56_185_153 _26_193_175_109_6 2_178_10_88_59_13 5_54_108_92_152_1 11_133_180_113_39 _91_159_149_150_1 14_60 | 0.924 | 81.1 | 86.1 | 0.932 | 90.9 | 81.8 | Age, number of ApoE4 alleles |
| 42 | 179_115_188_127_8 5_1_79_183_196_53 _187_189_194_110_ 200_185_193_175_1 19_105_97_145_125 _88_54_120_152_11 3_39_91_116_159_1 49_34_61_150_182_ 114_23 | 0.905 | 86.1 | 83.8 | 0.903 | 81.8 | 90.9 | Age, gender, number of ApoE4 alleles |
| 42 | 87_188_127_85_16_ 196_84_14_189_67_ 110_90_24_193_175 _43_100_109_145_4 8_63_10_88_59_54_ 174_108_152_111_1 33_39_91_159_149_ 82_104_61_182_114 | 0.962 | 97.3 | 91.9 | 0.983 | 77.3 | 95.5 | Age, gender, number of ApoE4 alleles |
| 43 | 155_137_142_188_1 27_85_5_1_16_196_ 84_53_187_38_67_1 10_4_176_24_177_1 01_193_175_100_10 9_145_48_88_54_71 _152_89_133_39_91 _159_149_104_182_ 93_114 | 0.925 | 89.2 | 81.1 | 0.93 | 77.3 | 95.5 | Age, number of ApoE4 alleles |
| 44 | 87_188_127_1_80_1 83_45_187_189_110 _128_4_200_102_26 _177_101_193_175_ 145_178_88_96_174 _117_92_152_111_1 33_39_91_159_149_ 82_171_51_55_150_ 182_93_114_12 | 0.942 | 83.8 | 94.4 | 0.911 | 86.4 | 86.4 | Age, number of ApoE4 alleles |
| 44 | 121_155_188_127_8 5_146_196_53_187_ 189_110_32_56_26_ 101_193_175_100_1 09_199_97_145_178 _48_63_10_88_59_5 4_108_152_111_133 _39_7_197_159_149 _82_182_114 | 0.935 | 91.9 | 80.6 | 0.955 | 90.9 | 86.4 | Age, gender, number of ApoE4 alleles |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 44 | 121_155_87_188_12 7_85_1_80_84_198_ 187_189_194_110_2 00_102_86_193_175 _118_75_97_145_12 5_178_88_59_37_92 _134_152_111_39_1 59_149_82_20_51_1 50_182_114 | 0.941 | 81.1 | 91.9 | 0.934 | 86.4 | 90.9 | Age, gender, number of ApoE4 alleles |
| 44 | 137_95_87_142_188 _127_85_1_79_196_ 187_189_67_110_13 6_41_4_24_19_177_ 101_193_118_100_1 05_97_48_54_191_1 74_108_152_111_13 3_39_159_149_171_ 18_182_114 | 0.939 | 86.1 | 91.9 | 0.911 | 77.3 | 86.4 | Age, gender, number of ApoE4 alleles |
| 44 | 95_87_188_127_146 _5_196_161_84_187 _14_189_154_194_1 10_128_176_24_68_ 200_185_193_175_4 3_109_97_145_178_ 48_54_174_92_152_ 113_39_159_149_82 _55_114_23 | 0.916 | 78.4 | 89.2 | 0.915 | 77.3 | 77.3 | Age, gender, number of ApoE4 alleles |
| 44 | 121_155_137_87_18 8_127_85_79_129_ 1 6 84 14 189 67 11 0_124_136_41_176_ 24_68_185_193_175 _109_97_145_178_1 20_73_152_111_133 _113_39_91_159_14 9_150_182_114 | 0.917 | 78.4 | 86.5 | 0.919 | 86.4 | 72.7 | Age, gender, number of ApoE4 alleles |
| 45 | 155_137_168_188_1 27_85_1_80_79_183 _196_172_84_187_1 89_110_41_68_185_ 175_75_100_109_97 _145_178_48_98_88 _135_54_108_92_73 _111_133_39_91_14 9_82_150_114 | 0.923 | 75.7 | 91.9 | 0.907 | 77.3 | 90.9 | Age, gender, number of ApoE4 alleles |
| 45 | 13_155_132_87_168 _188_127_5_1_196_ 84_110_6_124_41_9 0_176_81_24_143_6 8_200_101_193_175 _100_109_169_178_ 47_174_152_111_13 3_39_91_149_82_61 _18_182_114 | 0.911 | 67.6 | 94.4 | 0.907 | 86.4 | 81.8 | Age, gender, number of ApoE4 alleles |
| 46 | 121_115_155_168_1 42_188_127_85_1_1 6_196_161_187_14_ 189_67_110_4_176_ 200_193_175_25_10 0_167_145_178_59_ 54_174_108_152_11 1_133_113_39_91_1 59_82_104_182_114 _23 | 0.932 | 83.3 | 91.7 | 0.973 | 81.8 | 95.5 | Age, gender, number of ApoE4 alleles |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 46 | 179_115_142_188_1 27_146_79_196_161 _187_189_194_110_ 4_68_177_101_193_ 175_100_109_145_4 8_59_174_108_99_1 52_133_113_39_149 _70_82_104_140_61 _103_77_55_182_11 4_50 | 0.909 | 83.8 | 83.8 | 0.909 | 72.7 | 86.4 | Age, gender, number of ApoE4 alleles |
| 48 | 13_121_155_132_95 _87_168_188_127_1 _16_196_84_198_18 9_38_110_124_4_17 6_24_200_102_148_ 185_193_175_25_10 9_145_125_178_10_ 59_37_108_141_111 _133_39_159_149_8 2_150_182_114 | 0.913 | 83.3 | 83.8 | 0.913 | 86.4 | 86.4 | Age, number of ApoE4 alleles |
| 48 | 179_121_95 _188_12 7_85_1_79_16_84_5 3_198_187_38_194_ 110_136_78_4_176_ 193_175_75_100_10 9_97_145_48_88_59 _54_37_152_111_11 3_39_91_159_149_8 2_171_46_150_182_ 114 | 0.917 | 83.8 | 83.3 | 0.934 | 86.4 | 90.9 | Age, gender, number of ApoE4 alleles |
| 48 | 179 115 155 87 16 8_188_127_85_1_16 _196_198_14_110_1 76_24_143_68_200_ 193 175 118 109 9 7_125_178_88_83_5 9_47_54_120_108_1 63_111_133_39_91_ 159_149_82_46_55_ 182_114 | 0.942 | 81.1 | 86.5 | 0.94 | 95.5 | 81.8 | Age, gender, number of ApoE4 alleles |

As miRNA markers used for the discriminants exhibiting AUC of 0.8 or more concerning the discrimination performance in the cross validation cohort and the independent validation cohort, polynucleotides consisting of the nucleotide sequences shown by SEQ ID NOs: 1 to 28, 30 to 64, 67 to 73, 75 to 122, 124, 125, 127 to 156, 158 to 163, and 167 to 200 are selected. It was shown that the hippocampal atrophy group can be discriminated from the hippocampal non-atrophy group when using such polynucleotides in arbitrary combination.

As described above, at least, polynucleotides consisting of the nucleotide sequences shown by SEQ ID NOs: 1 to 7, 9 to 14, 16 to 21, 23 to 26, 30 to 32, 34, 35, 37 to 48, 50 to 56, 58 to 63, 67 to 73, 75, 77 to 93, 95 to 106, 108 to 111, 113 to 121, 124, 125, 127 to 129, 132 to 138, 140 to 143, 145, 146, 148 to 150, 152 to 155, 159 to 161, 163, 167 to 169, 171, 172, 174 to 189, 191 to 194, and 196 to 200 were shown to be capable of discriminating a subject with hippocampal atrophy from a subject with no hippocampal atrophy with high accuracy (AUC of 0.9 or more) when used in combinations of two or more.

[Example 6]

< Discrimination of two groups with more extremely different hippocampal volumes with the use of multiple miRNA markers in combination with hippocampal atrophy-associated factors (discrimination by logistic regression analysis) >

In Example 6, the analysis was performed in terms of the following two objectives. Objective 1: To perform the analysis more precisely than in Example 5 via comparison between two groups exhibiting extents of hippocampal atrophy that are more extremely different from each other, than that in Example 5.

Objective 2: To examine as to whether or not a set of markers exhibiting AUC exceeding 0.9 is obtained even when the number of samples used in training and validation cohorts is decreased when constructing discriminants.

The same samples as used in Example 3, i.e., 88 samples of the hippocampal atrophy group with the volume ratio of the hippocampus to the whole brain of 0.57% or less and 88 samples of the hippocampal non-atrophy group with the volume ratio of the hippocampus to the whole brain of 0.68% or more, were divided into the training and cross validation cohort and the independent validation cohort as shown in Table 7. The dividing of samples into the training and cross validation cohort and the independent validation cohort was carried out in 30 different ways while randomly shuffling the samples. Further, dividing of the samples of the training and cross validation cohort was performed into the training cohort and the cross validation cohort in the same manner as in Example 3. Logistic regression analysis was performed by the LASSO method using the measured expression levels of 210 miRNAs analyzed in Example 2 in addition to the age, the gender, and the number of ApoE4 alleles of the subjects as explanatory variables to construct discriminants to determine whether or not the hippocampus has become atrophic. The discrimination performance of the constructed discriminants was evaluated by calculating accuracy, sensitivity, and specificity using the training and cross validation cohort. The discrimination performance of the discriminants was further validated using the independent validation cohort.

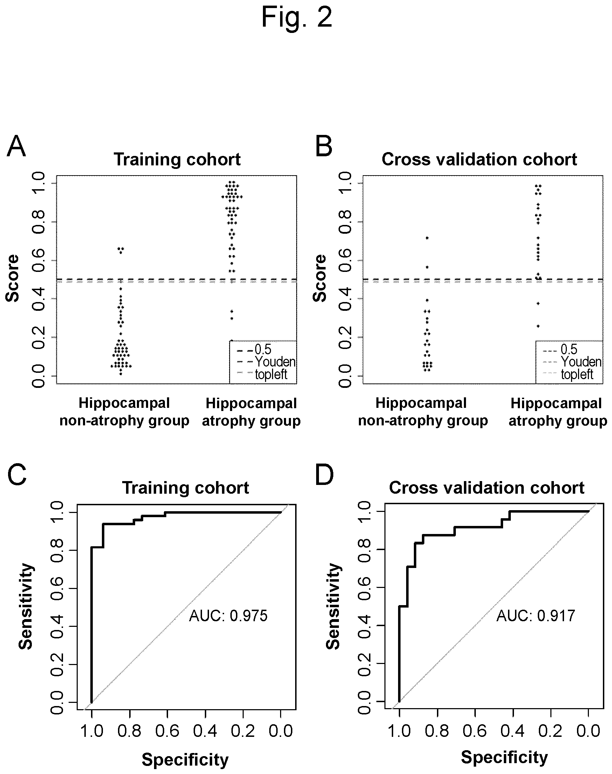

Table 12 shows combinations of miRNA markers (shown by SEQ ID NOs:) used in the discriminants exhibiting AUC of 0.9 or more in the cross validation cohort and the independent validation cohort among the constructed discriminants, and AUC, sensitivity, and specificity calculated for each group. For example, the discriminant was constructed using, as explanatory variables, the age, the gender, and the number of ApoE4 alleles, as well as the expression levels of miRNAs comprising the nucleotide sequences shown by SEQ ID NOs: 155, 137, 188, 127, 196, 154, 110, 128, 100, 152, 133, 159, and 114 in the training and cross validation cohort, of which the threshold discriminant score to discriminate the hippocampal atrophy group from the hippocampal non-atrophy group was set at 0.5. When the probability of correct classification for hippocampal atrophy is calculated using the discriminant, the AUC of 1.0, the sensitivity of 100.0%, and the specificity of 100.0% were shown as the discrimination performance in the independent validation cohort. Fig. 2 shows the results of analysis including the plots of discriminant scores (A, B) and the Receiver Operating Characteristic (ROC) curves (C, D). The discriminant scores range from 0 to 1. The dotted line (a discriminant score of 0.5) in each panel depicts a discriminant boundary for discriminating whether or not the hippocampus has become atrophic. The discriminant scores indicated in the plots of discriminant scores were calculated by assigning the measured miRNA expression levels of the samples and the age, the gender, and the number of ApoE4 alleles into the discriminant constructed with the use of the 13 miRNAs described above (SEQ ID NOs: 155, 137, 188, 127, 196, 154, 110, 128, 100, 152, 133, 159, and 114). In Figs. 2A and 2B, the vertical axis represents a discriminant score and the horizontal axis represents the sample group. An ROC curve was shown with the sensitivity calculated using miRNA markers on the vertical axis and the specificity on the horizontal axis (Figs. 2C and 2D).

Table 12

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 5 | 110_3_114 | 0.907 | 76.0 | 87.5 | 0.92 | 80.0 | 80.0 | Age, number of ApoE4 alleles |
| 5 | 127_1_110 | 0.903 | 83.3 | 83.3 | 0.947 | 80.0 | 100.0 | Age, number of ApoE4 alleles |
| 6 | 1_110_3_114 | 0.933 | 62.5 | 100.0 | 0.92 | 73.3 | 93.3 | Age, number of ApoE4 alleles |
| 7 | 137_1_110_111_114 | 0.948 | 91.7 | 83.3 | 0.987 | 100.0 | 86.7 | Age, number of ApoE4 alleles |
| 7 | 188_5_110_3_114 | 0.901 | 84.0 | 92.0 | 0.96 | 80.0 | 93.3 | Age, number of ApoE4 alleles |
| 8 | 137_127_5_110_145_ 152_114 | 0.902 | 87.5 | 88.0 | 0.907 | 66.7 | 86.7 | Age |
| 9 | 155_142_110_68_3_7 1_114 | 0.922 | 76.0 | 87.5 | 0.92 | 80.0 | 93.3 | Age, number of ApoE4 alleles |
| 9 | 155_127_5_1_154_11 0 | 0.9 | 84.0 | 79.2 | 0.902 | 86.7 | 93.3 | Age, gender, number of ApoE4 alleles |
| 10 | 155_87_1_154_110_7 3_133_149_114 | 0.905 | 79.2 | 92.0 | 0.902 | 80.0 | 93.3 | Age |
| 10 | 30_1_110_128_145_3 _159_114 | 0.907 | 68.0 | 87.5 | 0.92 | 73.3 | 86.7 | Age, number of ApoE4 alleles |
| 10 | 127_5_1_154_110_3_ 73_114 | 0.9 | 87.5 | 84.0 | 0.902 | 80.0 | 73.3 | Age, number of ApoE4 alleles |
| 11 | 155_5_1_110_100_14 5_54_133_114 | 0.93 | 80.0 | 91.7 | 0.92 | 93.3 | 93.3 | Age, number of ApoE4 alleles |
| 11 | 188_5_1_38_110_152 _133_159_114 | 0.922 | 83.3 | 83.3 | 0.907 | 86.7 | 93.3 | Age, number of ApoE4 alleles |
| 12 | 155_1_110_100_145_ 54_152_133_159_114 | 0.927 | 87.5 | 83.3 | 1 | 100.0 | 100.0 | Age, number of ApoE4 alleles |

(continued)

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 13 | 155_188_127_110_14 5_3_152_180_159_11 4 | 0.936 | 70.8 | 100.0 | 0.978 | 73.3 | 100.0 | Age, gender, number of ApoE4 alleles |
| 13 | 137_188_127_5_1_11 0_145_3_152_180_11 4 | 0.923 | 76.0 | 100.0 | 0.907 | 86.7 | 86.7 | Age, number of ApoE4 alleles |
| 14 | 155_188_1_110_75_3_71_152_133_159_14 9_114 | 0.955 | 91.7 | 79.2 | 0.92 | 86.7 | 93.3 | Age, number of ApoE4 alleles |
| 14 | 179_121_137_5_1_18 3_110_185_3_54_152_114 | 0.913 | 79.2 | 100.0 | 0.916 | 93.3 | 86.7 | Age, number of ApoE4 alleles |
| 14 | 155_188_127_30_5_1_53_110_109_145_15 2_159 | 0.947 | 76.0 | 92.0 | 0.92 | 86.7 | 86.7 | Age, number of ApoE4 alleles |
| 15 | 155_87_188_127_110_68_185_100_145_15 2_180_114 | 0.915 | 84.0 | 95.8 | 0.973 | 73.3 | 93.3 | Age, gender, number of ApoE4 alleles |
| 15 | 155_188_127_1_196_189_110_109_71_152_133_149_114 | 0.913 | 87.5 | 92.0 | 0.938 | 100.0 | 86.7 | Age, number of ApoE4 alleles |
| 15 | 155_137_87_188_5_1_94_110_68_109_145_71_152_2_114 | 0.95 | 84.0 | 91.7 | 0.911 | 86.7 | 86.7 | Age |
| 15 | 155_137_127_1_110_193_175_109_3_152_133_180_114 | 0.929 | 87.5 | 87.5 | 0.96 | 86.7 | 86.7 | Age, number of ApoE4 alleles |
| 15 | 155_142_1_154_110_100_109_145_152_13 3_39_104_114 | 0.96 | 92.0 | 79.2 | 0.956 | 93.3 | 80.0 | Age, number of ApoE4 alleles |
| 16 | 155_142_188_127_19 8_154_110_75_100_3_133_149_82_114 | 0.936 | 79.2 | 87.5 | 0.996 | 100.0 | 93.3 | Age, number of ApoE4 alleles |
| 16 | 155_137_188_127_5_1_187_189_110_26_1 09_71_152_114 | 0.937 | 91.7 | 80.0 | 0.92 | 93.3 | 80.0 | Age, number of ApoE4 alleles |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 16 | 155_5_198_110_75_1 09_54_174_71_152_1 33_91_82_114 | 0.918 | 84.0 | 87.5 | 0.907 | 73.3 | 86.7 | Age, number of ApoE4 alleles |
| 16 | 155_188_127_110_9_ 101_175_54_71_111_ 133_2_82_114 | 0.91 | 100.0 | 62.5 | 0.938 | 80.0 | 86.7 | Age, number of ApoE4 alleles |
| 16 | 155_137_188_127_19 6_154_110_128_100_ 152_133_159_114 | 0.917 | 83.3 | 91.7 | 1 | 100.0 | 100.0 | Age, gender, number of ApoE4 alleles |
| 16 | 155_188_127_1_189 _ 110_78_185_100_71_ 152_133_149_114 | 0.903 | 87.5 | 75.0 | 0.933 | 86.7 | 86.7 | Age, number of ApoE4 alleles |
| 16 | 121_155_188_1_79_1 98_154_110_100_109 _54_133_104_114 | 0.92 | 75.0 | 91.7 | 0.92 | 93.3 | 86.7 | Age, number of ApoE4 alleles |
| 16 | 155_137_188_127_5_ 110_68_145_71_73_1 52_159_149_114 | 0.932 | 80.0 | 87.5 | 0.973 | 80.0 | 93.3 | Age, number of ApoE4 alleles |
| 17 | 155_142_188_127_1_ 196_189_110_175_10 0_109_145_3_174_11 4 | 0.944 | 87.5 | 87.5 | 0.942 | 100.0 | 80.0 | Age, number of ApoE4 alleles |
| 17 | 121_155_188_127_1_ 110_4_100_109_145_ 71_152_133_82_114 | 0.943 | 91.7 | 88.0 | 0.907 | 93.3 | 66.7 | Age, number of ApoE4 alleles |
| 17 | 155_142_188_127_1_ 189_110_100_109_17 4_71_133_57_51_114 | 0.939 | 87.5 | 87.5 | 0.929 | 93.3 | 86.7 | Age, number of ApoE4 alleles |
| 17 | 155_188_127_1_154_ 110_58_100_109_54_ 71_73_133_149_114 | 0.965 | 87.5 | 88.0 | 0.973 | 93.3 | 86.7 | Age, number of ApoE4 alleles |
| 17 | 155_142_188_127_15 4_110_100_109_96_1 74_71_133_2_82_114 | 0.944 | 75.0 | 91.7 | 0.942 | 93.3 | 86.7 | Age, number of ApoE4 alleles |
| 17 | 121_155_188_196_19 8_110_193 100 145_ 3_152_133_39_182_1 14 | 0.941 | 80.0 | 100.0 | 0.924 | 86.7 | 86.7 | Age, number of ApoE4 alleles |
| 17 | 155_142_188_127_19 8_154_110_68_109_3 _54_71_111_82_114 | 0.901 | 79.2 | 79.2 | 0.942 | 86.7 | 86.7 | Age, number of ApoE4 alleles |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 17 | 155_142_188_127_1_ 189_110_100_145_17 4_152_133_149_51_1 14 | 0.941 | 87.5 | 91.7 | 0.956 | 86.7 | 93.3 | Age, number of ApoE4 alleles |
| 17 | 155_188_127_110_14 8_193_145_3_71_152 _180_159_2_114 | 0.97 | 83.3 | 91.7 | 0.924 | 86.7 | 93.3 | Age, gender, number of ApoE4 alleles |
| 17 | 155_137_188_127_19 6_110_75_145_71_73 _152_133_2_77_114 | 0.938 | 91.7 | 79.2 | 0.942 | 86.7 | 93.3 | Age, number of ApoE4 alleles |
| 17 | 179_155_137_188_12 7_1_138_110_193_10 0_145_10_152_51_11 4 | 0.938 | 83.3 | 91.7 | 0.911 | 86.7 | 93.3 | Age, number of ApoE4 alleles |
| 18 | 155_142_188_30_1_5 3_110_109145_54_9 6_174_152_2_51_114 | 0.908 | 79.2 | 79.2 | 0.929 | 93.3 | 86.7 | Age, number of ApoE4 alleles |
| 18 | 121_155_137_188_19 4 110 100 109 145_ 21_71_152_180_2_18 2_114 | 0.925 | 80.0 | 96.0 | 0.902 | 86.7 | 86.7 | Age, number of ApoE4 alleles |
| 18 | 155_137_87_188_127 _196_194_110_101_1 45_3_152_133_149_1 82_114 | 0.918 | 79.2 | 88.0 | 0.929 | 86.7 | 93.3 | Age, number of ApoE4 alleles |
| 18 | 155_188_127_30_1_1 89_194_110_100_109 _145_3_71_152_133_ 82_114 | 0.911 | 79.2 | 83.3 | 1 | 93.3 | 100.0 | Age |
| 18 | 155_137_188_127_85 _30_1_110_175_100_ 145_54_71_152_159_ 114 | 0.945 | 92.0 | 87.5 | 0.996 | 80.0 | 100.0 | Age, number of ApoE4 alleles |
| 18 | 155_188_127_1_110_ 78_185_175_100_145 _152_133_149_82_51 | 0.92 | 88.0 | 79.2 | 0.982 | 100.0 | 80.0 | Age, gender, number of ApoE4 alleles |
| 18 | 155_188_127_1_198_ 110_86_175_100_109 _174_152_133_159_1 49_93_114 | 0.988 | 84.0 | 100.0 | 0.991 | 80.0 | 100.0 | Age |
| 18 | 155_188_127_1_110_ 175_145_3_152_133_ 180_39_159_149_77_ 114 | 0.922 | 76.0 | 96.0 | 0.951 | 80.0 | 86.7 | Age, number of ApoE4 alleles |

EP 4 130 259 A1

136

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 18 | 155_188_127_110_68 _200_109_145_10_83 _71_152_180_159_82 _114 | 0.962 | 87.5 | 96.0 | 0.938 | 80.0 | 93.3 | Age, number of ApoE4 alleles |
| 18 | 155_188_127_5_1_19 6_53_154_110_58_54 _152_133_82_114 | 0.928 | 87.5 | 80.0 | 0.973 | 100.0 | 86.7 | Age, gender, number of ApoE4 alleles |
| 19 | 179_188_127_1_110_ 102_175_75_100_109 _3_54_152_190_133_ 159_2 | 0.931 | 83.3 | 87.5 | 0.956 | 80.0 | 86.7 | Age, number of ApoE4 alleles |
| 19 | 155_137_188_127_1_ 196 189 110 109 14 5_3_73_152_133_2_8 2_114 | 0.943 | 95.8 | 79.2 | 0.96 | 93.3 | 80.0 | Age, number of ApoE4 alleles |
| 19 | 155_188_127_1_198_ 110_68_175_75_100_ 54_71_73_133_149_7 7_114 | 0.917 | 75.0 | 79.2 | 0.987 | 80.0 | 93.3 | Age, number of ApoE4 alleles |
| 19 | 155_188_127_196_15 4_110_193_100_109_ 145_62_3_54_152_13 3_180_114 | 0.918 | 84.0 | 83.3 | 0.96 | 80.0 | 93.3 | Age, number of ApoE4 alleles |
| 19 | 155_137_188_127_5_ 194_110_100_109_15 6_3_71_152_133_82_ 150_114 | 0.923 | 88.0 | 88.0 | 1 | 100.0 | 93.3 | Age, number of ApoE4 alleles |
| 19 | 155_137_188_127_30 _5_110_193_71_152_ 180_159_149_2_51_1 82_114 | 0.904 | 76.0 | 92.0 | 0.969 | 80.0 | 100.0 | Age, number of ApoE4 alleles |
| 19 | 155_142_188_127_15 4_110_75_31_100_10 9_3_71_152_133_2_8 2_114 | 0.938 | 88.0 | 79.2 | 0.933 | 100.0 | 80.0 | Age, number of ApoE4 alleles |
| 19 | 121_155_87_188_127 _30_1_161_110_193_ 109_145_135_71_152 _82_114 | 0.905 | 70.8 | 88.0 | 0.987 | 73.3 | 100.0 | Age, number of ApoE4 alleles |
| 19 | 155_87_188_127_1_1 98_154_110_100_174 _73_152_133_159_82 _51_114 | 0.958 | 87.5 | 87.5 | 0.987 | 93.3 | 93.3 | Age, number of ApoE4 alleles |
| 19 | 155_137_87_188_127 _1_198_110_68_101_ 100_109_145_54_152 _149_51_114 | 0.91 | 76.0 | 87.5 | 0.956 | 86.7 | 93.3 | Age |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 19 | 155_137_188_127_85 _154_110_68_100_10 9_199_145_3_152_13 3_114 | 0.965 | 92.0 | 95.8 | 0.924 | 86.7 | 86.7 | Age, gender, number of ApoE4 alleles |
| 19 | 121_155_188_127_1_ 198_110_75_109_145 _54_152_133_82_104 _114 | 0.975 | 96.0 | 87.5 | 0.924 | 86.7 | 93.3 | Age, gender, number of ApoE4 alleles |
| 19 | 155_142_188_127_94 _196_189_110_143_6 8_75_71_152_133_2_ 77_114 | 0.917 | 92.0 | 68.0 | 0.911 | 86.7 | 86.7 | Age, gender, |
| 19 | 155_137_87_142_188 _127_1_110_185_175 _100_145_174_152_1 41_133_180 | 0.927 | 87.5 | 83.3 | 0.924 | 86.7 | 86.7 | Age, number of ApoE4 alleles |
| 19 | 155_142_188_127_1_ 189_154_110_71_73_ 152_159_149_82_51_ 182_114 | 0.942 | 70.8 | 92.0 | 0.942 | 80.0 | 93.3 | Age, number of ApoE4 alleles |
| 19 | 155_188_127_5_1_79 _154_110_75_3_54_1 08_71_73_152_133_1 14 | 0.924 | 91.7 | 70.8 | 0.911 | 86.7 | 86.7 | Age, number of ApoE4 alleles |
| 19 | 155_142_188_127_5_ 154_194_110_32_41_ 152_159_2_51_150_1 82_114 | 0.915 | 79.2 | 87.5 | 0.924 | 80.0 | 86.7 | Age, number of ApoE4 alleles |
| 20 | 121_155_188_127_19 8_110_68_175_109_1 35_3_174_71_133_18 0_149_82_114 | 0.927 | 83.3 | 84.0 | 0.978 | 93.3 | 93.3 | Age, number of ApoE4 alleles |
| 20 | 155_142_127_1_189_ 154_110_109_3_174_ 108_71_133_91_159_ 82_104_114 | 0.939 | 87.5 | 83.3 | 0.942 | 86.7 | 93.3 | Age, number of ApoE4 alleles |
| 20 | 121_155_188_127_15 4_110_41_100 145 7 1_152_133_159_2_82 _51_150_114 | 0.965 | 83.3 | 87.5 | 0.916 | 66.7 | 80.0 | Age, number of ApoE4 alleles |
| 20 | 155_142_188_127_15 4_110_177_100_109_ 125_54_71_152_2_82 _104_182_114 | 0.972 | 88.0 | 95.8 | 0.902 | 86.7 | 73.3 | Age, number of ApoE4 alleles |
| 20 | 155_137_188_5_198_ 110_41_100_109_145 _125_96_92_71_152_ 133_91_114 | 0.908 | 79.2 | 79.2 | 0.907 | 93.3 | 86.7 | Age, number of ApoE4 alleles |

EP 4 130 259 A1

138

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 20 | 121_137_188_127_1_ 196_189_110_175_10 9_3_54_191_152_133 _149_82_114 | 0.927 | 91.7 | 83.3 | 0.902 | 86.7 | 86.7 | Age, number of ApoE4 alleles |
| 20 | 142_188_127_1_198_ 110_175_100_109_14 5_133_180_91_159_1 49_82_182_114 | 0.917 | 79.2 | 84.0 | 0.978 | 100.0 | 93.3 | Age, number of ApoE4 alleles |
| 20 | 155_188_127_30_198 _189_110_193_175_1 00_3_71_133_159_2_ 82_182_114 | 0.977 | 91.7 | 95.8 | 0.947 | 80.0 | 100.0 | Age, number of ApoE4 alleles |
| 20 | 121_155_137_188_12 7_85_5_1_110_100_1 09_3_54_73_152_133 _159_114 | 0.925 | 80.0 | 95.8 | 1 | 100.0 | 100.0 | Age, number of ApoE4 alleles |
| 20 | 155_137_142_188_12 7 154 110 193 175_ 109_88_135_3_54_15 2_133_82_114 | 0.908 | 84.0 | 87.5 | 0.982 | 93.3 | 80.0 | Age, number of ApoE4 alleles |
| 20 | 155_142_188_127_5_ 154_110_175_25_100 _109_54_133_180_91 _159_2_114 | 0.918 | 83.3 | 83.3 | 0.96 | 93.3 | 86.7 | Age, number of ApoE4 alleles |
| 20 | 155_142_188_127_5_ 196_154_110_68_193 _109_3_71_133_180_ 77_114 | 0.93 | 88.0 | 79.2 | 0.947 | 93.3 | 86.7 | Age, gender, number of ApoE4 alleles |
| 20 | 155_188_127_1_138_ 198_110_68_100_109 _48_3_71_152_141_1 33_91_182 | 0.915 | 70.8 | 95.8 | 0.964 | 80.0 | 93.3 | Age, number of ApoE4 alleles |
| 20 | 155_137_142_188_12 7_1_110_109_174_71 _152_111_133_180_1 59_149_51_114 | 0.96 | 87.5 | 91.7 | 0.956 | 93.3 | 86.7 | Age, number of ApoE4 alleles |
| 20 | 155_137_188_127_30 _1_161_154_194_110 _178_10_152_111_13 3_180_114 | 0.912 | 88.0 | 84.0 | 0.987 | 86.7 | 100.0 | Age, gender, number of ApoE4 alleles |
| 20 | 179_155_188_127_1_ 161_110_175_109_62 _125_10_54_71_152_ 133_180_114 | 0.94 | 87.5 | 80.0 | 0.996 | 80.0 | 100.0 | Age, number of ApoE4 alleles |
| 20 | 155_188_127_1_110_ 193_175_145_48_10_ 71_152_141_133_180 _182_114 | 0.96 | 87.5 | 91.7 | 0.991 | 93.3 | 100.0 | Age, gender, number of ApoE4 alleles |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 20 | 155_137_87_188_127 _1_189_194_110_193 _100_10_111_133_18 0_159_149_114 | 0.925 | 83.3 | 83.3 | 0.956 | 86.7 | 93.3 | Age, number of ApoE4 alleles |
| 20 | 155_188_5_1_154_11 0_109_3_54_71_73_1 63_152_133_180_82_ 104_114 | 0.923 | 87.5 | 88.0 | 0.902 | 86.7 | 86.7 | Age, number of ApoE4 alleles |
| 20 | 155_137_188_127_30 _1_17_110_193_145_ 71_152_111_133_180 _77_150_114 | 0.949 | 88.0 | 92.0 | 0.964 | 93.3 | 73.3 | Age, number of ApoE4 alleles |
| 20 | 155_137_188_127_11 0_68_75_100_109_14 5_135_152_111_133_ 113_182_114 | 0.908 | 84.0 | 91.7 | 0.969 | 93.3 | 86.7 | Age, gender, number of ApoE4 alleles |
| 20 | 155_137_87_142_188 _127_1_198_110_58_ 175_109_10_152_133 _149_82_93_114 | 0.945 | 91.7 | 92.0 | 0.969 | 86.7 | 93.3 | Age |
| 20 | 155_188_127_5_1_19 6_110_68_193_109_1 0_54_152_133_180_8 2_114 | 0.95 | 91.7 | 84.0 | 0.902 | 86.7 | 93.3 | Age, gender, number of ApoE4 alleles |
| 20 | 155_142_188_127_1_ 110_75_109_145_10_ 3_71_152_133_82_51 _114 | 0.967 | 91.7 | 92.0 | 0.973 | 100.0 | 80.0 | Age, gender, number of ApoE4 alleles |
| 20 | 155_137_142_188_12 7_85_30_5_196_110_ 193_145_135_3_152_ 2_182_114 | 0.932 | 87.5 | 92.0 | 0.938 | 86.7 | 93.3 | Age, number of ApoE4 alleles |
| 20 | 121_155_142_188_12 7_1_161_154_110_17 5_109_71_73_152_13 3_149_51_114 | 0.928 | 92.0 | 87.5 | 0.96 | 86.7 | 93.3 | Age, number of ApoE4 alleles |
| 21 | 155_137_188_127_5_ 1_198_154_110_193_ 109_3_71_152_180_1 59_82_104_114 | 0.948 | 80.0 | 91.7 | 0.956 | 80.0 | 100.0 | Age, number of ApoE4 alleles |
| 21 | 155_188_127_5_84_1 98_110_185_75_109_ 145_71_152_180_159 _2_51_114 | 0.908 | 87.5 | 79.2 | 0.951 | 80.0 | 86.7 | Age, gender, number of ApoE4 alleles |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 21 | 121_155_137_142_18 8_127_1_154_110_17 5_109_108_133_159_ 149_82_77_114 | 0.906 | 75.0 | 79.2 | 0.987 | 100.0 | 93.3 | Age, gender, number of ApoE4 alleles |
| 21 | 155_188_127_1_189_ 110_26_193_175_109 _145_73_152_141_13 3_149_82_77_114 | 0.924 | 95.8 | 75.0 | 0.991 | 100.0 | 73.3 | Age, number of ApoE4 alleles |
| 21 | 155_188_127_1_162_ 198_189_154_110_75 _100_109_54_71_152 _133_82_104_114 | 0.903 | 84.0 | 87.5 | 0.933 | 100.0 | 86.7 | Age, number of ApoE4 alleles |
| 21 | 121_155_137_188_12 7_1_110_185_69_100 _109_3_174_71_133_ 180_159_82_114 | 0.962 | 84.0 | 100.0 | 0.947 | 60.0 | 86.7 | Age, number of ApoE4 alleles |
| 21 | 121_155_142_188_12 7_5_198_154_110_10 0_109_145_54_71_16 3_152_2_114 | 0.973 | 92.0 | 95.8 | 0.916 | 86.7 | 80.0 | Age, gender, number of ApoE4 alleles |
| 21 | 155_87_188_127_161 _198_38_110_185_10 9_10_152_180_91_15 9_104_77_182_114 | 0.935 | 79.2 | 96.0 | 0.991 | 86.7 | 93.3 | Age, number of ApoE4 alleles |
| 21 | 155 _137 _188 _ 127 _ 11 0_101_193_25_109_1 45_10_3_152_133_18 0_159_2_82_114 | 0.942 | 76.0 | 91.7 | 0.991 | 100.0 | 86.7 | Age, number of ApoE4 alleles |
| 21 | 179_155_188_127_1_ 154_110_86_193_175 _75_109_145_71_133 _159_82_114 | 0.917 | 88.0 | 87.5 | 0.969 | 93.3 | 86.7 | Age, gender, number of ApoE4 alleles |
| 21 | 155_87_188_127_1_8 4_110_193_175_100_ 109_71_152_133_149 _82_104_77_114 | 0.952 | 83.3 | 92.0 | 0.978 | 93.3 | 86.7 | Age, number of ApoE4 alleles |
| 21 | 155_87_188_127_161 _198_38_110_101_17 5_100_109_10_54_71 _133_91_82_114 | 0.925 | 79.2 | 87.5 | 0.969 | 86.7 | 93.3 | Age, number of ApoE4 alleles |
| 21 | 121_188_127_189_15 4_110_102_177_100_ 109_3_54_174_133_1 59_2_82_104_114 | 0.927 | 95.8 | 58.3 | 0.956 | 100.0 | 73.3 | Age, number of ApoE4 alleles |
| 21 | 155_137_142_188_12 7_85_5_154_110_128 _193_175_100_145_1 52_133_51_182_114 | 0.967 | 72.0 | 95.8 | 0.996 | 100.0 | 93.3 | Age, number of ApoE4 alleles |

(continued)

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 21 | 155_137_188_127_1_ 110_175_100_145_10 _135_3_71_152_133_ 180_149_77_114 | 0.925 | 88.0 | 92.0 | 0.938 | 80.0 | 93.3 | Age, number of ApoE4 alleles |
| 21 | 137_142_188_127_16 1_110_75_109 145 1 78_10_96_174_133_3 9_159_82_104_114 | 0.942 | 87.5 | 84.0 | 0.978 | 93.3 | 86.7 | Age, number of ApoE4 alleles |
| 21 | 155_87_142_188_127 _196_154_110_193_1 00_109_145_3_174_7 1_152_159_114 | 0.993 | 95.8 | 96.0 | 0.902 | 80.0 | 93.3 | Age, gender, number of ApoE4 alleles |
| 21 | 87_188_127_196_161 _154_110_100_109_1 35_71_73_152_159_2 _82_182_114 | 0.903 | 83.3 | 87.5 | 0.942 | 93.3 | 80.0 | Age, gender, number of ApoE4 alleles |
| 21 | 155_142_188_127_1_ 189 154 110 175 10 9_145_125_174_71_1 52_133_91_82_114 | 0.943 | 100.0 | 79.2 | 0.96 | 80.0 | 80.0 | Age, number of ApoE4 alleles |
| 21 | 121_142_188_127_5_ 1_189_154_110_100_ 109_145_125_174_10 8_71_152_82_114 | 0.906 | 83.3 | 79.2 | 0.978 | 93.3 | 80.0 | Age, number of ApoE4 alleles |
| 21 | 155_137_87_188_127 _1_198_110_185_175 _100_109_145_92_15 2_133_180_149_114 | 0.984 | 95.8 | 87.5 | 0.973 | 93.3 | 86.7 | Age, number of ApoE4 alleles |
| 21 | 155_137_87_188_127 _1_161_198_110_100 _109_73_152_141_13 3_149_182_114 | 0.905 | 70.8 | 88.0 | 0.92 | 86.7 | 93.3 | Age, gender, number of ApoE4 alleles |
| 21 | 155_142_188_1_79_1 98_154_110_78_185_ 175_100_10_152_133 _180_159_51_114 | 0.915 | 84.0 | 87.5 | 0.982 | 93.3 | 93.3 | Age, number of ApoE4 alleles |
| 21 | 155_87_188_127_198 _110_68_101_25_192 _100_145_3_152_180 _159_149_114 | 0.958 | 91.7 | 83.3 | 0.938 | 80.0 | 93.3 | Age, gender, number of ApoE4 alleles |
| 21 | 155_188_127_189_11 0_68_86_193_192_10 0_135_3_71_152_133 _180_159_77_114 | 0.993 | 92.0 | 100.0 | 0.929 | 80.0 | 93.3 | Age, number of ApoE4 alleles |

142

EP 4 130 259 A1

(continued)

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 21 | 155_137_142_188_12 7_1_196_189_110_78 _143_200_185_145_1 52_133_180_159 | 0.923 | 92.0 | 79.2 | 0.942 | 86.7 | 93.3 | Age, gender, number of ApoE4 alleles |
| 21 | 155_137_188_127_1_ 198_110_41_101_109 _145_174_73_152_15 9_82_51_182_114 | 0.938 | 88.0 | 83.3 | 0.973 | 86.7 | 93.3 | Age, number of ApoE4 alleles |
| 21 | 155_142_188_127_1_ 80_189_110_185_153 _109_145_178_174_1 11_133_82_104_114 | 0.932 | 92.0 | 83.3 | 0.982 | 93.3 | 93.3 | Age, number of ApoE4 alleles |
| 21 | 179 _155 _142 _ 188 _ 12 7_1_198_194_110_68 _185_101_100_109_9 6_152_133_180_149 | 0.913 | 83.3 | 83.3 | 0.96 | 80.0 | 100.0 | Age, number of ApoE4 alleles |
| 21 | 121_155_87_188_127 _5_1_198_194_110_6 8_193_145_152_180_ 159_149_182_114 | 0.923 | 76.0 | 100.0 | 0.916 | 86.7 | 93.3 | Age, number of ApoE4 alleles |
| 22 | 155_87_188_127_196 _194_110_4_193_175 _109_145_178_48_10 _59_71_159_82_114 | 0.912 | 79.2 | 80.0 | 0.916 | 86.7 | 66.7 | Age, number of ApoE4 alleles |
| 22 | 155_137_127_1_198_ 110_175_100_109_54 _152_133_180_39_91 _149_2_82_114 | 0.939 | 79.2 | 91.7 | 0.982 | 100.0 | 80.0 | Age, gender, number of ApoE4 alleles |
| 22 | 121_155_137_87_142 _188_127_1_198_110 _56_193_109_10_3_1 52_180_104_182_114 | 0.928 | 80.0 | 92.0 | 0.956 | 86.7 | 86.7 | Age, number of ApoE4 alleles |
| 22 | 155_142_188_127_19 6_198_154_110_101_ 75_109_3_174_71_15 2_133_159_2_82_114 | 0.918 | 92.0 | 83.3 | 0.978 | 86.7 | 86.7 | Age, number of ApoE4 alleles |
| 22 | 121_137_87_188_127 _1_187_189_110_193 _109_145_135_3_152 _133_159_2_82_114 | 0.912 | 79.2 | 88.0 | 0.911 | 80.0 | 73.3 | Age, number of ApoE4 alleles |
| 22 | 155_137_188_127_1_ 79_198_110_86_100_ 109_3_174_71_152_1 33_91_149_82_114 | 0.957 | 92.0 | 91.7 | 0.982 | 100.0 | 86.7 | Age, number of ApoE4 alleles |
| 22 | 155_137_188_127_1_ 198 110 193 100 10 9_145_98_3_71_152_ 133_159_82_150_114 | 0.948 | 95.8 | 79.2 | 0.964 | 93.3 | 80.0 | Age, number of ApoE4 alleles |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 22 | 155_137_188_127_5_ 198_189_154_110_12 8_193_100_109_3_15 2_133_82_182_114 | 0.952 | 83.3 | 92.0 | 1 | 100.0 | 93.3 | Age, gender, number of ApoE4 alleles |
| 22 | 155_137_188_127_1_ 110_153_193_75_109 _88_174_71_152_133 _180_149_82_51_114 | 0.913 | 88.0 | 70.8 | 0.951 | 93.3 | 80.0 | Age, number of ApoE4 alleles |
| 22 | 121_155_188_127_5_ 79_198_110_100_145 _59_54_108_73_152_ 133_159_2_82_114 | 0.906 | 83.3 | 87.5 | 0.92 | 80.0 | 86.7 | Age, number of ApoE4 alleles |
| 22 | 155_87_188_127_1_1 61_198_110_175_109 _169_145_178_10_92 133_159_82_104_114 | 0.92 | 87.5 | 75.0 | 0.978 | 100.0 | 86.7 | Age, number of ApoE4 alleles |
| 22 | 155_87_142_188_127 _1_154_194_110_101 _75_100_109_145_15 2_133_180_82_114 | 0.937 | 68.0 | 95.8 | 0.911 | 93.3 | 86.7 | Age, gender, number of ApoE4 alleles |
| 22 | 155_137_87_127_1_1 10_185_175_173_109 _145_133_180_91_15 9_149_104_77_114 | 0.929 | 91.7 | 79.2 | 0.987 | 100.0 | 86.7 | Age, gender, number of ApoE4 alleles |
| 22 | 121_155_87_188_127 _196_110_175_75_14 5_48_3_152_159_82_ 104_77_57_114 | 0.938 | 92.0 | 79.2 | 0.973 | 86.7 | 93.3 | Age, gender, number of ApoE4 alleles |
| 22 | 155_87_142_188_127 _198_154_110_185_1 09_178_54_174_152_ 133_159_82_77_51_1 14 | 0.945 | 83.3 | 88.0 | 0.982 | 86.7 | 93.3 | Age, number of ApoE4 alleles |
| 22 | 121_155_87_188_127 _79_53_154_110_185 _175_100_3_54_73_1 52_133_93_114 | 0.937 | 92.0 | 91.7 | 0.911 | 73.3 | 86.7 | Age, gender, number of ApoE4 alleles |
| 23 | 87_142_188_127_161 _198_187_110_175_1 00_109_145_178_174 _133_159_2_82_93_1 14 | 0.943 | 87.5 | 91.7 | 0.956 | 80.0 | 86.7 | Age, gender, number of ApoE4 alleles |
| 23 | 155_137_87_188_127 _189_154_194_110_2 00_148_86_193_3_15 2_159_82_104_77_93 _114 | 0.95 | 88.0 | 76.0 | 0.973 | 100.0 | 80.0 | Age, number of ApoE4 alleles |

144

EP 4 130 259 A1

(continued)

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 23 | 121_155_142_188_12 7_196_110_185_26_1 75_100_109_145_88_ 3_71_ 152_2_82_114 | 0.95 | 83.3 | 92.0 | 0.924 | 93.3 | 80.0 | Age, gender, number of ApoE4 alleles |
| 23 | 155_142_188_127_1_ 196_110_58_185_175 _75_100_109_145_12 5_3_91_2_82_114 | 0.927 | 84.0 | 79.2 | 0.938 | 100.0 | 80.0 | Age, gender, number of ApoE4 alleles |
| 23 | 121_137_142_188_12 7_1_154_110_175_10 0_109_145_88_3_54_ 174_152_133_82_182 _114 | 0.933 | 88.0 | 79.2 | 0.978 | 93.3 | 86.7 | Age, number of ApoE4 alleles |
| 23 | 121_137_87_188_127 _1_53_110_185_175_ 109_152_133_91_159 _149_82_104_77_150 _114 | 0.903 | 92.0 | 79.2 | 0.982 | 100.0 | 80.0 | Age, number of ApoE4 alleles |
| 23 | 155_142_188_127_30 _1_196_198_189_154 _110_109_145_10_ 174_152_133_159_82_1 14 | 0.976 | 92.0 | 88.0 | 0.956 | 73.3 | 93.3 | Age, gender, number of ApoE4 alleles |
| 23 | 179_155_137_ 142_ 18 8 127 110 109 145_ 125_10_88_3_174_15 2_180_113_82_51_15 0_114 | 0.913 | 87.5 | 75.0 | 0.933 | 86.7 | 86.7 | Age, number of ApoE4 alleles |
| 23 | 155_87_142_188_127 _198_194_110_136_6 8_175_109_145_3_71 _73_152_159_82_77_ 114 | 0.987 | 91.7 | 96.0 | 0.902 | 80.0 | 93.3 | Age, number of ApoE4 alleles |
| 23 | 179_155_188_127_1_ 198_110_101_175_11 8_109_145_3_37_73_ 133_91_149_82_77_1 14 | 0.924 | 95.8 | 75.0 | 0.924 | 86.7 | 86.7 | Age, number of ApoE4 alleles |
| 23 | 121_155_142_188_12 7_53_189_110_200_1 93_75_109_145_3_15 2_133_180_82_104_1 14 | 0.923 | 92.0 | 84.0 | 0.973 | 86.7 | 86.7 | Age, gender, number of ApoE4 alleles |
| 23 | 155_137_142_188_12 7_1_196_154_110_68_ 26_178_71_152_133_ 180_91_149_ 82_ 114 | 0.901 | 80.0 | 84.0 | 1 | 100.0 | 100.0 | Age, gender, number of ApoE4 alleles |
| 23 | 155_137_87_188_127_ 5_ 198 154 110 193 _175_100_145_178_1 52_91_159_2_82_77_ 114 | 0.981 | 80.0 | 96.0 | 0.973 | 93.3 | 93.3 | Age, number of ApoE4 alleles |

(continued)

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 23 | 121_155_137_188_12 7_1_79_198_110_86_ 75_100_109_3_152_1 33_149_2_82_182_11 4 | 0.962 | 87.5 | 91.7 | 1 | 100.0 | 86.7 | Age, number of ApoE4 alleles |
| 23 | 155_137_87_188_127 _196_189_110_193_1 00_109_48_71_73_15 2_133_91_159_2_82_ 114 | 0.955 | 87.5 | 87.5 | 0.92 | 80.0 | 86.7 | Age, number of ApoE4 alleles |
| 23 | 155_137_188_127_19 8_189_194_110_200_ 175_75_100_10_152_ 133_180_159_149_2_ 182_114 | 0.918 | 75.0 | 87.5 | 0.969 | 93.3 | 93.3 | Age, number of ApoE4 alleles |
| 23 | 155 _137 _188 _ 127 _ 5_ 1_84_198_194_110_7 5_100_109_145_48_7 1_152_133_82_114 | 0.933 | 84.0 | 87.5 | 0.982 | 93.3 | 86.7 | Age, gender, number of ApoE4 alleles |
| 23 | 155_142_188_127_1_ 79_196_154_110_41_ 31_109_145_48_54_1 74_108_71_133_82_1 14 | 0.924 | 95.8 | 79.2 | 0.929 | 93.3 | 86.7 | Age, number of ApoE4 alleles |
| 23 | 121_155_188_127_1_ 161_198_189_110_78 _100_178_71_152_13 3_159_149_104_182_ 114 | 0.946 | 79.2 | 100.0 | 0.969 | 93.3 | 93.3 | Age, gender, number of ApoE4 alleles |
| 23 | 155_188_127_1_79_1 89_110_176_175_109 _145_48_71_73_152_ 133_91_159_82_77_1 14 | 0.905 | 95.8 | 70.8 | 0.947 | 86.7 | 80.0 | Age, number of ApoE4 alleles |
| 23 | 155_87_142_188_127 _1_196_17_189_110_ 153_101_175_100_10 9_133_180_149_51_1 14 | 0.936 | 83.3 | 87.5 | 0.916 | 86.7 | 93.3 | Age, gender, number of ApoE4 alleles |
| 23 | 121_155_188_127_85 _1_198_ 189_ 154_ 110 _109_145_125_178_7 1_152_141_133_180_ 159_114 | 0.958 | 100.0 | 84.0 | 0.907 | 93.3 | 80.0 | Age, number of ApoE4 alleles |
| 23 | 155_142_188_127_53 _189_154_110_78_18 5_75_109_145_174_1 52_133_2_82_93_114 | 0.948 | 92.0 | 87.5 | 0.964 | 100.0 | 80.0 | Age, gender, number of ApoE4 alleles |
| 23 | 155_188_127_1_198_ 187_110_68_175_100 _109_145_125_10_3_ 71_152_133_82_182_ 114 | 0.97 | 95.8 | 91.7 | 0.987 | 100.0 | 86.7 | Age, number of ApoE4 alleles |

(continued)

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 23 | 121_155_87_142_188_127_1_84_110_26_75_100_71_106_152_1 80_159_149_2_114 | 0.912 | 68.0 | 96.0 | 0.956 | 93.3 | 100.0 | Age, gender, number of ApoE4 alleles |
| 23 | 155_142_188_127_1_189_154_110_78_185_153_177_109_178_5 4_174_92_133_82_57_114 | 0.922 | 92.0 | 84.0 | 0.956 | 80.0 | 93.3 | Age, number of ApoE4 alleles |
| 23 | 155_142_188_127_14 6_1_189_154_110_18 5_153_175_100_109_145_92_152_133_82_104_114 | 0.943 | 91.7 | 87.5 | 1 | 93.3 | 100.0 | Age, number of ApoE4 alleles |
| 23 | 155_137_188_5_1_19 6_198_154_110_193_109_145_135_3_152_159_104_171_182_11 4 | 0.936 | 72.0 | 92.0 | 0.96 | 93.3 | 93.3 | Age, gender, number of ApoE4 alleles |
| 23 | 121_155_188_127_1_198_110_175_75_109_145_3_96_71_152_1 33_180_104_51_182_114 | 0.981 | 91.7 | 91.7 | 0.956 | 86.7 | 86.7 | Age, number of ApoE4 alleles |
| 23 | 121_155_137_188_12 7_30_1_79_110_58_1 93_109_145_156_71_152_133_149_182_11 4 | 0.977 | 91.7 | 88.0 | 0.924 | 86.7 | 93.3 | Age, gender, number of ApoE4 alleles |
| 24 | 121_155_87_127_1_1 58_161_84_110_175_100_109_48_3_54_19 0_133_91_159_82_11 4 | 0.906 | 79.2 | 87.5 | 0.96 | 93.3 | 86.7 | Age, gender, number of ApoE4 alleles |
| 24 | 155_188_127_1_196_198_110_185_175_75_100_109_145_152_1 33_39_91_149_82_18 2_114 | 0.967 | 88.0 | 87.5 | 0.973 | 93.3 | 80.0 | Age, gender, number of ApoE4 alleles |
| 24 | 155_188_127_196_15 4_110_176_102_69_1 75_75_100_109_145_54_108_73_133_159_82_114 | 0.923 | 76.0 | 87.5 | 0.902 | 93.3 | 80.0 | Age, gender, number of ApoE4 alleles |
| 24 | 155_137_142_188_12 7_1_196_161_154_11 0_185_109_199_145_178_3_71_133_82_11 4_12 | 0.937 | 91.7 | 80.0 | 0.987 | 93.3 | 86.7 | Age, gender, number of ApoE4 alleles |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 24 | 121_155_137_188_12 7_194_110_148_100_ 109_145_125_88_135 _3_152_91_159_149_ 77_114 | 0.968 | 76.0 | 95.8 | 0.947 | 93.3 | 86.7 | Age, gender, number of ApoE4 alleles |
| 24 | 121_155_137_87_188 _127_5_1_198_189_1 94_110_75_100_109_ 145_3_152_159_82_1 14 | 0.945 | 68.0 | 91.7 | 0.978 | 93.3 | 86.7 | Age, gender, number of ApoE4 alleles |
| 24 | 155_137_87_188_127 _85_1_198_154_110_ 128_185_101_145_10 _3_152_180_159_149 _51_114 | 0.92 | 83.3 | 91.7 | 0.938 | 86.7 | 93.3 | Age, number of ApoE4 alleles |
| 24 | 121_155_137_87_142 _188_127_30_196_15 4_110_100_109_145_ 135_71_152_133_149 _2_82_114 | 0.955 | 87.5 | 96.0 | 0.942 | 93.3 | 93.3 | Age, number of ApoE4 alleles |
| 24 | 155_137_87_188_127 _196_187_110_102_1 93_175_100_109_145 _125_152_149_2_82_ 104_114 | 0.962 | 100.0 | 83.3 | 0.929 | 93.3 | 73.3 | Age, gender, number of ApoE4 alleles |
| 24 | 155_137_142_188_12 7_1_161_194_110_10 1_100_145_178_3_54 _152_133_180_149_5 1_182_114 | 0.946 | 68.0 | 96.0 | 0.951 | 80.0 | 100.0 | Age, number of ApoE4 alleles |
| 24 | 155_87_188_127_1_1 61_198_189_110_185 _193_175_109_145_7 3_133_159_149_82_6 1_18_114 | 0.953 | 91.7 | 83.3 | 0.964 | 93.3 | 93.3 | Age, number of ApoE4 alleles |
| 24 | 155_137_87_188_127 _196_189_194_110_6 8_200_175_100_145_10_152_133_180_159_82_104_114 | 0.9 | 91.7 | 76.0 | 1 | 100.0 | 93.3 | Age, number of ApoE4 alleles |
| 24 | 121_155_188_127_19 8_110_193_75_100_1 09_145_48_152_133_ 180_159_2_82_104_1 82_114 | 0.97 | 95.8 | 87.5 | 0.951 | 86.7 | 80.0 | Age, gender, number of ApoE4 alleles |
| 24 | 121_155_87_188_127 _1_16_161_198_154_ 110_109_145_178_10 _174_71_133_159_2_ 82_114 | 0.918 | 91.7 | 83.3 | 0.982 | 100.0 | 80.0 | Age, number of ApoE4 alleles |
| 24 | 155_87_188_127_110 _185_193_109_145_1 0_3_71_73_180_91_1 59_149_82_51_182_9 3_114 | 0.91 | 83.3 | 87.5 | 0.973 | 93.3 | 86.7 | Age, number of ApoE4 alleles |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 24 | 121_155_188_127_1_ 161_189_110_185_17 7_100_109_125_71_1 33_159_149_82_104_ 182_114 | 0.92 | 91.7 | 62.5 | 0.982 | 80.0 | 93.3 | Age, gender, number of ApoE4 alleles |
| 24 | 155_87_188_127_196 _161_198_110_193_1 75_109_178_174_152 _133_91_159_82_51_ 93_114 | 0.995 | 100.0 | 83.3 | 0.947 | 80.0 | 93.3 | Age, gender, number of ApoE4 alleles |
| 24 | 155_87_188_127_198 _110_175_109_145_1 78_48_10_3_152_133 _91_159_2_82_51_11 4 | 0.931 | 79.2 | 87.5 | 0.96 | 66.7 | 100.0 | Age, gender, number of ApoE4 alleles |
| 24 | 121_137_188_127_30 _1_110_128_193_175 _100_199_145_10_15 2_133_180_159_149_ 104_51_114 | 0.91 | 79.2 | 95.8 | 0.96 | 93.3 | 86.7 | Age, number of ApoE4 alleles |
| 24 | 155_188_127_85_1_1 61_198_154_110_175 _100_109_178_54_92 _152_133_82_104_77 _51_114 | 0.958 | 91.7 | 83.3 | 0.996 | 93.3 | 100.0 | Age, number of ApoE4 alleles |
| 24 | 121_155_188_127_19 6_189_110_68_193_1 00_109_145_135_3_7 1_152_133_180_159_ 82_182_114 | 0.945 | 87.5 | 96.0 | 0.929 | 86.7 | 86.7 | Age, number of ApoE4 alleles |
| 24 | 155_142_188_127_14 6_1_198_189_38_110 _68_193_192_100_10 9_145_10_152_133_1 59_149_82_114 | 0.981 | 95.8 | 95.8 | 0.956 | 86.7 | 86.7 | Age |
| 24 | 121_155_188_127_15 4_194_110_176_200_ 69_100_145_174_71_ 73_152_133_159_149 _77_51_114 | 0.915 | 83.3 | 79.2 | 0.933 | 80.0 | 93.3 | Age, number of ApoE4 alleles |
| 24 | 155_137_142_188_12 7_1_198_189_154_11 0_68_192_100_109_1 45_152_133_159_149 _82_114 | 0.947 | 96.0 | 79.2 | 0.96 | 80.0 | 93.3 | Age, gender, number of ApoE4 alleles |
| 24 | 155_137_87_188_127 _53_189_194_110_10 1_193_175_100_109_ 145_3_152_133_149_ 82_114 | 0.942 | 91.7 | 88.0 | 0.902 | 86.7 | 86.7 | Age, gender, number of ApoE4 alleles |
| 24 | 155_142_188_127_15 4_110_68_148_193_1 75_145_156_10_152_ 133_180_159_149_82_77_182_114 | 0.92 | 92.0 | 76.0 | 0.956 | 86.7 | 80.0 | Age, number of ApoE4 alleles |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 24 | 155_142_188_127_1_ 194_110_9_200_148_ 86_109_145_125_174 _92_163_152_159_14 9_82_114 | 0.943 | 91.7 | 83.3 | 0.973 | 93.3 | 93.3 | Age, number of ApoE4 alleles |
| 24 | 121_155_137_188_12 7_1_161_198_110_86 _109_135_3_71_152_ 133_180_149_82_104 _182_114 | 0.98 | 91.7 | 88.0 | 0.956 | 93.3 | 86.7 | Age, number of ApoE4 alleles |
| 24 | 121_155_87_188_127 _198_194_110_109_1 35_3_191_152_133_3 9_159_149_82_104_5 1_150_114 | 0.969 | 87.5 | 100.0 | 0.929 | 86.7 | 86.7 | Age, number of ApoE4 alleles |
| 24 | 155_142_188_127_14 6_1_189_110_143_10 1_175_75_109_145_1 35_174_152_133_159 _82_182_114 | 0.922 | 91.7 | 87.5 | 0.929 | 80.0 | 86.7 | Age, number of ApoE4 alleles |
| 24 | 155_137_87_142_188 _127_30_1_196_198_ 110_58_193_175_109 _145_73_133_180_14 9_182_114 | 0.936 | 87.5 | 87.5 | 0.938 | 86.7 | 93.3 | Age, number of ApoE4 alleles |
| 24 | 155_137_188_127_1_ 189_110_78_185_175 _100_109_145_178_1 52_133_180_159_149 _82_182_114 | 0.952 | 87.5 | 84.0 | 0.96 | 100.0 | 73.3 | Age, number of ApoE4 alleles |
| 25 | 155_87_142_188_127 _1_53_198_110_193_ 175_109_97_3_152_1 33_39_91_159_149_8 2_93_114 | 0.958 | 91.7 | 87.5 | 0.942 | 100.0 | 80.0 | Age, number of ApoE4 alleles |
| 25 | 155_137_87_107_188 _127_198_154_110_4 1_185_193_109_178_ 3_152_133_91_149_1 04_51_93_114 | 0.901 | 83.3 | 79.2 | 0.982 | 86.7 | 93.3 | Age, number of ApoE4 alleles |
| 25 | 121_155_87_142_188 _127_196_198_154_1 10_4_175_109_167_1 91_73_113_159_149_ 2_82_182_114 | 0.953 | 83.3 | 96.0 | 0.92 | 100.0 | 73.3 | Age, number of ApoE4 alleles |
| 25 | 121_155_137_87_188 _127_1_84_198_110_ 102_175_75_100_109 _145_54_152_133_14 9_77_114 | 0.973 | 80.0 | 91.7 | 0.978 | 93.3 | 80.0 | Age, gender, number of ApoE4 alleles |
| 25 | 179_155_137_87_188 _127_196_198_194_1 10_86_75_100_109_1 52_133_180_91_149_ 2_82_182_114 | 0.953 | 91.7 | 83.3 | 0.973 | 100.0 | 86.7 | Age, number of ApoE4 alleles |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 25 | 121_155_137_188_12 7_5_1_84_189_154_1 94_110_75_3_152_13 3_180_159_149_104_ 77_114 | 0.932 | 84.0 | 87.5 | 0.924 | 80.0 | 86.7 | Age, gender, number of ApoE4 alleles |
| 25 | 155_137_87_188_127 _1_196_161_110_143 _86_175_100_109_63 _71_133_39_91_159_ 149_82_114 | 0.994 | 92.0 | 92.0 | 0.929 | 86.7 | 80.0 | Age, number of ApoE4 alleles |
| 25 | 121_155_137_87_188 _127_1_196_110_9_1 93_100_109_135_54_ 71_73_111_133_149_ 104_182_114 | 0.922 | 79.2 | 96.0 | 0.902 | 86.7 | 86.7 | Age, number of ApoE4 alleles |
| 25 | 155_137_87_188_127 _1_53_198_189_110_ 153_175_100_109_14 5_10_88_190_133_18 0_91_149_114 | 0.943 | 87.5 | 87.5 | 0.933 | 86.7 | 93.3 | Age, number of ApoE4 alleles |
| 25 | 121_155_87_142_188 _127_1_196_198_110 _128_41_101_175_10 0_109_145_54_152_1 50_182_93_114 | 0.922 | 87.5 | 79.2 | 0.92 | 86.7 | 80.0 | Age, number of ApoE4 alleles |
| 25 | 179_155_87_142_188 _127_5_1_198_154_1 94_110_193_175_199 _145_3_71_152_133_ 159_82_114 | 0.933 | 72.0 | 91.7 | 0.978 | 73.3 | 100.0 | Age, number of ApoE4 alleles |
| 25 | 155_87_188_127_161 _198_110_153_175_1 92_109_145_178_10_ 152_133_91_149_82_ 104_182_114 | 0.968 | 87.5 | 96.0 | 0.987 | 93.3 | 93.3 | Age, gender, number of ApoE4 alleles |
| 25 | 121_155_87_127_1_1 89_154_110_193_100 _109_10_3_152_133_ 180_159_2_82_104_1 82_114 | 0.96 | 83.3 | 88.0 | 0.951 | 86.7 | 86.7 | Age, gender, number of ApoE4 alleles |
| 25 | 155_142_188_127_1_ 162_196_154_110_20 0_101_193_175_109_ 125_71_133_180_149 _82_77_114 | 0.905 | 84.0 | 87.5 | 0.982 | 100.0 | 86.7 | Age, gender, number of ApoE4 alleles |
| 25 | 155_137_87_142_188 _127_189_154_110_1 75_100_109_145_178 _10_3_174_152_133_ 91 82 93 114 | 0.953 | 95.8 | 88.0 | 1 | 93.3 | 100.0 | Age, number of ApoE4 alleles |
| 25 | 155_87_142_188_127 _94_138_189_110_19 3_109_145_10_135_3 _71_152_133_159_2_ 82_182_114 | 0.91 | 70.8 | 88.0 | 1 | 93.3 | 100.0 | Age, number of ApoE4 alleles |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 25 | 142_188_127_138_18 9_154_110_26_193_1 00_109_178_10_88_1 52_133_159_104_171 _77_182_114 | 0.94 | 100.0 | 83.3 | 0.916 | 80.0 | 93.3 | Age, gender, number of ApoE4 alleles |
| 25 | 142_188_127_196_18 9_154_110_185_193_ 109_145_178_88_3_1 52_133_180_159_82_ 150_182_114 | 0.953 | 100.0 | 84.0 | 0.938 | 86.7 | 73.3 | Age, gender, number of ApoE4 alleles |
| 25 | 121_155_87_188_127 _85_198_154_38_110 _185_193_109_145_1 25_178_3_152_133_1 59_82_182_114 | 0.973 | 91.7 | 88.0 | 0.956 | 86.7 | 100.0 | Age, number of ApoE4 alleles |
| 25 | 155_137_188_127_14 6_183_196_198_110_ 200_193_25_100_109 _152_133_180_91_15 9_149_77_114 | 0.948 | 79.2 | 100.0 | 0.982 | 86.7 | 93.3 | Age, gender, number of ApoE4 alleles |
| 25 | 179_121_155_87_142 _188_127_161_172_1 98_110_193_175_145 _54_73_152_159_149 _82_93_114 | 0.945 | 79.2 | 88.0 | 0.978 | 86.7 | 86.7 | Age, gender, number of ApoE4 alleles |
| 25 | 121_155_188_127_19 8_154_110_26_193_1 75_100_109_145_88_ 3_54_174_71_152_15 9_182_114 | 0.972 | 88.0 | 95.8 | 0.911 | 80.0 | 86.7 | Age, gender, number of ApoE4 alleles |
| 25 | 155_137_188_127_1_ 161_198_154_194_11 0_100_145_178_3_71 _73_152_133_159_14 9_82_114 | 0.972 | 88.0 | 87.5 | 0.964 | 86.7 | 93.3 | Age, gender, number of ApoE4 alleles |
| 25 | 179_155_87_188_127 _5_1_154_110_200_1 93_175_100_109_145 _3_133_91_159_149_ 82_182_114 | 0.941 | 91.7 | 83.3 | 0.987 | 100.0 | 80.0 | Age, number of ApoE4 alleles |
| 25 | 179_121_155_87_188 _127_1_161_198_154 _143_193_175_192_1 00_109_71_133_149_ 104_182_114 | 0.918 | 87.5 | 88.0 | 0.991 | 100.0 | 86.7 | Age, gender, number of ApoE4 alleles |

EP 4 130 259 A1

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 25 | 155_137_87_188_127 _1_198_189_38_194_ 110_175_100_109_14 5_10_152_133_149_8 2_57_114 | 0.951 | 87.5 | 87.5 | 0.947 | 86.7 | 80.0 | Age, gender, number of ApoE4 alleles |
| 25 | 155_137_87_142_188 _127_1_154_194_110 _193_175_145_156_5 4_152_133_180_159_ 149_77_57_114 | 0.93 | 92.0 | 87.5 | 0.996 | 86.7 | 100.0 | Age, number of ApoE4 alleles |
| 25 | 179 _155 _142 _ 188 _ 12 7_138_198_110_9_56 _175_100_109_145_1 0_54_174_141_133_8 2_182_114 | 0.933 | 83.3 | 80.0 | 0.973 | 86.7 | 86.7 | Age, gender, number of ApoE4 alleles |
| 25 | 155_137_188_127_5_ 1_84_154_110_175_1 92_100_109_145_10_ 108_152_133_180_15 9_149_82_114 | 0.951 | 87.5 | 87.5 | 0.973 | 93.3 | 93.3 | Age, number of ApoE4 alleles |
| 25 | 155_188_127_146_1_ 196_189_194_110_20 0_86_193_100_109_1 52_39_159_149_82_7 7_182_114 | 0.977 | 91.7 | 91.7 | 0.916 | 80.0 | 86.7 | Age, gender, number of ApoE4 alleles |
| 25 | 155_188_127_146_1_ 196_161_154_38_194 _110_56_78_68_175_ 100_73_152_133_159 _149_182_114 | 0.908 | 75.0 | 95.8 | 0.991 | 86.7 | 100.0 | Age, gender, |
| 25 | 121_155_87_142_188 _127_198_110_109_1 67_145_21_48_3_174 _71_152_133_149_2_ 82_182_114 | 0.945 | 88.0 | 87.5 | 0.973 | 93.3 | 86.7 | Age, number of ApoE4 alleles |
| 25 | 155_137_87_142_188 _127_5_1_84_189_15 4_194_110_175_75_1 45_152_133_180_159 _77_114 | 0.917 | 76.0 | 88.0 | 0.92 | 66.7 | 93.3 | Age, gender, number of ApoE4 alleles |
| 25 | 121_137_87_142_188 _127_1_154_194_110 _128_185_175_100_8 3_71_73_152_133_14 9_82_182_114 | 0.912 | 84.0 | 83.3 | 0.987 | 80.0 | 100.0 | Age, number of ApoE4 alleles |

(continued)

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 26 | 179_155_137_142_18 8_127_79_196_198_1 54_110_102_148_101 _75_100_109_145_3_ 54_152_2_114 | 0.95 | 76.0 | 95.8 | 0.911 | 93.3 | 73.3 | Age, gender, number of ApoE4 alleles |
| 26 | 155_188_127_5_1_84 _154_110_176_58_10 2_148_109_145_135_ 3_54_191_152_133_1 80_82_114 | 0.914 | 68.0 | 88.0 | 0.938 | 93.3 | 86.7 | Age, gender, number of ApoE4 alleles |
| 26 | 155_137_142_188_12 7_5_1_196_84_189_1 54_110_109_145_135 _96 _174 _152 _133 _ 18 0_91_159_82_114 | 0.918 | 72.0 | 88.0 | 0.987 | 93.3 | 86.7 | Age, number of ApoE4 alleles |
| 26 | 121_155_137_87_188 _127_1_198_189_110 _26_177_175_100_10 9_145_48_98_54_108 _133_159_82_93_114 | 0.938 | 83.3 | 84.0 | 0.951 | 86.7 | 66.7 | Age |
| 26 | 179 _155 _142 _ 188 _ 12 7_1_154_194_110_56 _175_109_145_88_3_ 174_73_152_133_82_ 171_150_114 | 0.942 | 91.7 | 88.0 | 0.947 | 86.7 | 86.7 | Age, gender, number of ApoE4 alleles |
| 26 | 179_155_137_142_18 8_127_1_84_189_110 _148_193_109_145_8 8_3_71_152_180_159 _82_150_114 | 0.933 | 84.0 | 84.0 | 0.964 | 80.0 | 93.3 | Age, gender, number of ApoE4 alleles |
| 26 | 179_155_87_142_188 _127_79_198_110_10 2_175_100_109_97_1 45_3_174_141_91_15 9_149_2_82_114 | 0.958 | 87.5 | 87.5 | 0.964 | 73.3 | 93.3 | Age, number of ApoE4 alleles |
| 26 | 179_155_137_87_142 _188_127_5_1_161_1 98_110_41_100_109_ 145_174_71_152_159 _149_82_182_114 | 0.928 | 87.5 | 92.0 | 0.951 | 93.3 | 86.7 | Age, number of ApoE4 alleles |
| 26 | 155_107_188_127_1_ 79_198_110_58_100_ 109_145_125_10_54_ 174_106_152_133_82 _57_51_114 | 0.96 | 100.0 | 79.2 | 0.973 | 80.0 | 93.3 | Age, gender, number of ApoE4 alleles |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 26 | 155_137_87_142_188 _127_1_53_198_110_ 41_185_175_100_109 _169_49_3_54_71_13 3_149_182_114 | 0.942 | 83.3 | 88.0 | 0.924 | 80.0 | 80.0 | Age, number of ApoE4 alleles |
| 26 | 121_155_137_87_188 _127_1_198_110_58_ 100_109_3_54_191_7 1_133_180_91_149_2 _77_182_114 | 0.95 | 87.5 | 87.5 | 0.947 | 80.0 | 80.0 | Age, number of ApoE4 alleles |
| 26 | 121_155_87_142_188 _127_5_196_198_110 _102_69_175_192_10 9_145_88_54_174_2_ 82_52_114 | 0.974 | 91.7 | 87.5 | 0.911 | 93.3 | 80.0 | Age, gender, number of ApoE4 alleles |
| 26 | 155_137_87_142_188 _127_1_198_110_26_ 101_175_109_145_17 8_152_133_180_149_ 61_150_182_114 | 0.95 | 87.5 | 84.0 | 0.929 | 80.0 | 86.7 | Age, gender, number of ApoE4 alleles |
| 26 | 121_155_87_188_127 _1_162_110_86_193_ 175_109_145_3_71_1 52_133_159_149_82_ 77_150_114 | 0.937 | 79.2 | 92.0 | 0.982 | 100.0 | 86.7 | Age, gender, number of ApoE4 alleles |
| 26 | 121_142_188_127_1_ 196_154_110_56_153 _177_109_21_10_3_5 4_174_71_163_159_8 2_104_182_114 | 0.915 | 79.2 | 83.3 | 0.973 | 100.0 | 80.0 | Age, number of ApoE4 alleles |
| 26 | 155_137_188_127_85 _1_138_110_86_26_1 93_175_100_145_156 _10_88_3_152_111_2 _51_182_114 | 0.917 | 83.3 | 84.0 | 0.987 | 86.7 | 100.0 | Age, number of ApoE4 alleles |
| 26 | 155_142_188_127_18 3_198_110_128_200_ 185_175_75_100_178 _152_133_180_159_1 49_82_77_51_114 | 0.939 | 79.2 | 87.5 | 0.964 | 93.3 | 93.3 | Age, gender, number of ApoE4 alleles |
| 26 | 155_137_87_188_127 _85_162_196_198_11 0_68_175_75_100_14 5_3_54_71_152_133_ 159_55_114_12 | 0.939 | 87.5 | 87.5 | 0.978 | 86.7 | 93.3 | Age, number of ApoE4 alleles |
| 26 | 155_137_87_142_188 _127_79_53_198_110 _193_100_109_62_13 5_174_152_133_39_9 1_82_51_182_114 | 0.943 | 80.0 | 91.7 | 0.973 | 100.0 | 93.3 | Age, number of ApoE4 alleles |

EP 4 130 259 A1

(continued)

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 26 | 155_137_188_127_1_ 189_110_136_68_26_ 193_175_100_105_14 5_3_71_152_111_133 _82_104_77_114 | 0.911 | 91.7 | 58.3 | 1 | 100.0 | 86.7 | Age, number of ApoE4 alleles |
| 26 | 155_137_87_188_127 _1_161_198_154_110 _68_185_175_100_10 9_178_10_54_108_71 _133_82_104_114 | 0.967 | 79.2 | 92.0 | 0.96 | 80.0 | 86.7 | Age, number of ApoE4 alleles |
| 26 | 121_155_137_87_188 _127_1_161_189_194 _110_186_200_100_1 45_3_191_108_133_9 1_159_149_82_114 | 0.907 | 88.0 | 75.0 | 0.964 | 86.7 | 93.3 | Age, number of ApoE4 alleles |
| 26 | 121_155_188_127_1_ 198_154_110_185_86 _193_175_109_145_1 78_135_3_73_152_13 3_159_82_182_114 | 0.975 | 95.8 | 84.0 | 0.969 | 100.0 | 73.3 | Age, number of ApoE4 alleles |
| 26 | 121_155_137_188_12 7_1_161_53_198_110 _102_175_100_109_1 78_54_174_71_133_8 2_150_182_114 | 0.927 | 64.0 | 91.7 | 0.956 | 93.3 | 86.7 | Age, gender, number of ApoE4 alleles |
| 26 | 155_142_188_127_85 _1_161_189_154_110 _185_175_109_145_1 78_54_174_71_133_9 1_82_57_93_114 | 0.965 | 96.0 | 88.0 | 0.938 | 80.0 | 80.0 | Age, number of ApoE4 alleles |
| 26 | 121_155_137_142_18 8_127_1_198_110_19 3_175_100_145_174_ 71_106_152_159_149 _82_55_182_93_114 | 0.978 | 80.0 | 96.0 | 0.987 | 93.3 | 86.7 | Age, number of ApoE4 alleles |
| 26 | 121_155_188_127_14 6_30_1_189_38_110_ 68_193_175_192_100 _145_152_133_180_1 59_149_150_114 | 0.925 | 80.0 | 87.5 | 0.964 | 93.3 | 93.3 | Age, gender, number of ApoE4 alleles |
| 26 | 155_137_87_188_127 _1_84_198_154-38_1 10_176_109_48_71_1 52_111_190_133_91_ 159_149_182_93_114 | 0.946 | 83.3 | 87.5 | 0.964 | 86.7 | 86.7 | Age |

(continued)

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 26 | 121_155_137_87_142_188_127_1_198_110_9_175_100_109_145_54_174_152_133_14 9_82_104_182_114 | 0.943 | 91.7 | 83.3 | 0.96 | 93.3 | 86.7 | Age, number of ApoE4 alleles |
| 26 | 121_155_188_127_1_196_172_198_154_11 0_145_48_135_3_152_133_39_159_82_104_51_182_114 | 0.908 | 75.0 | 91.7 | 0.911 | 86.7 | 86.7 | Age, gender, number of ApoE4 alleles |
| 26 | 155_188_127_146_5_1_189_110_200_185_26_193_100_109_145_96_71_152_133_159_82_77_114 | 0.965 | 95.8 | 66.7 | 0.938 | 93.3 | 93.3 | Age, gender, number of ApoE4 alleles |
| 26 | 155_137_188_127_19 6_198_110_68_86_19 3_100_109_145_88_73_152_39_159_149_2_82_182_114 | 0.97 | 83.3 | 92.0 | 0.942 | 86.7 | 86.7 | Age, gender, number of ApoE4 alleles |
| 26 | 121_155_188_127_1_198_110_123_41_58_175_100_109_145_12 5_21_54_147_152_13 3_149_82_182_114 | 0.946 | 95.8 | 87.5 | 0.938 | 86.7 | 86.7 | Age, number of ApoE4 alleles |
| 26 | 155_137_87_142_188_127_1_198_110_193 _175_100_109_145_178_10_3_54_174_152 _133_91_149_114 | 0.975 | 95.8 | 92.0 | 0.96 | 93.3 | 86.7 | Age, number of ApoE4 alleles |
| 27 | 155_87_188_127_1_1_96_161_198_154_110 _186_68_200_175_10 0_109_48_73_152_13 3_159_77_182_114 | 0.973 | 91.7 | 92.0 | 0.996 | 93.3 | 93.3 | Age, gender, number of ApoE4 alleles |
| 27 | 155_87_188_127_30_198_110_9_175_109_48_10_3_96_73_152_133_91_159_149_2_82_51_93_114 | 0.943 | 79.2 | 100.0 | 0.938 | 86.7 | 93.3 | Age, number of ApoE4 alleles |
| 27 | 121_155_87_142_188_127_79_196_198_11 0_101_175_109_178_3_54_152_133_39_15 9_149_82_150_182_1 14 | 0.951 | 83.3 | 91.7 | 0.942 | 86.7 | 86.7 | Age, number of ApoE4 alleles |

(continued)

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 27 | 121_155_87_188_127 _1_196_189_110_200 _175_109_145_48_3_ 54_191_71_133_159_ 149_82_61_77_114 | 0.931 | 83.3 | 87.5 | 0.991 | 93.3 | 86.7 | Age, number of ApoE4 alleles |
| 27 | 155_137_142_188_12 7_1_79_198_154_110 _75_100_109_145_17 8_96_174_92_152_13 3_180_91_159_182_1 14 | 0.97 | 83.3 | 88.0 | 0.938 | 93.3 | 86.7 | Age, number of ApoE4 alleles |
| 27 | 121_155_137_87_142 _ 188_127_1_161_154 _110_175_100_109_1 45_62_3_174_133_18 0_91_159_82_114 | 0.99 | 95.8 | 100.0 | 0.956 | 80.0 | 86.7 | Age, gender, number of ApoE4 alleles |
| 27 | 155_137_188_127_1_ 183_196_198_189_15 4_38_194_110_68_17 5_100_109_145_152_ 133_159_149_82_77_ 114 | 0.96 | 88.0 | 91.7 | 0.987 | 86.7 | 93.3 | Age, number of ApoE4 alleles |
| 27 | 155_142_188_127_1_ 138_196_189_154_11 0_185_101_175_75_1 00_109_145_62_178_ 3_133_149_82_114 | 0.927 | 92.0 | 79.2 | 0.973 | 93.3 | 93.3 | Age, gender, number of ApoE4 alleles |
| 27 | 121_155_137_87_188 _127_1_196_198_110 _175_100_109_145_3 _71_152_133_159_82 _77_150_182_93_114 | 0.99 | 95.8 | 95.8 | 1 | 100.0 | 93.3 | Age, number of ApoE4 alleles |
| 27 | 155_87_142_188_127 _1_189_110_193_175 _109_145_62_178_3_ 71_133_180_91_159_ 149_82_104_182_93_ 114 | 0.952 | 91.7 | 92.0 | 0.982 | 93.3 | 100.0 | Age |
| 27 | 179_155_137_87_188 _127_30_1_161_198_ 189_154_110_175_10 0_109_145_178_71_1 33_39_91_159_82_11 4 | 0.913 | 79.2 | 91.7 | 1 | 100.0 | 100.0 | Age, number of ApoE4 alleles |
| 27 | 155_87_188_127_196 _161_198_110_68_18 5_86_193_175_109_1 45_178_10_152_141_ 133_149_82_51_182_ 114 | 0.965 | 83.3 | 92.0 | 0.996 | 86.7 | 100.0 | Age, number of ApoE4 alleles |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 27 | 179_155_137_188_12 7_5_79_198_189_154 _110_177_193_100_1 09_145_178_3_152_1 33_149_82_104_150_ 114 | 0.913 | 70.8 | 83.3 | 0.947 | 80.0 | 100.0 | Age, gender, |
| 27 | 155_87_188_127_84_ 198_110_185_175_10 9_145_62_178_10_54 _174_152_133_91_15 9_104_51_182_114 | 0.925 | 70.8 | 96.0 | 0.987 | 86.7 | 100.0 | Age, gender, number of ApoE4 alleles |
| 27 | 179_121_155_142_18 8_127_1_161_172_53 _198_110_175_100_1 09_105_3_54_133_14 9_82_77_182_93_114 | 0.94 | 88.0 | 83.3 | 1 | 100.0 | 93.3 | Age, number of ApoE4 alleles |
| 27 | 179_121_155_188_12 7_5_1_154_110_68_1 93_175_100_109_145 _10_3_108_152_133_ 91_159_149_82_114 | 0.965 | 91.7 | 87.5 | 0.956 | 86.7 | 93.3 | Age, number of ApoE4 alleles |
| 27 | 155_87_188_127_5_1 96_161_84_198_38_1 10_68_175_109_145_ 3_73_133_180_91_15 9_149_82_104_114 | 0.974 | 91.7 | 95.8 | 0.96 | 86.7 | 93.3 | Age, number of ApoE4 alleles |
| 27 | 155_137_188_127_18 3 196 110 68 86 19 3_175_100_145_135_ 71_73_152_133_180_ 159_149_2_77_51_18 2 | 0.927 | 80.0 | 100.0 | 0.951 | 93.3 | 86.7 | Age, number of ApoE4 alleles |
| 27 | 155_142_188_127_5_ 194_110_68_86_26_1 93_175_75_100_145_ 10_3_71_152_111_13 3_149_77_150_114 | 0.948 | 91.7 | 87.5 | 0.987 | 93.3 | 86.7 | Age, number of ApoE4 alleles |
| 27 | 179_121_155_142_18 8_127_1_110_128_17 5_100_109_125_178_ 10_135_3_174_92_13 3_42_149_82_182_11 4 | 0.93 | 83.3 | 88.0 | 0.96 | 93.3 | 86.7 | Age, number of ApoE4 alleles |
| 27 | 121_155_188_127_85 _5_1_79_198_154_11 0_145_135_54_71_73 _152_133_39_82_104 51_182_114 | 0.947 | 76.0 | 95.8 | 0.947 | 80.0 | 86.7 | Age, gender, number of ApoE4 alleles |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 27 | 121_155_87_142_188 _127_1_161_198_154 _110_185_193_109_1 78_48_174_152_133_ 159_2_82_104_114 | 0.957 | 91.7 | 87.5 | 0.924 | 80.0 | 86.7 | Age, gender, number of ApoE4 alleles |
| 27 | 121_155_137_87_142 _188_127_183_196_8 4_189_154_110_143_ 145_135_71_152_91_ 159_2_82_182_114 | 0.941 | 84.0 | 96.0 | 0.929 | 73.3 | 86.7 | Age, gender, number of ApoE4 alleles |
| 27 | 155_142_188_127_1_ 198_189_110_193_17 5_100_109_62_48_19 1_174_71_152_133_9 1_159_149_82_104_1 82_114 | 0.993 | 91.7 | 95.8 | 0.996 | 93.3 | 100.0 | Age |
| 27 | 155_87_142_188_127 _85_30_1_198_194_1 10_78_58_148_105_1 45_3_174_152_133_1 80_159_149_57_114 | 0.922 | 88.0 | 95.8 | 0.907 | 86.7 | 93.3 | Age, number of ApoE4 alleles |
| 27 | 121_155_137_87_188 _127_1_161_53_198_ 194_110_102_175_17 3_100_109_3_73_133 _149_82_104_93_114 | 0.976 | 95.8 | 95.8 | 0.947 | 86.7 | 93.3 | Age, number of ApoE4 alleles |
| 27 | 121_155_137_87_188 _127_161_198_154_1 10_69_175_100_3_71 _73_152_133_91_159 _82_182_93_114 | 0.983 | 87.5 | 91.7 | 0.942 | 80.0 | 93.3 | Age, gender, number of ApoE4 alleles |
| 27 | 121_155_137_87_188 _127_1_196_198_189 _154_110_185_109_1 78_3_54_174_71_152 _133_149_82_114 | 0.937 | 87.5 | 84.0 | 0.969 | 93.3 | 100.0 | Age, gender, number of ApoE4 alleles |
| 28 | 179_121_155_188_12 7_84_198_110_78_68 _148_185_175_100_1 09_174_108_152_141 _133_91_149_82_182 _93_114 | 0.95 | 87.5 | 87.5 | 0.933 | 80.0 | 80.0 | Age, gender, |
| 28 | 155_87_188_127_5_1 _196_161_198_110_6 8_185_100_109_145_ 63_54_37_108_133_1 59_2_82_104_114 | 0.901 | 87.5 | 79.2 | 0.942 | 93.3 | 80.0 | Age, gender, number of ApoE4 alleles |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 28 | 155_142_188_85_1_1 38_196_53_198_189_ 110_44_101_175_100 _145_152_180_159_1 49_2_82_51_182_93_ 114 | 0.918 | 80.0 | 91.7 | 0.947 | 93.3 | 80.0 | Age, number of ApoE4 alleles |
| 28 | 155_137_87_188_127 _1_161_198_189_154 _194_110_143_193_1 75_109_145_3_111_1 33_39_91_149_104_9 3_114 | 0.958 | 88.0 | 91.7 | 0.969 | 80.0 | 93.3 | Age, number of ApoE4 alleles |
| 28 | 179_121_155_188_12 7_1_198_110_200_14 8_175_75_100_109_1 45_125_63_54_174_9 2_190_133_91_82_10 4 | 0.915 | 87.5 | 70.8 | 0.969 | 100.0 | 73.3 | Age, gender, number of ApoE4 alleles |
| 28 | 121_155_142_188_94 _1_161_154_110_185 _175_75_100_109_3_ 96_174_108_92_152_ 133_180_91_2_82_11 4 | 0.932 | 95.8 | 75.0 | 0.911 | 100.0 | 73.3 | Age, number of ApoE4 alleles |
| 28 | 121_155_137_87_188 _127_1_84_198_110_ 148_175_75_100_109 _145_178_3_54_174_ 152_133_180_91_149 _114 | 0.963 | 91.7 | 84.0 | 0.973 | 93.3 | 73.3 | Age, number of ApoE4 alleles |
| 28 | 121_155_87_188_127 _1_196_198_110_148 _185_75_109_145_3_ 54_96_174_71_152_1 33_91_159_2_82_114 | 0.948 | 91.7 | 91.7 | 0.96 | 100.0 | 80.0 | Age, number of ApoE4 alleles |
| 28 | 155_142_188_127_94 _1_84_189_194_110_ 9_185_175_75_109_1 45_88_135_71_152_1 90_133_180_82_182_ 114 | 0.905 | 83.3 | 80.0 | 0.951 | 93.3 | 86.7 | Age, number of ApoE4 alleles |
| 28 | 155_87_142_188_127 _1_79_161_198_154_ 110 100 109 178 17 4_92_152_133_91_15 9_149_82_55_93_114 | 1 | 95.8 | 100.0 | 0.951 | 86.7 | 86.7 | Age, gender, number of ApoE4 alleles |
| 28 | 179_155_137_87_188 _127_1_196_161_198 _189_110_86_175_10 0_109_108_152_133_ 39_159_149_82_77_9 3_114 | 0.998 | 100.0 | 95.8 | 0.956 | 86.7 | 93.3 | Age, number of ApoE4 alleles |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 28 | 121_155_87_142_188 _127_138_196_161_1 98_189_110_56_185_ 101_100_10_73_152_ 133_180_149_70_51_ 182_114 | 0.925 | 70.8 | 95.8 | 0.951 | 93.3 | 93.3 | Age, number of ApoE4 alleles |
| 28 | 155_188_127_1_45_1 94_110_157_68_200_ 86_193_75_109_145_ 125_3_152_133_159_ 149_2_82_51_150_11 4 | 0.967 | 91.7 | 75.0 | 0.982 | 100.0 | 86.7 | Age, number of ApoE4 alleles |
| 28 | 121_155_137_87_127 _1_84_198_189_110_ 185_177_193_100_10 9_62_10_88_191_133 _91_159_82_104 _182 _114 | 0.953 | 79.2 | 91.7 | 0.978 | 93.3 | 93.3 | Age, number of ApoE4 alleles |
| 28 | 121_155_87_142_188 _127_1_196_172_198 _110_102_26_193_17 5_109_145_62_71_73 _133_149_82_51_114 | 0.908 | 70.8 | 87.5 | 0.973 | 93.3 | 86.7 | Age, gender, number of ApoE4 alleles |
| 28 | 13_121_87_142_188_ 127_30_1_198_154_1 10_175_192_109_145 _178_10_174_152_18 0_91_159_149_82_18 2_114 | 0.917 | 75.0 | 84.0 | 0.991 | 100.0 | 86.7 | Age, number of ApoE4 alleles |
| 28 | 155_137_87_142_127 _196_198_189_38_11 0_68_175_100_109_1 45_174_71_152_133_ 91_159_2_82_77_182 _114 | 0.974 | 87.5 | 95.8 | 0.987 | 86.7 | 100.0 | Age, number of ApoE4 alleles |
| 28 | 155_137_87_188_127 _198_110_68_185_17 7_175_100_145_63_3 _174_71_152_91_159 _2_82_77_51_114 | 0.912 | 68.0 | 87.5 | 0.978 | 93.3 | 93.3 | Age, gender, number of ApoE4 alleles |
| 28 | 155_137_142_188_12 7_198_189_110_56_ 1 76_193_175_75_100_ 97_145_174_73_152_ 133_159_82_77_182_ 114 | 0.937 | 84.0 | 83.3 | 0.929 | 73.3 | 93.3 | Age, gender, number of ApoE4 alleles |
| 28 | 155_137_87_142_188 _127_1_79_196_84_1 98_110_175_100_109 _145_63_152_190_13 3_180_91_159_149_9 3_114 | 0.96 | 91.7 | 92.0 | 1 | 100.0 | 100.0 | Age, number of ApoE4 alleles |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 28 | 155_87_188_127_1_1 96_84_198_110_128_ 56_185_175_109_178 _3_174_133_180_39_ 159_149_82_77_182_ 114 | 0.953 | 79.2 | 96.0 | 0.982 | 93.3 | 93.3 | Age, gender, |
| 28 | 179_121_155_137_87 _188_127_196_198_1 54_110_193_175_25_ 100_109_178_48_3_1 52_133_91_82_182_1 14 | 0.977 | 100.0 | 88.0 | 0.978 | 93.3 | 80.0 | Age, gender, number of ApoE4 alleles |
| 28 | 155_87_142_188_127 _5_1_79_196_161_15 4_110_41_58_102_17 5_100_109_145_48_7 1_133_91_82_93_114 | 0.96 | 88.0 | 91.7 | 0.982 | 93.3 | 93.3 | Age, number of ApoE4 alleles |
| 28 | 155_188_127_1_194_ 110_200_86_193_175 _100_109_145_125_8 8_3_71_152_133_180 _39_159_149_77_114 _50 | 0.957 | 84.0 | 87.5 | 0.991 | 93.3 | 93.3 | Age, number of ApoE4 alleles |
| 28 | 179_155_137_87_188 _127_85_1_161_84_1 10_148_153_101_175 _109_178_63_71_152 _133_91_159_149_ 77 _114 | 0.938 | 95.8 | 84.0 | 0.982 | 86.7 | 93.3 | Age, number of ApoE4 alleles |
| 28 | 155_137_87_188_127 _1_161_198_110_193 _175_100_ 109_97_ 17 8_48_54_174_108_15 2_133_159_82_61_11 4 | 0.988 | 92.0 | 87.5 | 0.982 | 93.3 | 93.3 | Age, gender, number of ApoE4 alleles |
| 28 | 179_155_137_142_18 8_127_1_161_198_18 9_110_58_175_100_1 09_10_3_108_73_152 _133_159_82_ 77_182 _114 | 0.978 | 87.5 | 92.0 | 1 | 100.0 | 86.7 | Age, number of ApoE4 alleles |
| 28 | 121_155_87_142_188 _127_1_161_198_154 _110_193_175_192_1 00_109_169_73_163_ 133_159_149_82_77_ 182_93_114 | 0.962 | 91.7 | 84.0 | 0.96 | 80.0 | 100.0 | Age |
| 28 | 179_155_87_188_127 _1_161_198_189_154 _194_110_68_86_175 _25_199_152_133_18 0_149_2_104_77_114 | 0.957 | 87.5 | 100.0 | 0.964 | 86.7 | 93.3 | Age, gender, number of ApoE4 alleles |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 28 | 155_137_87_142_188 _127_1_198_154_110 _68_86_75_100_54_1 74_92_152_111_190_ 133_180_159_57_114 | 0.935 | 88.0 | 87.5 | 0.973 | 86.7 | 93.3 | Age, gender, number of ApoE4 alleles |
| 28 | 155_87_188_127_1_1 96_161_198_110_26_ 193_175_100_109_14 5_178_48_71_73_152 _133_149_82_104_18 2_114 | 0.958 | 75.0 | 96.0 | 0.947 | 93.3 | 86.7 | Age, number of ApoE4 alleles |
| 28 | 155_87_188_127_1_1 61_198_110_185_175 _25_100_109_178_54 _108_152_141_133_9 1_149_82_104_182_1 14 | 0.986 | 91.7 | 100.0 | 0.969 | 86.7 | 93.3 | Age, gender, number of ApoE4 alleles |
| 28 | 155_87_142_188_127 _5_1_198_154_110_1 48_101_193_175_109 _178_48_135_73_152 _133_91_159_82_51_ 182_114 | 0.973 | 87.5 | 92.0 | 1 | 100.0 | 100.0 | Age |
| 28 | 155_87_188_127_146 _30_1_196_198_154_ 38_110_193_175_100 _167_178_54_133_18 0_159_2_77_18_20_1 82_114 | 0.913 | 79.2 | 84.0 | 0.951 | 73.3 | 100.0 | Age |
| 28 | 121_155_87_142_188 _127_146_1_196_198 _189_154_38_110_17 7_100_109_178_174_ 152_133_91_159_82_ 182_114 | 0.985 | 91.7 | 96.0 | 0.987 | 86.7 | 100.0 | Age, number of ApoE4 alleles |
| 28 | 155_137_87_188_127 _196_84_198_154_19 4_110_68_175_73_15 2_133_180_159_149_ 2_82_104_77_182_11 _4 | 0.912 | 87.5 | 80.0 | 0.951 | 80.0 | 86.7 | Age, gender, number of ApoE4 alleles |
| 29 | 155_137_142_188_12 7_138_53_198_110_4 1_175_75_109_145_3 _174_71_152_133_18 0_159_149_2_82_51_ 182_114 | 0.946 | 87.5 | 79.2 | 0.951 | 80.0 | 80.0 | Age, number of ApoE4 alleles |
| 29 | 87_188_127_1_196_1 61_84_53_198_110_2 00_193_175_109_48_ 135_54_174_71_133_ 180_91_159_82_104_ 182_114 | 0.91 | 91.7 | 75.0 | 0.964 | 86.7 | 80.0 | Age, number of ApoE4 alleles |

164

EP 4 130 259 A1

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 29 | 155_142_188_127_1_ 53_110_81_185_153_ 101_175_173_109_11 7_152_133_180_39_9 1_159_149 _2_ 82 _182 _114 | 0.951 | 95.8 | 91.7 | 0.956 | 93.3 | 86.7 | Age, gender, number of ApoE4 alleles |
| 29 | 155_87_188_127_183 _198_189_154_194_1 10_56_58_175_75_10 9_199_145_3_174_73 _152_133_159_149_5 7_93_114 | 0.94 | 76.0 | 95.8 | 0.907 | 66.7 | 93.3 | Age, number of ApoE4 alleles |
| 29 | 121_155_87_188_127 _1_196_161_198_154 _110_58_175_109_14 5_125_178_3_191_92 _152_133_149_82_93 _114 | 0.905 | 88.0 | 87.5 | 0.991 | 100.0 | 93.3 | Age, gender, number of ApoE4 alleles |
| 29 | 179_155_137_87_188 _127_161_198_189_1 10_4_100_109_145_1 78_10_3_54_174_133 _39_91_159_149_82_ 51_114 | 0.911 | 66.7 | 87.5 | 0.969 | 80.0 | 93.3 | Age, number of ApoE4 alleles |
| 29 | 155_137_87_142_188 _127_1_198_154_110 _175_100_109_145_1 78_3_47_54_133_39_ 91_149_82_104_182_ 114 | 0.979 | 100.0 | 87.5 | 0.996 | 100.0 | 93.3 | Age, gender, number of ApoE4 alleles |
| 29 | 179 _155 _142 _ 188 _ 12 7_5_1_161_198_154_ 110_143_69_193_175 _173_109_145_178_1 0_174_71_159_82_93 _114 | 0.93 | 88.0 | 87.5 | 0.964 | 73.3 | 93.3 | Age, gender, number of ApoE4 alleles |
| 29 | 179_155_137_87_188 _127_1_161_84_194_ 110_185_175_109_17 8_63_71_152_133_18 0_91_159_149_82_77 _182_114 | 0.967 | 95.8 | 88.0 | 0.956 | 66.7 | 93.3 | Age, number of ApoE4 alleles |
| 29 | 155_87_142_188_127 _30_1_198_154_110_ 176_100_109_145_48 _135_3_71_152_180_ 39_149_82_51_93_11 4 | 0.982 | 88.0 | 95.8 | 0.902 | 73.3 | 86.7 | Age, gender, number of ApoE4 alleles |
| 29 | 121_155_137_87_142 _188_127_80_198_11 0_101_193_175_100_ 109_145_54_152_111 _133_159_149_82_18 2_93_114 | 0.997 | 87.5 | 100.0 | 0.938 | 93.3 | 86.7 | Age, gender, number of ApoE4 alleles |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 29 | 121_155_137_87_188 _127_161_189_38_11 0_56_193_175_109_6 2_178_3_191_133_18 0_91_159_149_82_77 _114 | 0.965 | 91.7 | 91.7 | 0.956 | 93.3 | 80.0 | Age, gender, number of ApoE4 alleles |
| 29 | 121_155_137_87_188 _127_1_198_194_110 _193_175_100_109_1 99_145_10_3_71_133 _149_2_82_77_93_11 4 | 0.976 | 100.0 | 87.5 | 0.996 | 100.0 | 93.3 | Age, gender, number of ApoE4 alleles |
| 29 | 121_155_142_188_12 7_5_196_161_198 _ 15 4_110_26_175_100 1 09_145_63_10_54_15 2_133_159_2_82_104 _114 | 0.983 | 95.8 | 95.8 | 0.991 | 100.0 | 93.3 | Age, gender, number of ApoE4 alleles |
| 29 | 121_155_137_87_127 _1_79_198_154_110_ 185_175_109_145_17 8_10_3_152_91_159_ 149_82_104_52_182_ 114 | 0.925 | 75.0 | 84.0 | 0.991 | 86.7 | 93.3 | Age, gender, number of ApoE4 alleles |
| 29 | 121_155_137_87_142 _127_1_194_110_86_ 26_193_175_25_75_1 09_145_3_174_152_1 33_180_39_159_82_1 82_114 | 0.99 | 87.5 | 95.8 | 0.951 | 86.7 | 86.7 | Age, number of ApoE4 alleles |
| 29 | 155_137_142_188_12 7_1_196_161_198_11 0_143_185_153_175_ 109_145_62_178_48_ 174_73_152_133_159 _82_104_114 | 0.966 | 96.0 | 76.0 | 0.973 | 100.0 | 80.0 | Age, number of ApoE4 alleles |
| 29 | 155_137_142_188_12 7_30_1_198_194_110 _9_58_86_175_100_1 67_145_98_152_133_ 180_159_82_104_51_ 150_114 | 0.905 | 79.2 | 87.5 | 0.942 | 86.7 | 93.3 | Age, number of ApoE4 alleles |
| 29 | 121_155_87_188_127 _1_79_161_198_154_ 110_78_175_100_109 _54_174_71_152_133 _180_149_82_182_93 _114 | 0.958 | 84.0 | 88.0 | 0.978 | 93.3 | 100.0 | Age, gender, number of ApoE4 alleles |
| 29 | 155_87_142_188_127 _1_80_161_53_187_1 89_154_110_175_75_ 31_109_62_48_54_19 1_92_71_133_149_82 _114 | 0.928 | 84.0 | 80.0 | 0.911 | 80.0 | 93.3 | Age, number of ApoE4 alleles |

(continued)

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 29 | 155_87_142_188_127 _146_158_196_53_19 8_110_68_101_25_10 0_109_145_3_54_152 _180_91_159_82_182 _114_12 | 0.911 | 79.2 | 87.5 | 0.924 | 86.7 | 86.7 | Age, number of ApoE4 alleles |
| 29 | 155_137_87_188_127 _5_1_161_84_198_19 4_110_175_100_109_ 145_3_133_180_159_ 149_82_104_150_182 _114 | 0.943 | 91.7 | 87.5 | 0.942 | 93.3 | 93.3 | Age, gender, number of ApoE4 alleles |
| 29 | 155_137_87_142_188 _127_1_53_198_189_ 154 110 175 192 10 0_109_145_125_178_ 54_174_133_159_82_ 182_114 | 0.955 | 92.0 | 87.5 | 0.973 | 93.3 | 73.3 | Age, gender, number of ApoE4 alleles |
| 29 | 155_142_188_127_14 6_1_53_189_154_38_ 194_110_185_175_10 0_109_145_135–3_96 _174_71_133_159_82 _182_114 | 0.939 | 95.8 | 83.3 | 0.973 | 93.3 | 86.7 | Age, number of ApoE4 alleles |
| 29 | 155_188_127_5_1_19 8_110_176_68_175_1 45_125_98_10_3_71_ 152_111_133_39_159 _149_82_104_51_150 _114 | 0.906 | 75.0 | 87.5 | 0.973 | 100.0 | 80.0 | Age, number of ApoE4 alleles |
| 29 | 155_137_142_188_12 7_146_1_189_194_11 0_143_200_193_175_ 100_109_145_156_10 _152_180_91_159_82_ 77_182_114 | 0.948 | 92.0 | 83.3 | 0.938 | 80.0 | 93.3 | Age, number of ApoE4 alleles |
| 29 | 155_137_142_188_12 7_146_196_53_154_1 10_41_68_200_175_1 00_109_97_145_63_1 52_190_180_91_159_ 182_114 | 0.922 | 83.3 | 87.5 | 0.942 | 80.0 | 93.3 | Age, gender, number of ApoE4 alleles |
| 29 | 155_137_87_188_127 _30_79_161_53_198_ 194_110_185_175_10 0_109_97_174_71_13 3_159_149_2_82_77_ 182_114 | 0.912 | 76.0 | 79.2 | 0.991 | 93.3 | 93.3 | Age, gender, |
| 29 | 179_155_142_188_12 7_1_196_198_189_11 0_44_78_148_185_17 5_100_109_105_167_ 145_62_152_133_39_ 82_114 | 0.935 | 92.0 | 83.3 | 0.964 | 100.0 | 80.0 | Age, gender, number of ApoE4 alleles |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 29 | 179_87_142_188_127 _1_196_161_53_198_ 154_110_58_153_175 _109_10_3_141_133_ 180_91_149_82_182_ 114 | 0.903 | 91.7 | 80.0 | 0.92 | 80.0 | 93.3 | Age, gender, number of ApoE4 alleles |
| 29 | 121_155_87_188_127 _1_79_158_196_161_ 198_38_110_102_175 _100_109_135_54_10 8_71_133_149_82_10 4_114 | 0.939 | 100.0 | 62.5 | 0.942 | 86.7 | 86.7 | Age, gender, number of ApoE4 alleles |
| 30 | 121_155_137_87_188 _127_94_1_84_194_1 10_148_193_175_75_ 100_109_145_178_3_ 54_152_133_149_2_8 2_77_114 | 0.974 | 95.8 | 87.5 | 0.969 | 100.0 | 86.7 | Age, number of ApoE4 alleles |
| 30 | 155_137_87_188_127 _1_161_198_154_110 _185_175_109_145_1 78_63_108_71_152_1 33_91_159_149_82_1 04 77 150 114 | 0.982 | 95.8 | 92.0 | 0.938 | 80.0 | 80.0 | Age, number of ApoE4 alleles |
| 30 | 121_155_137_142_18 8_127_161_198_187_ 194_110_148_26_177 _175_109_145_178_1 0_71_152_133_91_15 9_149_82_182_114 | 0.988 | 95.8 | 84.0 | 0.933 | 86.7 | 80.0 | Age, number of ApoE4 alleles |
| 30 | 121_155_87_142_188 _127_196_161_198_1 89_38_110_200_175_ 75_100_109_169_125 _71_152_133_91_149 _82_182_114 | 0.978 | 84.0 | 91.7 | 0.951 | 86.7 | 86.7 | Age, gender, number of ApoE4 alleles |
| 30 | 155_142_188_127_30 _1_161_189_110_153 _193_175_109_145_6 2_178_98_3_191_71_ 152_133_39_149_82_ 51_182_114 | 0.969 | 87.5 | 91.7 | 0.902 | 73.3 | 80.0 | Age, number of ApoE4 alleles |
| 30 | 121_155_87_142_188 _127_30_161_198_18 7_110_101_193_175_ 100_109_145_178_10 _152_111_133_39_14 9_82_51_182_114 | 0.957 | 83.3 | 88.0 | 0.916 | 80.0 | 80.0 | Age, number of ApoE4 alleles |
| 30 | 121_155_137_142_18 8_127_30_1_196_161 _198_110_193_175_1 00_109_10_174_152_ 133_180_91_149_82_ 51_182_114 | 0.908 | 83.3 | 76.0 | 0.907 | 86.7 | 80.0 | Age, gender, number of ApoE4 alleles |

(continued)

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 30 | 155_142_188_127_53 _198_154_110_176_ 6 9_193_192_100_109_ 145_48_10_152_133 39_91_159_149_82_7 7_182_114 | 0.971 | 80.0 | 100.0 | 1 | 100.0 | 86.7 | Age, gender, number of ApoE4 alleles |
| 30 | 121_155_87_142_188 _127_198_110_102_1 01_175_100_109_169 _145_3_174_108_71_ 152_133_91_159_2_8 2_57_182_114 | 0.94 | 88.0 | 87.5 | 1 | 93.3 | 100.0 | Age, number of ApoE4 alleles |
| 30 | 121_155_137_87_142 _188_127_196_161_1 98_110_41_193_175_ 173_75_100_109_97_ 71_73_133_91_159_1 49_82_77_114 | 0.969 | 95.8 | 87.5 | 0.996 | 93.3 | 93.3 | Age, number of ApoE4 alleles |
| 30 | 179_155_188_127_14 6_198_187_38_110_2 00_193_175_100_109 _199_178_71_152_13 3_180_91_159_149_7 7_51_93_114 | 0.915 | 88.0 | 88.0 | 0.951 | 86.7 | 93.3 | Age, gender, number of ApoE4 alleles |
| 30 | 155_137_142_188_12 7 196 198 189 110_ 78_185_175_100_109 _145_3_174_73_152_ 141_133_91_159_149 _82_51_182_114 | 0.955 | 75.0 | 92.0 | 0.982 | 93.3 | 100.0 | Age, number of ApoE4 alleles |
| 30 | 155_137_87_142_188 _127_1_196_161_198 _110_78_90_193_175 _100_109_145_71_73 _133_159_149_82_51 _182_114 | 0.918 | 79.2 | 84.0 | 1 | 100.0 | 93.3 | Age, gender, number of ApoE4 alleles |
| 30 | 179_155_87_188_127 _85_79_196_198_187 _154_110_86_175_10 9_145_88_3_174_71_ 134_152_39_159_82_ 57_114 | 0.983 | 92.0 | 91.7 | 0.951 | 80.0 | 80.0 | Age, gender, number of ApoE4 alleles |
| 30 | 155_137_87_188_127 _1_79_196_161_189_ 110_153_175_100_10 9_145_62_54_191_10 8_71_73_133_91_159 _149_82_114 | 0.952 | 80.0 | 87.5 | 0.938 | 66.7 | 93.3 | Age, gender, |
| 30 | 179_155_142_188_12 7_1_79_158_189_154 _110_4_185_175_100 _109_145_54_174_71 _152_133_159_82_51 _93_114 | 0.955 | 92.0 | 92.0 | 0.956 | 73.3 | 86.7 | Age, gender, number of ApoE4 alleles |

169

EP 4 130 259 A1

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 30 | 179 _155 _142 _ 188 _ 12 7_1_80_198_187_189 _110_148_175_100_1 09_97_145_135_54_1 74_92_152_133_149_ 82_51_114 | 0.938 | 91.7 | 79.2 | 0.973 | 93.3 | 86.7 | Age, gender, number of ApoE4 alleles |
| 30 | 179_121_155_142_18 8_127_5_1_194_110_ 9_4_75_100_109_145 _125_178_3_54_92_1 52_133_91_82_182_1 14 | 0.931 | 84.0 | 88.0 | 0.973 | 100.0 | 86.7 | Age, gender, number of ApoE4 alleles |
| 30 | 121_155_87_188_127 _198_110_102_185_8 6_175_109_145_178_ 96_71_190_133_180_ 91_159_149_82_20_9 3_114_12 | 0.91 | 87.5 | 79.2 | 0.973 | 100.0 | 93.3 | Age, gender, number of ApoE4 alleles |
| 30 | 179_121_155_142_18 8_127_5_84_198_189 _154_194_110_78_14 3_175_75_145_135_4 7_174_71_73_152_13 3_104_182_114 | 0.914 | 80.0 | 84.0 | 0.96 | 86.7 | 100.0 | Age, number of ApoE4 alleles |
| 30 | 179_155_188_127_1_ 161_198_154_110_58 _153_26_175_192_10 9_97_145_178_10_17 4_152_133_91_159_1 04_93_114 | 0.974 | 96.0 | 92.0 | 0.987 | 100.0 | 86.7 | Age, gender, number of ApoE4 alleles |
| 30 | 155_188_127_1_79_8 4_189_110_78_176_ 5 8_185_175_75_100_1 09_145_48_3_152_14 1_133_39_159_82_77 _51_114 | 0.976 | 87.5 | 91.7 | 0.982 | 100.0 | 86.7 | Age, number of ApoE4 alleles |
| 30 | 155_137_142_188_12 7_53_189_110_41_20 0_175_25_75_31_100 _125_156_54_71_106 _152_133_180_159_2 _77_182_114 | 0.901 | 87.5 | 87.5 | 0.907 | 93.3 | 93.3 | Age, number of ApoE4 alleles |
| 30 | 179_155_87_188_127 _1_79_196_198_189_ 110_153_193_175_10 0_109_3_152_190_13 3_39_91_159_149_82 _61_182_114 | 0.97 | 95.8 | 83.3 | 0.991 | 86.7 | 100.0 | Age, number of ApoE4 alleles |
| 30 | 155_87_142_188_127 _30_1_198_189_154_ 194_110_68_86_193_ 175_100_109_97_145 _10_152_133_159_77 _57_182_114 | 0.933 | 88.0 | 88.0 | 1 | 80.0 | 100.0 | Age, number of ApoE4 alleles |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 30 | 121_155_137_87_188 _127_1_198_110_56_ 193_175_192_100_10 9_145_178_48_10_83_71_152_133 _180_15 9_149_182_114 | 0.97 | 83.3 | 87.5 | 0.956 | 86.7 | 93.3 | Age, number of ApoE4 alleles |
| 30 | 121_155_87_188_127 _1_161_198_189_154 _38_110_175_109_48 _108_71_73_152_133 _91_159_82_77_182_ 114_12 | 0.97 | 87.5 | 95.8 | 0.933 | 86.7 | 80.0 | Age, gender, number of ApoE4 alleles |
| 30 | 121_155_137_87_188 _127_1_198_110_176 _185_175_109_48_10 _3_54_73_133_180_9 1_159_149_82_51_18 2_93_114 | 0.946 | 83.3 | 87.5 | 0.956 | 86.7 | 93.3 | Age, number of ApoE4 alleles |
| 31 | 179_155_137_188_12 7_5_198_154_110_68 _200_148_86_175_10 9_145_71_152_190_1 80_39_159_149_2_82 _150_182_114 | 0.97 | 84.0 | 92.0 | 0.969 | 86.7 | 86.7 | Age, gender, number of ApoE4 alleles |
| 31 | 155_87_142_188_127 _161_84_189_110_18 6_24_58_175_100_10 9_3_96_174_133_39_ 91_159_2_82_104_18 2_93_114 | 0.953 | 91.7 | 88.0 | 0.942 | 86.7 | 80.0 | Age, gender, number of ApoE4 alleles |
| 31 | 179_155_87_142_188 _127_1_183_53_198_ 189_110_68_148_175 _100_109_3_99_152_ 133_180_39_159_149 _2_82_182_114 | 0.986 | 79.2 | 100.0 | 0.964 | 86.7 | 86.7 | Age, gender, |
| 31 | 155_137_87_142_188 _127_1_79_196_198_ 189_38_110_75_109_ 145_10_174_92_71_1 52_190_133_180_91_ 104_182_114 | 0.928 | 91.7 | 76.0 | 0.96 | 86.7 | 93.3 | Age, gender, number of ApoE4 alleles |
| 31 | 121_155_137_87_188 _127_1_196_172_84_ 194_110_200_102_86 _193_75_109_3_71_1 33_159_149_2_82_77 _182_114 | 0.955 | 95.8 | 79.2 | 0.964 | 100.0 | 66.7 | Age, gender, number of ApoE4 alleles |
| 31 | 155_137_87_168_188 _127_161_53_198_18 7_110_101_193_175_ 100_109_10_152_141 _111_133_42_91_159 _82_51_93_114 | 0.965 | 83.3 | 91.7 | 0.942 | 73.3 | 93.3 | Age, gender, number of ApoE4 alleles |

(continued)

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 31 | 121_155_87_142_188 _127_94_1_196_161_ 198_189_110_102_17 5_100_109_145_48_5 4_174_39_91_159_82 _77_182_114 | 0.917 | 96.0 | 79.2 | 0.951 | 80.0 | 100.0 | Age, gender, number of ApoE4 alleles |
| 31 | 155_87_188_127_30_ 1_79_196_161_154_3 8_194_110_86_26_10 0_145_3_54_174_71_ 152_133_39_159_149 _104_57_114 | 0.927 | 87.5 | 83.3 | 0.991 | 93.3 | 100.0 | Age, number of ApoE4 alleles |
| 31 | 155_137_142_188_12 7_1_196_161_53_198 _189_154_38_110_68 _102_175_100_109_9 7_98_54_133_91_149 _82_182_114 | 0.953 | 83.3 | 87.5 | 0.973 | 86.7 | 93.3 | Age, gender, number of ApoE4 alleles |
| 31 | 155_137_87_142_188 _127_85_1_79_198_1 89_154_110_148_185 _100_109_145_135_1 74_71_152_133_91_1 59_149_82_182_114 | 0.968 | 87.5 | 88.0 | 0.902 | 73.3 | 86.7 | Age, number of ApoE4 alleles |
| 31 | 155_137_87_142_188 _127_146_30_1_79_5 3_189_154_110_192_ 100_109_145_48_174 _71_152_133_159_82 _150_182_93_114 | 0.978 | 100.0 | 92.0 | 0.924 | 80.0 | 93.3 | Age, number of ApoE4 alleles |
| 31 | 121_155_87_142_188 _79_53_198_154_110 _185_101_175_75_10 9_169_125_3_174_92 _71_152_141_133_91 _149_104_114 | 0.942 | 80.0 | 87.5 | 0.916 | 80.0 | 80.0 | Age, gender, number of ApoE4 alleles |
| 31 | 121_155_137_87_142 _188_127_1_110_177 _193_175_192_100_1 09_178_54_73_152_1 11_133_180_91_159_ 149_104_51_150_114 | 0.905 | 92.0 | 75.0 | 0.947 | 100.0 | 80.0 | Age, number of ApoE4 alleles |
| 31 | 121_155_188_127_5_ 196_84_198_187_189 _110_78_102_148_86 _177_175_100_109_1 45_3_174_133_180_1 49_2_182_114 | 0.917 | 87.5 | 72.0 | 0.96 | 86.7 | 93.3 | Age, gender, number of ApoE4 alleles |
| 31 | 155_137_87_142_188 _127_146_196_198_1 89_154_38_110_148_ 177_109_135_3_108_ 152_180_39_91_159_ 149_2_182_114 | 0.932 | 79.2 | 92.0 | 0.956 | 66.7 | 100.0 | Age, gender, number of ApoE4 alleles |

EP 4 130 259 A1

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 31 | 155_137_87_142_188_127_1_198_110_41_185_193_175_109_97_167_62_178_10_54_71_152_190_133_91_149_104_182_114 | 0.955 | 79.2 | 96.0 | 0.973 | 86.7 | 100.0 | Age, number of ApoE4 alleles |
| 31 | 121_155_137_142_18 8 127 1 189 194 11 0_58_193_175_109_1 69_145_125_178_54_174_133_180_91_159_82_104_51_182_114 | 0.931 | 79.2 | 87.5 | 0.96 | 93.3 | 86.7 | Age, number of ApoE4 alleles |
| 32 | 155_87_188_127_85_161_84_198_154_110_68_86_193_175_109_3_191_73_152_180_39_91_159_149_82_7 7_182_93_114 | 0.952 | 87.5 | 96.0 | 0.973 | 93.3 | 93.3 | Age, gender, number of ApoE4 alleles |
| 32 | 155_137_87_142_188_127_1_80_196_198_189_154_194_110_41_148_177_175_100_109_145_178_48_174_133_91_2_82_93_114 | 0.957 | 91.7 | 84.0 | 0.969 | 100.0 | 86.7 | Age, number of ApoE4 alleles |
| 32 | 121_155_137_87_142_188_127_1_84_198_194_110_153_193_17 5_100_109_48_135_3_96_174_133_39_91_149_2_82_93_114 | 0.957 | 79.2 | 91.7 | 0.947 | 93.3 | 86.7 | Age, number of ApoE4 alleles |
| 32 | 155_142_188_127_1_79_198_189_154_67_110_175_75_100_109_145_62_125_174_108_190_133_39_91_15 9_149_82_55_114 | 0.973 | 88.0 | 100.0 | 0.947 | 80.0 | 86.7 | Age, gender, number of ApoE4 alleles |
| 32 | 155_137_188_127_1_79_53_154_110_186_143_58_86_69_100_1 45_178_3_54_152_13 3_180_91_159_149_7 7_57_182_114 | 0.968 | 92.0 | 95.8 | 0.938 | 86.7 | 80.0 | Age, gender, number of ApoE4 alleles |
| 32 | 121_155_137_87_142_188_127_85_1_189_110_128_157_101_19 3_175_118_75_31_10 0_145_178_88_3_54_152_133_2_82_114 | 0.973 | 84.0 | 95.8 | 0.916 | 80.0 | 86.7 | Age, number of ApoE4 alleles |
| 32 | 179_121_155_142_18 8_127_1_161_53_154_110_148_193_175_3 1_100_109_62_48_15 6_88_54_71_152_111_133_149_82_182_11 4 | 0.948 | 95.8 | 80.0 | 0.978 | 100.0 | 80.0 | Age, number of ApoE4 alleles |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 32 | 155_87_188_127_161 _53_198_189_110_19 3_175_109_145_48_1 35_174_71_152_111_ 133_180_39_91_159_ 82_104_77_182_114 | 0.963 | 87.5 | 88.0 | 0.991 | 100.0 | 93.3 | Age, gender, number of ApoE4 alleles |
| 32 | 179_121_155_137_87 _142_188_127_198_1 89_110_148_192_100 _109_145_135_174_1 52_133_180_113_91_ 159_149_77_18_182_ 114 | 0.982 | 88.0 | 100.0 | 0.951 | 86.7 | 86.7 | Age, gender, number of ApoE4 alleles |
| 32 | 121_155_87_188_127 _1_161_198_110_128 _200_148_185_175_1 92_75_100_109_145_ 48_174_152_133_180 _149_82_104_182_11 4 | 0.952 | 96.0 | 79.2 | 0.938 | 86.7 | 86.7 | Age, gender, number of ApoE4 alleles |
| 33 | 155_137_188_127_5_ 1_196_53_198_154_1 10_74_68_101_175_1 92_109_145_10_3_10 8_71_133_159_149_8 2_77_150_182_93_11 _ 4 | 0.98 | 87.5 | 96.0 | 0.924 | 86.7 | 86.7 | Age, gender, |
| 33 | 155_137_87_127_5_1 _84 _198 _110 _ 185 _15 3_175_75_100_109_1 45_178_10_174_71_1 41_190_133_180_39_ 91_149_182_93_114 | 0.903 | 79.2 | 95.8 | 0.964 | 93.3 | 86.7 | Age, gender, number of ApoE4 alleles |
| 33 | 121_155_87_142_188 _127_94_79_196_198 _189_154_110_102_1 75_75_109_145_62_4 8_3_174_108_133_19 7_159_82_77_150_11 4 | 0.938 | 80.0 | 87.5 | 0.929 | 86.7 | 93.3 | Age, gender, number of ApoE4 alleles |
| 33 | 121_155_87_142_188 _127_94_1_161_198_ 187_110_185_175_10 9_167_145_178_54_1 52_133_39_91_159_1 49_2_82_61_51_114 | 0.951 | 91.7 | 83.3 | 0.973 | 93.3 | 86.7 | Age, gender, number of ApoE4 alleles |
| 33 | 121_155_137_142_18 8_127_5_1_161_198_ 189_154_110_185_15 3_177_175_100_109_ 178_10_54_174_152_ 133_91_159_149_82_ 104_114 | 0.95 | 87.5 | 91.7 | 1 | 100.0 | 100.0 | Age, number of ApoE4 alleles |
| 33 | 179_155_137_142_18 8_127_1_198_187_15 4_110_176_143_200_ 175_100_109_97_145 _62_178_48_108_71_ 152_111_133_91_159 _104_114 | 0.948 | 79.2 | 100.0 | 0.951 | 86.7 | 86.7 | Age, number of ApoE4 alleles |

174

EP 4 130 259 A1

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 33 | 155_142_188_127_1_ 161_198_189_154_11 0_78_143_175_100_1 09_178_10_3_191_17 4_108_71_73_152_13 3_149_2_82_104_182 _114 | 0.99 | 95.8 | 100.0 | 0.978 | 80.0 | 93.3 | Age, number of ApoE4 alleles |
| 33 | 121_155_87_188_127 _1_29_161_53_198_1 87_110_185_153_175 _100_109_145_178_4 8_59_152_141_133_1 80_91_149_82_104_1 14 | 0.957 | 87.5 | 95.8 | 0.942 | 80.0 | 80.0 | Age, gender, number of ApoE4 alleles |
| 33 | 155_142_188_127_1_ 189_154_110_78_193 _175_75_100_109_14 5_62_178_96_174_10 8_92_71_134_152_13 3_159_82_104_93_11 4 | 0.924 | 91.7 | 87.5 | 0.987 | 93.3 | 93.3 | Age, gender, number of ApoE4 alleles |
| 33 | 121_155_87_142_188 _127_1_161_198 _189 _194_110_185_193_1 75_75_100_109_125_ 54_191_174_152_190 _133_91_159_82_77_ 182_93_114 | 0.977 | 91.7 | 91.7 | 0.996 | 86.7 | 100.0 | Age |
| 34 | 155_137_87_188_127 _1_79_198_189_110_ 193_175_75_109_145 _178_48_98_135_3_7 1_133_39_91_159_14 9_2_82_77_93_114 | 0.944 | 79.2 | 91.7 | 0.942 | 93.3 | 86.7 | Age, gender, number of ApoE4 alleles |
| 34 | 179_121_155_137_87 _188_127_1_79_196_ 161 198 154 110 14 8_86_175_100_109_1 45_174_92_152_133_ 159_149_2_82_182_9 3_114 | 0.972 | 100.0 | 79.2 | 0.973 | 100.0 | 86.7 | Age, gender, number of ApoE4 alleles |
| 34 | 179_155_142_188_12 7_79_196_161_53_19 8_189_154_110_148 69_100_109_145_54_ 174_92_152_180_113 _91_159_149_2_82_7 7_57_114 | 0.952 | 84.0 | 100.0 | 0.942 | 80.0 | 86.7 | Age, number of ApoE4 alleles |
| 34 | 179_121_87_142_188 _127_5_1_183_161_1 54_110_153_175_109 _145_125_178_156_3 _54_174_71_133_159 _149_82_104_150_93 _114 | 0.946 | 92.0 | 80.0 | 0.982 | 100.0 | 93.3 | Age, gender, number of ApoE4 alleles |
| 35 | 121_155_137_87_188 _127_1_196_198_189 _194_110_68_193_17 5_100_109_169_145_ 10_3_174_152_133_3 9_91_159_149_82_77 _57_182_114 | 0.963 | 92.0 | 84.0 | 1 | 100.0 | 100.0 | Age, number of ApoE4 alleles |

| Number of markers | SEQ ID NO: | Training and cross validation cohort | | | Independent validation cohort | | | Hippocampal atrophy-associated factor |
|---|---|---|---|---|---|---|---|---|
| | | AUC | Sensitivity (%) | Specificity (%) | AUC | Sensitivity (%) | Specificity (%) | |
| 35 | 155_137_87_188_127 _1_80_161_198_189_ 110_176_102_185_17 5_100_109_97_145_5 4_92_152_133_180_9 1_149_104_61_18_57 _93_114 | 0.965 | 95.8 | 79.2 | 0.92 | 93.3 | 86.7 | Age, gender, number of ApoE4 alleles |
| 35 | 155_137_87_142_188 _127_146_196_161_1 7_198_187_194_110_ 56_200_148_86_193_ 109_54_73_152_159_ 149_2_82_104_77_15 0_182_114 | 0.967 | 91.7 | 83.3 | 0.96 | 80.0 | 93.3 | Age, gender, number of ApoE4 alleles |
| 36 | 121_155_137_87_188 _127_1_161_198_189 _38_110_185_175_10 0_109_145_62_125_1 78_48_10_3_108_71_ 152_133_91_159_104 _77_182_93_114 | 0.972 | 87.5 | 95.8 | 0.996 | 93.3 | 100.0 | Age, number of ApoE4 alleles |
| 36 | 179_121_155_87_188 _127_1_79_161_172_ 198_110_148_185_15 3_175_109_145_178_ 48_54_191_174_71_7 3_152_133_39_91_14 9_82_51_93_114 | 0.933 | 84.0 | 87.5 | 0.982 | 86.7 | 100.0 | Age, number of ApoE4 alleles |
| 39 | 121_155_137_87_188 _127_1_79_198_189_ 38_194_110_143_68_ 26_177_101_175_25_ 192_100_109_63_135 _54_108_71_152_133 _91_82_104_171_77_ 182_114 | 0.944 | 91.7 | 87.5 | 0.987 | 93.3 | 93.3 | Age, number of ApoE4 alleles |
| 39 | 179_155_137_87_142 _188_127_172_84_53 154_38_110_86_153 _193_175_75_100_10 9_145_125_156_3_54 _133_180_39_91_159 _149_2_82_77_93_11 4 | 0.92 | 91.7 | 80.0 | 0.933 | 93.3 | 80.0 | Age, gender, number of ApoE4 alleles |

As markers used for the discriminants exhibiting AUC of 0.8 or more concerning the discrimination performance in the cross validation cohort and the independent validation cohort, polynucleotides consisting of the nucleotide sequences shown by SEQ ID NOs: 1 to 5, 9 to 13, 16 to 18, 20, 21, 23 to 26, 29 to 32, 37 to 39, 41, 42, 44, 45, 47 to 63, 67 to 75, 77 to 88, 90 to 94, 96 to 102, 104 to 111, 113, 114, 117, 118, 120, 121, 123 to 125, 127 to 129, 133 to 138, 141 to 143, 145 to 150, 152 to 159, 161 to 163, 167 to 169, 171 to 183, and 185 to 200 were selected. Such polynucleotides were shown to be capable of discriminating a patient with hippocampal atrophy from a patient with no hippocampal atrophy when used in arbitrary combination.

In this Example, discriminants were constructed using samples of two groups exhibiting extremely different extents of hippocampal atrophy from each other. The discriminant scores obtained using the discriminant that was constructed and validated were regressed with high correlation to the actual volume ratio of the hippocampus to the whole brain obtained from brain MRI image. For example, the discriminant scores obtained using the discriminant constructed with the use of the combination of the expression levels of miRNAs consisting of the nucleotide sequences shown by SEQ ID NOs: 155, 137, 188, 127, 196, 154, 110, 128, 100, 152, 133, 159, and 114, the age, the gender, and the number of ApoE alleles in the training and cross validation cohort were correlated with the extent of hippocampal atrophy. Specifically, for example, when the discriminant score obtained using the discriminant is less than -2, the volume ratio of the hippocampus to the whole brain can be estimated to be 0.69% or more. When the discriminant score is from -2 to less than 0, the volume ratio of the hippocampus to the whole brain can be estimated to be 0.61% or more and less than 0.69%. When the discriminant score is from 0 to less than 2, the volume ratio of the hippocampus to the whole brain can be estimated to be 0.53% or more and less than 0.61%. When the discriminant score is from 2 to less than 4, the volume ratio of the hippocampus to the whole brain can be estimated to be 0.45% or more and less than 0.53%. When the discriminant score is 4 or more, the volume ratio of the hippocampus to the whole brain can be estimated to be less than 0.45%.

As described above, polynucleotides consisting of the nucleotide sequences shown by SEQ ID NOs: 1 to 5, 9,10, 12, 13, 16 to 18, 20, 21, 24 to 26, 29 to 32, 37 to 39, 41, 42, 44, 45, 47 to 59, 61 to 63, 67 to 71, 73 to 75, 77 to 88, 90 to 94, 96 to 102, 104 to 111, 113, 114, 117, 118, 121, 123, 125, 127, 128, 133 to 138, 141 to 143, 145 to 150, 152 to 159, 161 to 163, 167 to 169, 171 to 180, 182, 183, 185 to 194, and 196 to 200 were shown to be capable of discriminating a subject with hippocampal atrophy from a subject with no hippocampal atrophy with high accuracy (at AUC of 0.9 or more) when used in combinations of two or more.

It was also shown that discriminants based on a smaller number of markers and/or discriminants with higher performance could be obtained by also integrating a factor(s) highly associated with hippocampal atrophy, such as the age, the gender, and the number of ApoE4 alleles, as an explanatory variable.

[Example 7]

< Determination of extent of hippocampal atrophy and estimation of hippocampal volume through constructing regression equation >

In this Example, markers with higher quantitative performance capable of estimating the hippocampal volume were selected. Specifically, instead of comparing two groups: the hippocampal atrophy group and the hippocampal non-atrophy group, linear regression analysis to estimate the hippocampal volume as an objective variable was performed with the use of the miRNA expression levels, the age, the gender, and the number of ApoE4 alleles as explanatory variables.

Among the total of 264 samples selected and used for discriminant analysis of hippocampal atrophy in Example 1, 132 samples accounting for a half of the total samples were allocated to the training and cross validation cohort, and other 132 samples as the remaining half of the total were allocated to the independent validation cohort (Table 13).

Table 13

| Number of all samples | Example 7 | |
|---|---|---|
| | Training and cross validation cohort | Independent validation cohort |
| 264 | 132 | 132 |

(1) Searching for hippocampal atrophy marker by LASSO regression analysis

The optimal value for $\lambda$ to minimize the mean squared error (MSE) was determined by LASSO regression in the training and cross validation cohort. The cross validation was performed using one of 10 parts into which the training and cross validation cohort was divided. A regression equation with the optimal $\lambda$ value was evaluated in the independent validation cohort, and performance of the regression equation (correlation coefficient $R^2$, RMSE, and MAE) was calculated.

At the outset, the miRNA expression levels were corrected in the same manner as in Example 2. Subsequently, the expression levels of the 210 miRNAs subjected to the analysis in Example 2, the age, the gender, and the number of ApoE4 alleles were subjected to LASSO regression analysis. The regression equation capable of estimating the hippocampal volume was constructed, and the discrimination performance of the regression equation constructed was validated using the independent validation cohort.

Table 14 shows $R^2$, RMSE, and MAE of the constructed regression equation. When the age was added as an explanatory variable, the regression equation constructed with the use of the expression levels of miRNAs consisting of the nucleotide sequences shown by SEQ ID NOs: 155, 188, 127, 5, 198, 189, 110, 176, 143, 102, 148, 101, 75, 109, 199, 145, 54, 174, 106, 152, 133, 2, 20, 51, and 114 exhibited $R^2$ of 0.621, RMSE of 1.187, and MAE of 0.940 as the regression performance in the training and cross validation cohort, and $R^2$ of 0.488, RMSE of 1.393, and MAE of 1.117 as the regression performance in the independent validation cohort.

The discriminant score obtained using the regression equation was regressed to the actual quantified hippocampal volume obtained from brain MRI images with high correlation. For example, the discriminant score obtained using the discriminant constructed with the use of the combination of the expression levels of miRNAs consisting of the nucleotide sequences shown by SEQ ID NOs: 155, 137, 188, 127, 196, 154, 110, 128, 100, 152155, 188, 127, 5, 198, 189, 110, 176, 143, 102, 148, 101, 75, 109, 199, 145, 54, 174, 106, 152, 133, 2, 20, 51, and 114 in the training cohort, and the age, the gender, and the number of ApoE4 alleles was correlated with the quantified hippocampal volume and the extent of hippocampal atrophy.

Specifically, for example, when the discriminant score obtained using the discriminant is less than 6, the volume ratio of the hippocampus to the whole brain can be estimated to be less than 0.44%. When the discriminant score is from 6 to less than 8, the volume ratio of the hippocampus to the whole brain can be estimated to be 0.44% or more and less than 0.59%. When the discriminant score is from 8 to less than 10, the volume ratio of the hippocampus to the whole brain can be estimated to be 0.59% or more and less than 0.75%. When the discriminant score is 10 or more, the volume ratio of the hippocampus to the whole brain can be estimated to be 0.75% or more.

Table 14

| Number of markers | SEQ ID NO: | Training and cross validation cohort | | | Independent validation cohort | | | Hippocampal atrophy-associated factor |
|---|---|---|---|---|---|---|---|---|
| | | $R^2$ | RMSE | MAE | $R^2$ | RMSE | MAE | |
| 26 | 155_188_127_5_198_189_110_176_143_102_148_101_75_109_199_145_54_174_106_152_133_2_20_51_114 | 0.621 | 1.187 | 0.94 | 0.488 | 1.393 | 1.117 | Age |

(2) Searching for hippocampal atrophy marker by partial least squares regression analysis

In this Example, coordinate transformation was performed by regression analysis using the partial least squares (PLS) method in the training and cross validation cohort. The values of the explanatory variables were transformed into Z-scores using the mean and the standard deviation and regressed with the use of linear-converted variables to make the miRNA and the age factors to be uncorrelated to each other. The selection of an explanatory variable was performed using the threshold (= 0.6) for variable importance in projection (VIP), cross validation was performed using one sample from the training and cross validation cohort, a model using an optimal variable was evaluated using the independent validation cohort, and the regression performance (correlation coefficient $R^2$, RMSE, and MAE) was calculated.

At the outset, the miRNA expression levels were corrected in the same manner as in Example 2. Subsequently, the expression levels of the 210 miRNAs as used in Example 2, the age, the gender, and the number of ApoE4 alleles were subjected to partial least squares regression analysis to construct a regression equation capable of measuring (estimating) the hippocampal volume, and the regression performance thereof was validated using the independent validation cohort.

Table 15 shows $R^2$, RMSE, and MAE of the constructed regression equation. When the age, the gender, and the number of ApoE4 alleles were added as explanatory variables, the regression equation constructed with the use of the expression levels of miRNAs consisting of the nucleotide sequences shown by SEQ ID NOs: 179, 121, 155, 137, 132, 87, 168, 142, 188, 127, 85, 94, 5, 1, 162, 80, 196, 161, 160, 84, 53, 17, 198, 187, 189, 110, 6, 124, 9, 123, 136,

74, 78, 4, 90, 176, 143, 200, 102, 148, 185, 86, 76, 153, 193, 175, 192, 43, 75, 100, 109, 169, 199, 97, 145, 62, 125, 21, 48, 63, 98, 88, 83, 59, 135, 3, 47, 54, 96, 174, 131, 92, 71, 73, 106, 147, 152, 27, 133, 113, 39, 91, 197, 159, 149, 2, 82, 34, 104, 61, 77, 51, 150, 182, and 114 exhibited $R^2$ of 0.763, RMSE of 0.975, and MAE of 0.746 as the regression performance in the training and cross validation cohort, and $R^2$ of 0.508, RMSE of 1.307, and MAE of 1.021 as the regression performance in the independent validation cohort.

Table 15

| Number of markers | SEQ ID NO: | Training and cross validation cohort | | | Independent validation cohort | | | Hippocampal atrophy-associated factor |
|---|---|---|---|---|---|---|---|---|
| | | $R^2$ | RMSE | MAE | $R^2$ | RMSE | MAE | |
| 95 | 179_121_155_137_132 _87_168_142_188_127 _85_94_5_1_162_80_1 96_161_160_84_53_17 _198_187_189_110_6_ 124_9_123_136_74_78 _4_90_176_143_200_1 02_148_185_86_76_15 3_193_175_192_43_75 _100_109_169_199_97 _145_62_125_21_48_6 3_98_88_83_59_135_3 _47_54_96_174_131_9 2_71_73_106_147_152 _27_133_113_39_91_1 97_159_149_2_82_34_ 104_61_77_51_150_18 2_114 | 0.763 | 0.975 | 0.746 | 0.508 | 1.307 | 1.021 | Age, gender, number of ApoE4 alleles |

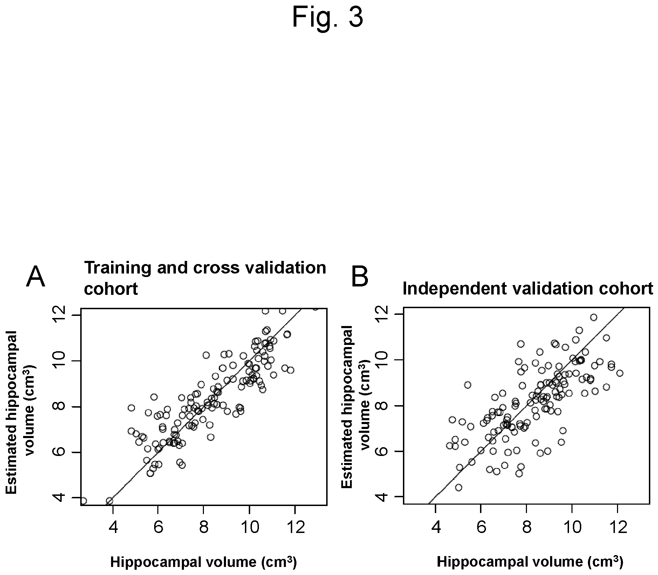

Fig. 3 shows regression lines based on the hippocampal volume (the quantified value) and the explanatory variables, as obtained in Example 7-(2).

In this section, a regression equation capable of estimating the quantified value for hippocampal atrophy (the hippocampal volume) on the basis of miRNA, the age, the gender, and the number of ApoE4 alleles was obtained. In addition, the discriminant score obtained using the regression equation was regressed to the actual quantified hippocampal volume obtained from brain MRI image with high correlation.

For example, the discriminant score obtained using the regression equation constructed with the use of the combination of the expression levels of miRNAs consisting of the nucleotide sequences shown by SEQ ID NOs: 179, 121, 155, 137, 132, 87, 168, 142, 188, 127, 85, 94, 5, 1, 162, 80, 196, 161, 160, 84, 53, 17, 198, 187, 189, 110, 6, 124, 9, 123, 136, 74, 78, 4, 90, 176, 143, 200, 102, 148, 185, 86, 76, 153, 193, 175, 192, 43, 75, 100, 109, 169, 199, 97, 145, 62, 125, 21, 48, 63, 98, 88, 83, 59, 135, 3, 47, 54, 96, 174, 131, 92, 71, 73, 106, 147, 152, 27, 133, 113, 39, 91, 197, 159, 149, 2, 82, 34, 104, 61, 77, 51, 150, 182, and 114, and the age, the gender, and the number of ApoE4 alleles in the training cohort was correlated with the quantified hippocampal volume and the extent of hippocampal atrophy.

Specifically, for example, when the discriminant score obtained using the discriminant is less than 6, the volume ratio of the hippocampus to the whole brain can be estimated to be less than 0.48%. When the discriminant score is from 6 to less than 8, the volume ratio of the hippocampus to the whole brain can be estimated to be 0.48% or more and less than 0.60%. When the discriminant score is from 8 to less than 10, the volume ratio of the hippocampus to the whole brain can be estimated to be 0.60% or more and less than 0.71%. When the discriminant score is 10 or more, the volume ratio of the hippocampus to the whole brain can be estimated to be 0.71% or more.

As described above, shown is the potential to estimate an extent of hippocampal atrophy of a subject based on miRNA measurement and detect a potential patient of hippocampal atrophy who has a relatively lesser extent of hippocampal atrophy.

The results obtained in Examples 1 to 7 indicate that all the polynucleotides consisting of the nucleotide sequences shown by SEQ ID NOs: 1 to 200 are useful as markers for determining an extent of hippocampal atrophy. The results also indicate that a subject with hippocampal atrophy can be discriminated from a subject with no hippocampal atrophy with higher accuracy, when using the polynucleotides in combination.

Industrial Applicability

The use of nucleic acids capable of specifically binding to the hippocampal atrophy markers of the present invention enables objective and quantitative detection of hippocampal atrophy in a simple manner without differences caused among physicians or medical institutions. For example, the measured expression levels of from one to several miRNAs in the blood, serum, and/or plasma sample from a subject, which can be obtained in a less invasive manner, may be used as an indicator to determine an extent of hippocampal atrophy of the subject in a simple manner. With the use of the kit, the device, and the method of the present invention, in addition, hippocampal atrophy can be detected less invasively and more easily, compared with conventional methods for detection of hippocampal atrophy, such as brain MRI imaging or brain PET imaging examination. Accordingly, the kit, the device, and the method of the present invention are useful for screening for hippocampal atrophy. The present invention enables early detection of hippocampal atrophy, which leads to early diagnosis of dementia and the like. With the use of the diagnostic marker(s) of the present invention, whether or not the hippocampus has become atrophic can be accurately evaluated using a blood sample, and thus, suppression of the progression of hippocampal atrophy through medical intervention can be expected. The present invention can provide a test method that is advantageous in the following respects: 1) the method being objective in that the test results would not be affected by differences in medical institutions, equipment, and physicians; 2) the method being applicable to a patient who cannot undergo MRI; 3) the method being able to evaluate gradual changes of the extent of atrophy; and 4) the method being less invasive.

All publications, patents, and patent applications cited herein are incorporated herein by reference in their entirety.

**Claims**

1. A kit for detection of hippocampal atrophy, comprising a nucleic acid(s) capable of specifically binding to one or more polynucleotide(s) selected from the group consisting of hippocampal atrophy markers: miR-3131, miR-6757-5p, miR-4706, miR-5001-5p, miR-3180-3p, miR-642b-3p, miR-4655-5p, miR-6819-5p, miR-937-5p, miR-4688, miR-6741-5p, miR-7107-5p, miR-4271, miR-1229-5p, miR-4707-5p, miR-6808-5p, miR-4656, miR-6076, miR-6762-5p, miR-7109-5p, miR-6732-5p, miR-3195, miR-7150, miR-642a-3p, miR-1249-5p, miR-3185, miR-4689, miR-3141, miR-6840-3p, miR-3135b, miR-1914-3p, miR-4446-3p, miR-4433b-3p, miR-6877-5p, miR-6848-5p, miR-3620-5p, miR-6825-5p, miR-5739, miR-3663-3p, miR-4695-5p, miR-3162-5p, miR-3679-5p, miR-8059, miR-7110-5p, miR-1275, miR-6779-5p, miR-197-5p, miR-6845-5p, miR-4327, miR-4723-5p, miR-4530, miR-6771-5p, miR-614, miR-92a-2-5p, miR-6891-5p, miR-6124, miR-4687-3p, miR-4442, miR-7977, miR-6785-5p, miR-4497, miR-8071, miR-663b, miR-3180, miR-4251, miR-1285-3p, miR-6870-5p, miR-4484, miR-4476, miR-6749-5p, miR-4454, miR-6893-5p, miR-6085, miR-4787-5p, miR-149-3p, miR-7704, miR-6125, miR-6090, miR-3197, miR-6850-5p, miR-4467, miR-6885-5p, miR-6803-5p, miR-6798-5p, miR-6780b-5p, miR-6768-5p, miR-5100, miR-6724-5p, miR-6879-5p, miR-7108-5p, miR-4649-5p, miR-4739, miR-6089, miR-1908-5p, miR-4516, miR-2861, miR-4492, miR-4294, miR-6791-5p, miR-1469, miR-6752-5p, miR-4730, miR-6126, miR-6869-5p, miR-1268a, miR-6799-5p, miR-8069, miR-3621, and miR-4763-3p, or to a complementary strand(s) of the polynucleotide(s).

2. The kit according to claim 1, wherein the nucleic acid(s) are polynucleotide(s) selected from the group consisting of the following polynucleotides (a) to (e):

   (a) a polynucleotide consisting of a nucleotide sequence shown by any of SEQ ID NOs: 1 to 109 or a nucleotide sequence derived from the nucleotide sequence by the replacement of u with t, a variant thereof, a derivative thereof, or a fragment thereof comprising 15 or more consecutive nucleotides;
   (b) a polynucleotide comprising a nucleotide sequence shown by any of SEQ ID NOs: 1 to 109, a variant thereof, a derivative thereof, or a fragment thereof comprising 15 or more consecutive nucleotides;
   (c) a polynucleotide consisting of a nucleotide sequence complementary to a nucleotide sequence shown by any of SEQ ID NOs: 1 to 109 or a nucleotide sequence derived from the nucleotide sequence by the replacement of u with t, a variant thereof, a derivative thereof, or a fragment thereof comprising 15 or more consecutive nucleotides;
   (d) a polynucleotide comprising a nucleotide sequence complementary to a nucleotide sequence shown by any of SEQ ID NOs: 1 to 109 or a nucleotide sequence derived from the nucleotide sequence by the replacement of u with t, a variant thereof, a derivative thereof, or a fragment thereof comprising 15 or more consecutive nucleotides; and
   (e) a polynucleotide hybridizing under stringent conditions to any of the polynucleotides (a) to (d).

3. The kit according to claim 1 or 2, wherein the kit further comprises a nucleic acid(s) capable of specifically binding to one or more polynucleotide(s) selected from the group consisting of other hippocampal atrophy markers: miR-1228-5p, miR-760, miR-187-5p, miR-7111-5p, miR-6088, miR-6805-3p, miR-4640-5p, miR-6721-5p, miR-6880-5p, miR-711, miR-128-1-5p, miR-4525, miR-486-3p, miR-6756-5p, miR-1260b, miR-3184-5p, miR-6075, miR-204-3p, miR-4728-5p, miR-4534, miR-4758-5p, miR-8063, miR-6836-3p, miR-6789-5p, miR-744-5p, miR-1909-3p, miR-887-3p, miR-4745-5p, miR-4433a-3p, miR-5090, miR-296-5p, miR-939-5p, miR-3648, miR-3196, miR-6722-3p, miR-6805-5p, miR-1202, miR-6775-5p, miR-6087, miR-6765-5p, miR-6875-5p, miR-4674, miR-1233-5p, miR-7114-5p, miR-5787, miR-8072, miR-3619-3p, miR-4632-5p, miR-6800-5p, miR-4634, miR-4486, miR-6727-5p, miR-4505, miR-4725-3p, miR-1538, miR-320b, miR-1915-5p, miR-328-5p, miR-6820-5p, miR-6726-5p, miR-3665, miR-638, miR-762, miR-4466, miR-3940-5p, miR-1237-5p, miR-575, miR-3656, miR-4488, miR-4281, miR-6781-5p, miR-4532, miR-4665-5p, miR-6816-5p, miR-4508, miR-6784-5p, miR-6786-5p, miR-4741, miR-1343-5p, miR-1227-5p, miR-4734, miR-3960, miR-128-2-5p, miR-6743-5p, miR-663a, miR-6729-5p, miR-1915-3p, miR-1268b, miR-4651, miR-3178, and miR-4463, or to a complementary strand(s) of the polynucleotide(s).

4. The kit according to claim 3, wherein the nucleic acid(s) are polynucleotide(s) selected from the group consisting of the following polynucleotides (f) to (j):

(f) a polynucleotide consisting of a nucleotide sequence shown by any of SEQ ID NOs: 110 to 200 or a nucleotide sequence derived from the nucleotide sequence by the replacement of u with t, a variant thereof, a derivative thereof, or a fragment thereof comprising 15 or more consecutive nucleotides;
(g) a polynucleotide comprising a nucleotide sequence shown by any of SEQ ID NOs: 110 to 200, a variant thereof, a derivative thereof, or a fragment thereof comprising 15 or more consecutive nucleotides;
(h) a polynucleotide consisting of a nucleotide sequence complementary to a nucleotide sequence shown by any of SEQ ID NOs: 110 to 200 or a nucleotide sequence derived from the nucleotide sequence by the replacement of u with t, a variant thereof, a derivative thereof, or a fragment thereof comprising 15 or more consecutive nucleotides;
(i) a polynucleotide comprising a nucleotide sequence complementary to a nucleotide sequence shown by any of SEQ ID NOs: 110 to 200 or a nucleotide sequence derived from the nucleotide sequence by the replacement of u with t, a variant thereof, a derivative thereof, or a fragment thereof comprising 15 or more consecutive nucleotides; and
(j) a polynucleotide hybridizing under stringent conditions to any of the polynucleotides (f) to (i).

5. A device for detection of hippocampal atrophy comprising a nucleic acid(s) capable of specifically binding to one or more polynucleotide(s) selected from the group consisting of hippocampal atrophy markers: miR-3131, miR-6757-5p, miR-4706, miR-5001-5p, miR-3180-3p, miR-642b-3p, miR-4655-5p, miR-6819-5p, miR-937-5p, miR-4688, miR-6741-5p, miR-7107-5p, miR-4271, miR-1229-5p, miR-4707-5p, miR-6808-5p, miR-4656, miR-6076, miR-6762-5p, miR-7109-5p, miR-6732-5p, miR-3195, miR-7150, miR-642a-3p, miR-1249-5p, miR-3185, miR-4689, miR-3141, miR-6840-3p, miR-3135b, miR-1914-3p, miR-4446-3p, miR-4433b-3p, miR-6877-5p, miR-6848-5p, miR-3620-5p, miR-6825-5p, miR-5739, miR-3663-3p, miR-4695-5p, miR-3162-5p, miR-3679-5p, miR-8059, miR-7110-5p, miR-1275, miR-6779-5p, miR-197-5p, miR-6845-5p, miR-4327, miR-4723-5p, miR-4530, miR-6771-5p, miR-614, miR-92a-2-5p, miR-6891-5p, miR-6124, miR-4687-3p, miR-4442, miR-7977, miR-6785-5p, miR-4497, miR-8071, miR-663b, miR-3180, miR-4251, miR-1285-3p, miR-6870-5p, miR-4484, miR-4476, miR-6749-5p, miR-4454, miR-6893-5p, miR-6085, miR-4787-5p, miR-149-3p, miR-7704, miR-6125, miR-6090, miR-3197, miR-6850-5p, miR-4467, miR-6885-5p, miR-6803-5p, miR-6798-5p, miR-6780b-5p, miR-6768-5p, miR-5100, miR-6724-5p, miR-6879-5p, miR-7108-5p, miR-4649-5p, miR-4739, miR-6089, miR-1908-5p, miR-4516, miR-2861, miR-4492, miR-4294, miR-6791-5p, miR-1469, miR-6752-5p, miR-4730, miR-6126, miR-6869-5p, miR-1268a, miR-6799-5p, miR-8069, miR-3621, and miR-4763-3p, or to a complementary strand(s) of the polynucleotide(s).

6. The device according to claim 5, wherein the nucleic acid(s) are polynucleotide(s) selected from the group consisting of the following polynucleotides (a) to (e):

(a) a polynucleotide consisting of a nucleotide sequence shown by any of SEQ ID NOs: 1 to 109 or a nucleotide sequence derived from the nucleotide sequence by the replacement of u with t, a variant thereof, a derivative thereof, or a fragment thereof comprising 15 or more consecutive nucleotides;
(b) a polynucleotide comprising a nucleotide sequence shown by any of SEQ ID NOs: 1 to 109, a variant thereof, a derivative thereof, or a fragment thereof comprising 15 or more consecutive nucleotides;
(c) a polynucleotide consisting of a nucleotide sequence complementary to a nucleotide sequence shown by any of SEQ ID NOs: 1 to 109 or a nucleotide sequence derived from the nucleotide sequence by the replacement of u with t, a variant thereof, a derivative thereof, or a fragment thereof comprising 15 or more consecutive nucleotides;

(d) a polynucleotide comprising a nucleotide sequence complementary to a nucleotide sequence shown by any of SEQ ID NOs: 1 to 109 or a nucleotide sequence derived from the nucleotide sequence by the replacement of u with t, a variant thereof, a derivative thereof, or a fragment thereof comprising 15 or more consecutive nucleotides; and

(e) a polynucleotide hybridizing under stringent conditions to any of the polynucleotides (a) to (d).

7. The device according to claim 5 or 6, wherein the device further comprises a nucleic acid(s) capable of specifically binding to one or more polynucleotide(s) selected from the group consisting of other hippocampal atrophy markers: miR-1228-5p, miR-760, miR-187-5p, miR-7111-5p, miR-6088, miR-6805-3p, miR-4640-5p, miR-6721-5p, miR-6880-5p, miR-711, miR-128-1-5p, miR-4525, miR-486-3p, miR-6756-5p, miR-1260b, miR-3184-5p, miR-6075, miR-204-3p, miR-4728-5p, miR-4534, miR-4758-5p, miR-8063, miR-6836-3p, miR-6789-5p, miR-744-5p, miR-1909-3p, miR-887-3p, miR-4745-5p, miR-4433a-3p, miR-5090, miR-296-5p, miR-939-5p, miR-3648, miR-3196, miR-6722-3p, miR-6805-5p, miR-1202, miR-6775-5p, miR-6087, miR-6765-5p, miR-6875-5p, miR-4674, miR-1233-5p, miR-7114-5p, miR-5787, miR-8072, miR-3619-3p, miR-4632-5p, miR-6800-5p, miR-4634, miR-4486, miR-6727-5p, miR-4505, miR-4725-3p, miR-1538, miR-320b, miR-1915-5p, miR-328-5p, miR-6820-5p, miR-6726-5p, miR-3665, miR-638, miR-762, miR-4466, miR-3940-5p, miR-1237-5p, miR-575, miR-3656, miR-4488, miR-4281, miR-6781-5p, miR-4532, miR-4665-5p, miR-6816-5p, miR-4508, miR-6784-5p, miR-6786-5p, miR-4741, miR-1343-5p, miR-1227-5p, miR-4734, miR-3960, miR-128-2-5p, miR-6743-5p, miR-663a, miR-6729-5p, miR-1915-3p, miR-1268b, miR-4651, miR-3178, and miR-4463, or to a complementary strand(s) of the polynucleotide(s).

8. The device according to claim 7, wherein the nucleic acid(s) are polynucleotide(s) selected from the group consisting of the following polynucleotides (f) to (j):

(f) a polynucleotide consisting of a nucleotide sequence shown by any of SEQ ID NOs: 110 to 200 or a nucleotide sequence derived from the nucleotide sequence by the replacement of u with t, a variant thereof, a derivative thereof, or a fragment thereof comprising 15 or more consecutive nucleotides;

(g) a polynucleotide comprising a nucleotide sequence shown by any of SEQ ID NOs: 110 to 200, a variant thereof, a derivative thereof, or a fragment thereof comprising 15 or more consecutive nucleotides;

(h) a polynucleotide consisting of a nucleotide sequence complementary to a nucleotide sequence shown by any of SEQ ID NOs: 110 to 200 or a nucleotide sequence derived from the nucleotide sequence by the replacement of u with t, a variant thereof, a derivative thereof, or a fragment thereof comprising 15 or more consecutive nucleotides;

(i) a polynucleotide comprising a nucleotide sequence complementary to a nucleotide sequence shown by any of SEQ ID NOs: 110 to 200 or a nucleotide sequence derived from the nucleotide sequence by the replacement of u with t, a variant thereof, a derivative thereof, or a fragment thereof comprising 15 or more consecutive nucleotides; and

(j) a polynucleotide hybridizing under stringent conditions to any of the polynucleotides (f) to (i).

9. The device according to any one of claims 5 to 8, wherein the device is a device for measurement by a hybridization technique.

10. The device according to claim 9, wherein the hybridization technique is a nucleic acid array technique.

11. A method for detecting hippocampal atrophy, comprising: measuring an expression level(s) of one or more polynucleotide(s) selected from the group consisting of hippocampal atrophy markers: miR-3131, miR-6757-5p, miR-4706, miR-5001-5p, miR-3180-3p, miR-642b-3p, miR-4655-5p, miR-6819-5p, miR-937-5p, miR-4688, miR-6741-5p, miR-7107-5p, miR-4271, miR-1229-5p, miR-4707-5p, miR-6808-5p, miR-4656, miR-6076, miR-6762-5p, miR-7109-5p, miR-6732-5p, miR-3195, miR-7150, miR-642a-3p, miR-1249-5p, miR-3185, miR-4689, miR-3141, miR-6840-3p, miR-3135b, miR-1914-3p, miR-4446-3p, miR-4433b-3p, miR-6877-5p, miR-6848-5p, miR-3620-5p, miR-6825-5p, miR-5739, miR-3663-3p, miR-4695-5p, miR-3162-5p, miR-3679-5p, miR-8059, miR-7110-5p, miR-1275, miR-6779-5p, miR-197-5p, miR-6845-5p, miR-4327, miR-4723-5p, miR-4530, miR-6771-5p, miR-614, miR-92a-2-5p, miR-6891-5p, miR-6124, miR-4687-3p, miR-4442, miR-7977, miR-6785-5p, miR-4497, miR-8071, miR-663b, miR-3180, miR-4251, miR-1285-3p, miR-6870-5p, miR-4484, miR-4476, miR-6749-5p, miR-4454, miR-6893-5p, miR-6085, miR-4787-5p, miR-149-3p, miR-7704, miR-6125, miR-6090, miR-3197, miR-6850-5p, miR-4467, miR-6885-5p, miR-6803-5p, miR-6798-5p, miR-6780b-5p, miR-6768-5p, miR-5100, miR-6724-5p, miR-6879-5p, miR-7108-5p, miR-4649-5p, miR-4739, miR-6089, miR-1908-5p, miR-4516, miR-2861, miR-4492, miR-4294, miR-6791-5p, miR-1469, miR-6752-5p, miR-4730, miR-6126, miR-6869-5p, miR-1268a, miR-6799-5p, miR-8069, miR-3621, and miR-4763-3p in a sample from a subject; and evaluating whether or not the subject's hippocampus has become atrophic by using the measured expression level(s).

**12.** The method according to claim 11, wherein the expression level(s) of the polynucleotide(s) are measured using the kit according to any one of claims 1 to 4 or the device according to any one of claims 5 to 10.

**13.** The method according to claim 12, comprising: assigning the measured expression level(s) of the polynucleotide(s) to a discriminant capable of distinctively discriminating between atrophy and non-atrophy of hippocampus, wherein the discriminant is prepared using expression level(s) of the polynucleotide(s) in samples from subjects known to have hippocampal atrophy and expression level(s) of the polynucleotide(s) in samples from subjects known to have no hippocampal atrophy as training samples; and thereby evaluating whether there is atrophy or non-atrophy of hippocampus.

**14.** The method according to claim 12 or 13, wherein the nucleic acid(s) are polynucleotide(s) selected from the group consisting of the following polynucleotides (a) to (e):

(a) a polynucleotide consisting of a nucleotide sequence shown by any of SEQ ID NOs: 1 to 109 or a nucleotide sequence derived from the nucleotide sequence by the replacement of u with t, a variant thereof, a derivative thereof, or a fragment thereof comprising 15 or more consecutive nucleotides;
(b) a polynucleotide comprising a nucleotide sequence shown by any of SEQ ID NOs: 1 to 109, a variant thereof, a derivative thereof, or a fragment thereof comprising 15 or more consecutive nucleotides;
(c) a polynucleotide consisting of a nucleotide sequence complementary to a nucleotide sequence shown by any of SEQ ID NOs: 1 to 109 or a nucleotide sequence derived from the nucleotide sequence by the replacement of u with t, a variant thereof, a derivative thereof, or a fragment thereof comprising 15 or more consecutive nucleotides;
(d) a polynucleotide comprising a nucleotide sequence complementary to a nucleotide sequence shown by any of SEQ ID NOs: 1 to 109 or a nucleotide sequence derived from the nucleotide sequence by the replacement of u with t, a variant thereof, a derivative thereof, or a fragment thereof comprising 15 or more consecutive nucleotides; and
(e) a polynucleotide hybridizing under stringent conditions to any of the polynucleotides (a) to (d).

**15.** The method according to any one of claims 12 to 14, wherein the kit or device further comprises nucleic acid(s) capable of specifically binding to one or more polynucleotide(s) selected from the group consisting of other hippocampal atrophy markers: miR-1228-5p, miR-760, miR-187-5p, miR-7111-5p, miR-6088, miR-6805-3p, miR-4640-5p, miR-6721-5p, miR-6880-5p, miR-711, miR-128-1-5p, miR-4525, miR-486-3p, miR-6756-5p, miR-1260b, miR-3184-5p, miR-6075, miR-204-3p, miR-4728-5p, miR-4534, miR-4758-5p, miR-8063, miR-6836-3p, miR-6789-5p, miR-744-5p, miR-1909-3p, miR-887-3p, miR-4745-5p, miR-4433a-3p, miR-5090, miR-296-5p, miR-939-5p, miR-3648, miR-3196, miR-6722-3p, miR-6805-5p, miR-1202, miR-6775-5p, miR-6087, miR-6765-5p, miR-6875-5p, miR-4674, miR-1233-5p, miR-7114-5p, miR-5787, miR-8072, miR-3619-3p, miR-4632-5p, miR-6800-5p, miR-4634, miR-4486, miR-6727-5p, miR-4505, miR-4725-3p, miR-1538, miR-320b, miR-1915-5p, miR-328-5p, miR-6820-5p, miR-6726-5p, miR-3665, miR-638, miR-762, miR-4466, miR-3940-5p, miR-1237-5p, miR-575, miR-3656, miR-4488, miR-4281, miR-6781-5p, miR-4532, miR-4665-5p, miR-6816-5p, miR-4508, miR-6784-5p, miR-6786-5p, miR-4741, miR-1343-5p, miR-1227-5p, miR-4734, miR-3960, miR-128-2-5p, miR-6743-5p, miR-663a, miR-6729-5p, miR-1915-3p, miR-1268b, miR-4651, miR-3178, and miR-4463, or to a complementary strand(s) of the polynucleotide(s).

**16.** The method according to claim 15, wherein the nucleic acid(s) are polynucleotide(s) selected from the group consisting of the following polynucleotides (f) to (j):

(f) a polynucleotide consisting of a nucleotide sequence shown by any of SEQ ID NOs: 110 to 200 or a nucleotide sequence derived from the nucleotide sequence by the replacement of u with t, a variant thereof, a derivative thereof, or a fragment thereof comprising 15 or more consecutive nucleotides;
(g) a polynucleotide comprising a nucleotide sequence shown by any of SEQ ID NOs: 110 to 200, a variant thereof, a derivative thereof, or a fragment thereof comprising 15 or more consecutive nucleotides;
(h) a polynucleotide consisting of a nucleotide sequence complementary to a nucleotide sequence shown by any of SEQ ID NOs: 110 to 200 or a nucleotide sequence derived from the nucleotide sequence by the replacement of u with t, a variant thereof, a derivative thereof, or a fragment thereof comprising 15 or more consecutive nucleotides;

(i) a polynucleotide comprising a nucleotide sequence complementary to a nucleotide sequence shown by any of SEQ ID NOs: 110 to 200 or a nucleotide sequence derived from the nucleotide sequence by the replacement of u with t, a variant thereof, a derivative thereof, or a fragment thereof comprising 15 or more consecutive nucleotides; and

(j) a polynucleotide hybridizing under stringent conditions to any of the polynucleotides (f) to (i).

17. The method according to any one of claims 11 to 16, wherein the subject is a human.

18. The method according to any one of claims 11 to 17, wherein the sample is blood, serum, or plasma.

EP 4 130 259 A1

Fig. 1

hsa-miR-1915-5p
(SEQ ID NO: 166)

hsa-mir-1915
(SEQ ID NO: 379)

hsa-miR-1915-3p
(SEQ ID NO: 196)

# Fig. 2

# Fig. 3

**A** Training and cross validation cohort

**B** Independent validation cohort

<table>
<tr><td colspan="2" align="center">**INTERNATIONAL SEARCH REPORT**</td><td>International application No.<br>PCT/JP2021/013807</td></tr>
</table>

| A. CLASSIFICATION OF SUBJECT MATTER |
|---|
| Int.Cl. C12N15/09(2006.01)i, C12N15/113(2010.01)i, C12Q1/6837(2018.01)i, C12Q1/6883(2018.01)i<br>FI: C12N15/09200, C12Q1/6837Z, C12N15/113ZZNA, C12Q1/6883Z<br>According to International Patent Classification (IPC) or to both national classification and IPC |

| B. FIELDS SEARCHED |
|---|
| Minimum documentation searched (classification system followed by classification symbols)<br>Int.Cl. C12N15/09, C12N15/113, C12Q1/6837, C12Q1/6883 |
| Documentation searched other than minimum documentation to the extent that such documents are included in the fields searched<br>Published examined utility model applications of Japan　　　1922-1996<br>Published unexamined utility model applications of Japan　　1971-2021<br>Registered utility model specifications of Japan　　　　　　1996-2021<br>Published registered utility model applications of Japan　　1994-2021 |
| Electronic data base consulted during the international search (name of data base and, where practicable, search terms used)<br>JSTPlus/JMEDPlus/JST7580(JDreamIII), CAplus/MEDLINE/EMBASE/BIOSIS(STN) |

| C. DOCUMENTS CONSIDERED TO BE RELEVANT | | |
|---|---|---|
| Category* | Citation of document, with indication, where appropriate, of the relevant passages | Relevant to claim No. |
| Y | WO 2019/159884 A1 (TORAY INDUSTRIES, INC.) 22 August 2019 (2019-08-22), claims, paragraphs [0052], [0534], [1160]-[1167], [1191] | 1-18 |
| Y | 羽生春夫ほか, アルツハイマ一病における海馬病変と大脳皮質機能との関連, 日本老年医学会雑誌, 2000, vol. 37, no. 11, pp. 921-927, particularly, abstract, non-official translation (HANYU, Haruo et al., Relationship between hippocampal lesions and cerebral cortex function in Alzheimer's disease, Journal of the Japan Geriatrics Society) | 1-18 |
| Y | WO 2018/199275 A1 (TORAY INDUSTRIES, INC.) 01 November 2018 (2018-11-01), claim 1 | 11-18 |
| Y | WO 2019/004436 A1 (TORAY INDUSTRIES, INC.) 03 January 2019 (2019-01-03), claims 1, 3 | 11-18 |

| ☐ Further documents are listed in the continuation of Box C. | ☒ See patent family annex. |
|---|---|

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search<br>07 May 2021 | Date of mailing of the international search report<br>18 May 2021 |
|---|---|
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

<table>
<tr><td colspan="2"><strong>INTERNATIONAL SEARCH REPORT</strong><br>Information on patent family members</td><td>International application No.<br>PCT/JP2021/013807</td></tr>
</table>

```
WO 2019/159884 A1  22 August 2019      (Family: none)

WO 2018/199275 A1  01 November 2018    US 2020/0140956 A1
                                       claim 1
                                       EP 3628736 A1
                                       CN 110546263  A
                                       KR 10-2020-0002809 A

WO 2019/004436 A1  03 January 2019     US 2020/0140958 A1
                                       claims 1, 3
                                       EP 3647422 A1
                                       KR 10-2020-0019849 A
                                       CN 110799648 A
```

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2019159884 A
- JP 2017184642 A
- WO 2019014457 A
- US 2019136320
- US 2014272993
- WO 2019048500 A
- US 2019249250

### Non-patent literature cited in the description

- **DANIEL H.S. et al.** *The Journal of Nuclear Medicine,* April 2004, vol. 45 (4
- **NAKAMURA A. et al.** *Nature,* 31 January 2018, vol. 554, 249-254
- **GRASSO M. et al.** *Neurobiol. Aging.,* December 2019, vol. 84, e1-e12
- **KUMAR S. et al.** *Hum. Mol. Genet.,* October 2017, vol. 26 (19), 3808-3822
- **BHAGAT R. et al.** *Cell Death Differ,* November 2018, (10), 1837-1854
- **EMRAH DUZEL et al.** *Alzhemer's & Dementia Monitoring,* 2018, vol. 10, 782-790
- **ZHENG ZHANG et al.** *J. Comput. Biol.,* 2000, vol. 7, 203-214
- **ALTSCHUL, S.F. et al.** *Journal of Molecular Biology,* 1990, vol. 215, 403-410
- **PEARSON, W.R. et al.** *Proc. Natl. Acad. Sci. U.S.A.,* 1988, vol. 85, 2444-2448
- **NIELSEN, P.E. et al.** *Science,* 1991, vol. 254, 1497-500
- **OBIKA, S. et al.** *Tetrahedron Lett.,* 1998, vol. 39, 5401-5404
- **STARK MS et al.** *PLoS One,* 2010, vol. 5, e9685
- **LADEWIG E et al.** *Genome Res.,* 2012, vol. 22, 1634-1645
- **PERSSON H et al.** *Cancer Res.,* 2011, vol. 71, 78-86
- **HANSEN TB et al.** *RNA Biol,* 2011, vol. 8, 378-383
- **CREIGHTON CJ et al.** *PLoS One,* 2010, vol. 5, e9637
- **WITTEN D et al.** *BMC Biol,* 2010, vol. 8, 58
- **LADEWIG E et al.** *Genome Res,* 2012, vol. 22, 1634-1645
- **LUI WO et al.** *Cancer Res.,* 2007, vol. 67, 6031-6043
- **GOFF LA et al.** *PLoS One,* 2009, vol. 4, e7192
- **BEREZIKOV E et al.** *Mol. Cell,* 2007, vol. 28, 328-336
- **VOELLENKLE C et al.** *RNA,* 2012, vol. 18, 472-484
- **OULAS A et al.** *Nucleic Acids Res.,* 2009, vol. 37, 3276-3287
- **CUMMINS JM et al.** *Proc. Natl. Acad. Sci., U.S.A,* 2006, vol. 103, 3687-3692
- **MORIN RD et al.** *Genome Res.,* 2008, vol. 18, 610-621
- **JIMA DD et al.** *Blood,* 2010, vol. 116, e118-e127
- **BAR M et al.** *Stem Cells,* 2008, vol. 26, 2496-2505
- **PLE H et al.** *PLoS One,* 2012, vol. 7
- **YOO JK et al.** *Biochem. Biophys. Res. Commun,* 2011, vol. 415, 258-262
- **LIAO JY et al.** *PLoS One,* 2010, vol. 5, e10563
- **WANG HJ et al.** *Shock,* 2013, vol. 39, 480-487
- **LAGOS-QUINTANA M et al.** *RNA,* 2003, vol. 9, 175-179
- **CUMMINS JM et al.** *Proc. Natl. Acad. Sci., U.S.A.,* vol. 103, 3687-3692
- **MOURELATOS Z et al.** *Genes Dev.,* 2002, vol. 16, 720-728

- **SMITH JL et al.** *J. Virol.,* 2012, vol. 86, 5278-5287
- **VELTHUT-MEIKAS A et al.** *Mol. Endocrinol.,* 2013
- **TAKADA S et al.** *Leukemia,* 2008, vol. 22, 1274-1278
- **MORIN RD et al.** *Genome Res,* 2008, vol. 18, 10-621
- **LAGOS-QUINTANA M et al.** *Curr. Biol.,* 2002, vol. 12, 735-739
- **SWAMINATHAN S et al.** *Biochem. Biophys. Res. Commun.,* 2013, vol. 434, 228-234
- **YOO JK et al.** *Stem Cells Dev,* 2012, vol. 21, 2049-2057
- **TANDON M et al.** *Oral Dis,* 2012, vol. 18, 127-131
- **LI Y et al.** *Gene,* vol. 497, 330-335
- **YOO JK et al.** *Stem Cells Dev.,* 2012, vol. 21, 2049-2057
- **LI H et al.** *J. Clin. Invest,* 2009, vol. 119, 3666-3677
- **KAWAJI H et al.** *BMC Genomics,* 2008, vol. 9, 157
- **MORIN RD et al.** *Genome Res,* 2008, vol. 18, 610-621
- **WITTEN D et al.** *BMC Biol.,* 2010, vol. 8, 58
- **PERSSON H et al.** *Cancer Res.,* vol. 71, 78-86
- **BEREZIKOV E et al.** *Genome Res.,* 2006, vol. 16, 1289-1298
- **LIM LP et al.** *Science,* 2003, vol. 299, 1540
- **LI Y et al.** *Gene,* 2012, vol. 497, 330-335
- **ARTZI S et al.** *BMC Bioinformatics,* 2008, vol. 9, 39
- **LAGOS-QUINTANA M et al.** *Curr. Biol,* 2002, vol. 12, 735-739
- **FU H et al.** *FEBS Lett.,* 2005, vol. 579, 3849-3854
- **JIMADD et al.** *Blood,* 2010, vol. 116, e118-e127
- **DING N et al.** *J. Radiat. Res.,* 2011, vol. 52, 425-432
- **HOUBAVIY HB et al.** *Dev. Cell.,* 2003, vol. 5, 351-358
- **MEIRI E et al.** *Nucleic Acids Res,* 2010, vol. 38, 6234-6246
- **MARTON S et al.** *Leukemia,* 2008, vol. 22, 330-338
- **YOO H et al.** *Biochem. Biophys. Res. Commun,* 2011, vol. 415, 567-572
- **AZUMA-MUKAI, A et al.** *Proc. Natl. Acad. Sci. U.S.A.,* 2008, vol. 105, 7964-7969
- **BEREZIKOV E et al.** *Genome Res,* 2006, vol. 16, 1289-1298
- **KIM J et al.** *Proc. Natl. Acad. Sci. U.S.A.,* 2004, vol. 101, 360-365
- **XIE X et al.** *Nature,* vol. 434, 338-345
- **CUMMINS JM et al.** *Proc. Natl. Acad. Sci., U.S.A.,* 2006, vol. 103, 3687-3692
- **MEIRI E et al.** *Nucleic Acids Res.,* 2010, vol. 38, 6234-6246
- **HU R et al.** *J. Biol. Chem.,* 2011, vol. 286, 12328-12339
- **MORIN RD. et al.** *Genome Res,* 2008, vol. 18, 610-621
- **SAMBROOK, J. ; RUSSEL, D.** Molecular Cloning, A LABORATORY MANUAL. Cold Spring Harbor Laboratory Press, 15 January 2001, vol. 1-2
- **RICK KAMPS et al.** *Int. J. Mol. Sci.,* 2017, vol. 18 (2), 308
- *Int. Neurourol. J.,* 2016, vol. 20, S76-83
- **SPEED T.** Statistical Analysis of Gene Expression Microarray Data. Chapman and Hall/CRC
- **CAUSTON H. C. et al.** A Beginner's guide Microarray Gene Expression data Analysis. Blackwell publishing
- **BOLSTAD, B.M. et al.** *Bioinformatics,* 2003, vol. 19, 185-193
- **VENABLES, W. N. et al.** Modern Applied Statistics with S. Springer, 2002
- **TAKAFUMI KANAMORI et al.** Pattern Recognition. KYORITSU SHUPPAN CO., LTD, 2009
- **RICHARD O. et al.** Pattern Classification. Wiley-Interscience, 2000
- **V. VAPNIK.** The Nature of Statistical Leaning Theory. Springer, 1995
- Statistics of pattern recognition and learning - New concepts and approaches. **HIDEKI ASO et al.** Frontier of Statistical Science 6. Iwanami Shoten, Publishers, 2004
- **NELLO CRISTIANINI et al.** Introduction to SVM. Kyoritsu Shuppan Co., Ltd, 2008
- **C. CORTES et al.** *Machine Learning,* 1995, vol. 20, 273-297
- **TIBSHIRANI R.** *J R Stat Soc Ser B,* 1996, vol. 58, 267-88
- **HOERL, A.E.** *Technometrics,* 1970, vol. 12, 55-67
- **ZOU, H.** *J R Stat Soc Ser B,* 2005, vol. 67, 301-320
- **PEARSON, K.** *Philosophical Magazine,* 1901, vol. 2 (11), 559-572
- **HOTELLING, H.** *Journal of Educational Psychology,* 1933, vol. 24, 498-520
- **YASUSHI NAGATA et al.** Basics of statistical multiple comparison methods. Scientist Press Co, 2007
- **FUREY TS et al.** *Bioinformatics,* 2000, vol. 16, 906-14

• **R CORE TEAM.** R: A language and environment for statistical computing. *R Foundation for Statistical Computing, Vienna, Austria,* 2018, URL https://www.R-project.org

• **VENABLES, W.N. ; RIPLEY, B.D.** Modern Applied Statistics. Springer, 2002